# FASCICULE DE BREVET EUROPEEN

(11) **EP 2 079 722 B1**
(45) Date de publication et mention de la délivrance du brevet: **17.06.2015**
(21) Numéro de dépôt: 07872038.0
(22) Date de dépôt: 28.12.2007
(51) Int. Cl.: C07D 333/22

(54) **DERIVES DE 3-PHENYL-1- (PHENYLTHIENYL) PROPAN-1-ONE ET DE 3-PHENYL-1- (PHENYLFURANYL) PROPAN-1-ONE SUBSTITUES, PREPARATION ET UTILISATION**
SUBSTITUIERTE 3-PHENYL-1-(PHENYLTHIENYL)PROPAN-1-ON- UND 3-PHENYL-1-(PHENYLFURANYL)PROPAN-1-ON-DERIVATE, IHRE HERSTELLUNG UND VERWENDUNG
SUBSTITUTED 3-PHENYL-1-(PHENYLTHIENYL)PROPAN-1-ONE AND 3-PHENYL-1-(PHENYLFURANYL)PROPAN-1-ONE DERIVATIVES, AND PREPARATION AND USE OF SAME

(30) Priorité: 29.12.2006 FR 0656067
(43) Date de publication de la demande: 22.07.2009
(73) Titulaire: Genfit, 59120 Loos (FR)
(72) Inventeur: DELHOMEL, Jean-François, F-62144 Acq (FR)
(74) Mandataire: Sekhri, Redha
(86) Numéro de dépôt international: PCT/FR2007/052634
(87) Numéro de publication internationale: WO 2008/087366

(56) Documents cités:
- WO-A-2004/063184
- WO-A-2006/090920
- DATABASE CA [Online] CHEMICAL ABSTRACTS SERVICE, COLUMBUS, OHIO, US; FISERA, L. ET AL: "Thiophene derivatives. IV. .alpha.,.beta.-Unsaturated ketones of the phenylthiophene series" XP002443677 extrait de STN Database accession no. 1979:137601 & ZBORNIK PRAC CHEMICKOTECHNOLOGICKEJ FAKULTY SVST , VOLUME DATE 1975-1976 91-6 CODEN: ZPCTA7; ISSN: 0524-2185, 1978,

## Description

La présente invention concerne des composés dérivés de 3-phényl-1-(phénylthiènyl)propan-1-one et de 3-phényl-1-(phénylfuranyl)propan-1-one substitués, les compositions pharmaceutiques les comprenant ainsi que leurs applications thérapeutiques, notamment dans les domaines de la santé humaine et animale.

Les inventeurs ont mis en évidence, de manière surprenante, que les composés selon l'invention possèdent de manière intrinsèque des propriétés agonistes PPAR (Peroxisome Proliferator-Activated Receptor).

Les molécules décrites dans l'invention sont donc d'un intérêt particulier pour traiter les complications associées au syndrome métabolique, l'athérosclérose, les maladies cardiovasculaires, l'insulino-résistance, l'obésité, l'hypertension, le diabète, les dyslipidémies, les maladies inflammatoires (asthme, etc.), l'ischémie cérébrale, les maladies autoimmunes, les pathologies neurodégénératives (Alzheimer, etc.), les cancers, etc., ainsi que pour permettre la diminution du risque cardiovasculaire global. Préférentiellement, les composés selon l'invention sont utilisables pour le traitement des dyslipidémies et l'amélioration du risque global cardiovasculaire.

Le diabète, l'obésité et les dyslipidémies (taux plasmatiques de cholestérol LDL et de triglycérides élevés, taux plasmatiques de cholestérol HDL faible, etc.) font partie des facteurs de risque cardiovasculaire clairement identifiés qui prédisposent un individu à développer une pathologie cardiovasculaire (Mensah M, 2004). Ces facteurs de risque s'additionnent aux facteurs de risque liés au mode de vie tels que le tabagisme, l'inactivité physique et les régimes alimentaires déséquilibrés. Un effet synergique existe entre ces différents facteurs : la présence concomitante de plusieurs d'entre eux conduit à une aggravation dramatique du risque cardiovasculaire et il convient alors de parler de risque global (« *global risk* ») pour les maladies cardiovasculaires. La prévalence des dyslipidémies atteignait 43,6% de la population en 2004 dans les principaux pays développés. La prévalence du diabète, actuellement en nette augmentation, est en passe de devenir de plus en plus significative dans l'épidémiologie des maladies cardiovasculaires : la prévalence du diabète est en effet estimée à 7,6% de la population pour 2010 (Fox-Tucker J, 2005).

Selon l'International Atherosclerosis Society (International Atherosclerosis Society, 2003), les maladies cardiovasculaires représentent la première cause de mortalité dans les pays industrialisés et deviennent de plus en plus fréquentes dans les pays en voie de développement. Ces maladies sont notamment les maladies coronariennes, l'ischémie cérébrale et les maladies artérielles périphériques.

Ces données justifient donc l'adoption de mesures énergiques pour réduire significativement la morbidité et la mortalité dues aux pathologies cardiovasculaires et la nécessité de trouver des traitements efficaces, complémentaires d'une modification de l'hygiène de vie, agissant sur les facteurs de risque des maladies cardiovasculaires et sur leurs conséquences devient une urgence mondiale.

Les composés selon l'invention, par leurs propriétés d'agonistes PPAR, présentent un intérêt particulier pour le traitement des pathologies liées aux dérèglements du métabolisme lipidique et/ou glucidique, telles que le diabète, l'obésité, les dyslipidémies ou l'inflammation, ainsi que pour la diminution du risque cardiovasculaire global.

Les PPAR (α, γ et δ) sont en effet connus comme étant impliqués dans ce type de pathologies (Kota BP *et al.,* 2005) : des ligands de ces récepteurs sont commercialisés pour traiter de telles pathologies (Lefebvre P *et al.,* 2006) et de nombreux modulateurs PPAR, agonistes ou antagonistes, sélectifs ou non, sont actuellement en développement pharmaceutique avancé. Un modulateur PPAR ayant des effets bénéfiques sur la résistance à l'insuline, l'obésité, les dyslipidémies, l'hypertension et/ou l'inflammation pourrait être utilisé dans le traitement du syndrome métabolique (ou syndrome X) (Liu Y and Miller A, 2005).

La famille des PPAR comprend trois isoformes, désignés α, γ et δ (également appelé β), chacun codé par un gène différent. Ces récepteurs font partie de la superfamille des récepteurs nucléaires et des facteurs de transcription qui sont activés par la liaison de certains acides gras et/ou de leurs métabolites lipidiques. Les PPAR activés forment des hétérodimères avec les récepteurs de l'acide rétinoïque 9-cis (RXR ou Retinoid X Receptor) et se fixent sur des éléments de réponse spécifiques (PPRE ou Peroxisome Proliferator Response Element) au niveau du promoteur de leurs gènes cibles, permettant ainsi le contrôle de la transcription.

PPARα contrôle principalement le métabolisme lipidique (hépatique et musculaire) et l'homéostasie du glucose, par un contrôle direct de la transcription de gènes codant pour des protéines impliquées dans l'homéostasie lipidique. Il exerce des effets anti-inflammatoires et anti-prolifératifs et prévient les effets pro-athérogéniques de l'accumulation du cholestérol dans les macrophages en stimulant l'efflux du cholestérol (Lefebvre P, Chinetti G, Fruchart JC and Staels B, 2006). Les fibrates (fénofibrate, bézafibrate, ciprofibrate, gemfibrozil), par l'intermédiaire de PPARα, sont ainsi utilisés en clinique dans le traitement de certaines dyslipidémies en baissant les triglycérides et en augmentant les taux plasmatiques de cholestérol HDL (High Density Lipoprotein).

PPARγ est impliqué dans le métabolisme lipidique des adipocytes matures (régulateur-clé de l'adipogenèse), dans l'homéostasie du glucose (notamment dans la résistance à l'insuline), dans l'inflammation, dans l'accumulation de cholestérol au niveau des macrophages et dans la prolifération cellulaire (Lehrke M and Lazar MA, 2005). PPARγ joue par conséquent un rôle dans la pathogenèse de l'obésité, de l'insulino-résistance et du diabète. Les thiazolidinediones (Rosiglitazone, Troglitazone, etc.) sont des ligands du récepteur PPARγ utilisés dans le traitement du diabète de type 2.

Il existe des ligands de PPARδ actuellement en développement clinique (par exemple le GW501516 (CAS Registry Number 317318-70-0)), mais aucun ligand PPARδ n'est actuellement utilisé comme médicament. Ce récepteur est une cible attractive pour le développement de médicaments utilisables dans le traitement des facteurs de risques associés au syndrome métabolique et à l'athérosclérose tels que les dyslipidémies, l'obésité, l'inflammation et la résistance à l'insuline. PPARδ est en effet impliqué dans le contrôle des métabolismes lipidique et glucidique, dans la balance énergétique, dans la prolifération et la différentiation de neurones, et dans la réponse inflammatoire (Gross B *et al.,* 2005).

Au-delà du rôle direct joué par les ligands PPAR sur la régulation du métabolisme des lipides et des glucides, ces molécules ont un spectre d'action pléiotropique dû à la grande diversité des gènes cibles des PPAR. Ces multiples propriétés font des PPAR des cibles thérapeutiques d'intérêt pour le traitement de diverses pathologies, notamment des pathologies cardio-métaboliques (i.e. pathologies cardiovasculaires et métaboliques) ainsi que pour permettre la diminution du risque cardiovasculaire global.

Les ligands PPAR ont un rôle neuroprotecteur dans la maladie d'Alzheimer, la sclérose en plaque, la maladie de Parkinson et plus généralement dans toute pathologie impliquant une mort ou une dégénérescence neuronale, qu'il s'agisse des neurones du système nerveux central ou périphérique, une mort ou une dégénérescence des oligodendrocytes, une mort ou une dégénérescence de cellules gliales, une inflammation des cellules gliales (c'est-à-dire les astrocytes, la microglie ou les oligodendrocytes) ou des cellules de Schwann. Ainsi, il a été récemment montré que les agonistes PPARδ permettaient de préserver l'apprentissage et la mémoire chez des rats pour lesquels la maladie d'Alzheimer avait été induite (de la Monte SM *et al.,* 2006). Il a également été montré que l'administration orale d'agonistes de PPARδ réduisait les symptômes cliniques et l'activation de l'inflammation astrogliale et microgliale dans un modèle de sclérose en plaques (Polak, 2005).

Les documents WO 2004/063181 et WO 2006/090920 décrivent des composés agonistes de PPAR.

Les composés selon l'invention, par leurs propriétés agonistes PPAR, représentent donc un outil thérapeutique avantageux pour l'amélioration des pathologies liées aux dérèglements du métabolisme lipidique et/ou glucidique, pour la diminution du risque cardiovasculaire global ainsi que pour la neuroprotection.

Notamment, les composés selon l'invention possèdent des propriétés agonistes PPARδ et PPARα et présentent donc un intérêt dans le traitement des pathologies métaboliques telles que le syndrome métabolique (dont les caractéristiques sont l'obésité (en particulier l'obésité abdominale), une concentration anormale de lipides sanguins (taux élevé de triglycérides et/ou faible taux de cholestérol HDL (dyslipidémie)), une glycémie élevée et/ou une résistance à l'insuline et une hypertension) et dans le traitement des dyslipidémies.

La présente invention a pour objet des composés dérivés de 3-phényl-1-(phénylthiènyl)propan-1-one et de 3-phényl-1-(phénylfuranyl)propan-1-one substitués de formule générale (I) suivante : 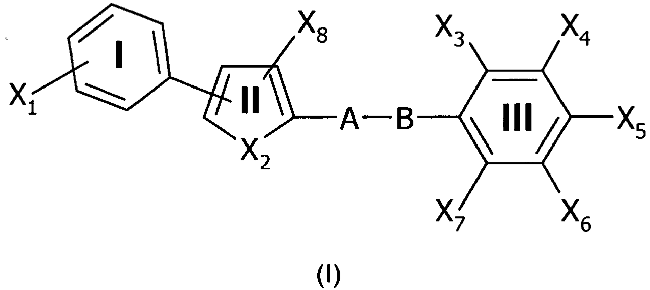 dans laquelle :
X1 représente un halogène, un groupement R1, SR1 ou OR1 ;
X2 représente un atome de soufre ou d'oxygène ;
X3 représente un halogène ou un groupement R3 ;
X4 représente un halogène ou un groupement R4 ;
X5 représente un groupement OR5 ;
X6 représente un halogène ou un groupement R6 ;
X7 représente un halogène ou un groupement R7 ;
X8 représente un atome d'hydrogène ;

R1 représentant un hydrogène ou un groupement alkyle ayant 1 à 4 atomes de carbone, ledit groupement alkyle étant éventuellement halogéné ;
R3, R4, R6 et R7 identiques ou différents, choisis parmi un hydrogène ou un groupement alkyle ayant 1 à 4 atomes de carbone ;
R5 représentant un radical alkyle formé d'une chaîne carbonée linéaire et saturée, ayant 1 à 4 atomes de carbone, ladite chaîne carbonée étant :
   - liée, par son extrémité opposée au groupement phényle (III), à un substituant - COOR12, R12 représentant un hydrogène ou un groupement alkyle ayant 1 à 4 atomes de carbone ;
   - non ramifiée ou ramifiée avec au moins un groupement alkyle ayant 1 à 4 atomes de carbone ;
A représente :
   (i) un groupement carbonyle (CO), ou
   (ii) un groupement -CR9R10, R9 représentant un atome d'hydrogène et R10 représentant un groupement -OR11, R11 étant choisi parmi un atome d'hydrogène ou un groupement alkyle (linéaire ou ramifié) ayant 1 à 7 atomes de carbone, ledit groupement alkyle étant non substitué ou substitué par un groupement cycloalkyle, notamment cyclohexyl, un groupement aryle, notamment phényle ou un groupement hétéroaryle, notamment pyridinyl;
B représente un groupement alkyle non substitué, saturé présentant deux atomes de carbone (CH2-CH2);
leurs stéréoisomères (diastéréoisomères, énantiomères), purs ou en mélange, mélanges racémiques, isomères géométriques, tautomères, sels, hydrates, solvates, formes solides ainsi que leurs mélanges.

Dans le cadre de la présente invention :
- le terme « alkyle » désigne un radical hydrocarboné saturé, linéaire, ramifié, halogéné ou non, ayant plus particulièrement de 1 à 24 atomes de carbone, de préférence de 1 à 10, et ayant plus particulièrement 1, 2, 3, 4, 5, 6, 7, 8, 9 ou 10 atomes de carbone. On peut citer, par exemple, les radicaux méthyle, trifluorométhyle, éthyle, n-propyle, isopropyle, n-butyle, isobutyle, tertiobutyle, sec-butyle, pentyle, néopentyle ou n-hexyle.

En particulier, un radical alkyle ou alcényle ayant 1 à 4 atomes de carbone est de préférence choisi parmi les groupes méthyle, éthyle, *n*-propyle, n-butyle, isopropyle, sec-butyle, isobutyle tertiobutyle et leurs dérivés insaturés, présentant au moins une double liaison (telle que notamment : CH=CH).
- le terme « cycloalkyle » désigne un groupe alkyle tel que défini ci-dessus et formant au moins un cycle. On peut citer, à titre de groupes cycloalkyle ayant de 3 à 8 atomes de carbone, le cyclopropyle, cyclobutyle, cyclopentyle, cyclohexyle, cycloheptyle et cyclooctyle.
- le terme « hétérocycloalkyle » désigne un groupe alkyle saturé ou non et formant au moins un cycle interrompu par un ou plusieurs hétéroatomes choisis parmi N, O, S ou P. On peut citer, à titre de groupes hétérocycloalkyle, l'aziridine, la pyrrolidine, le tetrahydrothiophene, l'imidazoline, la pipéridine, la pipérazine et la morpholine.
- le terme « aryle » fait référence à des groupes aromatiques comprenant de préférence 5 à 14 atomes de carbone, avantageusement 6 à 14 atomes de carbone (i.e. 6, 7, 8, 9, 10, 11, 12, 13 ou 14 atomes de carbone). Ils sont généralement mono- ou bi-cycliques. On peut citer par exemple le phényle, benzyle, α-naphtyle, β-naphtyle, anthracényle ou fluorényle. Dans le cadre de la présente invention, les groupements aryles peuvent être substitués par un ou plusieurs substituants, identiques ou différents. Parmi les substituants des groupes aryles, on peut citer à titre d'exemple, les halogènes, les groupements alkyle (tels que définis ci-dessus) et alkyloxy (défini comme une chaîne alkyle (telle que définie ci-dessus) liée à la molécule par l'intermédiaire d'un oxygène (liaison éther)) les groupements alkylthio (défini comme une chaîne alkyle (telle que définie ci-dessus) liée à la molécule par l'intermédiaire d'un soufre (liaison thioéther)) tels que méthyle, trifluorométhyle, méthoxy et trifluorométhoxy, méthylthio et trifluorométhylthio, les amines, les groupements nitro, les groupements hydroxy, les groupements aryle, hétéroaryle et hétérocycle.
- le terme « hétéroaryle » fait référence à des groupes aromatiques comprenant de préférence 3 à 14 atomes de carbone, avantageusement 3 à 8 atomes de carbone (i.e. 3, 4, 6, 7 ou 8 atomes de carbone), interrompus par un ou plusieurs hétéroatomes choisis parmi N, O, S ou P. On peut citer, à titre de groupes hétéroaryles ayant de 3 à 8 atomes de carbone, le pyrrole, l'imidazole, et la pyridine.

Parmi les substituants des groupes hétéroaryles, on peut citer à titre d'exemple, les halogènes, les groupements alkyle (tels que définis ci-dessus) et alkyloxy (défini comme une chaîne alkyle (telle que définie ci-dessus) liée à la molécule par l'intermédiaire d'un oxygène (liaison éther)) les groupements alkylthio (défini comme une chaîne alkyle (telle que définie ci-dessus) liée à la molécule par l'intermédiaire d'un soufre (liaison thioéther)). Des exemples de ces substituants sont le méthyle, le trifluorométhyle, le méthoxy et le trifluorométhoxy, le méthylthio et le trifluorométhylthio, les amines, les groupements nitro, les groupements hydroxy, les groupements aryle, hétéroaryle et hétérocycle.

Les atomes d'halogène sont choisis parmi les atomes de brome, fluor, iode, chlore.

Dans le cadre de la présente invention, dans la formule générale (I), les atomes du cycle Il sont numérotés à partir de l'atome X₂, qui porte le numéro 1, les autres atomes du cycle étant numérotés à partir du carbone du cycle II lié au groupement A-B. Ainsi, le carbone du cycle II lié au groupement A-B est le carbone 2 (ou C₂), le carbone adjacent au C₂ est le carbone 3 (ou C₃), etc.

Un aspect particulier de l'invention concerne les composés de formule générale (I) dans laquelle A représente un groupement carbonyle (CO).

Un autre aspect particulier de l'invention concerne les composés de formule générale (I) dans laquelle A représente un groupement -CR9R10, R9 représentant un hydrogène et R10 représentant un groupement hydroxy, un groupement alkyle ou un groupement -OR11, R11 représentant un groupement alkyle, substitué ou non par un groupement cycloalkyle, hétérocycloalkyle, aryle ou hétéroaryle.

En particulier, R11 représente un groupement alkyle, linéaire ou ramifié, ayant 1 à 7 atomes de carbone, de préférence 1, 2, 3 ou 4 atomes de carbone, de préférence 1 ou 2 atomes de carbone, avantageusement R11 représente le groupement méthyle ou éthyle. Par exemple, R11 peut également être un isopropyle.

Avantageusement R11 est substitué par un groupement cycloalkyle, notamment cyclohexyle, un groupement aryle, notamment phényle, un groupement hétéroaryle, notamment pyridinyle. De manière encore plus préférentielle, R11 représente un groupement alkyle, comprenant de préférence un atome de carbone, substitué par un groupement phényle, cyclohexyle, ou pyridinyle.

Un autre aspect particulier de l'invention concerne les composés de formule générale (I) dans laquelle A représente un groupement -CR9R10, R9 représentant un hydrogène et R10 représentant un groupement hydroxy.

R12 étant tel que défini ci-avant et représente un hydrogène ou un groupement alkyle comprenant 1, 2, 3 ou 4 atomes de carbone, en particulier un groupement tertiobutyle.

Dans un aspect particulier de l'invention, X5 est choisi parmi les groupements: -OC(CH₃)₂COOR12, -OCH(CH₂CH₃)COOR12, et -OCH₂COOR12. Avantageusement, R12 peut notamment être choisi parmi l'hydrogène et les groupements -CH₃, -C(CH₃)₃ et -CH₂CH₃.

Encore plus préférentiellement, X5 représente un groupement -OC(CH₃)₂COOH, -OC(CH₃)₂COOC(CH₃)₃, -OCH(CH₂CH₃)COOC(CH₃)₃, -OCH(CH₂CH₃)COOH, -OCH₂COOH, ou -OCH₂COOC(CH₃)₃.

Selon un autre aspect de l'invention, les composés de formule générale (I) présentent au moins un des groupements X3, X4, X6 et X7 désignant un atome d'halogène ou un groupement alkyle ayant 1 à 4 atomes de carbone, de préférence un halogène.

Un autre objet particulier de l'invention concerne les composés de formule générale (I) dans laquelle X3 et/ou X4, identiques ou différents, représentent un halogène, de préférence le chlore ou le fluor.

De préférence, X3 et X4 sont identiques et représentent un halogène, préférentiellement un atome de chlore ou de fluor, encore plus préférentiellement de chlore.

Un autre objet particulier de l'invention concerne les composés de formule générale (I) dans laquelle X3 représente un atome d'hydrogène et X4 représente un atome de brome ou de fluor.

Selon un autre aspect de l'invention, les composés de formule générale (I) présentent au moins un des groupements X3, X4, X6 et X7 désignant un atome d'halogène ou un groupement alkyle ayant 1 à 4 atomes de carbone et le(s) groupement(s) restant (c'est-à-dire le(s) groupement(s) non halogéné(s) ou non alkylé(s) choisi(s) parmi X3, X4, X6 et X7) désignent un(des) atome(s) d'hydrogène.

Un autre objet particulier de l'invention concerne les composés de formule (I) dans lesquels X4 et/ou X6 désigne(nt) un groupement alkyle, en particulier des composés dans lesquels X4 et X6 sont deux groupements méthyles, et X3 et X7 sont des atomes d'hydrogène.

Un autre objet particulier de l'invention concerne les composés de formule générale (I) dans laquelle X6 et X7 représentent un atome d'hydrogène.

De préférence, X6 et X7 représente un hydrogène et X3 et/ou X4, identiques ou différents, représentent un halogène, de préférence le chlore ou le fluor.

Un autre aspect préféré concerne les composés de formule générale (I) dans laquelle X1 représente un groupement R1 ou -OR1, R1 représentant un hydrogène ou un groupement alkyle. Préférentiellement, R1 représente un groupement alkyle comportant 1, 2 ou 3 atomes de carbone, encore plus préférentiellement le groupement alkyle est halogéné.

De manière préférée, X1 est choisi parmi un groupement trifluorométhyle, un atome de brome, un groupement méthyloxy, un groupement méthylthio, un groupement trifluorométhoxy et un atome d'hydrogène. Facultativement, X1 représente un groupement -CF₃, -OCF₃, -SCH₃.

Un autre aspect préféré concerne les composés de formule générale (I) dans laquelle X1 représente un halogène, préférentiellement un brome.

Un autre objet particulier de l'invention concerne les composés de formule générale (I) dans laquelle X2 représente un atome de soufre.

Un autre objet particulier de l'invention concerne les composés de formule générale (I) dans laquelle le cycle Il est substitué par le cycle I en position C₄.

Un autre objet particulier de l'invention concerne les composés de formule générale (I) dans laquelle le cycle Il est substitué par le cycle I en position C₅.

Un autre objet particulier de l'invention concerne les composés de formule générale (I) dans laquelle le cycle I est substitué par le groupement X1 en position C₃ (ou en méta par rapport au cycle II).

Un autre objet particulier de l'invention concerne les composés de formule générale (I) dans laquelle le cycle I est substitué par le groupement X1 en position C₄ (ou en para par rapport au cycle II).

Un autre aspect particulier de l'invention concerne les composés de formule générale (I) dans laquelle R1 représente un hydrogène ou un groupement alkyle ayant 1 à 4 atomes de carbone, ledit groupement alkyle pouvant éventuellement être halogéné et R3, R4, R6, et R7, identiques ou différents, sont choisis parmi un hydrogène ou un groupement alkyle ayant 1 à 4 atomes de carbone.

De manière encore plus préférentielle, l'invention a pour objet les composés de formule générale (I) dans laquelle au moins l'une des conditions suivantes, de préférence toutes les conditions, est remplie :
X6 et X7, identiques représentent un hydrogène ; et/ou
X3 et/ou X4, identiques ou différents, représentent un halogène, de préférence le chlore ou le fluor ; et/ou
le cycle Il est substitué par le cycle I en position C₄ ou en position C₅. ; et/ou
le cycle I est substitué par le groupement X1 en position C₃ ou en position C₄.

Une autre variante particulièrement préféré de l'invention concerne les composés de formule générale (I) dans laquelle A représente un groupement -CHOR11, R11 étant de préférence choisi parmi un atome d'hydrogène, un groupement méthyle, éthyle, isopropyle, cyclohexylméthyle, et benzyle.

Avantageusement, X5 est choisi parmi les groupements : -OC(CH₃)₂COOR12, -OCH(CH₂CH₃)COOR12 et -OCH₂COOR12.

Avantageusement, R12 est choisi parmi l'hydrogène et les groupements-CH₃, -C(CH₃)₃ et -CH₂CH₃.

Dans un mode particulier de réalisation de l'invention, X1 est choisi parmi un groupement trifluorométhyle, un atome de brome, un groupement méthyloxy, un groupement méthylthio, un groupement trifluorométhoxy et un atome d'hydrogène.

Selon un aspect particulier de l'invention, les composés selon l'invention présentent au moins un des groupements X3, X4, X6 et X7 désignant un atome d'halogène ou un groupement alkyle ayant 1 à 4 atomes de carbone. De préférence, le(s) groupement(s) restant (c'est-à-dire le(s) groupement(s) non halogéné(s) ou non alkylé(s) choisi(s) parmi X3, X4, X6 et X7) désignent, un(des) atome(s) d'hydrogène

A titre d'exemple, il peut aussi s'agir de composés pour lesquels X3 et X4 sont identiques et correspondent à des atomes d'halogène (chlore, fluor, brome ou iode), notamment de chlore ou de fluor.

Il peut aussi s'agir de composés pour lesquels X4 et/ou X6 désigne(nt) un groupement alkyle, en particulier des composés pour lesquels X4 et X6 sont deux groupements méthyle, et X3 et X7 sont des atomes d'hydrogène.

Selon un aspect particulier de l'invention, dans le mode particulièrement préféré de l'invention, X4 et/ou X6 désigne(nt) un groupement alkyle, en particulier X4 et X6 sont deux groupements méthyles, et X3 et X7 sont des atomes d'hydrogène.

De manière préférée, les composés selon l'invention sont choisis parmi :
- le 2-(2,3-dichloro-4-(3-oxo-3-(5-(4-(trifluorométhyl)phényl)thièn-2-yl)propyl)-phénoxy)-2-méthylpropanoate de tertiobutyle ;
- l'acide 2-(2,3-dichloro-4-(3-oxo-3-(5-(4-(trifluorométhyl)phényl)thièn-2-yl)-propyl)phénoxy)-2-méthylpropanoïque ;
- le 2-(2,3-dichloro-4-(3-hydroxy-3-(5-(4-(trifluorométhyl)phényl)thièn-2-yl)-propyl)phénoxy)-2-méthylpropanoate de tertiobutyle ;
- l'acide 2-(2,3-dichloro-4-(3-hydroxy-3-(5-(4-(trifluorométhyl)phényl)thièn-2-yl)-propyl)phénoxy)-2-méthylpropanoïque ;
- l'acide 2-(4-(3-(benzyloxy)-3-(5-(4-(trifluorométhyl)phényl)thièn-2-yl)propyl)-2,3-dichlorophénoxy)-2-méthylpropanoïque ;
- le 2-(2,3-dichloro-4-(3-oxo-3-(5-(4-(trifluorométhyl)phényl)thièn-2-yl)propyl)-phénoxy)butanoate de tertiobutyle ;
- l'acide 2-(2,3-dichloro-4-(3-oxo-3-(5-(4-(trifluorométhyl)phényl)thièn-2-yl)-propyl)phénoxy)butanoïque ;
- le 2-(2,3-dichloro-4-(3-oxo-3-(4-(4-(trifluorométhyl)phényl)thièn-2-yl)propyl)-phénoxy)-2-méthylpropanoate de tertiobutyle ;
- l'acide 2-(2,3-dichloro-4-(3-oxo-3-(4-(4-(trifluorométhyl)phényl)thièn-2-yl)-propyl)phénoxy)-2-méthylpropanoïque ;
- le 5-(2,3-dichloro-4-(3-oxo-3-(5-(4-(trifluorométhyl)phényl)thièn-2-yl)propyl)-phénoxy)-2,2-diméthylpentanoate de méthyle ;
- l'acide 5-(2,3-dichloro-4-(3-oxo-3-(5-(4-(trifluorométhyl)phényl)thièn-2-yl)-propyl)phénoxy)-2,2-diméthylpentanoïque ;
- le 2-(2,3-dichloro-4-(3-oxo-3-(5-(4-(trifluorométhyl)phényl)thièn-2-yl)propyl)-phénoxy)acétate de tertiobutyle ;
- l'acide 2-(2,3-dichloro-4-(3-oxo-3-(5-(4-(trifluorométhyl)phényl)thièn-2-yl)-propyl)phénoxy)acétique ;
- le 2-(2-chloro-4-(3-oxo-3-(5-(4-(trifluorométhyl)phényl)thièn-2-yl)propyl)-phénoxy)-2-méthylpropanoate de tertiobutyle ;
- l'acide 2-(2-chloro-4-(3-oxo-3-(5-(4-(trifluorométhyl)phényl)thièn-2-yl)propyl) phénoxy)-2-méthylpropanoïque ;
- le 2-(3-chloro-4-(3-oxo-3-(5-(4-(trifluorométhyl)phényl)thièn-2-yl)propyl)-phénoxy)-2-méthylpropanoate de tertiobutyle ;
- l'acide 2-(3-chloro-4-(3-oxo-3-(5-(4-(trifluorométhyl)phényl)thièn-2-yl)propyl)-phénoxy)-2-méthylpropanoïque ;
- le 2-(2-fluoro-4-(3-oxo-3-(5-(4-(trifluorométhyl)phényl)thièn-2-yl)propyl)-phénoxy)-2-méthylpropanoate de tertiobutyle ;
- l'acide 2-(2-fluoro-4-(3-oxo-3-(5-(4-(trifluorométhyl)phényl)thièn-2-yl)propyl)-phénoxy)-2-méthylpropanoïque ;
- le 2-(2-fluoro-4-(3-oxo-3-(5-(4-(trifluorométhyl)phényl)thièn-2-yl)propyl)-phénoxy)acétate de tertiobutyle ;
- l'acide 2-(2-fluoro-4-(3-oxo-3-(5-(4-(trifluorométhyl)phényl)thièn-2-yl)propyl)-phénoxy)acétique ;
- l'acide 2-(2-fluoro-4-(3-hydroxy-3-(5-(4-(trifluorométhyl)phényl)thièn-2-yl)-propyl)phénoxy)acétique ;
- l'acide 2-(4-(3-(benzyloxy)-3-(5-(4-(trifluorométhyl)phényl)thièn-2-yl)propyl)-2-fluorophénoxy)acétique ;
- le 2-(2-fluoro-4-(3-oxo-3-(5-(4-(trifluorométhyl)phényl)thièn-2-yl)propyl)-phénoxy)butanoate de tertiobutyle ;
- l'acide 2-(2-fluoro-4-(3-oxo-3-(5-(4-(trifluorométhyl)phényl)thièn-2-yl)propyl)-phénoxy)butanoïque ;
- l'acide 2-(2-fluoro-4-(3-hydroxy-3-(5-(4-(trifluorométhyl)phényl)thièn-2-yl)-propyl)phénoxy)butanoïque ;
- le 2-(2-bromo-4-(3-oxo-3-(5-(4-(trifluorométhyl)phényl)thièn-2-yl)propyl)-phénoxy)butanoate de tertiobutyle ;
- l'acide 2-(2-bromo-4-(3-oxo-3-(5-(4-(trifluorométhyl)phényl)thièn-2-yl)propyl)-phénoxy)butanoïque ;
- le 2-(2-bromo-4-(3-oxo-3-(5-(4-(trifluorométhyl)phényl)thièn-2-yl)propyl)-phénoxy)acétate de tertiobutyle ;
- l'acide 2-(2-bromo-4-(3-oxo-3-(5-(4-(trifluorométhyl)phényl)thièn-2-yl)propyl)-phénoxy)acétique ;
- le 2-(2-bromo-4-(3-oxo-3-(5-(4-(trifluorométhyl)phényl)thièn-2-yl)propyl)-phénoxy)-2-méthylpropanoate de tertiobutyle ;
- l'acide 2-(2-bromo-4-(3-oxo-3-(5-(4-(trifluorométhyl)phényl)thièn-2-yl)propyl)-phénoxy)-2-méthylpropanoïque ;
- le 2-(2,3-dichloro-4-(3-oxo-3-(5-(4-(trifluorométhyl)phényl)fur-2-yl)propyl)-phénoxy)-2-méthylpropanoate de tertiobutyle ;
- l'acide 2-(2,3-dichloro-4-(3-oxo-3-(5-(4-(trifluorométhyl)phényl)fur-2-yl)propyl)-phénoxy)-2-méthylpropanoïque ;
- l'acide 2-(2,3-dichloro-4-(3-(pyridin-3-ylméthoxy)-3-(5-(4-(trifluorométhyl)-phényl)thièn-2-yl)propyl)phénoxy)-2-méthylpropanoïque ;
- l'acide 2-(2,3-dichloro-4-(3-méthoxy-3-(5-(4-(trifluorométhyl)phényl)thièn-2-yl)propyl)phénoxy)-2-méthylpropanoïque ;
- l'acide 2-(2,3-dichloro-4-(3-éthoxy-3-(5-(4-(trifluorométhyl)phényl)thièn-2-yl)-propyl)phénoxy)-2-méthylpropanoique ;
- l'acide 2-(2,3-dichloro-4-(3-(cyclohexylméthoxy)-3-(5-(4-(trifluorométhyl)-phényl)-thièn-2-yl)propyl)phénoxy)-2-méthylpropanoïque ;
- le 2-(2,3-dichloro-4-(3-oxo-3-(5-(4-(trifluorométhoxy)phényl)thièn-2-yl)propyl)-phénoxy)-2-méthylpropanoate de tertiobutyle ;
- l'acide 2-(2,3-dichloro-4-(3-oxo-3-(5-(4-(trifluorométhoxy)phényl)thièn-2-yl)-propyl)phénoxy)-2-méthylpropanoïque ;
- le 2-(2,3-difluoro-4-(3-oxo-3-(5-(4-(trifluorométhyl)phényl)thièn-2-yl)propyl)-phénoxy)-2-méthylpropanoate de tertiobutyle ;
- l'acide 2-(2,3-difluoro-4-(3-oxo-3-(5-(4-(trifluorométhyl)phényl)thièn-2-yl)-propyl)phénoxy)-2-méthylpropanoïque ;
- le 2-(2,6-diméthyl-4-(3-oxo-3-(5-(4-(trifluorométhyl)phényl)thièn-2-yl)propyl)-phénoxy)-2-méthylpropanoate de tertiobutyle ;
- l'acide 2-(2,6-diméthyl-4-(3-oxo-3-(5-(4-(trifluorométhyl)phényl)thièn-2-yl)-propyl)-phénoxy)-2-méthylpropanoïque ;
- le 2-(4-(3-(5-(4-bromophényl)thièn-2-yl)-3-oxopropyl)-2,3-dichlorophénoxy)-2-méthylpropanoate de tertiobutyle ;
- l'acide 2-(4-(3-(5-(4-bromophényl)thièn-2-yl)-3-oxopropyl)-2,3-dichloro-phénoxy)-2-méthylpropanoïque ;
- le 2-(2,3-dichloro-4-(3-(5-(4-(méthylthio)phényl)thièn-2-yl)-3-oxopropyl)-phénoxy)-2-méthylpropanoate de tertio-butyle ;
- l'acide 2-(2,3-dichloro-4-(3-(5-(4-(méthylthio)phényl)thièn-2-yl)-3-oxo-propyl)phénoxy)-2-méthylpropanoïque ;
- l'acide 2-(2,3-dichloro-4-(3-isopropoxy-3-(5-(4-(trifluorométhyl)phényl)thièn-2-yl)propyl)phénoxy)-2-méthylpropanoïque ;
- le 2-(2,3-dichloro-4-(3-oxo-3-(5-phénylthiophèn-2-yl)propyl)phénoxy)-2-méthyl-propanoate de tertiobutyle ;
- l'acide 2-(2,3-dichloro-4-(3-oxo-3-(5-phénylthièn-2-yl)propyl)phénoxy)-2-méthylpropanoïque ;
- le 2-méthyl-2-(2-méthyl-4-(3-oxo-3-(5-(4-(trifluorométhyl)phényl)thiophèn-2-yl)-propyl)phénoxy) propanoate de tertiobutyle ;
- l'acide 2-méthyl-2-(2-méthyl-4-(3-oxo-3-(5-(4-(trifluorométhyl)phényl)-thiophèn-2-yl)propyl)phénoxy)propanoïque ;
- l'acide 2-(2,3-dichloro-4-(3-hydroxy-3-(4-(4-(trifluorométhyl)phényl)thièn-2-yl)-propyl)phénoxy)-2-méthylpropanoïque ;
- l'acide 2-(4-(3-(benzyloxy)-3-(4-(4-(trifluorométhyl)phényl)thièn-2-yl)propyl)-2,3-dichlorophénoxy)-2-méthylpropanoïque ;
- l'acide 2-(2,3-difluoro-4-(3-hydroxy-3-(5-(4-(trifluorométhyl)phényl)thièn-2-yl)-propyl)phénoxy)-2-méthylpropanoïque ;
- l'acide 2-(4-(3-(benzyloxy)-3-(5-(4-(trifluorométhyl)phényl)thièn-2-yl)propyl)-2,3-difluorophénoxy)-2-méthylpropanoïque ;
- le 2-(4-(3-oxo-3-(5-(4-(trifluorométhyl)phényl)thièn-2-yl)propyl)phénoxy)-butanoate de tertiobutyle ;
- l'acide 2-(4-(3-oxo-3-(5-(4-(trifluorométhyl)phényl)thièn-2-yl)propyl)-phénoxy)-butanoïque ;
- le 2-méthyl-2-(4-(3-oxo-3-(5-(4-(trifluorométhyl)phényl)thièn-2-yl)propyl)-phénoxy)propanoate de tertiobutyle ;
- l'acide 2-(4-(3-oxo-3-(5-(4-(trifluorométhyl)phényl)thièn-2-yl)propyl)-phénoxy)-2-méthylpropanoïque ;
- le 2-(4-(3-oxo-3-(5-(4-(trifluorométhyl)phényl)thièn-2-yl)propyl)phénoxy)-acétate de tertiobutyle ;
- l'acide 2-(4-(3-oxo-3-(5-(4-(trifluorométhyl)phényl)thièn-2-yl)propyl)phénoxy)-acétique ;
- l'acide 2-(4-(3-(benzyloxy)-3-(5-(4-(trifluorométhyl)phényl)thièn-2-yl)propyl)-2-fluorophénoxy)butanoïque ;
- l'acide 2-(2,3-dichloro-4-(3-hydroxy-3-(5-(4-(trifluorométhyl)phényl)furan-2-yl)-propyl)phénoxy)-2-méthylpropanoïque ;
- l'acide 2-(2,3-dichloro-4-(3-méthoxy-3-(5-(4-(trifluorométhyl)phényl)fur-2-yl)-propyl)phénoxy)-2-méthylpropanoïque ;
- l'acide 2-(4-(3-hydroxy-3-(5-(4-(trifluorométhyl)phényl)thièn-2-yl)propyl)-2,6-diméthylphénoxy)-2-méthylpropanoïque ;
- l'acide 2-(4-(3-méthoxy-3-(5-(4-(trifluorométhyl)phényl)thièn-2-yl)propyl)-2,6-diméthylphénoxy)-2-méthylpropanoïque ;
- le 2-(2,3-dichloro-4-(3-oxo-3-(5-(3-(trifluorométhyl)phényl)thièn-2-yl)propyl)-phénoxy)-2-méthylpropanoate d'éthyle ;
- l'acide 2-(2,3-dichloro-4-(3-oxo-3-(5-(3-(trifluorométhyl)phényl)thièn-2-yl)-propyl)phénoxy)-2-méthylpropanoïque ;
- le 2-(2,3-dichloro-4-(3-hydroxy-3-(5-(3-(trifluorométhyl)phényl)thièn-2-yl)-propyl)phénoxy)-2-méthylpropanoate d'éthyle ;
- le 2-(2,3-dichloro-4-(3-méthoxy-3-(5-(3-(trifluorométhyl)phényl)thièn-2-yl)-propyl)phenoxy)-2-méthylpropanoate d'éthyle ;
- l'acide 2-(2,3-dichloro-4-(3-hydroxy-3-(5-(4-(trifluorométhoxy)phényl)thièn-2-yl)propyl)phénoxy)-2-méthylpropanoïque ;
- l'acide 2-(2,3-dichloro-4-(3-méthoxy-3-(5-(4-(trifluorométhoxy)phényl)thièn-2-yl)propyl)phénoxy)-2-méthylpropanoïque.

Les composés selon l'invention peuvent contenir un ou plusieurs centres asymétriques. La présente invention inclut les stéréoisomères (diastéréoisomères, énantiomères), purs ou en mélange, ainsi que les mélanges racémiques et les isomères géométriques. Quand un mélange énantiomériquement pur (ou enrichi) est souhaité, il pourra être obtenu soit par purification du produit final ou d'intermédiaires chiraux, soit par synthèse asymétrique suivant des méthodes connues de l'homme du métier (utilisant par exemple des réactifs et catalyseurs chiraux). Certains composés selon l'invention peuvent avoir différentes formes tautomères stables et toutes ces formes ainsi que leurs mélanges sont inclus dans l'invention.

La présente invention concerne également les sels « pharmaceutiquement acceptables » des composés selon l'invention. D'une manière générale, ce terme désigne les sels peu ou non toxiques obtenus à partir de bases ou d'acides, organiques ou inorganiques. Ces sels peuvent être obtenus lors de l'étape de purification finale du composé selon l'invention ou par incorporation du sel sur le composé déjà purifié.

Certains composés selon l'invention et leurs sels pourraient être stables sous plusieurs formes solides. La présente invention inclut toutes les formes solides des composés selon l'invention, ce qui inclut les formes amorphes, polymorphes, mono- et poly-cristallines.

Les composés selon l'invention peuvent exister sous forme libre ou sous forme solvatée, par exemple avec des solvants pharmaceutiquement acceptables tels que l'eau (hydrates) ou l'éthanol.

Les composés selon l'invention marqués par un ou des isotopes sont également inclus dans l'invention : ces composés sont structurellement identiques mais diffèrent par le fait qu'au moins un atome de la structure est remplacé par un isotope (radioactif ou non). Des exemples d'isotopes pouvant être inclus dans la structure des composés selon l'invention peuvent être choisis parmi l'hydrogène, le carbone, l'oxygène, le soufre tels que ²H, ³H, ¹³C, ¹⁴C, ¹⁸O, ¹⁷O, ³⁵S respectivement. Les isotopes radioactifs ³H et ¹⁴C sont particulièrement préférés car faciles à préparer et à détecter dans le cadre d'études de biodisponibilité *in vivo* des substances. Les isotopes lourds (tels que ²H) sont particulièrement préférés car ils sont utilisés comme standards internes dans des études analytiques.

La présente invention a également pour objet le procédé de synthèse des composés de formule générale (I), qui comprend:
1. une étape de mise en contact en milieu basique ou en milieu acide d'au moins un composé de formule (C) avec au moins un composé de formule (D) 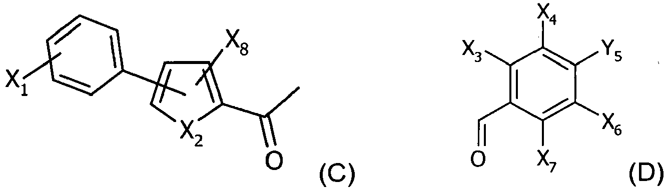
   dans lesquelles X1, X2, X3, X4, X6, X7 et X8 sont tels que définis précédemment,
   Y5 représente un groupement -OR5, hydroxy ou thiol, R5 étant tel que défini précédemment ;
2. éventuellement une étape de réduction des composés obtenus à l'étape (1),
3. et éventuellement une étape d'insertion de groupements fonctionnels.

Les conditions de mise en oeuvre de l'étape (1) en milieu acide ou basique et de l'étape (2) sont connues de l'homme du métier et peuvent varier dans une large mesure. Les protocoles de synthèse peuvent être en particulier ceux présentés dans la partie « exemples » de la présente invention.

La mise en contact de ces deux composés est avantageusement réalisée de manière stoechiométrique. Elle est réalisée de préférence à une température appropriée (entre environ 18°C et 100°C) et de préférence à pression atmosphérique.

En milieu basique, la réaction est de préférence réalisée en présence d'une base forte, tel qu'un hydroxyde de métal alcalin, comme l'hydroxyde de sodium ou un alcoolate de métal alcalin comme l'éthylate de sodium.

En milieu acide, la réaction est de préférence réalisée en présence d'un acide fort, tel que l'acide chlorhydrique.

Les composés ainsi obtenus peuvent être isolés par des méthodes classiques et connues de l'homme du métier.

La présente invention a aussi pour objet les composés tels que décrits ci-avant, à titre de médicaments.

La présente invention a également pour objet un composé tel que décrit ci-avant, pour le traitement des complications associées au syndrome métabolique, de l'athérosclérose, de l'ischémie cérébrale, des maladies autoimmunes, des maladies cardiovasculaires, de l'insulino-résistance, de l'obésité, de l'hypertension, du diabète, des dyslipidémies, des maladies inflammatoires (tel que l'asthme), des pathologies neurodégénératives (en particulier la sclérose en plaques, la maladie de Parkinson, la maladie d'Alzheimer, les tauopathies (démences fronto-temporales, maladie de Pick, dégénérescence cortico-basale, paralysie supranucléaire progressive), les démences corticales, les amyotrophies spinales, les troubles cognitifs légers (MCI : Mild Cognitive Impairment), les synucléopathies, les pathologies à corps de Lewy, la chorée d'Huntington, les épilepsies, la sclérose latérale amyotrophique, les maladies à prions (Maladie de Creutzfeld-Jakob), le syndrome de Down, l'Ataxie de Friedreich, les ataxies spino-cérébelleuses, la maladie de Charcot-Marie-Tooth, les complications neurologiques associées au SIDA, les douleurs chroniques, la dégénérescence cérébelleuse, l'hypoxie cérebelleuse, les neuropathies associées au diabète), des cancers, etc., ainsi que pour permettre la diminution du risque cardiovasculaire global.

Préférentiellement, l'invention a pour objet un composé tel que décrit ci-avant, pour traiter les facteurs de risque cardiovasculaire liés aux dérèglements du métabolisme lipidique et/ou glucidique, notamment les hyperlipidémies et l'obésité, et en particulier le diabète (diabète de type II).

Encore plus préférentiellement, l'invention concerne un composé tel que décrit ci-avant, pour le traitement des dyslipidémies.

La présente invention a également pour objet une composition pharmaceutique comprenant, dans un support pharmaceutiquement acceptable, au moins un composé tel que décrit ci-dessus, éventuellement en association avec un ou plusieurs autres principes actifs thérapeutiques et/ou cosmétiques.

Il s'agit avantageusement d'une composition pharmaceutique pour le traitement des complications associées au syndrome métabolique, de l'athérosclérose, de l'ischémie cérébrale, des maladies autoimmunes, des maladies cardiovasculaires, de l'insulino-résistance, de l'obésité, de l'hypertension, du diabète, des dyslipidémies, des maladies inflammatoires (tel que l'asthme), des pathologies neurodégénératives (en particulier la sclérose en plaques, la maladie de Parkinson, la maladie d'Alzheimer, les tauopathies (démences fronto-temporales, maladie de Pick, dégénérescence cortico-basale, paralysie supranucléaire progressive), les démences corticales, les amyotrophies spinales, les troubles cognitifs légers (MCI: Mild Cognitive Impairment), les synucléopathies, les pathologies à corps de Lewy, la chorée d'Huntington, les épilepsies, la sclérose latérale amyotrophique, les maladies à prions (Maladie de Creutzfeld-Jakob), le syndrome de Down, l'Ataxie de Friedreich, les ataxies spino-cérébelleuses, la maladie de Charcot-Marie-Tooth, les complications neurologiques associées au SIDA, les douleurs chroniques, la dégénérescence cérébelleuse, l'hypoxie cérebelleuse, les neuropathies associées au diabète), des cancers, etc., ainsi que pour permettre la diminution du risque cardiovasculaire global.

Il s'agit préférentiellement d'une composition pharmaceutique pour traiter les facteurs de risque cardiovasculaire liés aux dérèglements du métabolisme lipidique et/ou glucidique, notamment les hyperlipidémies et l'obésité, et en particulier le diabète (diabète de type II).

Encore plus préférentiellement, la composition pharmaceutique selon l'invention est destinée au traitement des dyslipidémies.

Un autre objet de l'invention concerne une composition nutritionnelle comprenant au moins un composé tel que décrit ci-dessus.

La présente invention a aussi pour objet les composés tels que décrits ci-avant, à titre de produits cosmétiques.

Un autre objet de l'invention réside dans l'utilisation d'au moins un composé tel que décrit ci-avant pour la préparation de compositions pharmaceutiques destinées au traitement de diverses pathologies telles que définies ci-dessus, notamment liées à des troubles du métabolisme des lipides et/ou des glucides parmi lesquelles on peut citer les dyslipidémies. Plus généralement, l'invention a pour objet l'utilisation d'au moins un composé tel que décrit ci-avant pour la préparation de compositions pharmaceutiques destinées à traiter les facteurs de risques pour les maladies cardiovasculaires liés aux dérèglements du métabolisme des lipides et/ou des glucides et destinées à diminuer ainsi le risque cardiovasculaire global.

A titre d'exemple (et de manière non limitative), les composés selon l'invention pourront de manière avantageuse être administrés en combinaison avec un ou plusieurs autres agents thérapeutiques et/ou cosmétiques, commercialisés ou en développement, tels que :
- des anti-diabétiques : les insulinosécréteurs (sulfonylurées (glibenclamide, glimépiride, gliclazide, etc.) et glinides (répaglinide, natéglinide, etc.)), les inhibiteurs de l'alpha-glucosidase, les agonistes PPARγ (thiazolidinediones telles que rosiglitazone, pioglitazone), les agonistes mixtes PPARα/PPARγ (tesaglitazar, muraglitazar), les pan-PPAR (composés activant simultanément les 3 isoformes PPAR), des biguanides (metformine), les inhibiteurs de la Dipeptidyl Peptidase IV (sitagliptin, vildagliptin), les agonistes du Glucagon-Like Peptide-1 (GLP-1) (exenatide), etc.
- l'insuline
- des molécules hypolipémiantes et/ou hypocholestérolémiantes : les fibrates (fenofibrate, gemfibrozil), les inhibiteurs de la HMG CoA réductase ou hydroxylméthylglutaryl Coenzyme A reductase (les statines telles que atorvastatine, simvastatine, fluvastatine), les inhibiteurs de l'absorption du cholestérol (ezetimibe, phytostérols), les inhibiteurs de la CETP ou Cholesteryl Ester Transfer Protein (torcetrapib), les inhibiteurs de l'ACAT ou Acyl-Coenzyme A cholesterol acylTransferase (Avasimibe, Eflucimibe), les inhibiteurs MTP (Microsomal Triglycéride Transfer Protein), les agents séquestrants des acides biliaires (cholestyramine), la vitamine E, les acides gras poly-insaturés, les acides gras oméga 3, les dérivés de type acide nicotinique (niacine), etc.
- des agents anti-hypertenseurs et les agents hypotenseurs : les inhibiteurs ACE (Angiotensin-Converting Enzyme) (captopril, enalapril, ramipril ou quinapril), les antagonistes du récepteur de l'angiotensine Il (losartan, valsartan, telmisartan, eposartan, irbesartan, etc.), les béta-bloquants (atenolol, metoprolol, labetalol, propranolol), les diurétiques thiazidiques et non thiazidiques (furosemide, indapamide, hydrochlorthiazide, anti-aldosterone), les vasodilatateurs, les bloquants des canaux calciques (nifedipine, felodipine ou amlodipine, diltiazem ou verapamil), etc.
- des agents anti-plaquettaires : Aspirine, Ticlopidine, Dipyridamol, Clopidogrel, flurbiprofen, etc.
- des agents anti-obésité : Sibutramine, les inhibiteurs de lipases (orlistat), les agonistes et antagonistes PPARδ, les antagonistes du récepteur cannabinoïde CB1 (rimonabant), etc.
- des agents anti-inflammatoires : par exemple, les corticoïdes (prednisone, betamethasone, dexamethasone, prednisolone, méthylprednisolone, hydrocortisone, etc.), les AINS ou Anti-Inflammatoires Non Stéroidiens dérivés de l'indole (indomethacine, sulindac), les AINS du groupe des arylcarboxyliques (acide tiaprofenique, diclofenac, etodolac, flurbiprofen, ibuprofen, ketoprofen, naproxen, nabumetone, alminoprofen), les AINS dérivés de l'oxicam (meloxicam, piroxicam, tenoxicam), les AINS du groupe des fénamates, les inhibiteurs sélectifs de la COX2 (celecoxib, rofecoxib), etc.
- des agents anti-oxydants : par exemple le probucol, etc.
- des agents utilisés dans le traitement de l'insuffisance cardiaque : les diurétiques thiazidiques ou non thiazidiques (furosemide, indapamide, hydrochlorthiazide, anti-aldosterone), les inhibiteurs de l'ACE (captopril, enalapril, ramipril ou quinapril), les digitaliques (digoxin, digitoxin), les béta bloquants (atenolol, metoprolol, labetalol, propranolol), les inhibiteurs de Phosphodiesterases (enoximone, milrinone), etc.
- des agents utilisés pour le traitement de l'insuffisance coronaire : les béta-bloquants (atenolol, metoprolol, labetalol, propranolol), les bloquants des canaux calciques (nifedipine, felodipine ou amlodipine, bepridil, diltiazem ou verapamil), les agents donneurs de NO (trinitrine, isosorbide dinitrate, molsidomine), l'Amiodarone, etc.
- des anticancéreux : les agents cytotoxiques (agents intéragissants avec l'ADN, agents alkylants, cisplatine et dérivés), les agents cytostatiques (les analogues GnRH (Gonatropin-Releasing Hormone), les analogues de la somatostatine, les progestatifs, les anti-oestrogènes, les inhibiteurs de l'aromatase, etc.), les modulateurs de la réponse immunitaire (interférons, IL2, etc.), etc.
- des anti-asthmatiques tels que des bronchodilatateurs (agonistes des récepteurs béta 2), des corticoïdes, le cromoglycate, les antagonistes du récepteur aux leucotriènes (montelukast), etc.
- des corticoïdes utilisés dans le traitement des pathologies de la peau telles que le psoriasis et les dermatites
- des vasodilatateurs et/ou des agents anti-ischémiques (buflomedil, extrait de Ginkgo Biloba, naftidrofuryl, pentoxifylline, piribédil), etc.

L'invention concerne également une méthode de traitement de diverses pathologies telles que définies ci-dessus, notamment liées à des troubles du métabolisme des lipides et/ou des glucides comprenant l'administration à un sujet, notamment humain, d'une quantité efficace d'un composé ou d'une composition pharmaceutique tels que définis ci-avant.

Au sens de l'invention, le terme «une quantité efficace » se réfère à une quantité du composé suffisante pour produire le résultat biologique désiré.

Le terme « sujet » désigne un mammifère et plus particulièrement un humain.

Le terme « traitement » désigne le traitement curatif, symptomatique et/ou préventif. Les composés de la présente invention peuvent ainsi être utilisés chez des sujets (comme les mammifères, en particulier humains) atteints d'une maladie déclarée. Les composés de la présente invention peuvent aussi être utilisés pour retarder ou ralentir la progression ou prévenir une progression plus en avant de la maladie, améliorant ainsi la condition des sujets. Les composés de la présente invention peuvent enfin être administrés aux sujets non malades, mais qui pourraient développer normalement la maladie ou qui ont un risque important de développer la maladie.

Les compositions pharmaceutiques selon l'invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, l'acacia, les liposomes, etc. Les compositions peuvent être formulées sous forme de suspensions injectables, gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Les composés ou compositions selon l'invention peuvent être administrés de différentes manières et sous différentes formes. Ainsi, ils peuvent être par exemple administrés de manière systémique, par voie orale, parentérale, par inhalation ou par injection, comme par exemple par voie intraveineuse, intramusculaire, sous-cutanée, trans-dermique, intra-artérielle, etc. Pour les injections, les composés sont généralement conditionnés sous forme de suspensions liquides, qui peuvent être injectées au moyen de seringues ou de perfusions, par exemple.

Il est entendu que le débit et/ou la dose injectée peuvent être adaptés par l'homme du métier en fonction du patient, de la pathologie, du mode d'administration, etc. Typiquement, les composés sont administrés à des doses pouvant varier entre 1µg et 2g par administration, préférentiellement de 0,01 mg à 1 g par administration. Les administrations peuvent être quotidiennes voire répétées plusieurs fois par jour, le cas échéant. D'autre part, les compositions selon l'invention peuvent comprendre, en outre, d'autres agents ou principes actifs.

### LEGENDES DES FIGURES

### Abréviations employées sur les figures et dans les tableaux :

- Cpd = composés ;
- HDL-cholesterol : High-Density-Lipoprotein cholesterol
- LDL-cholesterol : Low-Density-Lipoprotein cholesterol
- VLDL-cholesterol : Very-Low-Density-Lipoprotein cholesterol
- mpk =mg/kg/jour.

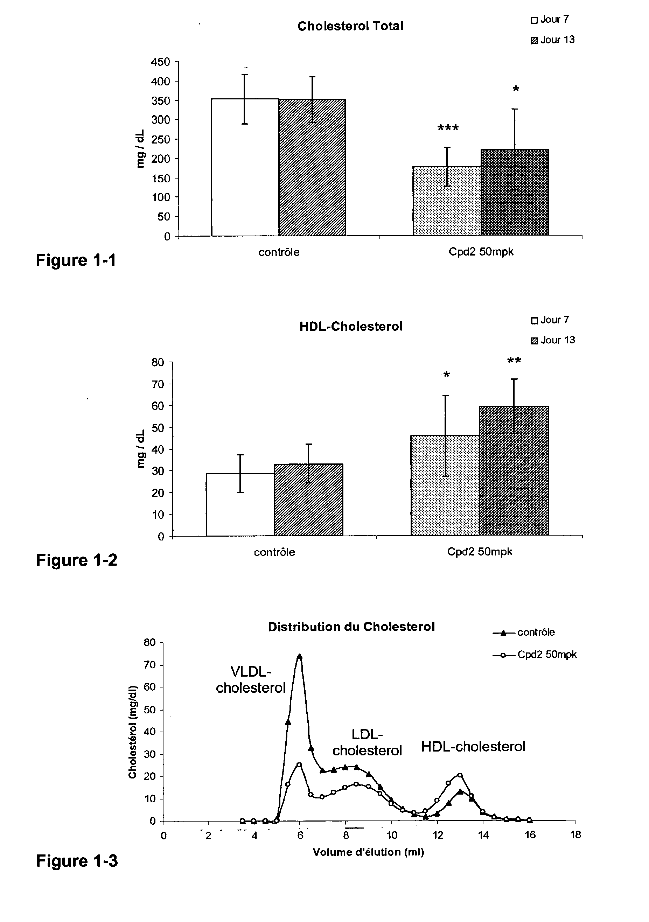

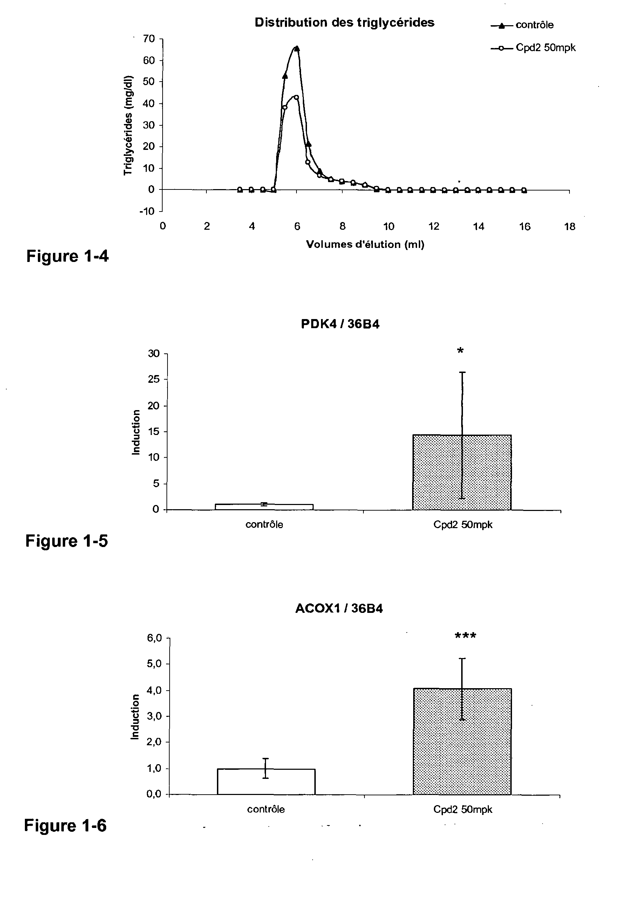

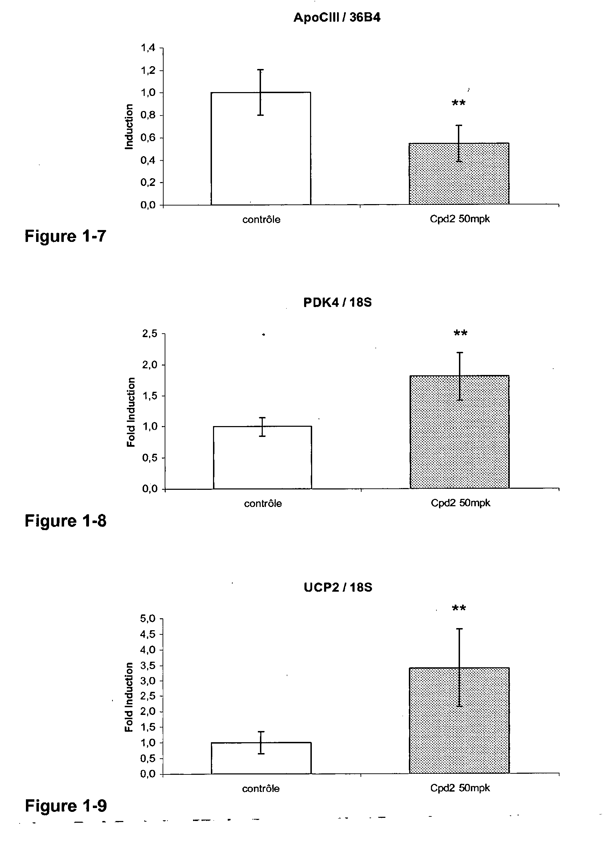

### Figures 1-1 à 1-9 : Evaluation in vivo, chez la souris E2/E2, des propriétés hypolipémiantes et stimulatrices de la synthèse de HDL-cholestérol des composés selon l'invention par dosages lipidiques et mesure de l'expression de gènes impliqués dans le métabolisme lipidique et glucidique et la dissipation d'énergie

L'effet hypolipémiant des composés selon l'invention a été évalué *in vivo* chez la souris E2/E2 (humanisée pour l'isoforme E2 de l'apolipoproteine E) par l'analyse de la répartition du cholestérol et des triglycérides dans les différentes fractions lipoprotéiques plasmatiques et par la mesure des taux de cholestérol total et de HDL-cholestérol plasmatiques après 7 et 13 jours de traitement par voie orale ; ces taux sont comparés à ceux obtenus avec des animaux contrôles (non traités par les composés selon l'invention). La différence mesurée témoigne de l'effet hypolipémiant des composés selon l'invention.
- Figure 1-1 : taux de cholestérol total plasmatique après 7 et 13 jours de traitement avec le composé 2, administré à 50 mpk ;
- Figure 1-2 : taux de HDL-cholestérol plasmatique après 7 et 13 jours de traitement avec le composé 2, administré à 50 mpk ;
- Figure 1-3: répartition du cholestérol dans les différentes fractions lipoprotéiques plasmatiques après 13 jours de traitement avec le composé 2, administré à 50 mpk ;
- Figure 1-4: répartition des triglycérides dans les différentes fractions lipoprotéiques plasmatiques après 13 jours de traitement avec le composé 2, administré à 50 mpk.

L'efficacité des composés selon l'invention a aussi été évaluée par la mesure, dans les tissus hépatiques et musculaires (squelettiques), de l'expression de gènes impliqués dans le métabolisme lipidique, glucidique et la dissipation d'énergie. Les niveaux d'expression de chaque gène ont été normalisés par rapport au niveau d'expression des gènes de référence 36B4 dans le tissu hépatique ou 18S dans le muscle squelettique gastrocnémien. Le facteur d'induction, c'est-à-dire le rapport entre le signal relatif (induit par le composé selon l'invention) et la moyenne des valeurs relatives du groupe contrôle a ensuite été calculé. Plus ce facteur est élevé, plus le composé a un caractère activateur d'expression génique. Le résultat final est représenté comme moyenne des valeurs d'induction dans chaque groupe expérimental.
- Figure 1-5: expression de PDK4 (Pyruvate Deshydrogenase Kinase, isoforme 4) dans le tissu hépatique, chez la souris E2/E2, après 13 jours de traitement avec le composé 2 (50 mpk) ;
- Figure 1-6 : expression de l'Acox1 dans le tissu hépatique, chez la souris E2/E2, après 13 jours de traitement avec le composé 2 (50 mpk) ;
- Figure 1-7 : expression de l'ApoCIII dans le tissu hépatique, chez la souris E2/E2, après 13 jours de traitement avec le composé 2 (50 mpk) ;
- Figure 1-8: expression de PDK4 (Pyruvate Deshydrogenase Kinase, isoforme 4) dans le muscle squelettique, chez la souris E2/E2, après 13 jours de traitement avec le composé 2 (50 mpk) ;
- Figure 1-9: expression d'UCP2 (uncoupling protein 2 dans le muscle squelettique, chez la souris E2/E2, après 13 jours de traitement avec le composé 2 (50 mpk).

### Figures 2-1 à 2-6 : Evaluation in vivo, chez la souris C57BI6, des propriétés hypolipémiantes et stimulatrices de la synthèse de HDL-cholestérol des composés selon l'invention par dosages lipidiques et mesure de l'expression de gènes impliqués dans le métabolisme lipidique, glucidique et la dissipation d'énergie.

L'effet du composé 2 selon l'invention, administré en effet dose, a été évalué *in vivo* chez la souris C57BI6 après 14 jours de traitement par voie orale. A l'issue du traitement, l'effet hypolipémiant du composé 2 selon l'invention a été évalué par la mesure des taux de cholestérol total, de HDL-cholestérol, de triglycérides et d'acides gras libres plasmatiques.

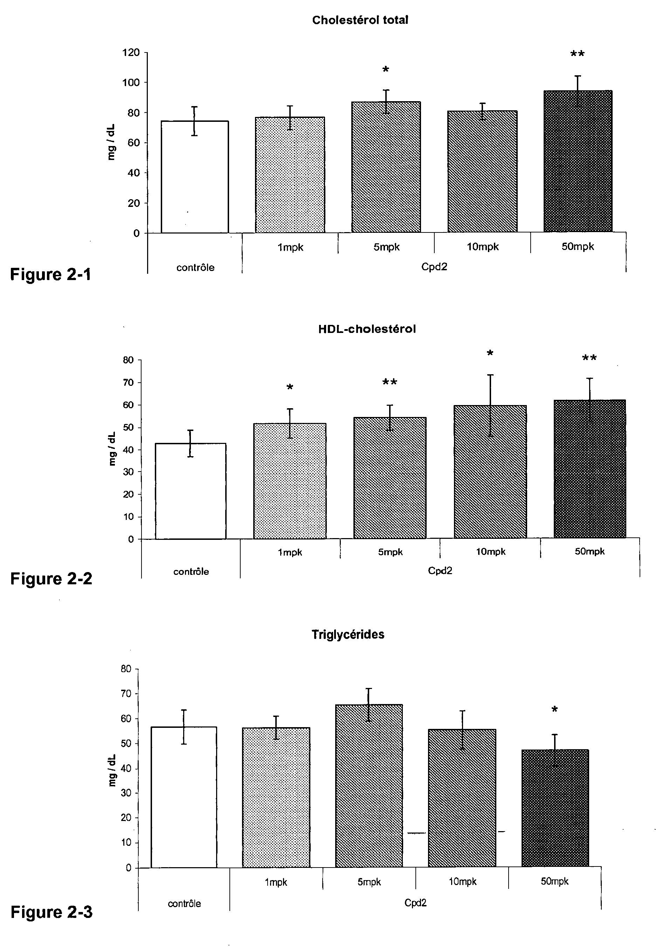

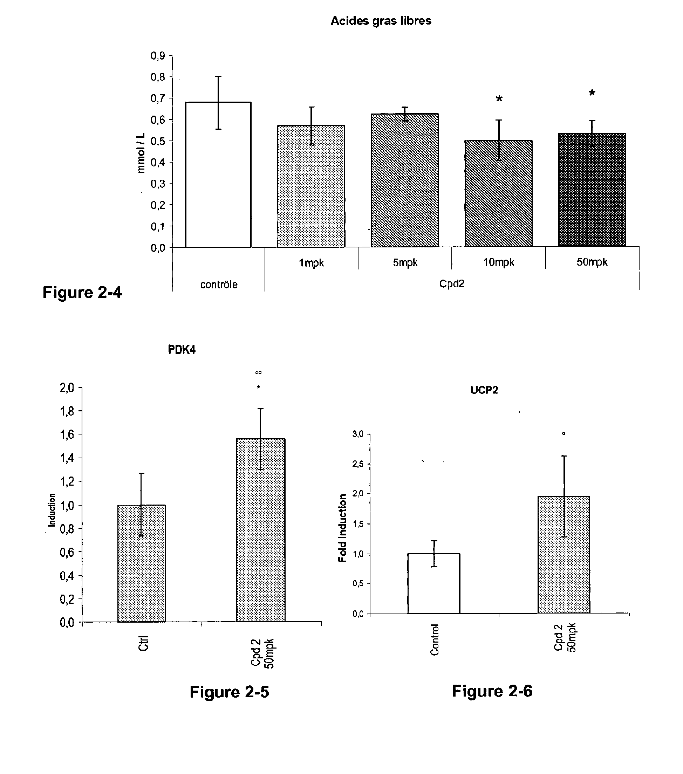

- Figure 2-1 : taux de cholestérol total plasmatique après 14 jours de traitement avec le composé 2 selon l'invention, administré à 1, 5, 10 et 50 mpk, chez la souris C57BI6 ;
- Figure 2-2 : taux de HDL-cholestérol plasmatique après 14 jours de traitement avec le composé 2 selon l'invention, administré à 1, 5, 10 et 50 mpk, chez la souris C57BL6 ;
- Figure 2-3: taux de Triglycérides plasmatiques après 14 jours de traitement avec le composé 2 selon l'invention, administré à 1, 5, 10 et 50 mpk, chez la souris C57BI6 ;
- Figure 2-4 : taux d'acides gras libres plasmatiques après 14 jours de traitement avec le composé 2 selon l'invention, administré à 1, 5, 10 et 50 mpk, chez la souris C57BI6.

L'efficacité des composés selon l'invention a aussi été évaluée par la mesure, dans le tissu musculaire (squelettique), de l'expression de gènes impliqués dans le métabolisme glucidique et la dissipation d'énergie. Les niveaux d'expression de chaque gène ont été normalisés par rapport au niveau d'expression du gène de référence 18S. Le facteur d'induction, a ensuite été calculé. Plus ce facteur est élevé, plus les composés ont un caractère activateur d'expression génique. Le résultat final est représenté comme moyenne des valeurs d'induction dans chaque groupe expérimental.
- Figure 2-5 : expression de PDK4 dans le muscle squelettique, chez la souris C57BI6, après 14 jours de traitement par voie orale avec le composé 2 (50 mpk) ;
- Figure 2-6: expression d'UCP2 dans le muscle squelettique, chez la souris C57BI6, après 14 jours de traitement par voie orale avec le composé 2 (50mpk).

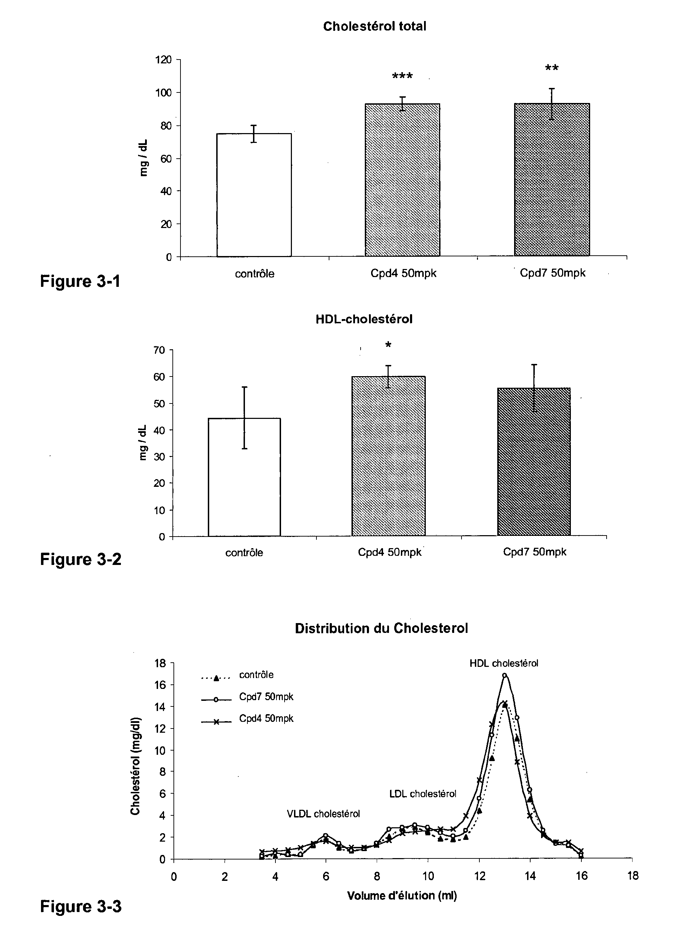

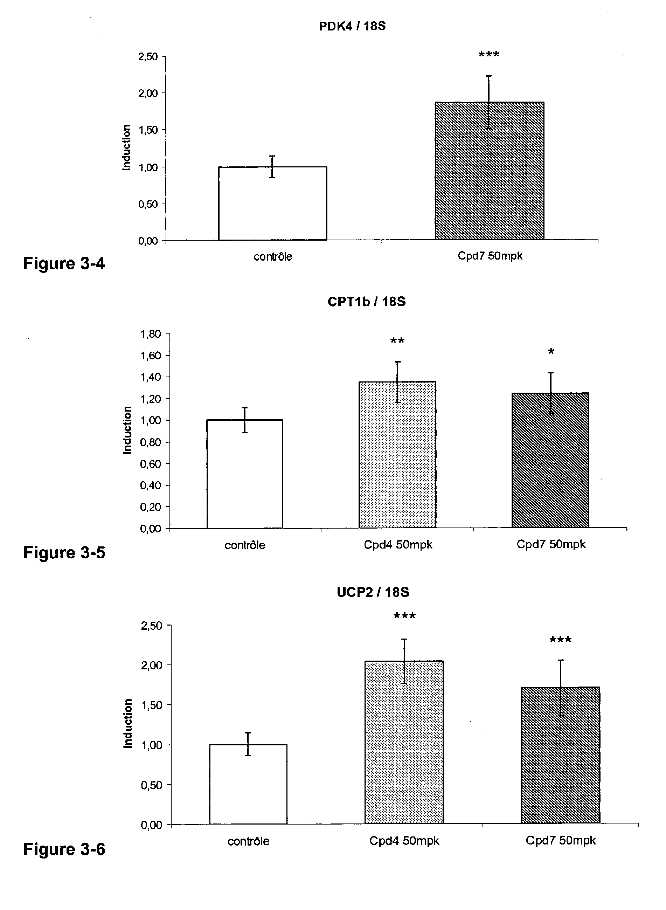

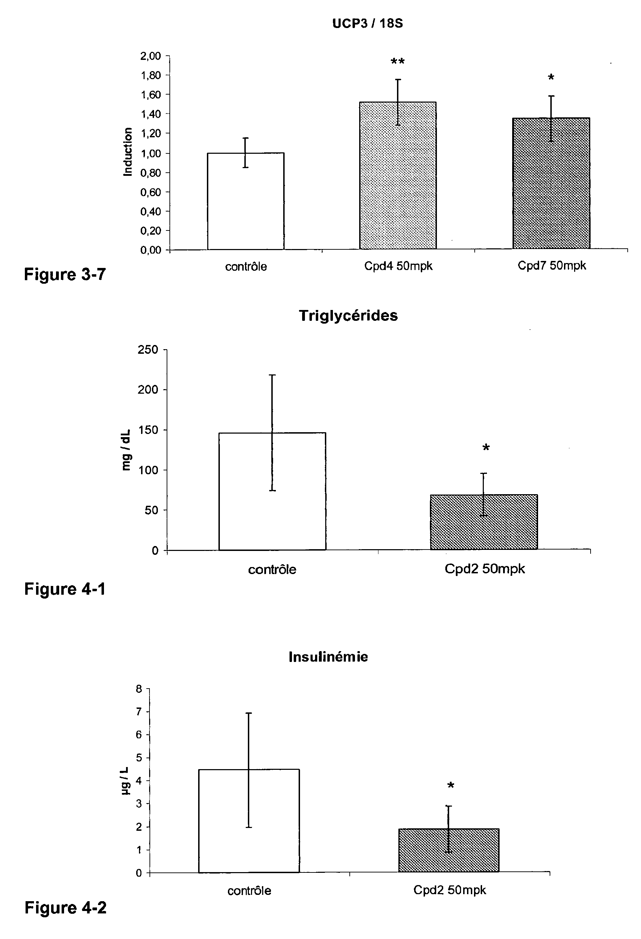

### Figures 3-1 à 3-7: Evaluation in vivo, chez la souris C57BI6, des propriétés stimulatrices de la synthèse de HDL-cholestérol des composés selon l'invention par dosages lipidiques et mesure de l'expression de gènes impligués dans le métabolisme lipidique, glucidique et la dissipation d'énergie.

L'effet des composés selon l'invention a été évalué *in vivo* chez la souris C57BI6 après 14 jours de traitement par voie orale. A l'issu du traitement, la répartition du cholestérol dans les différentes fractions lipoprotéiques plasmatiques a été déterminée. Celle-ci a été comparée au profil obtenu pour les animaux contrôles (non traités par les composés selon l'invention). L'effet des composés selon l'invention a aussi été évalué *in vivo* chez la souris C57BI6 par la mesure des taux plasmatiques de cholestérol total et de HDL-cholestérol après 14 jours de traitement par voie orale. Ces taux ont été comparés à ceux obtenus pour des animaux contrôles (non traités par les composés selon l'invention). La différence mesurée témoigne de l'effet hypolipémiant des composés selon l'invention.
- Figure 3-1 : taux de cholestérol total plasmatique après 14 jours de traitement avec les composés 4 et 7 selon l'invention, administrés à 50 mpk;
- Figure 3-2: taux de HDL cholestérol plasmatique après 14 jours de traitement avec les composés 4 et 7 selon l'invention, administrés à 50 mpk ;
- Figure 3-3: répartition du cholestérol dans les différentes fractions lipoprotéiques plasmatiques après 14 jours de traitement avec les composés 4 et 7 selon l'invention, administrés à 50 mpk.

L'efficacité des composés selon l'invention a aussi été évaluée par la mesure, dans le tissu musculaire (squelettique), de l'expression de gènes impliqués dans le métabolisme lipidique, glucidique et la dissipation d'énergie. Les niveaux d'expression de chaque gène ont été normalisés par rapport au niveau d'expression du gène de référence 18S. Le facteur d'induction a ensuite été calculé. Plus ce facteur est élevé, plus les composés ont un caractère activateur d'expression génique. Le résultat final est représenté comme moyenne des valeurs d'induction dans chaque groupe expérimental.
- Figure 3-4 : expression de PDK4 dans le tissu musculaire, chez la souris C57BI6, après 14 jours de traitement avec le composé 7 (50 mpk) ;
- Figure 3-5 : expression de CPT1 b dans le tissu musculaire, chez la souris C57BI6, après 14 jours de traitement avec les composés 4 et 7 (50 mpk) ;
- Figure 3-6 : expression de UCP2 dans le tissu musculaire, chez la souris C57BI6, après 14 jours de traitement avec les composés 4 et 7 (50 mpk) ;
- Figure 3-7 : expression de UCP3 dans le tissu musculaire, chez la souris C57BI6, après 14 jours de traitement avec les composés 4 et 7 (50 mpk).

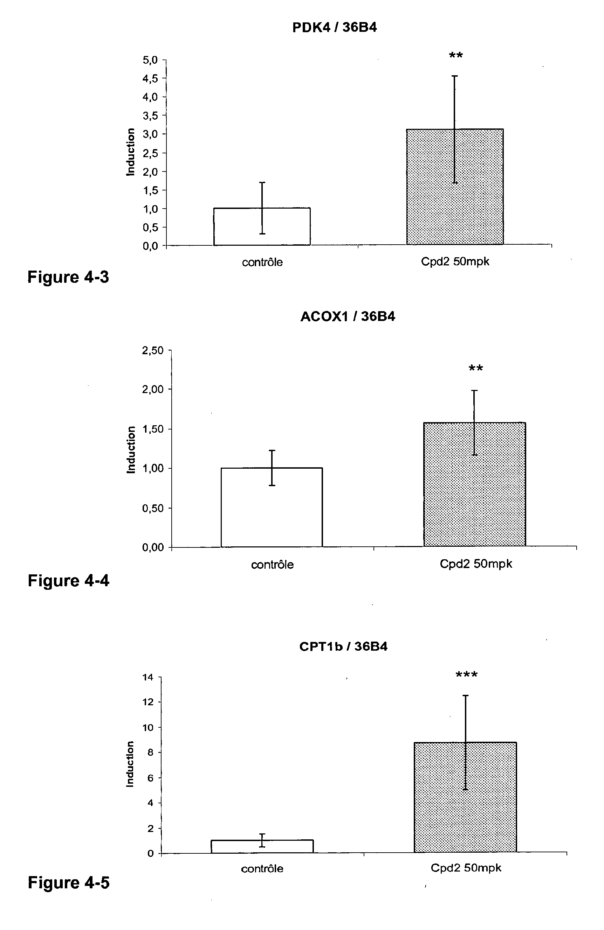

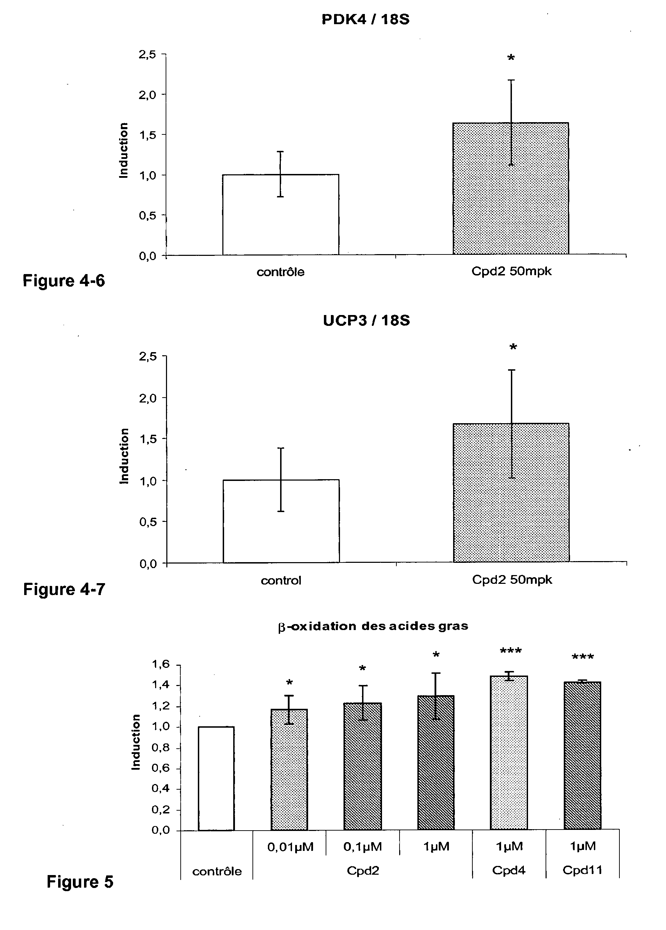

### Figures 4-1 à 4-7: Evaluation in vivo, chez la souris db/db, des propriétés hypolipémiantes, antidiabétiques et activatrices des PPAR des composés selon l'invention.

L'effet des composés selon l'invention a été évalué *in vivo* chez la souris db/db par la mesure des triglycérides plasmatiques et de l'insulinémie après 28 jours de traitement par voie orale avec le composé 2. Ces taux ont été comparés à ceux obtenus avec des animaux contrôles (non traités par le composé selon l'invention). La différence mesurée témoigne de l'effet hypolipémiant et sur l'insulino-résistance du composé selon l'invention.
- Figure 4-1 : taux de triglycérides plasmatiques après 28 jours de traitement par le composé 2, administré à 50 mpk chez la souris db/db ;
- Figure 4-2 : taux d'insuline plasmatique après 28 jours de traitement par le composé 2, administré à 50 mpk chez la souris db/db.

L'efficacité du composé 2 a aussi été évaluée par la mesure, dans les tissus hépatiques et musculaires de l'expression de gènes impliqués dans le métabolisme glucidique, lipidique, et la dissipation d'énergie. Les niveaux d'expression de chaque gène ont été normalisés par rapport au niveau d'expression des gènes de référence 36B4 dans le foie et 18S dans le muscle squelettique. Le facteur d'induction, c'est-à-dire le rapport entre le signal relatif (induit par le composé selon l'invention) et la moyenne des valeurs relatives du groupe contrôle, a ensuite été calculé. Plus ce facteur est élevé, plus le composé a un caractère activateur d'expression génique. Le résultat final est représenté comme moyenne des valeurs d'induction dans chaque groupe expérimental.
- Figure 4-3 : expression de PDK4 dans le tissu hépatique, chez la souris db/db, après 28 jours de traitement avec le composé 2, administré à 50 mpk;
- Figure 4-4 : expression d'ACOX1 dans le tissu hépatique, chez la souris db/db, après 28 jours de traitement avec le composé 2, administré à 50 mpk;
- Figure 4-5 : expression de CPT1 b dans le tissu hépatique, chez la souris db/db, après 28 jours de traitement avec le composé 2, administré à 50 mpk ;
- Figure 4-6 : expression de PDK4 dans le tissu musculaire, chez la souris db/db, après 28 jours de traitement avec le composé 2, administré à 50 mpk ;
- Figure 4-7 : expression de UCP3 dans le tissu musculaire, chez la souris db/db, après 28 jours de traitement avec le composé 2, administré à 50 mpk.

### Figure 5 : Evaluation in vitro des propriétés métaboliques des composés selon l'invention par mesure de la β-oxidation des acides gras dans des myocytes murins

Les effets stimulateurs des composés selon l'invention ont été évalués par la mesure de la 5-oxydation des acides gras dans des myocytes murins pré-traités pendant 24 heures avec les composés selon l'invention. Plus l'induction de la β-oxidation des acides gras est augmentée, plus les composés selon l'invention sont stimulateurs de la dégradation des acides gras dans les cellules musculaires.

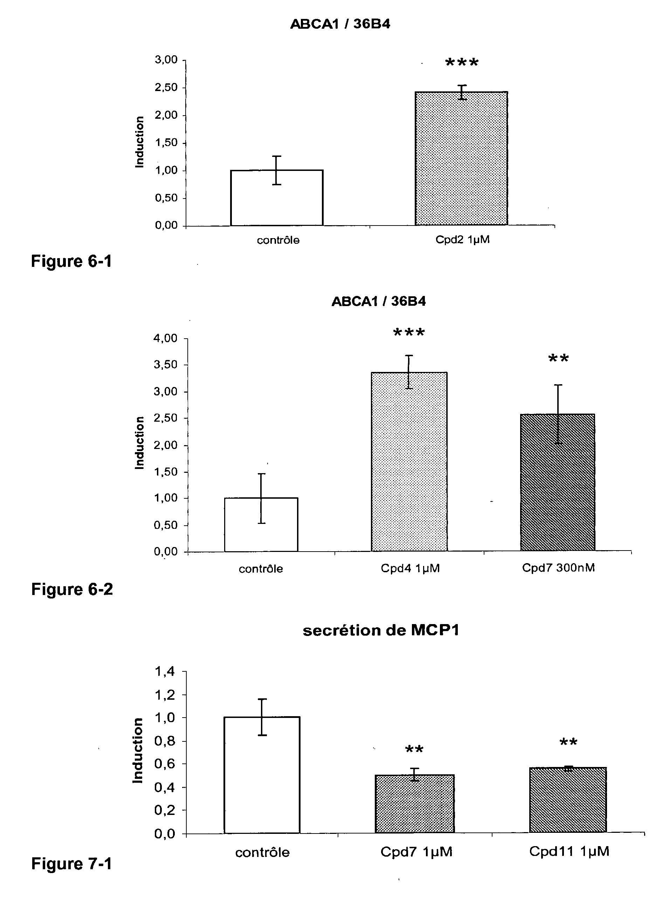

### Figures 6-1 et 6-2: Evaluation in vitro des propriétés activatrices du transport inverse du cholestérol des composés selon l'invention par mesure de l'expression du gène ABCA1 dans les macrophages.

L'effet des composés selon l'invention sur le transport inverse du cholestérol a été évalué par la mesure de l'expression du gène ABCA1 (ATP-binding cassette, sub-family A, member 1 ; transporteur membranaire impliqué dans l'efflux de cholestérol) dans des macrophages humains. Plus l'expression d'ABCA1 est augmentée, plus le composé selon l'invention stimule le transport inverse du cholestérol.
- Figure 6-1 : expression de ABCA1 dans les macrophages humains, après 24 heures de traitement avec le composé 2, à 1 µM ;
- Figure 6-2 : expression de ABCA1 dans les macrophages humains, après 24 heures de traitement avec les composés 4 et 7 selon l'invention, à 1 µM et 300nM, respectivement.

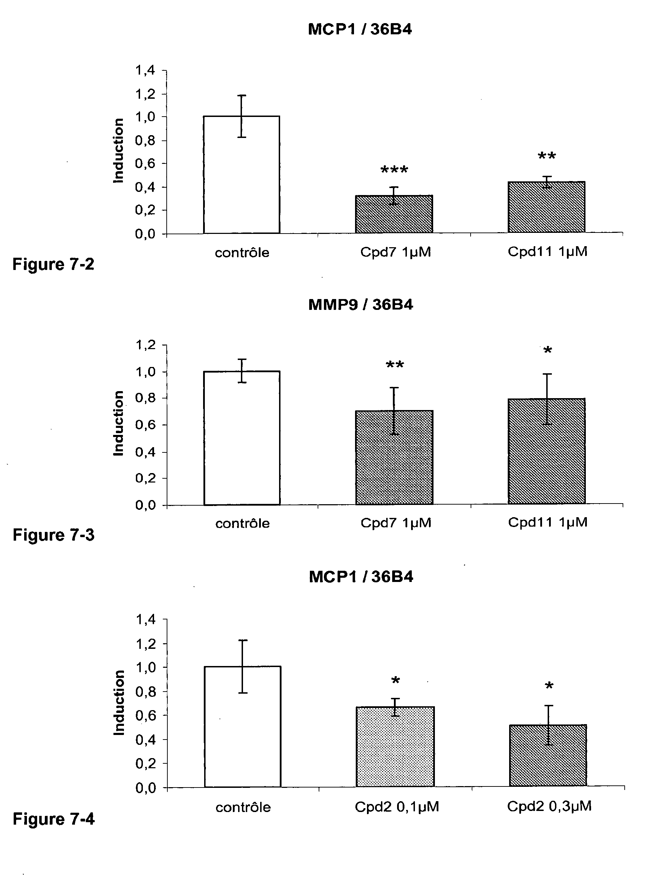

### Figures 7-1 à 7-4 : Evaluation in vitro des propriétés anti-inflammatoires des composés selon l'invention par mesure de la sécrétion et de l'expression de MCP1 et MMP9 par des monocytes humains traités avec les composés selon l'invention et stimulés avec du PMA

Les effets anti-inflammatoires des composés selon l'invention ont été évalués par la mesure de la sécrétion et de l'expression de la Monocyte Chemoattractant Protein-1 (MCP1) ainsi que par la mesure de l'expression de la matrix metalloproteinase 9 (MMP9) par des monocytes humains traités pendant 24 heures avec les composés selon l'invention et stimulés avec du PMA (phorbol 12-myristate 13-acétate, qui provoque une réponse inflammatoire des cellules). Plus la quantité de MCP1 sécrétée est diminuée, plus le composé selon l'invention inhibe la réponse inflammatoire. De la même manière, plus l'expression des gènes MCP1 et MMP9 est inhibée, plus le composé selon l'invention est anti-inflammatoire.
- Figure 7-1 : sécrétion de MCP1 (Monocyte Chemoattractant Protein-1) dans les monocytes humains, traités avec les composés 7 et 11 selon l'invention à 1µM ;
- Figure 7-2 : expression de MCP1 (Monocyte Chemoattractant Protein-1) dans les monocytes humains, traités avec les composés 7 et 11 selon l'invention à 1µM ;
- Figure 7-3 : expression de MMP9 (matrix metalloproteinase 9) dans les monocytes humains, traités avec les composés 7 et 11 selon l'invention à 1µM ;
- Figure 7-4 : expression de MCP1 (Monocyte Chemoattractant Protein-1) dans les monocytes humains, traités avec le composé 2 à 0,1 et 0,3µM.

D'autres avantages et aspects de l'invention apparaîtront à la lecture des exemples qui suivent, qui doivent être considérés comme illustratifs et non limitatifs.

### ANALYSES STATISTIQUES

Les études statistiques réalisées consistent en un test T de Student et/ou une Analyse de la Variance univariée à un facteur (ANOVA), suivie d'un test de Tukey. Les résultats sont comparés par rapport au groupe contrôle selon la valeur du paramètre p :
* : p<0,05 ; ** : p<0,01 ; *** : p<0,001.

### EXEMPLES

Les réactifs et catalyseurs usuels sont disponibles commercialement (Aldrich, Alfa Aesar, Acros, Fluka ou Lancaster selon les cas).

Dans ces exemples, différentes analyses sont réalisées pour l'identification des composés.

Les points de fusion (F) sont donnés en degrés Celsius.

La pureté des produits est vérifiée par Chromatographie sur Couche Mince (CCM) et/ou par HPLC (chromatographie liquide haute performance).

Les spectres de masse sont réalisés par ESI-MS (Electrospray Ionisation - Mass Spectroscopy), Q-TOF (Quadripol - Time of Flight) ou MALDI-TOF (Matrix Assisted Laser Desorption/Ionization - Time of Flight).

Les spectres de Résonance Magnétique Nucléaire du Proton (RMN ¹H) ont été enregistrés sur un spectromètre Bruker AC300P. Les déplacements chimiques sont exprimés en ppm (partie par million) et sont enregistés à 300 MHz dans un solvant deutéré qui est précisé pour chaque analyse : DMSO-*d*₆, MeOD ou CDCl₃.

Pour l'interprétation des spectres, sont utilisées les abréviations suivantes : s pour singulet, sl pour singulet large, d pour doublet, dd pour doublet dédoublé, ddd pour doublet dédoublé dédoublé, t pour triplet, td pour triplet dédoublé, q pour quadruplet, quint pour quintuplet, sext pour sextuplet, m pour multiplet ou massif.

### Exemple 1 : Description des protocoles généraux de synthèse selon l'invention

### Procédure générale A :

Le dérivé bromé (0,5 à 75 g, 0,05 à 0,5 mol/mL), le carbonate de potassium (3 éq.) et l'eau (11 éq.) sont solubilisés dans du N,N-diméthylformamide sous atmosphère inerte. L'acétate de palladium (0,1 éq.) est additionné, puis la solution d'acide boronique dans du N,N-diméthylformamide (1,5 éq., 0,25g/mL) est ajoutée goutte à goutte. Le milieu est laissé sous agitation, sous atmosphère inerte à température ambiante.

### Procédure générale B :

La cétone (1 éq.) et l'aldéhyde (1 éq.) sont solubilisés dans une solution d'éthanol saturée d'acide chlorhydrique gazeux (0,2 g à 38 g, 0,2 à 0,5 mol/L). Après 16 heures d'agitation à température ambiante le solvant est éliminé par évaporation sous pression réduite.

### Procédure générale C :

La propénone est solubilisée dans un mélange 2 :1 chloroforme/méthanol (0,2 à 14 g, 0,01 à 0,2 mol/L) puis, une quantité catalytique de palladium sur charbon est ajoutée. L'ensemble est placé sous atmosphère d'hydrogène à pression atmosphérique.

### Procédure générale D :

Le phénol ou le thiophénol est solubilisé dans du N,N-diméthylformamide (0,3 à 12 g, 0,06 à 0,2 mol/L), puis le dérivé halogéné (5 éq.) et le carbonate de potassium (5 éq.) sont ajoutés. Le milieu réactionnel est maintenu sous vive agitation à 70°C.

### Procédure générale E :

L'ester de tertiobutyle est solubilisé dans du dichlorométhane (0,2 à 15 g, 0,1 à 1 mol/L) puis, l'acide trifluoroacétique (10 à 17 éq.) est additionné. L'agitation est maintenue à température ambiante.

### Procédure générale F :

L'ester est solubilisé dans de l'éthanol (0,2 à 0,4 g, 0,1 à 0,2 mol/L) puis une solution de soude 2N est additionnée.

### Procédure générale G :

La propanone est solubilisée dans de l'éthanol (0,2 à 4 g, 0,1 à 0,5 mol/L). Le borohydrure de sodium (3 éq.) est ajouté. L'ensemble est maintenu 3 heures sous agitation à température ambiante. Le solvant est éliminé par évaporation sous pression réduite, le résidu d'évaporation est repris par une solution aqueuse diluée d'acide chlorhydrique et extrait par du dichlorométhane.

### Procédure générale H :

L'alcool est solubilisé dans du N,N-diméthylformamide (0,2 à 3 g, 0,1 à 0,5 mol/L), la solution est refroidie à 0°C puis l'hydrure de sodium est ajouté. Après 20 minutes d'agitation, l'halogénure d'alkyle approprié est ajouté. Le milieu est laissé 4 heures sous agitation à température ambiante. Les solvants sont éliminés par évaporation sous pression réduite.

### Procédure générale I :

La propanone est solubilisée dans de la pyridine (0,3 g, 0,1 mol/L). Le chlorhydrate de O-alkylhydroxylamine (5 à 10 équivalents) est ajouté. Après 18 h de reflux, le milieu est évaporé sous pression réduite, repris par de l'acétate d'éthyle et lavé par une solution d'acide chlorhydrique diluée. La phase organique est concentrée sous pression réduite. Le résidu d'évaporation est purifié par chromatographie flash sur gel de silice.

### Exemple 2 : Synthèse des matières premières intervenant dans la synthèse des composés selon l'invention :

### 2,3-dichloro-4-hydroxybenzaldéhyde

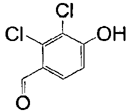

Le carbonate de sodium (3,5 éq.), l'hydroxyde de calcium (4,5 éq.) et le 2,3-dichlorophénol (0,15 g/L) sont ajoutés à l'eau, la suspension est chauffée à 70°C pendant 4 heures. Le chloroforme (2 éq.) est additionné goutte à goutte et l'ensemble est laissé sous agitation à 70°C pendant 16 heures.

Le milieu réactionnel est refroidi à 0°C, acidifié (pH=2) par une solution d'acide chlorhydrique concentrée. L'ensemble est extrait par de l'acétate d'éthyle ; les phases organiques sont lavées avec de l'eau, séchées sur sulfate de magnésium et concentrées sous pression réduite. Le résidu d'évaporation est purifié par chromatographie flash sur gel de silice. Le solide obtenu est recristallisé dans l'isopropanol.

Elution : cyclohexane/acétate d'éthyle : gradient 9/1 à 7/3. Silice 40-63µm. RMN ¹H (300MHz, DMSO-d₆, δ en ppm) : 7,20 (d, 1 H, J=8,8Hz); 7,70 (d, 1 H, J=8,8Hz); 10,13 (s, 1 H).

### 4-hydroxy-3,5-diméthylbenzaldéhyde

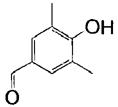

Le 2,6-diméthylphénol (0,34 g/mL) et l'hexaméthylènetétramine (2 éq.) sont solubilisés dans un mélange acide acétique/eau : 2/1. L'ensemble est chauffé à 100°C pendant 4 heures. Le milieu réactionnel est refroidi à température ambiante versé sur un mélange eau/glace. Le précipité est essoré.

RMN ¹H (300MHz, CDCl₃, δ en ppm) : 2,33 (s, 6H); 5,90 (s, 1 H); 7,55 (s, 2H); 9,81 (s, 1 H).

### 1-(5-(4-(trifluorométhyl)phényl)thièn-2-yl)éthanone

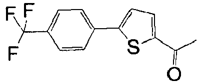

La 1-(5-(4-(trifluorométhyl)phényl)thièn-2-yl)éthanone est préparée à partir d'acide 5-acétyl-2-thiophéneboronique et de 4-bromobenzotrifluoride selon la procédure générale A.

Après 2 heures d'agitation, les solvants sont éliminés par évaporation sous pression réduite et le résidu d'évaporation est purifié par chromatographie sur gel de silice.

Elution : cyclohexane/acétate d'éthyle : 8/2. Silice 40-63µm.

RMN ¹H (300MHz, CDCl₃, δ en ppm) : 2,60 (s, 3H); 7,40 (d, 1 H, J=3,8Hz); 7,66-7,70 (m, 3H); 7,76 (d, 2H, J=8,2Hz).

### 1-(4-(4-(trifluorométhyl)phényl)thièn-2-yl)éthanone

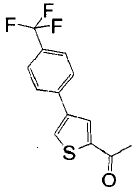

La 1-(4-(4-(trifluorométhyl)phényl)thièn-2-yl)éthanone est préparée à partir de la (4-bromothièn-2-yl)éthanone et d'acide 4-trifluorométhylphénylboronique selon la procédure générale A.

Après 18 heures d'agitation, les solvants sont éliminés par évaporation sous pression réduite et le résidu d'évaporation est purifié par chromatographie sur gel de silice.

Elution : cyclohexane/acétate d'éthyle : 9/1. Silice 40-63µm.

RMN ¹H (300MHz, CDCl₃, δ en ppm) : 2,63 (s, 3H); 7,70 (m, 4H); 7,82 (d, 1 H, J=1,5Hz); 7,97 (d, 1H, J=1,5Hz).

### 1-(5-(4-(trifluorométhoxy)phényl)thièn-2-yl)éthanone

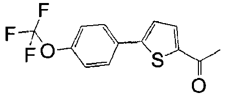

La 1-(5-(4-(trifluorométhoxy)phényl)thièn-2-yl)éthanone est préparée à partir d'acide 5-acétyl-2-thiophèneboronique et de 1-bromo-4-(trifluorométhoxy)benzène selon la procédure générale A.

Après 1 heure d'agitation, le milieu réactionnel est dilué par de l'eau, filtré sur célite puis extrait par de l'acétate d'éthyle. La phase organique est séchée sur sulfate de magnésium, filtrée puis concentrée sous pression réduite. Le résidu d'évaporation est recristallisé dans du cyclohexane.

RMN ¹H (300MHz, DMSO-*d₆*, δ en ppm) : 2,56 (s, 3H); 7,47 (d, 2H, J=8,4Hz); 7,71 (d, 1 H, J=3,9Hz); 7,91 (d, 2H, J=8,4Hz); 7,98 (d, 1 H, J=3,9Hz).

### 1-(5-(4-bromophénvl)thièn-2-yl)éthanone

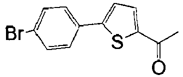

La 1-(5-(4-bromophényl)thièn-2-yl)éthanone est préparée à partir d'acide 5-acétyl-2-thiophèneboronique et de 1-bromo-4-iodobenzène selon la procédure générale A.

Après 12 heures d'agitation, le milieu réactionnel est dilué par de l'eau, filtré sur célite puis extrait par de l'acétate d'éthyle. La phase organique est séchée sur sulfate de magnésium, filtrée puis concentrée sous pression réduite. Le résidu d'évaporation est purifié par chromatographie sur gel de silice.

Elution : cyclohexane/acétate d'éthyle : 9/1 à 8/2. Silice 40-63µm.

RMN ¹H (300MHz, CDCl₃, δ en ppm) : 2,57 (s, 3H); 7,31 (d, 1 H, J=3,9Hz); 7,51 (d, 2H, J=9,0Hz); 7,55 (d, 2H, J=9,0Hz); 7,65 (d, 1 H, J=3,9Hz).

### 5-bromo-2-acétylfurane

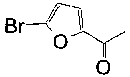

Le 2-acétylfurane est solubilisé dans du N,N-diméthylformamide (5 g, 1,1 mol/L) et le N-bromosuccinimide (1 éq.) est ajouté.

Après 5 heures d'agitation à température ambiante, le milieu réactionnel est versé sur de l'eau glacée et le précipité formé est essoré.

RMN ¹H (300MHz, CDCl₃, δ en ppm) : 2,47 (s, 3H); 6,49 (d, 1H, J=3,6Hz); 7,12 (d, 1 H, J=3,6Hz).

### 1-(5-(4-(trifluorométhyl)phényl)fur-2-yl)éthanone

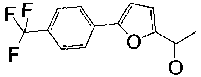

La 1-(5-(4-(trifluorométhyl)phényl)fur-2-yl)éthanone est préparée à partir d'acide 4-trifluorométhylbenzèneboronique et de 5-bromo-2-acétylfurane selon la procédure générale A.

Après 18 heures d'agitation, le milieu réactionnel est dilué par de l'eau puis extrait par de l'acétate d'éthyle. La phase organique est séchée sur sulfate de magnésium, filtrée puis concentrée sous pression réduite. Le résidu d'évaporation est purifié par chromatographie sur gel de silice.

Elution : cyclohexane/acétate d'éthyle : 95/5 à 8/2. Silice 40-63µm.

RMN ¹H (300MHz, CDCl₃, δ en ppm) : 2,55 (s, 3H); 6,89 (d, 1H, J=3,7Hz); 7,28 (d, 1 H, J=3,7Hz); 7,68 (d, 1H, J=8,1 Hz); 7,89 (d, 1H, J=8,1 Hz).

### 1-(5-(4-(méthylthio)phényl)thièn-2-yl)éthanone

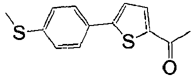

La 1-(5-(4-(méthylthio)phényl)thièn-2-yl)éthanone est préparée à partir d'acide 5-acétyl-2-thiophèneboronique et de 1-bromo-4-(méthylthio)benzène selon la procédure générale A.

Après 18 heures d'agitation à 100°C, le milieu réactionnel est dilué par de l'eau, filtré sur célite puis extrait par de l'acétate d'éthyle. La phase organique est séchée sur sulfate de magnésium, filtrée puis concentrée sous pression réduite. Le résidu d'évaporation est purifié par chromatographie sur gel de silice.

Elution : cyclohexane/acétate d'éthyle : 9/1 à 8/2. Silice 40-63µm.

RMN ¹H (300MHz, DMSO-*d₆*, δ en ppm) : 2,54 (s, 3H); 7,34 (d, 2H, J=8,7Hz); 7,63 (d, 1 H, J=3,9Hz); 7,75 (d, 2H, J=8,7Hz); 7,94 (d, 1 H, J=3,9Hz).

### 1-(5-(3-(trifluorométhyl)phényl)thièn-2-yl)éthanone

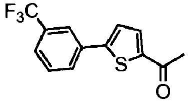

La 1-(5-(3-(trifluorométhyl)phényl)thièn-2-yl)éthanone est préparée à partir d'acide 2-acétyl-5-bromothiophène et d'acide 3-trifluorométhylbenzèneboronique selon la procédure générale A.

Après 4 heures d'agitation, les solvants sont éliminés par évaporation sous pression réduite et le résidu d'évaporation est purifié par chromatographie sur gel de silice.

Elution : cyclohexane/acétate d'éthyle : 8/2. Silice 40-63µm.

RMN ¹H (300MHz, CDCl₃, δ en ppm) : 2,59 (s, 3H); 7,39 (d, 1 H, J=3,9Hz); 7,53-7,65 (m, 2H); 7,69 (d, 1 H, J=3,9Hz); 7,83 (d, 1 H, J=7,6Hz); 7,90 (s, 1 H).

### Exemple 3 : Synthèse des composés intermédiaires intervenant dans la synthèse des composés selon l'invention :

### Composé intermédiaire 1 : 3-(2,3-dichloro-4-hydroxyphényl)-1-(5-(4-(trifluorométhyl)-phényl)thièn-2-yl)prop-2-èn-1-one

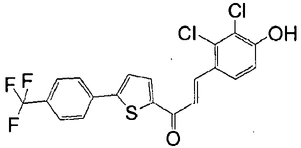

La 3-(2,3-dichloro-4-hydroxyphényl)-1-(5-(4-(trifluorométhyl)phényl)thièn-2-yl)prop-2-èn-1-one est préparée à partir de 1-(5-(4-(trifluorométhyl)phényl)thièn-2-yl)éthanone et de 2,3-dichloro-4-hydroxybenzaldéhyde selon la procédure générale B. Le résidu d'évaporation est cristallisé dans l'acétonitrile.

RMN ¹H (300MHz, CDCl₃, δ en ppm) : 6,02 (sl, 1H); 7,06 (d, 1 H, J=8,8Hz); 7,34 (d, 1 H, J=15,5Hz); 7,48 (d, 1H, J=3,8Hz); 7,67-7,72 (m, 3H); 7,81 (d, 2H, J=8,2Hz); 7,86 (d, 1 H, J=3,8Hz); 8,22 (d, 1 H, J=15,5Hz).

### Composé intermédiaire 2: 3-(2,3-dichloro-4-hydroxyphényl)-1-(5-(4-(trifluorométhyl)-phényl)thièn-2-yl)propan-1-one

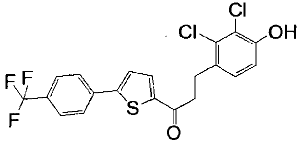

La 3-(2,3-dichloro-4-hydroxyphényl)-1-(5-(4-(trifluorométhyl)phényl)thièn-2-yl)propan-1-one est préparée à partir de la 3-(2,3-dichloro-4-hydroxyphényl)-1-(5-(4-(trifluorométhyl)phényl)thièn-2-yl)prop-2-èn-1-one selon la procédure générale C.

Après 1 heure 30 d'agitation à 42°C, le catalyseur est éliminé par filtration et le solvant est éliminé par évaporation sous pression réduite. Le résidu d'évaporation est purifié par chromatographie sur gel de silice.

Elution : cyclohexane/acétate d'éthyle : 7/3. Silice 40-63µm.

RMN ¹H (300MHz, CDCl₃, δ en ppm) : 3,14-3,27 (m, 4H); 5,64 (sl, 1H); 6,92 (d, 1 H, J=8,5Hz); 7,18 (d, 1 H, J=8,5Hz); 7,39 (d, 1 H, J=3,8Hz); 7,67-7,71 (m, 3H); 7,77 (d, 2H, J=8,2Hz).

### Composé intermédiaire 3: 3-(2,3-dichloro-4-hydroxyphényl)-1-(4-(4-(trifluorométhyl)-phényl)thièn-2-yl)prop-2-èn-1-one

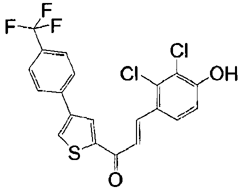

La 3-(2,3-dichloro-4-hydroxyphényl)-1-(4-(4-(trifluorométhyl)phényl)thièn-2-yl)prop-2-èn-1-one est préparée à partir de 1-(4-(4-(trifluorométhyl)phényl)thièn-2-yl)éthanone et de 2,3-dichloro-4-hydroxybenzaldéhyde selon la procédure générale B. Le résidu d'évaporation est purifié par chromatographie sur gel de silice.

Elution : cyclohexane/acétate d'éthyle : 8/2. Silice 40-63µm.

RMN ¹H (300MHz, DMSO-*d₆*, δ en ppm) : 7,08 (d, 1H, J=8,8Hz); 7,82-8,10 (m, 7H); 8,58 (s, 1 H); 8,86 (s, 1 H).

### Composé intermédiaire 4 : 3-(2,3-dichloro-4-hydroxyphényl)-1-(4-(4-(trifluorométhyl)-phényl)thièn-2-yl)propan-1-one

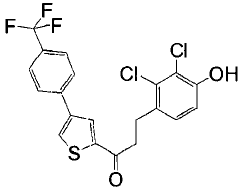

La 3-(2,3-dichloro-4-hydroxyphényl)-1-(4-(4-(trifluorométhyl)phényl)thièn-2-yl)propan-1-one est préparée à partir de la 3-(2,3-dichloro-4-hydroxyphényl)-1-(4-(4-(trifluorométhyl)phényl)thièn-2-yl)prop-2-èn-1-one selon la procédure générale C.

Après 6 heures à température ambiante, le catalyseur est éliminé par filtration et le solvant est éliminé par évaporation sous pression réduite. Le résidu d'évaporation est purifié par chromatographie sur gel de silice.

Elution : cyclohexane/acétate d'éthyle : 8/2. Silice 40-63µm.

RMN ¹H (300MHz, CDCl₃, δ en ppm) : 3,15-3,21 (m, 2H); 3,25-3,30 (m, 2H); 5,60 (s, 1 H); 6,91 (d, 1H, J=8,5Hz); 7,17 (d, 1H, J=8,5Hz); 7,67 (m, 4H); 7,81 (d, 1H, J=1,3 Hz); 7,95 (d, 1 H, J=1,3Hz).

### Composé intermédiaire 5 : 3-(3-chloro-4-hydroxyphényl)-1-(5-(4-(trifluorométhyl)-phényl)thièn-2-yl)prop-2-èn-1-one

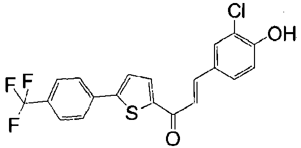

La 3-(3-chloro-4-hydroxyphényl)-1-(5-(4-(trifluorométhyl)phényl)thièn-2-yl)prop-2-èn-1-one est préparée à partir de 1-(5-(4-(trifluorométhyl)phényl)thièn-2-yl)éthanone et de 3-chloro-4-hydroxybenzaldéhyde selon la procédure générale B. Le résidu d'évaporation est purifié par chromatographie sur gel de silice.

Elution : cyclohexane/acétate d'éthyle : 6/4. Silice 40-63µm.

RMN ¹H (300MHz, DMSO-*d₆*, δ en ppm) : 7,03 (d, 1H, J=8,5Hz); 7,62-7,67 (m, 2H); 7,77-7,84 (m, 3H); 7,9 (d, 1 H, J=4,1 Hz); 8,03 (m, 3H); 8,39 (d, 1 H, J=4,1 Hz).

### Composé intermédiaire 6 : 3-(3-chloro-4-hydroxyphényl)-1-(5-(4-(trifluorométhyl)-phényl)thièn-2-yl)propan-1-one

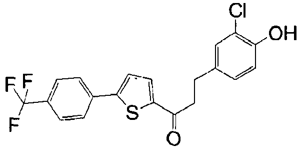

La 3-(3-chloro-4-hydroxyphényl)-1-(5-(4-(trifluorométhyl)phényl)thièn-2-yl)propan-1-one est préparée à partir de la 3-(3-chloro-4-hydroxyphényl)-1-(5-(4-(trifluorométhyl)phényl)thièn-2-yl)prop-2-èn-1-one selon la procédure générale C. Après 3 heures d'agitation à température ambiante, le catalyseur est éliminé par filtration et le solvant est éliminé par évaporation sous pression réduite. Le résidu d'évaporation est purifié par chromatographie sur gel de silice.

Elution : chlorure de méthylène. Silice 40-63µm.

RMN ¹H (300MHz, CDCl₃, δ en ppm) : 3,01 (t, 2H, J=7,7Hz); 3,21 (t, 2H, J=7,7Hz); 5,58 (s, 1 H); 6,95 (d, 1 H, J=8,2Hz); 7,07 (dd, 1 H, J=2,1Hz, J=8,2Hz); 7,22 (d, 1H, J=2,1Hz); 7,38 (d, 1H, J=4,2Hz); 7,63-7,68 (m, 3H); 7,75 (d, 2H, J=8,2Hz).

### Composé intermédiaire 7 : 3-(2-chloro-4-hydroxyphényl)-1-(5-(4-(trifluorométhyl)-phényl)thièn-2-yl)prop-2-èn-1-one

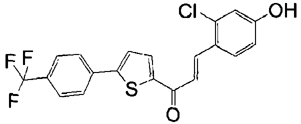

La 3-(2-chloro-4-hydroxyphényl)-1-(5-(4-(trifluorométhyl)phényl)thièn-2-yl)prop-2-èn-1-one est préparée à partir de 1-(5-(4-(trifluorométhyl)phényl)thièn-2-yl)éthanone et de 2-chloro-4-hydroxybenzaldéhyde selon la procédure générale B. Le résidu d'évaporation est purifié par chromatographie sur gel de silice.

Elution : cyclohexane/acétate d'éthyle : 6/4. Silice 40-63µm.

RMN ¹H (300MHz, DMSO-*d₆*, δ en ppm) : 6,86 (dd, 1 H, J=2,3Hz, J=8,5Hz); 6,94 (d, 1 H, J=2,3Hz); 7,78-7,84 (m, 3H); 7,90 (d, 1 H, J=4,1 Hz); 7,97-8,05 (m, 3H); 8,12 (d, 1 H, J=8,8Hz); 8,37 (d, 1 H, J=4,1 Hz).

### Composé intermédiaire 8 : 3-(2-chloro-4-hydroxyphényl)-1-(5-(4-(trifluorométhyl)-phényl)thièn-2-yl)propan-1-one

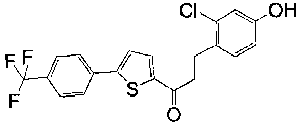

La 3-(2-chloro-4-hydroxyphényl)-1-(5-(4-(trifluorométhyl)phényl)thièn-2-yl)propan-1-one est préparée à partir de la 3-(2-chloro-4-hydroxyphényl)-1-(5-(4-(trifluorométhyl)phényl)thièn-2-yl)prop-2-èn-1-one selon la procédure générale C. Après 3 heures d'agitation à température ambiante, le catalyseur est éliminé par filtration et le solvant est éliminé par évaporation sous pression réduite. Le résidu d'évaporation est purifié par chromatographie sur gel de silice.

Elution : chlorure de méthylène. Silice 40-63µm.

RMN ¹H (300MHz, CDCl₃, δ en ppm) : 3,09-3,15 (m, 2H); 3,19-3,25 (m, 2H); 6,69 (dd, 1, J=2,5Hz, J=8,2Hz); 6,9 (d, 1 H, J=2,5Hz); 7,16 (d, 1 H); 7,38 (d, 1 H, J=3,8Hz); 7,66-7,71 (m, 3H); 7,76 (d, 1 H, J=8,5Hz).

### Composé intermédiaire 9 : 3-(3-fluoro-4-hydroxyphényl)-1-(5-(4-(trifluorométhyl)-phényl)thièn-2-yl)prop-2-èn-1-one

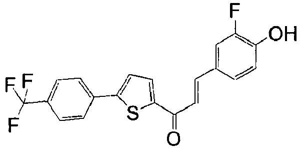

La 3-(3-fluoro-4-hydroxyphényl)-1-(5-(4-(trifluorométhyl)phényl)thièn-2-yl)prop-2-èn-1-one est préparée à partir de 1-(5-(4-(trifluorométhyl)phényl)thièn-2-yl)éthanone et de 3-fluoro-4-hydroxybenzaldéhyde selon la procédure générale B. Le résidu est cristallisé dans l'acétonitrile.

RMN ¹H (300MHz, CDCl₃, δ en ppm) : 5,51 (s, 1 H); 7,08 (d, 1 H, J=8,5Hz); 7,33-7,48 (m, 3H); 7,71 (m, 1 H); 7,77-7,82 (m, 3H); 7,86 (d, 2H, J=8,5Hz).

### Composé intermédiaire 10: 3-(3-fluoro-4-hydroxyphényl)-1-(5-(4-(trifluorométhyl)-phényl)thièn-2-yl)propan-1-one

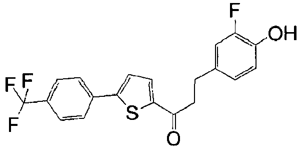

La 3-(3-fluoro-4-hydroxyphényl)-1-(5-(4-(trifluorométhyl)phényl)thièn-2-yl)propan-1-one est préparée à partir de la 3-(3-fluoro-4-hydroxyphényl)-1-(5-(4-(trifluorométhyl)phényl)thièn-2-yl)prop-2-èn-1-one selon la procédure générale C. Après 2 heures 30 à 42°C, le catalyseur est éliminé par filtration et le solvant est éliminé par évaporation sous pression réduite. Le résidu d'évaporation est purifé par chromatographie sur gel de silice.

Elution : cyclohexane/acétate d'éthyle : 7/3. Silice 40-63µm.

RMN ¹H (300MHz, CDCl₃, δ en ppm) : 3,02 (t, 2, J=7,5Hz); 3,21 (t, 2, J=7,5Hz); 5,01 (d, 1 H, J=3,8Hz); 6,97 (m, 3H); 7,39 (d, 1 H, J=3,9Hz); 7,68 (m, 3H); 7,75 (d, 2H, J=8,2Hz).

### Composé intermédiaire 11 : 3-(3-bromo-4-hydroxyphényl)-1-(5-(4-(trifluorométhyl)-phényl)thièn-2-yl)prop-2-èn-1-one

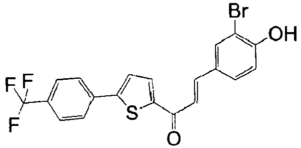

La 3-(3-bromo-4-hydroxyphényl)-1-(5-(4-(trifluorométhyl)phényl)thièn-2-yl)prop-2-èn-1-one est préparée à partir de 1-(5-(4-(trifluorométhyl)phényl)thièn-2-yl)éthanone et de 3-bromo-4-hydroxybenzaldéhyde selon la procédure générale B. Le résidu d'évaporation est cristallisé dans l'éthanol.

RMN ¹H (300MHz, DMSO-*d₆*, δ en ppm) : 7,01 (d, 1 H, J=8,2Hz); 7,65 (d, 1 H, J=15,5Hz); 7,71 (dd, 1H, J=1,9Hz, J=8,6Hz); 7,77-7,84 (m, 3H); 7,89 (d, 1H, J=4,1 Hz); 8,03 (d, 2H, J=8,2Hz); 8,19 (d, 1H, J=1,7Hz); 8,40 (d, 1H, J=4,1Hz).

### Composé intermédiaire 12: 3-(3-bromo-4-hydroxyphényl)-1-(5-(4-(trifluorométhyl)-phényl)thièn-2-yl)propan-1-one

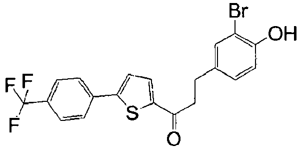

La 3-(3-bromo-4-hydroxyphényl)-1-(5-(4-(trifluorométhyl)phényl)thièn-2-yl)propan-1-one est préparée à partir de la 3-(3-bromo-4-hydroxyphényl)-1-(5-(4-(trifluorométhyl)-phényl)thièn-2-yl)prop-2-èn-1-one selon la procédure générale C.

Après 4 heures d'agitation à température ambiante, le catalyseur est éliminé par filtration, le solvant est éliminé par évaporation sous pression réduite, le résidu d'évaporation est purifié par chromatographie sur gel de silice.

Elution : cyclohexane/acétate d'éthyle : 85/15. Silice 40-63µm.

RMN ¹H (300MHz, CDCl₃, δ en ppm) : 2,99-3,05 (m, 2H); 3,18-3,23 (m, 2H); 6,96 (d, 1 H, J=8,2Hz); 7,10-7,14 (m, 1 H); 7,36-7,41 (m, 2H); 7,66-7,69 (m, 3H); 7,75 (d, 2H, J=8,2Hz).

### Composé intermédiaire 13: 3-(2,3-dichloro-4-hydroxyphényl)-1-(5-(4-(trifluoro-méthyl)phényl)fur-2-yl)prop-2-èn-1-one

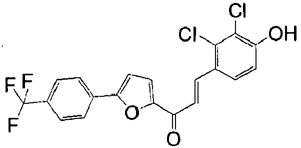

La 3-(2,3-dichloro-4-hydroxyphényl)-1-(5-(4-(trifluorométhyl)phényl)fur-2-yl)prop-2-èn-1-one est préparée à partir de 1-(5-(4-(trifluorométhyl)phényl)fur-2-yl)éthanone et de 2,3-dichloro-4-hydroxybenzaldéhyde selon la procédure générale B. Le résidu d'évaporation est lavé par du dichlorométhane.

RMN ¹H (300MHz, DMSO-*d₆*, δ en ppm) : 7,07 (d, 1 H, J=8,8Hz); 7,51 (d, 1 H, J=3,8Hz); 7,71 (d, 1H, J=15,5Hz); 7,88 (d, 2H, J=8,3Hz); 7,93 (d, 1H, J=3,8Hz); 8,03 (d, 1H, J=15,5Hz); 8,06 (d, 1H, J=8,8Hz); 8,14 (d, 2H, J=8,3Hz).

### Composé intermédiaire 14: 3-(2,3-dichloro-4-hydroxyphényl)-1-(5-(4-(trifluoro-méthyl)phényl)fur-2-yl)propan-1-one

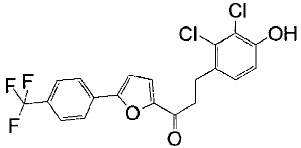

La 3-(2,3-dichloro-4-hydroxyphényl)-1-(5-(4-(trifluorométhyl)phényl)fur-2-yl)propan-1-one est préparée à partir de la 3-(2,3-dichloro-4-hydroxyphényl)-1-(5-(4-(trifluoro-méthyl)phényl)fur-2-yl)prop-2-èn-1-one selon la procédure générale C.

Après 24 heures d'agitation à 40 °C sous 5 bars de pression d'hydrogène, le catalyseur est éliminé par filtration et les solvants sont éliminés par évaporation sous pression réduite. Le résidu d'évaporation est purifé par chromatographie sur gel de silice.

Elution : cyclohexane/acétate d'éthyle : 8/2. Silice 40-63µm.

RMN ¹H (300MHz, CDCl₃, δ en ppm) : 3,19 (m, 4H); 6,88 (d, 1 H, J=4,1 Hz); 6,90 (d, 1 H, J=8,9Hz); 7,17 (d, 1 H, J=8,9Hz); 7,29 (d, 1 H, J=4,1 Hz); 7,69 (d, 2H, J=8,2Hz); 7,88 (d, 2H, J=8,2Hz).

### Composé intermédiaire 15: 3-(2,3-dichloro-4-hydroxyphényl)-1-(5-(4-(trifluoro-méthoxy)phényl)thièn-2-yl)prop-2-èn-1-one

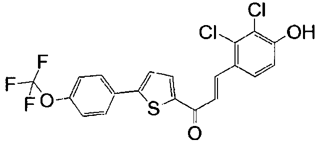

La 3-(2,3-dichloro-4-hydroxyphényl)-1-(5-(4-(trifluorométhoxy)phényl)thièn-2-yl)prop-2-èn-1-one est préparée à partir de 1-(5-(4-(trifluorométhoxy)phényl)thièn-2-yl)-éthanone et de 2,3-dichloro-4-hydroxybenzaldéhyde selon la procédure générale B. Le résidu d'évaporation est cristallisé dans de l'acétonitrile.

RMN ¹H (300MHz, DMSO-d₆, δ en ppm) : 7,07 (d, 1H, J=9,0Hz); 7,49 (d, 2H, J=8,4Hz); 7,80-7,86 (m, 3H); 7,93-7,98 (m, 2H); 8,09 (d, 1H, J=9,0Hz); 8,36 (d, 1 H, J=4,2Hz).

### Composé intermédiaire 16: 3-(2,3-dichloro-4-hydroxyphényl)-1-(5-(4-(trifluoro-méthoxy)phényl)thièn-2-yl)propan-1-one

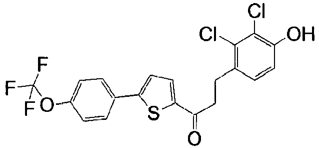

La 3-(2,3-dichloro-4-hydroxyphényl)-1-(5-(4-(trifluorométhoxy)phényl)thièn-2-yl)-propan-1-one est préparée à partir de la 3-(2,3-dichloro-4-hydroxyphényl)-1-(5-(4-(trifluorométhoxy)phényl)thièn-2-yl)prop-2-èn-1-one selon la procédure générale C.

Après 24 heures d'agitation à température ambiante sous 10 bars de pression d'hydrogène, le catalyseur est éliminé par filtration et les solvants sont éliminés par évaporation sous pression réduite. Le résidu d'évaporation est purifé par chromatographie sur gel de silice.

Elution : cyclohexane/acétate d'éthyle : 8/2. Silice 40-63µm.

RMN ¹H (300MHz, CDCl₃, δ en ppm) : 3,13-3,23 (m, 4H); 5,61 (s, 1 H); 6,90 (d, 1 H, J=8,6Hz); 7,16 (d, 1 H, J=8,6Hz); 7,25-7,29 (m, 3H); 7,64-7,69 (m, 3H).

### Composé intermédiaire 17 : 3-(2,3-difluoro-4-hydroxyphényl)-1-(5-(4-(trifluorométhyl)-phényl)thièn-2-yl)prop-2-èn-1-one

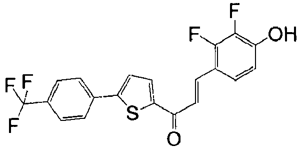

La 3-(2,3-difluoro-4-hydroxyphényl)-1-(5-(4-(trifluorométhyl)phényl)thièn-2-yl)prop-2-èn-1-one est préparée à partir de 1-(5-(4-(trifluorométhyl)phényl)thièn-2-yl)éthanone et de 2,3-difluoro-4-hydroxybenzaldéhyde selon la procédure générale B. Le résidu d'évaporation est cristallisé dans de l'acétonitrile.

RMN ¹H (300MHz, DMSO-*d₆*, δ en ppm) : 6,88 (t, 1 H, J=8,3Hz); 7,79 (m, 5H); 7,88 (d, 1 H, J=3,5Hz); 8,04 (d, 2H, J=7,9Hz); 8,31 (d, 1 H, J=3,5Hz).

### Composé intermédiaire 18: 3-(2,3-difluoro-4-hydroxyphényl)-1-(5-(4-(trifluorométhyl)-phényl)thièn-2-yl)propan-1-one

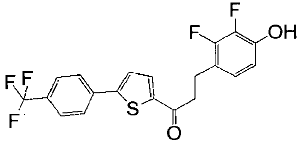

La 3-(2,3-difluoro-4-hydroxyphényl)-1-(5-(4-(trifluorométhyl)phényl)thièn-2-yl)propan-1-one est préparée à partir de la 3-(2,3-difluoro-4-hydroxyphényl)-1-(5-(4-(trifluoro-méthyl)phényl)thièn-2-yl)prop-2-èn-1-one selon la procédure générale C.

Après 1 heure d'agitation à 45°C, le catalyseur est éliminé par filtration et les solvants sont éliminés par évaporation sous pression réduite. Le résidu d'évaporation est purifié par chromatographie sur gel de silice.

Elution : cyclohexane/acétate d'éthyle : 7/3. Silice 40-63µm.

RMN ¹H (300MHz, DMSO-d₆, δ en ppm) : 2,89 (t, 2H, J=7,3Hz); 3,27 (t, 2H, J=7,3Hz); 6,69 (t, 1H, J=8,5Hz); 6,93 (t, 1H, J=8,5Hz); 7,80 (m, 3H); 8,01 (m, 3H).

### Composé intermédiaire 19: 3-(4-hydroxy-3,5-diméthylphényl)-1-(5-(4-(trifluoro-méthyl)phényl)thièn-2-yl)prop-2-èn-1-one

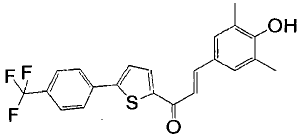

La 3-(4-hydroxy-3,5-diméthylphényl)-1-(5-(4-(trifluorométhyl)phényl)thièn-2-yl)prop-2-èn-1-one est préparée à partir de 1-(5-(4-(trifluorométhyl)phényl)thièn-2-yl)éthanone et de 4-hydroxy-3,5-diméthylbenzaldéhyde selon la procédure générale B. Le résidu d'évaporation est lavé par du dichlorométhane.

RMN ¹H (300MHz, CDCl₃, δ en ppm) : 2,22 (s, 6H); 7,52 (s, 2H); 7,62 (d, 1 H, J=15,5Hz); 7,71 (d, 1 H, J=15,5Hz); 7,83 (d, 2H, J=8,3Hz); 7,90 (d, 1 H, J=4,1 Hz); 8,03 (d, 2H, J=8,3Hz); 8,36 (d, 1 H, J=4,1 Hz); 9,01 (s, 1 H).

### Composé intermédiaire 20: 3-(4-hydroxy-3,5-diméthylphényl)-1-(5-(4-(trifluoro-méthyl)phényl)thièn-2-yl)propan-1-one

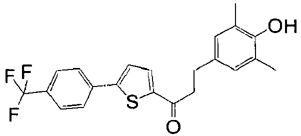

La 3-(4-hydroxy-3,5-diméthylphényl)-1-(5-(4-(trifluorométhyl)phényl)thièn-2-yl)-propan-1-one est préparée à partir de la 3-(4-hydroxy-3,5-diméthylphényl)-1-(5-(4-(trifluorométhyl)phényl)thièn-2-yl)prop-2-èn-1-one selon la procédure générale C.

Après 24 heures d'agitation à température ambiante sous 5 bars de pression d'hydrogène, le catalyseur est éliminé par filtration et les solvants sont éliminés par évaporation sous pression réduite. Le résidu d'évaporation est utilisé tel quel pour la réalisation de l'étape suivante.

RMN ¹H (300MHz, DMSO-*d₆*, δ en ppm) : 2,11 (s, 6H); 2,71-2,87 (m, 2H); 3,18-3,27 (m, 2H); 6,80 (s, 2H); 7,75-8,12 (m, 6H).

### Composé intermédiaire 21 : 1-(5-(4-bromophényl)thièn-2-yl)-3-(2,3-dichloro-4-hydroxyphényl)prop-2-èn-1-one

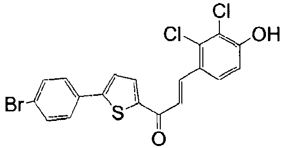

La 1-(5-(4-bromophényl)thièn-2-yl)-3-(2,3-dichloro-4-hydroxyphényl)prop-2-èn-1-one est préparée à partir de 1-(5-(4-bromophényl)thièn-2-yl)éthanone et de 2,3-dichloro-4-hydroxybenzaldéhyde selon la procédure générale B. Le résidu d'évaporation est lavé par du dichlorométhane.

RMN ¹H (300MHz, DMSO-*d₆*, δ en ppm) : 7,08 (d, 1H, J=8,7Hz); 7,68 (d, 2H, J=8,7Hz); 7,77-7,82 (m, 4H); 7,99 (d, 1H, J=15,5Hz); 8,09 (d, 1H, J=9,0Hz); 8,35 (d, 1H, J=4,2Hz); 11,42 (s, 1H).

### Composé intermédiaire 22: 1-(5-(4-bromophényl)thièn-2-yl)-3-(2,3-dichloro-4-hydroxyphényl)propan-1-one

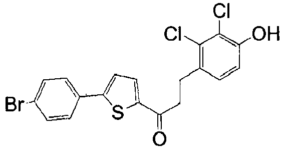

La 1-(5-(4-bromophényl)thièn-2-yl)-3-(2,3-dichloro-4-hydroxyphényl)prop-2-èn-1-one est solubilisée dans du dichlorométhane (0,7 g, 1,5 mol/L). Le triéthylsilane (2,1 éq.) et l'acide trifluoroacétique (8 éq.) sont successivement addtionnés goutte à goutte. Après 16 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice

Elution : cyclohexane/acétate d'éthyle : 9/1 à 8/2. Silice 40-63µm.

RMN ¹H (300MHz, CDCl₃, δ en ppm) : 3,15-3,20 (m, 4H); 6,90 (d, 1H, J=8,6Hz); 7,16 (d, 1 H, J=8,6Hz); 7,29 (d, 1 H, J=4,2Hz); 7,50 (d, 2H, J=9,0Hz); 7,55 (d, 2H, J=9,0Hz); 7,65 (d, 1 H, J=4,2Hz).

### Composé intermédiaire 23: 3-(2,3-dichloro-4-hydroxyphényl)-1-(5-(4-(méthylthio)-phényl)thièn-2-yl)prop-2-èn-1-one

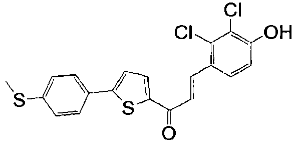

La 3-(2,3-dichloro-4-hydroxyphényl)-1-(5-(4-(méthylthio)phényl)thièn-2-yl)prop-2-èn-1-one est préparée à partir de 1-(5-(4-(méthylthio)phényl)thièn-2-yl)éthanone et de 2,3-dichloro-4-hydroxybenzaldéhyde selon la procédure générale B. Le résidu d'évaporation est utilisé tel quel pour la réalisation de l'étape suivante. RMN ¹H (300MHz, DMSO-*d₆*, δ en ppm) : 2,53 (s, 3H); 7,07 (d, 1H, J=8,8Hz); 7,35 (d, 2H, J=8,7Hz); 7,32-7,78 (m, 3H); 7,83 (d, 1 H, J=15,3Hz); 7,99 (d, 1H, J=15,3Hz); 8,09 (d, 1 H, J=8,8Hz); 8,33 (d, 1 H, J=3,9Hz).

### Composé intermédiaire 24: 3-(2,3-dichloro-4-hydroxyphényl)-1-(5-(4-(méthylthio)-phényl)thièn-2-yl)propan-1-one

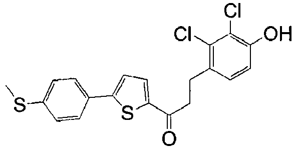

La 3-(2,3-dichloro-4-hydroxyphényl)-1-(5-(4-(méthylthio)phényl)thièn-2-yl)propan-1-one est préparée à partir de la 3-(2,3-dichloro-4-hydroxyphényl)-1-(5-(4-(méthylthio)phényl)thièn-2-yl)prop-2-èn-1-one selon la procédure générale C en solution dans du tétrahydrofurane.

Après 24 heures d'agitation à 50 °C sous 10 bars de pression d'hydrogène, le catalyseur est éliminé par filtration et le solvant est éliminé par évaporation sous pression réduite. Le résidu d'évaporation est purifié par chromatographie sur gel de silice.

Elution : cyclohexane/acétate d'éthyle : 8/2. Silice 40-63µm.

RMN ¹H (300MHz, CDCl₃, δ en ppm) : 2,52 (s, 3H); 3,11-3,24 (m, 4H); 5,61 (sl, 1 H); 6,91 (d, 1 H, J=8,6Hz); 7,17 (d, 1H, J=8,6Hz); 7,23-7,29 (m, 3H); 7,56 (d, 2H, J=8,5Hz); 7,64 (d, 1 H, J=4,1 Hz).

### Composé intermédiaire 25 : 3-(2,3-dichloro-4-hydroxyphényl)-1-(5-phénylthièn-2-yl)-propan-1-one

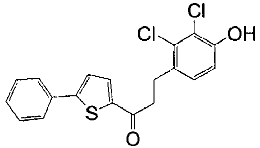

La 3-(2,3-dichloro-4-hydroxyphényl)-1-(5-phénylthièn-2-yl)propan-1-one est préparée à partir de la 1-(5-(4-bromophényl)thièn-2-yl)-3-(2,3-dichloro-4-hydroxyphényl)prop-2-èn-1-one selon la procédure générale C.

Après 24 heures d'agitation à 40 °C sous 10 bars de pression d'hydrogène, le catalyseur est éliminé par filtration et le solvant est éliminé par évaporation sous pression réduite. Le résidu d'évaporation est purifié par chromatographie sur gel de silice.

Elution : cyclohexane/acétate d'éthyle : 9/1 à 8/2. Silice 40-63µm.

RMN ¹H (300MHz, CDCl₃, δ en ppm): 3,16-3,21 (m, 4H); 6,91 (d, 1 H, J=8,6Hz); 7,17 (d, 1H, J=8,6Hz); 7,31 (d, 1 H, J=3,9Hz); 7,39-7,42 (m, 3H); 7,64-7,67 (m, 3H).

### Composé intermédiaire 26: 3-(4-hydroxy-3-méthylphényl)-1-(5-(4-(trifluorométhyl)-phényl)thièn-2-yl)prop-2-èn-1-one

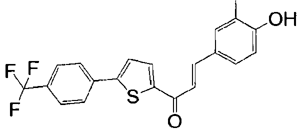

La 3-(4-hydroxy-3-méthylphényl)-1-(5-(4-(trifluorométhyl)phényl)thièn-2-yl)prop-2-èn-1-one est préparée à partir de 1-(5-(4-(trifluorométhyl)phényl)thièn-2-yl)éthanone et de 4-hydroxy-3-méthylbenzaldéhyde selon la procédure générale B. Le résidu d'évaporation est lavé par du dichlorométhane.

RMN ¹H (300MHz, DMSO-*d₆*, δ en ppm) : 2,18 (s, 3H); 6,86 (d, 1 H, J=8,3Hz); 7,55 (dd, 1 H, J=2,4Hz, J=8,3Hz); 7,65 (d, 1 H, J=15,6Hz); 7,71 (d, 1H, J=15,6Hz); 7,72 (d, 1 H, J=2,4Hz); 7,84 (d, 2H, J=8,2Hz); 7,91 (d, 1 H, J=4,1 Hz); 8,04 (d, 2H, J=8,2Hz); 8,35 (d, 1H, J=4,1Hz); 10,10 (sl, 1H).

### Composé intermédiaire 27: 3-(4-hydroxy-3-méthylphényl)-1-(5-(4-(trifluorométhyl)-phényl)thièn-2-yl)propan-1-one

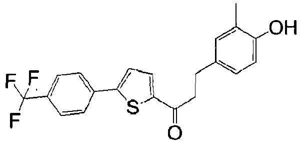

La 3-(4-hydroxy-3-méthylphényl)-1-(5-(4-(trifluorométhyl)phényl)thièn-2-yl)propan-1-one est préparée à partir de la 3-(4-hydroxy-3-méthylphényl)-1-(5-(4-(trifluorométhyl)-phényl)thièn-2-yl)prop-2-èn-1-one selon la procédure générale C.

Après 40 heures d'agitation à 40°C sous 5 bars de pression d'hydrogène, le catalyseur est éliminé par filtration et les solvants sont éliminés par évaporation sous pression réduite. Le résidu d'évaporation est purifié par chromatographie sur gel de silice.

Elution : cyclohexane/acétate d'éthyle : 9/1 à 8/2. Silice 40-63µm.

RMN ¹H (300MHz, CDCl₃, δ en ppm) : 2,24 (s, 3H); 2,96-3,01 (m, 2H); 3,17-3,22 (m, 2H); 4,68 (s, 1 H); 6,71 (d, 1 H, J=8,1 Hz); 6,96 (dd, 1 H, J=2,3Hz, J=8,1 Hz); 7,01 (d, 1H, J=2,3Hz); 7,38 (d, 1H, J=3,9Hz); 7,66-7,68 (m, 3H); 7,75 (d, 2H, J=8,4Hz).

### Composé intermédiaire 28 : 3-(4-hydroxyphényl)-1-(5-(4-(trifluorométhyl)phényl)-thièn-2-yl)prop-2-èn-1-one

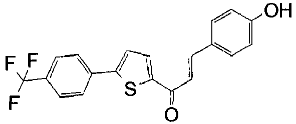

La 3-(4-hydroxyphényl)-1-(5-(4-(trifluorométhyl)phényl)thièn-2-yl)prop-2-èn-1-one est préparée à partir de 1-(5-(4-(trifluorométhyl)phényl)thièn-2-yl)éthanone et de 4-hydroxybenzaldéhyde selon la procédure générale B. Le résidu d'évaporation est purifé par chromatographie sur gel de silice.

Elution : cyclohexane/acétate d'éthyle : 6/4. Silice 40-63µm.

RMN ¹H (300MHz, CDCl₃, δ en ppm) : 6,91 (d, 1 H, J=8,4Hz); 7,32 (d, 1 H, J=15,4Hz); 7,46 (d, 1H, J=4,1 Hz); 7,59 (d, 2H, J=8,4Hz); 7,79-7,88 (m, 4H).

### Composé intermédiaire 29 : 3-(4-hydroxyphényl)-1-(5-(4-(trifluorométhyl)phényl)-thièn-2-yl)propan-1-one

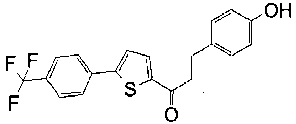

La 3-(4-hydroxyphényl)-1-(5-(4-(trifluorométhyl)phényl)thièn-2-yl)propan-1-one est préparée à partir de la 3-(4-hydroxyphényl)-1-(5-(4-(trifluorométhyl)phényl)thièn-2-yl)prop-2-èn-1-one selon la procédure générale C. Après 1,5 heures d'agitation à température ambiante, le catalyseur est éliminé par filtration et les solvants sont éliminés par évaporation sous pression réduite. Le résidu d'évaporation est purifé par chromatographie sur gel de silice.

Elution : cyclohexane/acétate d'éthyle : 8/2. Silice 40-63µm.

RMN ¹H (300MHz, CDCl₃, δ en ppm) : 3,01-3,06 (m, 2H); 3,19-3,24 (m, 2H); 4,72 (sl, 1 H); 6,78 (d, 2H, J=8,5Hz); 7,13 (d, 2H, J=8,5Hz); 7,38 (d, 1H, J=4,1 Hz); 7,67-7,68 (m, 3H); 7,76 (d, 2H, J=8,2Hz).

### Composé intermédiaire 30: 3-(2,3-dichloro-4-hydroxyphényl)-1-(5-(3-(trifluoro-méthyl)phényl)thièn-2-yl)prop-2-èn-1-one

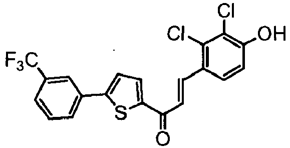

La 3-(2,3-dichloro-4-hydroxyphényl)-1-(5-(3-(trifluorométhyl)phényl)thièn-2-yl)prop-2-èn-1-one est préparée à partir de 1-(5-(3-(trifluorométhyl)phényl)thièn-2-yl)éthanone et de 2,3-dichloro-4-hydroxybenzaldéhyde selon la procédure générale B, à 50°C pendant 30 heures. Le résidu d'évaporation est purifé par chromatographie sur gel de silice.

Elution : cyclohexane/acétate d'éthyle : 7/3. Silice 40-63µm.

RMN ¹H (300MHz, CDCl₃, δ en ppm) : 5,95 (s, 1H); 7,06 (d, 1H, J=8,8Hz); 7,33 (d, 1 H, J=15,5Hz); 7,46 (d, 1H, J=4,1 Hz); 7,55-7,70 (m, 3H); 7,85 (d, 1H, J=4,1Hz); 7,88-7,93 (m, 2H); 8,21 (d, 1H, J=15,5Hz).

### Composé intermédiaire 31 : 3-(2,3-dichloro-4-hydroxyphényl)-1-(5-(3-(trifluoro-méthyl)phényl)thièn-2-yl)propan-1-one

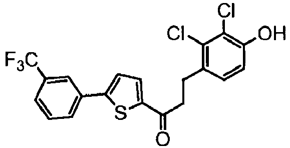

La 3-(2,3-dichloro-4-hydroxyphényl)-1-(5-(3-(trifluorométhyl)phényl)thièn-2-yl)propan-1-one est préparée à partir de la 3-(2,3-dichloro-4-hydroxyphényl)-1-(5-(3-(trifluoro-méthyl)phényl)thièn-2-yl)prop-2-èn-1-one selon la procédure générale C.

Après 3 heures d'agitation à 40°C, le catalyseur est éliminé par filtration et les solvants sont éliminés par évaporation sous pression réduite. Le résidu d'évaporation est purifié par chromatographie sur gel de silice.

Elution : cyclohexane/acétate d'éthyle : 8/2. Silice 40-63µm.

RMN ¹H (300MHz, CDCl₃, δ en ppm) : 3,15-3,25 (m, 4H); 5,57 (s, 1H); 6,92 (d, 1 H, J=8,4Hz); 7,17 (d, 1 H, J=8,4Hz); 7,38 (d, 1 H, J=4,1 Hz); 7,56 (m, 1 H); 7,63 (d, 1 H, J=7,9Hz); 7,69 (d, 1 H, J=4,1 Hz); 7,82 (d, 1 H, J=7,9Hz); 7,89 (s, 1 H).

### Exemple 4 : Synthèse des composés selon l'invention :

### Composé 1 : 2-(2,3-dichloro-4-(3-oxo-3-(5-(4-(trifluorométhyl)phényl)thièn-2-yl)-propyl)phénoxy)-2-méthylpropanoate de tertiobutyle

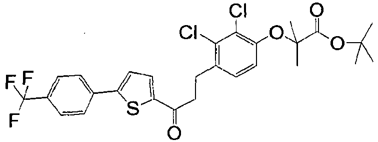

Le 2-(2,3-dichloro-4-(3-oxo-3-(5-(4-(trifluorométhyl)phényl)thièn-2-yl)propyl)phénoxy)-2-méthylpropanoate de tertiobutyle est préparé à partir de la 3-(2,3-dichloro-4-hydroxyphényl)-1-(5-(4-(trifluorométhyl)phényl)thièn-2-yl)propan-1-one et du bromoisobutyrate de tertiobutyle selon la procédure générale D.

Après 16 heures d'agitation, le solvant est éliminé par évaporation sous pression réduite et le résidu d'évaporation est purifié par chromatographie sur gel de silice.

Elution : cyclohexane/acétate d'éthyle : 95/5. Silice 40-63µm.

RMN ¹H (300MHz, CDCl₃, δ en ppm) : 1,46 (s, 9H); 1,61 (s, 6H); 3,16-3,26 (m, 4H); 6,81 (d, 1 H, J=8,5Hz); 7,11 (d, 1 H, J=8,5Hz); 7,39 (d, 1 H, J=3,8Hz); 7,66-7,69 (m, 3H); 7,77 (d, 2H, J=8,2Hz).

### Composé 2 : Acide 2-(2,3-dichloro-4-(3-oxo-3-(5-(4-(trifluorométhyl)phényl)thièn-2-yl)propyl)phénoxy)-2-méthylpropanoïque

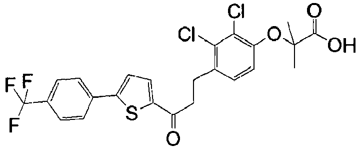

L'acide 2-(2,3-dichloro-4-(3-oxo-3-(5-(4-(trifluorométhyl)phényl)thièn-2-yl)propyl)-phénoxy)-2-méthylpropanoïque est préparé à partir du 2-(2,3-dichloro-4-(3-oxo-3-(5-(4-(trifluorométhyl)phényl)thièn-2-yl)propyl)phénoxy)-2-méthylpropanoate de tertio-butyle selon la procédure générale E au moyen de 10 équivalents d'acide trifluoroacétique.

Après 16 heures à température ambiante, le milieu réactionnel est lavé avec de l'eau puis le dichlorométhane est éliminé par évaporation sous pression réduite. Le résidu d'évaporation est recristallisé dans du chlorure de méthylène.

RMN ¹H (300MHz, CDCl₃, δ en ppm) : 1,64 (s, 6H); 3,19-3,28 (m, 4H); 6,96 (d, 1 H, J=8,5Hz); 7,21 (d, 1 H, J=8,5Hz); 7,39 (d, 1H, J=4,1 Hz); 7,67-7,69 (m, 3H); 7,76 (d, 2H, J=8,5Hz).

Masse (ES⁺) : 531 / 533 (M+1).

F = 153-154°C.

### Composé 3 : 2-(2,3-dichloro-4-(3-hydroxy-3-(5-(4-(trifluorométhyl)phényl)thièn-2-yl)-propyl)phénoxy)-2-méthylpropanoate de tertiobutyle

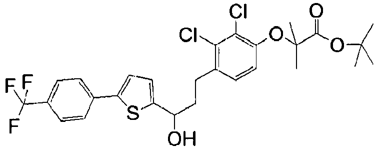

Le 2-(2,3-dichloro-4-(3-hydroxy-3-(5-(4-(trifluorométhyl)phényl)thièn-2-yl)propyl)-phénoxy)-2-méthylpropanoate de tertiobutyle est préparé à partir du 2-(2,3-dichloro-4-(3-oxo-3-(5-(4-(trifluorométhyl)phényl)thièn-2-yl)propyl)phénoxy)-2-méthylpropanoate de tertiobutyle selon la procédure générale G.

RMN ¹H (300MHz, CDCl₃, δ en ppm) : 1,46 (s, 9H); 1,60 (s, 6H); 2,15-2,22 (m, 2H); 2,79-3,01 (m, 2H); 4,95 (t, 1 H, J=6,6Hz); 6,83 (d, 1 H, J=8,5Hz); 7,01 (d, 1 H, J=3,8Hz); 7,05 (d, 1 H, J=8,5Hz); 7,28 (m, 1 H); 7,63 (d, 2H, J=8,5Hz); 7,69 (d, 2H, J=8,5Hz).

### Composé 4: Acide 2-(2,3-dichloro-4-(3-hydroxy-3-(5-(4-(trifluorométhyl)phényl)thièn-2-yl)propyl)phénoxy)-2-méthylpropanoïque

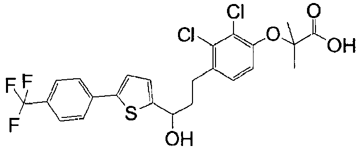

L'acide 2-(2,3-dichloro-4-(3-hydroxy-3-(5-(4-(trifluorométhyl)phényl)thièn-2-yl)propyl)-phénoxy)-2-méthylpropanoïque est préparé à partir de l'acide 2-(2,3-dichloro-4-(3-oxo-3-(5-(4-(trifluorométhyl)phényl)thièn-2-yl)propyl)phénoxy)-2-méthylpropanoïque selon la procédure générale G.

Le résidu d'évaporation est purifé par chromatographie sur gel de silice.

Elution : dichlorométhane/méthanol : 95/5. Silice 40-63µm.

RMN ¹H (300MHz, CDCl₃, δ en ppm) : 1,65 (s, 6H); 2,17-2,24 (m, 2H); 2,82-3,04 (m, 2H); 4,98 (t, 1H, J=6,6Hz); 6,97 (d, 1H, J=8,5Hz); 7,02 (d, 1H, J=3,8Hz); 7,14 (d, 1 H, J=8,5Hz); 7,28 (m, 1 H); 7,64 (d, 2H, J=8,3Hz); 7,69 (d, 2H, J=8,3Hz). Masse (ES⁺) : 515/517 (M-OH); 550 (M+NH₄)⁺; 555/557 (M+Na)⁺.

F = 54-56°C.

### Composé 5 : 2-(4-(3-(4-iodobenzyloxy)-3-(5-(4-(trifluorométhyl)phényl)thièn-2-yl)-propyl)-2,3-dichlorophénoxy)-2-méthylpropanoate de tertiobutyle

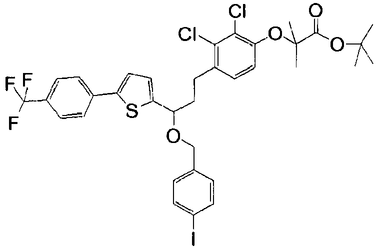

Le 2-(4-(3-(4-iodobenzyloxy)-3-(5-(4-(trifluorométhyl)phényl)thièn-2-yl)propyl)-2,3-dichlorophénoxy)-2-méthylpropanoate de tertiobutyle est préparé à partir du 2-(2,3-dichloro-4-(3-hydroxy-3-(5-(4-(trifluorométhyl)phényl)thièn-2-yl)propyl)phénoxy)-2-méthylpropanoate de tertiobutyle au moyen de 1,1 équivalents d'hydrure de sodium et de 1,1 équivalents de bromure de 4-iodobenzyle selon la procédure générale H.

Le résidu d'évaporation est purifié par chromatographie sur gel de silice.

Elution : cyclohexane/acétate d'éthyle : 95/5. Silice 40-63µm.

RMN ¹H (300MHz, CDCl₃, δ en ppm) : 1,46 (s, 9H); 1,58 (s, 6H); 2,05-2,31 (m, 2H); 2,71-2,97 (m, 2H); 4,33 (d, 1 H, J=11,7Hz); 4,53-4,59 (m, 2H); 6,79 (d, 1 H, J=8,6Hz); 6,95 (d, 1H, J=8,6Hz); 6,99 (d, 1H, J=3,5Hz); 7,11 (d, 2H, J=7,9Hz); 7,28 (m, 1 H); 7,64 (d, 2H, J=8,5Hz); 7,70 (m, 4H).

### Composé 6: Acide 2-(4-(3-(4-iodobenzyloxy)-3-(5-(4-(trifluorométhyl)phényl)thièn-2-yl)propyl)-2,3-dichlorophénoxy)-2-méthylpropanoïque

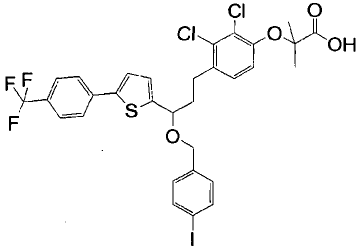

L'acide 2-(4-(3-(4-iodobenzyloxy)-3-(5-(4-(trifluorométhyl)phényl)thièn-2-yl)propyl)-2,3-dichlorophénoxy)-2-méthylpropanoïque est préparé à partir du 2-(4-(3-(4-iodobenzyloxy)-3-(5-(4-(trifluorométhyl)phényl)thièn-2-yl)propyl)-2,3-dichlorophénoxy)-2-méthylpropanoate de tertiobutyle selon la procédure générale E au moyen de 10 équivalents d'acide trifluoroacétique.

Après 16 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice.

Elution : chlorure de méthylène/méthanol : 9/1. Silice 40-63µm.

RMN ¹H (300MHz, CDCl₃, δ en ppm) : 1,61 (s, 6H); 2,02-2,25 (m, 2H); 2,70-2,95 (m, 2H); 4,32 (d, 1H, J=11,9Hz); 4,52-4,58 (m, 2H); 6,91 (d, 1H, J=8,6Hz); 6,87 (d, 1 H, J=8,6Hz); 6,98 (d, 1H, J=3,5Hz); 7,09 (d, 2H, J=8,2Hz); 7,27 (m, 1H); 7,61-7,72 (m, 6H).

Masse (ES⁻) : 747/748/749 (M-1).

Aspect : huile visqueuse

### Composé 7: Acide 2-(4-(3-(benzyloxy)-3-(5-(4-(trifluorométhyl)phényl)thièn-2-yl)propyl)-2,3-dichlorophénoxy)-2-méthylpropanoïque

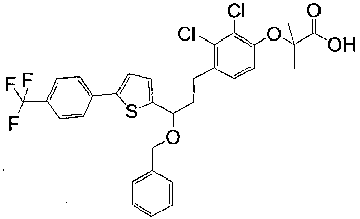

L'acide 2-(4-(3-(benzyloxy)-3-(5-(4-(trifluorométhyl)phényl)thièn-2-yl)propyl)-2,3-di-chlorophénoxy)-2-méthylpropanoïque est préparé à partir de l'acide 2-(2,3-dichloro-4-(3-hydroxy-3-(5-(4-(trifluorométhyl)phényl)thièn-2-yl)propyl)phénoxy)-2-méthyl-propanoïque au moyen de 2,2 équivalents d'hydrure de sodium et de 2,2 équivalents de bromure de benzyle selon la procédure générale H.

Le résidu d'évaporation est solubilisé dans de l'éthanol en présence de soude 2N (20 éq.). Après 16 heures sous agitation, les solvants sont éliminés par évaporation sous pression réduite. Le résidu d'évaporation est acidifié à l'aide d'une solution diluée d'acide chlorhydrique puis extrait par du dichlorométhane. La phase organique est séchée sur sulfate de magnésium, filtrée puis concentrée sous pression réduite. Le résidu d'évaporation est purifié par chromatographie sur gel de silice (HPLC préparative, lichrospher (Merck) RP18 12µm 100A, colonne : 25*250 mm).

Elution : gradient eau, méthanol + 0,1% acide trifluoroacétique : 22/78 à 0/100. RMN ¹H (300MHz, CDCl₃, δ en ppm) : 1,64 (s, 6H); 2,31-2,08 (m, 2H); 3,01-2,74 (m, 2H); 4,39 (d, 1H, J=11,7Hz); 4,57-4,66 (m, 2H); 6,91 (d, 1H, J=8,5Hz); 6,99 (d, 1 H, J=8,5Hz); 7,01 (d, 1 H, J=3,5Hz); 7,27-7,38 (m, 6H); 7,63 (d, 2H, J=8,5Hz); 7,70 (d, 2H, J=8,5Hz).

Masse (ES⁺) : 640 / 642 (M+NH₄)⁺, 645/647 (M+Na)⁺.

F = 46-48°C.

### Composé 7a et 7b :

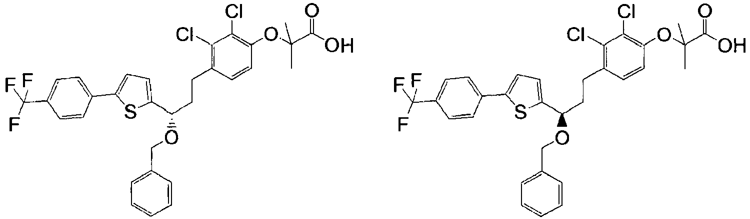

Les deux énantiomères du composé 7 sont séparés par HPLC semi-préparative sur colonne chirale Chiralpak®AD-H (250*20mm, 5µm, Chiral Technologies Europe) à température ambiante. L'élution est réalisée en mode isocratique avec une phase mobile n-heptane-éthanol (95-5) additionnée de 0,1% d'acide trifluoroacétique au débit de 18 ml/min.

La pureté énantiomérique de chacun des deux énantiomères ainsi obtenus est contrôlée par HPLC analytique : colonne Chiralpak®AD-H (250*46mm, 5µm, Daicel Chemical Industries, LTD) à 30°C ; élution isocratique avec phase mobile n-heptane-éthanol (92-8) additionnée de 0.1% d'acide trifluoroacétique ; débit 1 ml/min ; détection UV à 298 nm.

Composé 7a : tR = 11,85 min, ee = 100 %

Composé 7b : tR = 16,28 min, ee = 99,6%

### Composé 8: 2-(2,3-dichloro-4-(3-oxo-3-(5-(4-(trifluorométhyl)phényl)thièn-2-yl)-propyl)phénoxy)butanoate de tertiobutyle

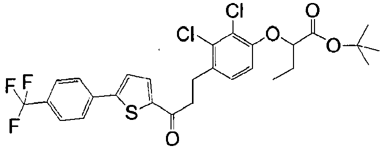

Le 2-(2,3-dichloro-4-(3-oxo-3-(5-(4-(trifluorométhyl)phényl)thièn-2-yl)propyl)phénoxy)-butanoate de tertiobutyle est préparé à partir de la 3-(2,3-dichloro-4-hydroxyphényl)-1-(5-(4-(trifluorométhyl)phényl)thièn-2-yl)propan-1-one et du 2-bromobutanoate de tertiobutyle selon la procédure générale D.

Après 2 heures d'agitation, le milieu est acidifié à l'aide d'une solution diluée d'acide chlorhydrique puis extrait par de l'acétate d'éthyle. La phase organique est séchée sur sulfate de magnésium, filtrée puis concentrée sous pression réduite. Le résidu d'évaporation est purifié par chromatographie sur gel de silice. Elution : cyclohexane/acétate d'éthyle : 9/1. Silice 40-63µm.

RMN ¹H (300MHz, CDCl₃, δ en ppm) : 1,11 (t, 3H, J=7,6Hz); 1,43 (s, 9H); 1,97-2,07 (m, 2H); 3,14-3,26 (m, 4H); 4,45 (t, 1H, J=5,8Hz); 6,66 (d, 1H, J=8,8Hz); 7,13 (d, 1H, J=8,8Hz); 7,38 (d, 1H, J=4,1 Hz); 7,66-7,69 (m, 3H); 7,75 (d, 2H, J=8,2Hz).

### Composé 9 : Acide 2-(2,3-dichloro-4-(3-oxo-3-(5-(4-(trifluorométhyl)phényl)thièn-2-yl)propyl)phénoxy)butanoïque

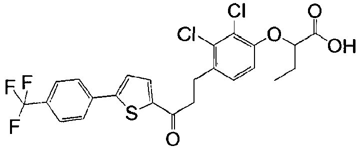

L'acide 2-(2,3-dichloro-4-(3-oxo-3-(5-(4-(trifluorométhyl)phényl)thièn-2-yl)propyl)-phénoxy)butanoïque est préparé à partir du 2-(2,3-dichloro-4-(3-oxo-3-(5-(4-(trifluorométhyl)phényl)thièn-2-yl)propyl)phénoxy)butanoate de tertiobutyle selon la procédure générale E au moyen de 17 équivalents d'acide trifluoroacétique. Après 4 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice (HPLC préparative, lichrospher (Merck) RP18 12µm 100A, colonne : 25*250 mm).

Elution : gradient eau, méthanol + 0,1% acide trifluoroacétique : 30/70 à 10/90.

RMN ¹H (300MHz, DMSO-*d₆*, δ en ppm) : 1,01 (t, 3H, J=7,3Hz); 1,84-1,98 (m, 2H); 3,03 (t, 2H, J=7,5Hz); 3,31 (t, 2H, J=7,5Hz); 4,82 (t, 1 H, J=6,4Hz); 6,92 (d, 1 H, J=8,8Hz); 7,32 (d, 1H, J=8,8Hz); 7,79-7,83 (m, 3H); 7,99 (d, 2H, J=8,2Hz); 8,05 (d, 1 H, J=4,2Hz); 13,15 (s, 1 H).

Masse (ES⁺) : 531 (M+1).

F = 169-171 °C.

### Composé 10: 2-(2,3-dichloro-4-(3-oxo-3-(4-(4-(trifluorométhyl)phényl)thièn-2-yl)-propyl)phénoxy)-2-méthylpropanoate de tertiobutyle

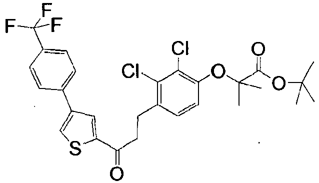

Le 2-(2,3-dichloro-4-(3-oxo-3-(4-(4-(trifluorométhyl)phényl)thièn-2-yl)propyl)phénoxy)-2-méthylpropanoate de tertiobutyle est préparé à partir de la 3-(2,3-dichloro-4-hydroxyphényl)-1-(4-(4-(trifluorométhyl)phényl)thièn-2-yl)propan-1-one et du bromoisobutyrate de tertiobutyle selon la procédure générale D.

Après 18 heures d'agitation, le milieu est acidifié à l'aide d'une solution diluée d'acide chlorhydrique puis extrait par de l'acétate d'éthyle. La phase organique est séchée sur sulfate de magnésium, filtrée puis concentrée sous pression réduite. Le résidu d'évaporation est purifié par chromatographie sur gel de silice. Elution : cyclohexane/acétate d'éthyle : 9/1. Silice 40-63µm.

RMN ¹H (300MHz, CDCl₃, δ en ppm): 1,45 (s, 9H); 1,57 (s, 6H); 3,16-3,21 (m, 2H); 3,25-3,30 (m, 2H); 6,79 (d, 1 H, J=8,5Hz); 7,09 (d, 1 H, J=8,5Hz); 7,68 (m, 4H); 7,8 (d, 1H, J=1,5); 7,95 (d, 1H, J=1,5Hz).

### Composé 11 : Acide 2-(2,3-dichloro-4-(3-oxo-3-(4-(4-(trifluorométhyl)phényl)thièn-2-yl)propyl)phénoxy)-2-méthylpropanoïque

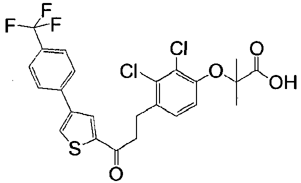

L'acide 2-(2,3-dichloro-4-(3-oxo-3-(4-(4-(trifluorométhyl)phényl)thièn-2-yl)propyl)-phénoxy)-2-méthylpropanoïque est préparé à partir du 2-(2,3-dichloro-4-(3-oxo-3-(4-(4-(trifluorométhyl)phényl)thièn-2-yl)propyl)phénoxy)-2-méthylpropanoate de tertiobutyle selon la procédure générale E au moyen de 17 équivalents d'acide trifluoroacétique.

Après 4 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice. Elution : chlorure de méthylène/méthanol : 9/1. Silice 40-63µm.

RMN ¹H (300MHz, DMSO-*d₆*, δ en ppm) : 1,52 (s, 6H); 3,03 (t, 2H, J=7,9Hz); 3,38 (t, 2H, J=7,9Hz); 6,88 (d, 1 H, J=8,6Hz); 7,32 (d, 1 H, J=8,6Hz); 7,78 (d, 2H, J=8,3Hz); 8,03 (d, 2H, J=8,3Hz); 8,51 (s, 1 H); 8,57 (s, 1 H).

Masse (ES⁻) : 529 (M-1).

F=99-101°C.

### Composé 12 : 5-(2,3-dichloro-4-(3-oxo-3-(5-(4-(trifluorométhyl)phényl)thièn-2-yl)-propyl)phénoxy)-2,2-diméthylpentanoate de méthyle

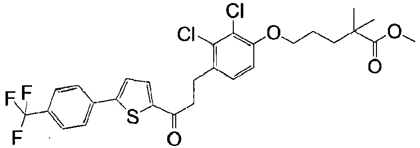

Le 5-(2,3-dichloro-4-(3-oxo-3-(5-(4-(trifluorométhyl)phényl)thièn-2-yl)propyl)phénoxy)-2,2-diméthylpentanoate de méthyle est préparé à partir de la 3-(2,3-dichloro-4-hydroxyphényl)-1-(5-(4-(trifluorométhyl)phényl)thièn-2-yl)propan-1-one et du 5-iodo-2,2-diméthylpentanoate de méthyle selon la procédure générale D.

Après 2 heures d'agitation, le milieu est acidifié à l'aide d'une solution diluée d'acide chlorhydrique puis extrait par de l'acétate d'éthyle. La phase organique est séchée sur sulfate de magnésium, filtrée puis concentrée sous pression réduite. Le résidu d'évaporation est purifié par chromatographie sur gel de silice. Elution : cyclohexane/acétate d'éthyle : 8/2. Silice 40-63µm.

RMN ¹H (300MHz, CDCl₃, δ en ppm) : 1,22 (s, 6H); 1,72-1,81 (m, 6H); 3,16-3,25 (m, 4H); 3,67 (s, 3H); 6,76 (d, 1 H, J=8,5Hz); 7,18 (d, 1 H, J=8,5Hz); 7,39 (d, 1 H, J=4,1 Hz); 7,66-7,69 (m, 3H); 7,75 (d, 2H, J=8,5Hz).

### Composé 13: Acide 5-(2,3-dichloro-4-(3-oxo-3-(5-(4-(trifluorométhyl)phényl)thièn-2-yl)propyl)phénoxy)-2,2-diméthylpentanoïque

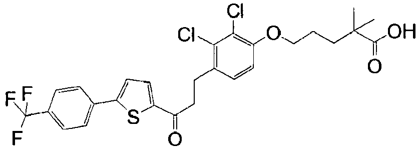

L'acide 5-(2,3-dichloro-4-(3-oxo-3-(5-(4-(trifluorométhyl)phényl)thièn-2-yl)propyl)-phénoxy)-2,2-diméthylpentanoïque est préparé à partir du 5-(2,3-dichloro-4-(3-oxo-3-(5-(4-(trifluorométhyl)phényl)thièn-2-yl)propyl)phénoxy)-2,2-diméthylpentanoate de méthyle selon la procédure générale F au moyen de 10 équivalents d'une solution de soude 2N.

Après 18 heures d'agitation à 50°C, les solvants sont éliminés par évaporation sous pression réduite. Le résidu d'évaporation est repris par une solution diluée d'acide chlorhydrique puis extrait par du chlorure de méthylène. La phase organique est séchée sur sulfate de magnésium, filtrées puis concentrées sous pression réduite. Le résidu d'évaporation est purifié par chromatographie sur gel de silice.

Elution : chlorure de méthylène/méthanol : 95/5. Silice 40-63µm.

RMN ¹H (300MHz, DMSO-*d₆*, δ en ppm) : 1,10 (s, 6H); 1,58-1,70 (m, 4H); 3,03 (t, 2H, J=7,6Hz); 3,31 (t, 2H, J=5,9Hz); 4,03 (t, 2H, J=5,6Hz); 7,08 (d, 1 H, J=8,8Hz); 7,33 (d, 1 H, J=8,8Hz); 7,79-7,82 (m, 3H); 7,99 (d, 2H, J=8,2Hz); 8,04 (d, 1 H, J=4,1 Hz); 12,12 (s, 1 H).

Masse (ES⁻) : 571 (M-1).

F=167-169°C.

### Composé 14: 2-(2,3-dichloro-4-(3-oxo-3-(5-(4-(trifluorométhyl)phényl)thièn-2-yl)propyl)phénoxy)acétate de tertiobutyle

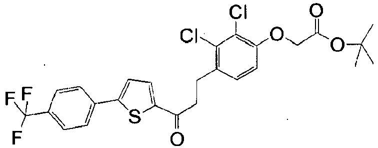

Le 2-(2,3-dichloro-4-(3-oxo-3-(5-(4-(trifluorométhyl)phényl)thièn-2-yl)propyl)phénoxy)-acétate de tertiobutyle est préparé à partir de la 3-(2,3-dichloro-4-hydroxyphényl)-1-(5-(4-(trifluorométhyl)phényl)thièn-2-yl)propan-1-one et du bromoacétate de tertiobutyle selon la procédure générale D.

Après 2 heures d'agitation, le milieu est acidifié à l'aide d'une solution diluée d'acide chlorhydrique puis extrait par de l'acétate d'éthyle. La phase organique combinées est séchée sur sulfate de magnésium, filtrée puis concentrée sous pression réduite. Le résidu d'évaporation est purifié par chromatographie sur gel de silice.

Elution : cyclohexane/acétate d'éthyle : 9/1. Silice 40-63µm.

RMN ¹H (300MHz, CDCl₃, δ en ppm) : 1,45 (s, 9H); 3,16-3,26 (m, 4H); 4,60 (s, 2H); 6,68 (d, 1H, J=8,4Hz); 7,18 (d, 1H, J=8,4Hz); 7,39 (d, 1 H, J=3,8Hz); 7,66-7,69 (m, 3H); 7,76 (d, 2H, J=8,2Hz).

### Composé 15: Acide 2-(2,3-dichloro-4-(3-oxo-3-(5-(4-(trifluorométhyl)phényl)thièn-2-yl)propyl)phénoxy)acétique

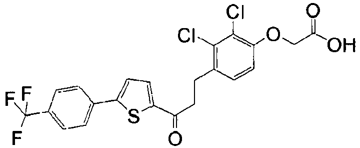

L'acide 2-(2,3-dichloro-4-(3-oxo-3-(5-(4-(trifluorométhyl)phényl)thièn-2-yl)propyl)-phénoxy)acétique est préparé à partir du 2-(2,3-dichloro-4-(3-oxo-3-(5-(4-(trifluorométhyl)phényl)thièn-2-yl)propyl)phénoxy)acétate de tertiobutyle selon la procédure générale E au moyen de 17 équivalents d'acide trifluoroacétique. Après 4 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice (HPLC préparative, lichrospher (Merck) RP18 12µm 100A, colonne : 25*250 mm).

Elution : gradient eau, méthanol + 0,1% acide trifluoroacétique : 14/86 à 10/90. RMN ¹H (300MHz, DMSO-*d₆*, δ en ppm) : 3,04 (t, 2H, J=7,5Hz); 3,31 (t, 2H, J=7,5Hz); 4,83 (s, 2H); 7,01 (d, 1H, J=8,6Hz); 7,33 (d, 1H, J=8,6Hz); 7,79-7,83 (m, 3H); 7,99 (d, 2H, J=8,2Hz); 8,06 (d, 1 H, J=4,1 Hz); 13,13 (s, 1 H).

Masse (ES⁻) : 501 (M-1).

F=217-219°C.

### Composé 16 : 2-(2,3-dichloro-4-(3-oxo-3-(5-(4-(trifluorométhyl)phényl)thièn-2-yl)-propyl)phénoxy)-2-phénylacétate d'éthyle

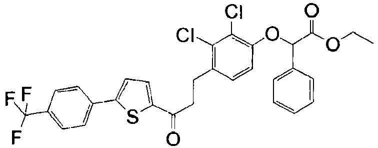

Le 2-(2,3-dichloro-4-(3-oxo-3-(5-(4-(trifluorométhyl)phényl)thièn-2-yl)propyl)phénoxy)-2-phénylacétate d'éthyle est préparé à partir de la 3-(2,3-dichloro-4-hydroxyphényl)-1-(5-(4-(trifluorométhyl)phényl)thièn-2-yl)propan-1-one et du 2-bromophénylacétate d'éthyle selon la procédure générale D.

Après 16 heures d'agitation, le milieu est acidifié à l'aide d'une solution diluée d'acide chlorhydrique puis extrait par de l'acétate d'éthyle. La phase organique est séchée sur sulfate de magnésium, filtrée puis concentrée sous pression réduite. Le résidu d'évaporation est purifié par chromatographie sur gel de silice. Elution : cyclohexane/acétate d'éthyle : 9/1. Silice 40-63µm.

RMN ¹H (300MHz, CDCl₃, δ en ppm) : 1,24 (t, 3H, J=7,6Hz); 3,17-3,22 (m, 4H); 4,15 (q, 2H, J=7,6Hz); 5,64 (s, 1 H); 6,75 (d, 1 H, J=8,7Hz); 7,13 (d, 1 H, J=8,7Hz); 7,32-7,46 (m, 6H); 7,62-7,67 (m, 3H); 7,72-7,77 (m, 2H).

### Composé 17: Acide 2-(2,3-dichloro-4-(3-oxo-3-(5-(4-(trifluorométhyl)phényl)thièn-2-yl)propyl)phénoxy)-2-phénylacétique

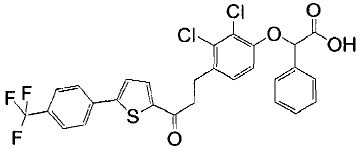

L'acide 2-(2,3-dichloro-4-(3-oxo-3-(5-(4-(trifluorométhyl)phényl)thièn-2-yl)propyl)-phénoxy)-2-phénylacétique est préparé à partir du 2-(2,3-dichloro-4-(3-oxo-3-(5-(4-(trifluorométhyl)phényl)thièn-2-yl)propyl)phénoxy)-2-phénylacétate d'éthyle selon la procédure générale F au moyen de 20 équivalents d'une solution de soude 2N.

Après 16 heures à 40°C, le milieu est acidifié à l'aide d'une solution diluée d'acide chlorhydrique puis extrait par de l'acétate d'éthyle. La phase organique est séchée sur sulfate de magnésium, filtrée puis concentrée sous pression réduite. Le résidu d'évaporation est purifié par chromatographie sur gel de silice (HPLC préparative, lichrospher (Merck) RP18 12µm 100A, colonne : 25*250 mm).

Elution : gradient eau, méthanol + 0,1% acide trifluoroacétique : 14/86 à 10/90.

RMN ¹H (300MHz, DMSO-*d₆*, δ en ppm) : 3,03 (t, 2H, J=7,6Hz); 3,31 (t, 2H, J=7,6Hz); 6,02 (s, 1H); 7,07 (d, 1H, J=8,8Hz); 7,35 (d, 1H, J=8,8Hz); 7,38-7,47 (m, 3H); 7,58 (d, 2H, J=6,5Hz); 7,79-7,82 (m, 3H); 7,99 (d, 2H, J=8,2Hz); 8,06 (d, 1 H, J=4,1 Hz); 13,35 (s, 1 H).

Masse (ES⁻) : 577 (M-1).

F=189-191°C.

### Composé 18 : 2-(2-chloro-4-(3-oxo-3-(5-(4-(trifluorométhyl)phényl)thièn-2-yl)propyl)-phénoxy)-2-méthylpropanoate de tertiobutyle

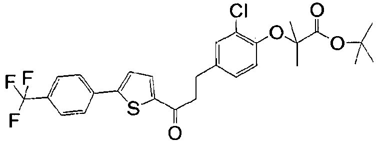

Le 2-(2-chloro-4-(3-oxo-3-(5-(4-(trifluorométhyl)phényl)thièn-2-yl)propyl)phénoxy)-2-méthylpropanoate de tertiobutyle est préparé à partir de la 3-(3-chloro-4-hydroxyphényl)-1-(5-(4-(trifluorométhyl)phényl)thièn-2-yl)propan-1-one et du bromoisobutyrate de tertiobutyle selon la procédure générale D. Après 2 heures d'agitation, le milieu est acidifié à l'aide d'une solution diluée d'acide citrique 1 N puis extrait par de l'acétate d'éthyle. La phase organique est séchée sur sulfate de magnésium, filtrée puis concentrée sous pression réduite. Le résidu d'évaporation est purifié par chromatographie flash sur gel de silice.

Elution : cyclohexane/dichlorométhane: 5/5. Silice 40-63µm.

RMN ¹H (300MHz, CDCl₃, δ en ppm) : 1,46 (s, 9H); 1,57 (s, 6H); 3,01 (t, 2H, J=7,3Hz); 3,2 (t, 2H, J=7,3Hz); 6,88 (d, 1H, J=8,5Hz); 7,01 (dd, 1H, J=2,1 Hz, J=8,5Hz); 7,26 (m, 1H); 7,39 (d, 1H, J=3,8Hz); 7,66-7,69 (m, 3H); 7,76 (d, 2H, J=8,2Hz).

### Composé 19 : Acide 2-(2-chloro-4-(3-oxo-3-(5-(4-(trifluorométhyl)phényl)thièn-2-yl)-propyl)phénoxy)-2-méthylpropanoïque

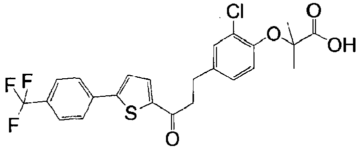

L'acide 2-(2-dichloro-4-(3-oxo-3-(5-(4-(trifluorométhyl)phényl)thièn-2-yl)propyl)-phénoxy)-2-méthylpropanoïque est préparé à partir du 2-(2-chloro-4-(3-oxo-3-(5-(4-(trifluorométhyl)phényl)thièn-2-yl)propyl)phénoxy)-2-méthylpropanoate de tertiobutyle selon la procédure générale E au moyen de 17 équivalents d'acide trifluoroacétique.

Après 1 heure à température ambiante, le milieu réactionnel est lavé avec de l'eau puis le dichlorométhane est éliminé par évaporation sous pression réduite. Le résidu d'évaporation est cristallisé dans un mélange chlorure de méthylène/heptane : 5/5.

RMN ¹H (300MHz, DMSO-*d₆*, δ en ppm) : 1,49 (s, 6H); 2,87 (t, 2H, J=7,7Hz); 3,32 (t, 2H, J=7,7Hz); 6,84 (d, 1H, J=8,3Hz); 7,15 (dd, 1 H, J=2,3Hz, J=8,3Hz); 7,39 (d, 1H, J=2,3Hz); 7,79-7,82 (m, 3H); 7,99 (d, 2H, J=8,2Hz); 8,05 (d, 1H, J=4,1 Hz).

Masse (ES⁻) : 495 (M-1).

F=136-137°C.

### Composé 20: 2-(3-chloro-4-(3-oxo-3-(5-(4-(trifluorométhyl)phényl)thièn-2-yl)propyl)-phénoxy)-2-méthylpropanoate de tertiobutyle

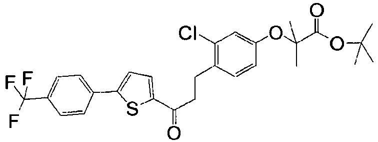

Le 2-(3-chloro-4-(3-oxo-3-(5-(4-(trifluorométhyl)phényl)thièn-2-yl)propyl)phénoxy)-2-méthylpropanoate de tertiobutyle est préparé à partir de la 3-(2-chloro-4-hydroxyphényl)-1-(5-(4-(trifluorométhyl)phényl)thièn-2-yl)propan-1-one et du bromoisobutyrate de tertiobutyle selon la procédure générale D. Après 2 heures d'agitation, le milieu est acidifié à l'aide d'une solution diluée d'acide citrique 1 N puis extrait par de l'acétate d'éthyle. La phase organique est séchée sur sulfate de magnésium, filtrée puis concentrée sous pression réduite. Le résidu d'évaporation est purifié par chromatographie flash sur gel de silice.

Elution : cyclohexane/dichlorométhane: 5/5. Silice 40-63µm.

RMN ¹H (300MHz, CDCl₃, δ en ppm) : 1,50 (s, 9H); 1,55 (s, 6H); 3,09-3,14 (m, 2H); 3,18-3,24 (m, 2H); 6,7 (dd, 1, J=2,4Hz, J=8,5Hz); 6,91 (d, 1H, J=2,6Hz); 7,15 (d, 1H, J=8,5Hz); 7,38 (d, 1H, J=3,8Hz); 7,66-7,69 (m, 3H); 7,75 (d, 2H, J=8,2Hz).

### Composé 21 : Acide 2-(3-chloro-4-(3-oxo-3-(5-(4-(trifluorométhyl)phényl)thièn-2-yl)-propyl)phénoxy)-2-méthylpropanoïque

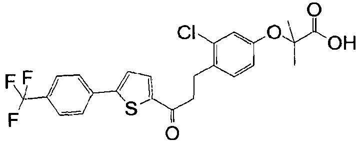

L'acide 2-(3-dichloro-4-(3-oxo-3-(5-(4-(trifluorométhyl)phényl)thièn-2-yl)propyl)-phénoxy)-2-méthylpropanoïque est préparé à partir du 2-(3-chloro-4-(3-oxo-3-(5-(4-(trifluorométhyl)phényl)thièn-2-yl)propyl)phénoxy)-2-méthylpropanoate de tertiobutyle selon la procédure générale E au moyen de 17 équivalents d'acide trifluoroacétique.

Après 1 heure à température ambiante, le milieu réactionnel est lavé avec de l'eau puis le dichlorométhane est éliminé par évaporation sous pression réduite. Le résidu d'évaporation est cristallisé dans un mélange chlorure de méthylène/heptane : 5/5.

RMN ¹H (300MHz, DMSO-*d₆*, δ en ppm) : 1,49 (s, 6H); 2,97 (t, 2H, J=8,2Hz); 3,28 (t, 2H, J=8,2Hz); 6,76 (dd, 1, J=2,6Hz, J=8,6Hz); 6,88 (d, 1H, J=2,6Hz); 7,32 (d, 1 H, J=8,6Hz); 7,79-7,83 (m, 3H); 7,99 (d, 2H, J=8,2Hz); 8,04 (d, 1H, J=4,1 Hz).

Masse (ES⁻) : 495 (M-1).

F=104-106°C.

### Composé 22: 2-(2-fluoro-4-(3-oxo-3-(5-(4-(trifluorométhyl)phényl)thièn-2-yl)propyl)-phénoxy)-2-méthylpropanoate de tertiobutyle

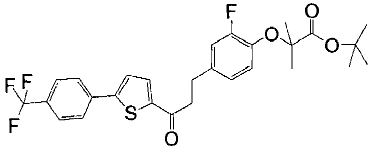

Le 2-(2-fluoro-4-(3-oxo-3-(5-(4-(trifluorométhyl)phényl)thièn-2-yl)propyl)phénoxy)-2-méthylpropanoate de tertiobutyle est préparé à partir de la 3-(3-fluoro-4-hydroxyphényl)-1-(5-(4-(trifluorométhyl)phényl)thièn-2-yl)propan-1-one et du bromoisobutyrate de tertiobutyle selon la procédure générale D.

Après 16 heures d'agitation, le solvant est éliminé par évaporation sous pression réduite et le résidu d'évaporation est repris par du dichlorométhane. La phase organique est lavée par une solution saturée de chlorure d'ammonium, séchée sur sulfate de magnésium, filtrée puis concentrée sous pression réduite. Le résidu d'évaporation est purifié par chromatographie sur gel de silice.

Elution : cyclohexane/acétate d'éthyle : gradient 97/3 à 9/1. Silice 40-63µm. RMN ¹H (300MHz, CDCl₃, δ en ppm) : 1,46 (s, 9H); 1,54 (s, 6H); 3,02 (m, 2H); 3,21 (m, 2H); 6,9 (m, 3H); 7,36 (d, 1H, J=4,1Hz); 7,67 (m, 3H); 7,76 (d, 2H J=8,1 Hz).

### Composé 23 : Acide 2-(2-fluoro-4-(3-oxo-3-(5-(4-(trifluorométhyl)phényl)thièn-2-yl)propyl)phénoxy)-2-méthylpropanoïque

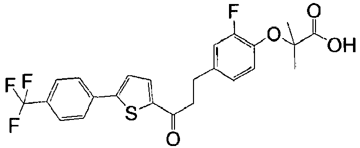

L'acide 2-(2-fluoro-4-(3-oxo-3-(5-(4-(trifluorométhyl)phényl)thièn-2-yl)propyl)-phénoxy)-2-méthylpropanoïque est préparé à partir du 2-(2-fluoro-4-(3-oxo-3-(5-(4-(trifluorométhyl)phényl)thièn-2-yl)propyl)phénoxy)-2-méthylpropanoate de tertiobutyle selon la procédure générale E au moyen de 17 équivalents d'acide trifluoroacétique.

Après 4 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice. Elution : chlorure de méthylène/méthanol : 95/5. Silice 40-63µm.

RMN ¹H (300MHz, CDCl₃, δ en ppm) : 1,57 (s, 6H); 3,07 (m, 2H); 3,24 (m, 2H); 6,99 (m, 3H); 7,39 (d, 1H, J=4,1Hz); 7,68 (m, 3H); 7,76 (d, 2H, J=8,2Hz).

Masse (ES⁺) : 481 (M+1); 498 (M+NH₄)⁺; 503 (M+Na)⁺.

F=150-151°C.

### Composé 24 : 2-(2-fluoro-4-(3-oxo-3-(5-(4-(trifluorométhyl)phényl)thièn-2-yl)propyl)-phénoxy)acétate de tertiobutyle

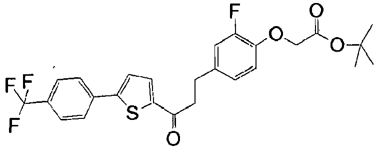

Le 2-(2-fluoro-4-(3-oxo-3-(5-(4-(trifluorométhyl)phényl)thièn-2-yl)propyl)phénoxy)-acétate de tertiobutyle est préparé à partir de la 3-(3-fluoro-4-hydroxyphényl)-1-(5-(4-(trifluorométhyl)phényl)thièn-2-yl)propan-1-one et du bromoacétate de tertiobutyle selon la procédure générale D.

Après 18 heures d'agitation, le solvant est éliminé par évaporation sous pression réduite et le résidu d'évaporation est repris par de l'acétate d'éthyle. La phase organique est lavée par une solution saturée de chlorure d'ammonium, séchée sur sulfate de magnésium, filtrée puis concentrée sous pression réduite. Le résidu d'évaporation est purifié par chromatographie sur gel de silice.

Elution : cyclohexane/acétate d'éthyle : 95/5. Silice 40-63µm.

RMN ¹H (300MHz, CDCl₃, δ en ppm) : 1,46 (s, 9H); 3,01-3,06 (m, 2H); 3,21 (m, 2H); 4,56 (s, 2H); 6,79-6,98 (m, 3H); 7,39 (d, 1H, J=4,1 Hz); 7,65-7,69 (m, 3H); 7,75 (m, 2H).

### Composé 25 : Acide 2-(2-fluoro-4-(3-oxo-3-(5-(4-(trifluorométhyl)phényl)thièn-2-yl)-propyl)phénoxy)acétique

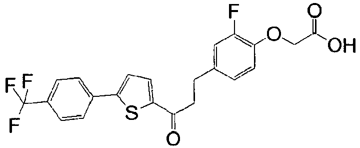

L'acide 2-(2-fluoro-4-(3-oxo-3-(5-(4-(trifluorométhyl)phényl)thièn-2-yl)propyl)-phénoxy)acétique est préparé à partir du 2-(2-fluoro-4-(3-oxo-3-(5-(4-(trifluorométhyl)phényl)thièn-2-yl)propyl)phénoxy)acétate de tertiobutyle selon la procédure générale E au moyen de 10 équivalents d'acide trifluoroacétique. Après 18 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice.

Elution : chlorure de méthylène/méthanol : gradient 95/5 à 9/1. Silice 40-63µm. RMN ¹H (300MHz, CDCl₃, δ en ppm) : 3,04 (t, 2H, J=4,5Hz); 3,22 (t, 2H, J=4,5Hz); 4,72 (s, 2H); 6,89-7,06 (m, 3H); 7,39 (d, 1H, J=3,8Hz); 7,69 (m, 3H); 7,76 (d, 2H, J=8,2Hz).

Masse (ES⁺) : 453 (M+1); 470 (M+NH₄)⁺; 475 (M+Na)⁺.

F=170-171°C.

### Composé 26 : Acide 2-(2-fluoro-4-(3-hydroxy-3-(5-(4-(trifluorométhyl)phényl)thièn-2-yl)propyl)phénoxy)acétique

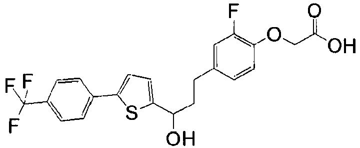

L'acide 2-(2-fluoro-4-(3-hydroxy-3-(5-(4-(trifluorométhyl)phényl)thièn-2-yl)propyl)-phénoxy)acétique est préparé à partir de l'acide 2-(2-fluoro-4-(3-oxo-3-(5-(4-(trifluorométhyl)phényl)thièn-2-yl)propyl)phénoxy)acétique selon la procédure générale G.

RMN ¹H (300MHz, CDCl₃, δ en ppm) : 2,11-2,22 (m, 2H); 2,67-2,76 (m, 2H); 4,69 (s, 2H); 4,91 (m, 1 H); 6,86-6,99 (m, 4H); 7,25 (d, 1 H, J=3,8Hz); 7,62 (d, 2H, J=8,5Hz); 7,68 (d, 2H, J=8,5Hz).

### Composé 27: Acide 2-(4-(3-(benzyloxy)-3-(5-(4-(trifluorométhyl)phényl)thièn-2-yl)-propyl)-2-fluorophénoxy)acétique

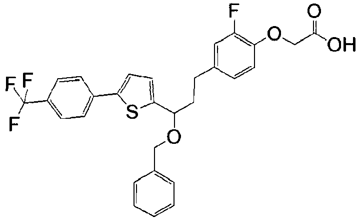

L'acide 2-(4-(3-(benzyloxy)-3-(5-(4-(trifluorométhyl)phényl)thièn-2-yl)propyl)-2-fluoro-phénoxy)acétique est préparé à partir de l'acide 2-(2-fluoro-4-(3-hydroxy-3-(5-(4-(trifluorométhyl)phényl)thièn-2-yl)propyl)phénoxy)acétique au moyen de 2,2 équivalents d'hydrure de sodium et de 2,2 équivalents de bromure de benzyle selon la procédure générale H.

Le résidu d'évaporation est solubilisé dans de l'éthanol en présence de soude 2N (20 éq.). Après 16 heures sous agitation, les solvants sont éliminés par évaporation sous pression réduite. Le résidu d'évaporation est acidifié à l'aide d'une solution diluée d'acide chlorhydrique puis extrait par du chlorure de méthylène. La phase organique est séchée sur sulfate de magnésium, filtrée puis concentrée sous pression réduite. Le résidu d'évaporation est purifié par chromatographie sur gel de silice (HPLC préparative, lichrospher (Merck) RP18 12µm 100A, colonne : 25*250 mm).

Elution : gradient eau, méthanol + 0,1% acide trifluoroacétique : 22/78 à 10/90.

RMN ¹H (300MHz, CDCl₃, δ en ppm) : 1,99-2,11 (m, 1 H); 2,23-2,35 (m, 1 H); 2,58-2,80 (m, 2H); 4,35 (d, 1 H, J=11,4Hz); 4,53 (m, 1 H); 4,61 (d, 1 H, J=11,4Hz); 4,71 (s, 2H); 6,82-6,93 (m, 3H); 6,97 (d, 1H, J=3,5Hz); 7,27-7,39 (m, 6H) ; 7,63 (d, 2H, J=8,5Hz); 7,71 (d, 2H, J=8,5Hz).

Masse (MALDI-TOF) : 566 (M+Na)⁺.

### Composé 28 : 2-(2-fluoro-4-(3-oxo-3-(5-(4-(trifluorométhyl)phényl)thièn-2-yl)propyl)-phénoxy)butanoate de tertiobutyle

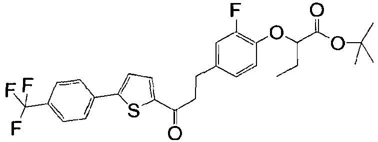

Le 2-(2-fluoro-4-(3-oxo-3-(5-(4-(trifluorométhyl)phényl)thièn-2-yl)propyl)phénoxy)-butanoate de tertiobutyle est préparé à partir de la 3-(3-fluoro-4-hydroxyphényl)-1-(5-(4-(trifluorométhyl)phényl)thièn-2-yl)propan-1-one et de 2-bromobutanoate de tertiobutyle selon la procédure générale D.

Après 16 heures d'agitation, le solvant est éliminé par évaporation sous pression réduite. Le résidu d'évaporation est repris par de l'acétate d'éthyle. La phase organique est lavée par une solution saturée de chlorure d'ammonium, séchée sur sulfate de magnésium, filtrée puis concentrée sous pression réduite. Le résidu d'évaporation est purifié par chromatographie sur gel de silice.

Elution : cyclohexane/acétate d'éthyle : gradient 95/5 à 9/1. Silice 40-63µm. RMN ¹H (300MHz, CDCl₃, δ en ppm) : 1,09 (t, 3H, J=7,3Hz); 1,43 (s, 9H); 1,98 (m, 2H); 3,02 (t, 2H, J=4,5Hz); 3,23 (t, 2H, J=4,5Hz); 4,42 (t, 1 H, J=6,1 Hz); 6,81-7,01 (m, 3H); 7,38 (d, 1 H, J=4,1 Hz); 7,66-7,69 (m, 3H); 7,76 (d, 2H, J=8,2Hz).

### Composé 29 : Acide 2-(2-fluoro-4-(3-oxo-3-(5-(4-(trifluorométhyl)phényl)thièn-2-yl)-propyl)phénoxy)butanoïque

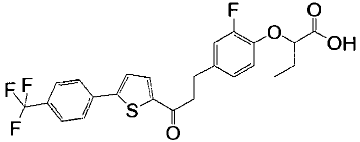

L'acide 2-(2-fluoro-4-(3-oxo-3-(5-(4-(trifluorométhyl)phényl)thièn-2-yl)propyl)-phénoxy)butanoïque est préparé à partir du 2-(2-fluoro-4-(3-oxo-3-(5-(4-(trifluorométhyl)phényl)thièn-2-yl)propyl)phénoxy)butanoate de tertiobutyle selon la procédure générale E au moyen de 17 équivalents d'acide trifluoroacétique. Après 6 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice. L'huile obtenue est cristallisée dans du dichlorométhane.

Elution : chlorure de méthylène/méthanol : 9/1. Silice 40-63µm.

RMN ¹H (300MHz, CDCl₃, δ en ppm) : 1,11 (t, 3H, J=7,3Hz); 2,03 (m, 2H); 3,01 (t, 2H, J=7,4Hz); 3,19 (t, 2H, J=7,4Hz); 4,58 (t, 1 H, J=5,7Hz); 6,86-7,01 (m, 3H); 7,37 (d, 1 H, J=3,8Hz); 7,66 (m, 3H); 7,76 (d, 2H, J=7,9Hz).

Masse (MALDI-TOF) : 481 (M+1).

F=119-120°C.

### Composés 30 : Acide 2-(2-fluoro-4-(3-hydroxy-3-(5-(4-(trifluorométhyl)phényl)thièn-2-yl)propyl)phénoxy)butanoïque

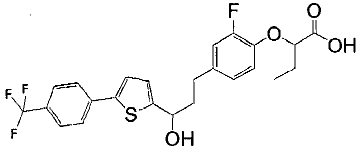

L'acide 2-(2-fluoro-4-(3-hydroxy-3-(5-(4-(trifluorométhyl)phényl)thièn-2-yl)propyl)-phénoxy)butanoïque est préparé à partir de l'acide 2-(2-fluoro-4-(3-oxo-3-(5-(4-(trifluorométhyl)phényl)thièn-2-yl)propyl)phénoxy)butanoïque selon la procédure générale G.

Le résidu d'évaporation est purifié par chromatographie sur gel de silice. Elution : chlorure de méthylène/méthanol : 95/5. Silice 40-63µm.

RMN ¹H (300MHz, CDCl₃, δ en ppm) : 1,12 (t, 3H, J=7,6Hz); 1,98-2,25 (m, 4H); 2,69 (m, 2H); 4,59 (t, 1 H, J=6,6Hz); 4,9 (t, 1H, J=5,8Hz); 6,83-6,96 (m, 4H); 7,23 (d, 1H, J=3,8Hz); 7,61 (d, 2H, J=8,7Hz); 7,66 (d, 2H, J=8,7Hz).

Masse (ES⁺) : 483 (M+1).

Aspect : huile visqueuse incolore.

### Composé 31 : 2-(2-bromo-4-(3-oxo-3-(5-(4-(trifluorométhyl)phényl)thièn-2-yl)propyl)-phénoxy)butanoate de tertiobutyle

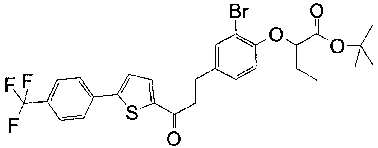

Le 2-(2-bromo-4-(3-oxo-3-(5-(4-(trifluorométhyl)phényl)thièn-2-yl)propyl)phénoxy)-butanoate de tertiobutyle est préparé à partir de la 3-(3-bromo-4-hydroxyphényl)-1-(5-(4-(trifluorométhyl)phényl)thièn-2-yl)propan-1-one et du 2-bromobutanoate de tertiobutyle selon la procédure générale D.

Après 2 heures d'agitation, le milieu est acidifié à l'aide d'une solution diluée d'acide citrique 1 N puis extrait par de l'acétate d'éthyle. La phase organique est séchée sur sulfate de magnésium, filtrée puis concentrée sous pression réduite. Le résidu d'évaporation est purifié par chromatographie flash sur gel de silice.

Elution : cyclohexane/acétate d'éthyle : 8/2. Silice 40-63µm.

RMN ¹H (300MHz, CDCl₃, δ en ppm) : 0,95-1,25 (m, 3H); 1,45 (s, 9H); 1,84-2,06 (m, 2H); 3,01 (m, 2H); 3,2 (t, 2H, J=7,3Hz); 4,45 (t, 1 H, J=6,1 Hz); 6,69 (d, 1 H, J=8,5Hz); 7,09 (dd, 1 H, J=2,1 Hz, J=8,5Hz); 7,37-7,39 (m, 1 H); 7,45 (d, 1 H, J=2,1 Hz); 7,65-7,69 (m, 3H); 7,76 (d, 2H, J=8,5Hz).

### Composé 32 : Acide 2-(2-bromo-4-(3-oxo-3-(5-(4-(trifluorométhyl)phényl)thièn-2-yl)propyl)phénoxy)butanoïque

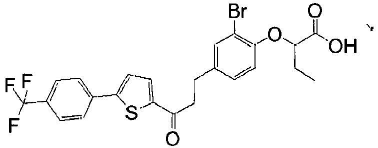

L'acide 2-(2-bromo-4-(3-oxo-3-(5-(4-(trifluorométhyl)phényl)thièn-2-yl)propyl)-phénoxy)butanoïque est préparé à partir du 2-(2-bromo-4-(3-oxo-3-(5-(4-(trifluorométhyl)phényl)thièn-2-yl)propyl)phénoxy)butanoate de tertiobutyle selon la procédure générale E au moyen de 17 équivalents d'acide trifluoroacétique. Après 1 heure à température ambiante, le milieu réactionnel est lavé avec de l'eau puis le dichlorométhane est éliminé par évaporation sous pression réduite. Le résidu d'évaporation est purifié par chromatographie sur gel de silice (HPLC préparative, lichrospher (Merck) RP18 12µm 100A, colonne : 25*250 mm).

Elution : gradient eau, méthanol + 0,1% acide trifluoroacétique : 30/70 à 0/100.

RMN ¹H (300MHz, CDCl₃, δ en ppm) : 1,13 (t, 3H, J=7,3Hz); 2,11 (m, 2H); 3,02 (t, 2H, J=7,4Hz); 3,21 (t, 2H, J=7,4Hz); 4,68 (t, 1H, J=5,6Hz); 6,78 (d, 1H, J=8,5Hz); 7,14 (dd, 1H, J=2,1 Hz, J=8,5Hz); 7,39 (d, 1H, J=4,1 Hz); 7,48 (d, 1H, J=2,1 Hz); 7,67-7,69 (m, 3H); 7,76 (d, 2H, J=8,5Hz).

Masse (ES⁺) : 541 /543 (M+1).

F=126-128°C.

### Composé 33 : 2-(2-bromo-4-(3-oxo-3-(5-(4-(trifluorométhyl)phényl)thièn-2-yl)propyl)phénoxy)acétate de tertiobutyle

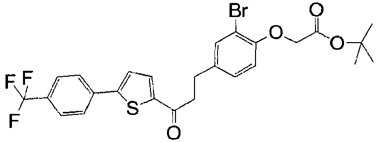

Le 2-(2-bromo-4-(3-oxo-3-(5-(4-(trifluorométhyl)phényl)thièn-2-yl)propyl)phénoxy)-acétate de tertiobutyle est préparé à partir de la 3-(3-bromo-4-hydroxyphényl)-1-(5-(4-(trifluorométhyl)phényl)thièn-2-yl)propan-1-one et du bromoacétate de tertiobutyle selon la procédure générale D.

Après 12 heures d'agitation, le milieu est acidifié à l'aide d'une solution diluée d'acide citrique 1 N puis extrait par de l'acétate d'éthyle. La phase organique est séchée sur sulfate de magnésium, filtrée puis concentrée sous pression réduite. Le résidu d'évaporation est purifié par chromatographie flash sur gel de silice.

Elution : cyclohexane/acétate d'éthyle : 85/15. Silice 40-63µm.

RMN ¹H (300MHz, CDCl₃, δ en ppm) : 1,48 (s, 9H); 2,98-3,05 (m, 2H); 3,2 (t, 2H, J=7,1 Hz); 4,57 (s, 2H); 6,72 (d, 1 H, J=8,2Hz); 7,12 (dd, 1 H, J=2,1 Hz, J=8,2Hz); 7,38 (d, 1H, J=3,8Hz); 7,46 (d, 1H, J=2,1 Hz); 7,64-7,68 (m, 3H); 7,74 (d, 2H, J=8,2Hz).

### Composé 34 : Acide 2-(2-bromo-4-(3-oxo-3-(5-(4-(trifluorométhyl)phényl)thièn-2-yl)propyl)phénoxy)acétique

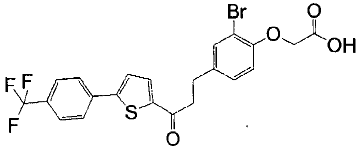

L'acide 2-(2-bromo-4-(3-oxo-3-(5-(4-(trifluorométhyl)phényl)thièn-2-yl)propyl)-phénoxy)acétique est préparé à partir du 2-(2-bromo-4-(3-oxo-3-(5-(4-(trifluorométhyl)phényl)thièn-2-yl)propyl)phénoxy)acétate de tertiobutyle selon la procédure générale E au moyen de 17 équivalents d'acide trifluoroacétique. Après 1 heure à température ambiante, le milieu réactionnel est lavé avec de l'eau puis le dichlorométhane est éliminé par évaporation sous pression réduite. Le résidu d'évaporation est purifié par chromatographie sur gel de silice (HPLC préparative, lichrospher (Merck) RP18 12µm 100A, colonne : 25*250 mm).

Elution : gradient eau, méthanol + 0,1% acide trifluoroacétique : 30/70 à 0/100.

RMN ¹H (300MHz, CDCl₃, δ en ppm) : 3,04 (t, 2H, J=7,6Hz); 3,22 (t, 2H, J=7,6Hz); 4,7 (s, 2H); 6,82 (d, 1H, J=8,2Hz); 7,19 (dd, 1, J=2,1 Hz, J=8,2Hz); 7,39 (d, 1H, J=4,1 Hz); 7,49 (d, 1H, J=2,1 Hz); 7,67-7,70 (m, 3H); 7,76 (d, 2H, J=8,5Hz).

Masse (ES⁺) : 513/515 (M+1).

F=180-182°C.

### Composé 35 : 2-(2-bromo-4-(3-oxo-3-(5-(4-(trifluorométhyl)phényl)thièn-2-yl)propyl)-phénoxy)-2-méthylpropanoate de tertiobutyle

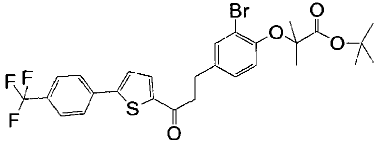

Le 2-(2-bromo-4-(3-oxo-3-(5-(4-(trifl uorométhyl)phényl)th ièn-2-yl)propyl)phénoxy)-2-méthylpropanoate de tertiobutyle est préparé à partir de la 3-(3-bromo-4-hydroxyphényl)-1-(5-(4-(trifluorométhyl)phényl)thièn-2-yl)propan-1-one et du bromoisobutyrate de tertiobutyle selon la procédure générale D. Après 2 heures d'agitation, le milieu est acidifié à l'aide d'une solution diluée d'acide citrique 1 N puis extrait par de l'acétate d'éthyle. La phase organique est séchée sur sulfate de magnésium, filtrée puis concentrée sous pression réduite. Le résidu d'évaporation est purifié par chromatographie flash sur gel de silice.

Elution : cyclohexane/acétate d'éthyle : 9/1. Silice 40-63µm.

RMN ¹H (300MHz, CDCl₃, δ en ppm) : 1,46 (s, 9H); 1,58 (s, 6H); 2,98-3,05 (m, 2H); 3,2 (t, 2H, J=7,1 Hz); 6,86 (d, 1 H, J=8,5Hz); 7,05 (dd, 1 H, J=2,1 Hz, J=8,5Hz); 7,39 (d, 1 H, J=4,1 Hz); 7,44 (d, 1 H, J=2,1 Hz); 7,65-7,69 (m, 3H); 7,76 (d, 2H, J=8,2Hz).

### Composé 36 : Acide 2-(2-bromo-4-(3-oxo-3-(5-(4-(trifluorométhyl)phényl)thièn-2-yl)propyl)phénoxy)-2-méthylpropanoïque

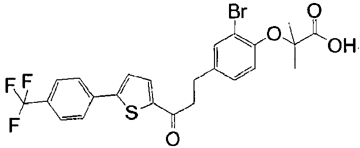

L'acide 2-(2-bromo-4-(3-oxo-3-(5-(4-(trifluorométhyl)phényl)thièn-2-yl)propyl)-phénoxy)-2-méthylpropanoïque est préparé à partir du 2-(2-bromo-4-(3-oxo-3-(5-(4-(trifluorométhyl)phényl)thièn-2-yl)propyl)phénoxy)-2-méthylpropanoate de tertiobutyle selon la procédure générale E au moyen de 17 équivalents d'acide trifluoroacétique.

Après 1 heure d'agitation à température ambiante, le milieu réactionnel est lavé avec de l'eau puis le dichlorométhane est éliminé par évaporation sous pression réduite. Le résidu d'évaporation est purifié par chromatographie sur gel de silice (HPLC préparative, lichrospher (Merck) RP18 12µm 100A, colonne : 25*250 mm).

Elution : gradient eau, méthanol + 0,1% acide trifluoroacétique : 30/70 à 0/100. RMN ¹H (300MHz, CDCl₃, δ en ppm) : 1,64 (s, 6H); 3,05 (t, 2H, J=7,6Hz); 3,23 (t, 2H, J=7,6Hz); 7,01 (d, 1H, J=8,3Hz); 7,15 (dd, 1 H, J=2,1 Hz, J=8,3Hz); 7,39 (d, 1 H, J=4,1 Hz); 7,5 (d, 1 H, J=2,1 Hz); 7,67-7,70 (m, 3H); 7,76 (d, 2H, J=8,5Hz). Masse (ES⁺) : 541/543 (M+1).

F=102-104°C.

### Composé 37: 2-(2,3-dichloro-4-(3-oxo-3-(5-(4-(trifluorométhyl)phényl)fur-2-yl)propyl)-phénoxy)-2-méthylpropanoate de tertiobutyle

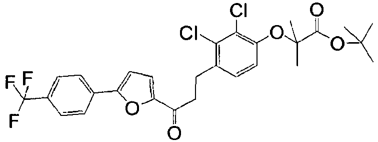

Le 2-(2,3-dichloro-4-(3-oxo-3-(5-(4-(trifluorométhyl)phényl)fur-2-yl)propyl)phénoxy)-2-méthylpropanoate de tertiobutyle est préparé à partir de 3-(2,3-dichloro-4-hydroxyphényl)-1-(5-(4-(trifluorométhyl)phényl)fur-2-yl)propan-1-one et du bromoisobutyrate de tertiobutyle selon la procédure générale D.

Après 16 heures d'agitation, le solvant est éliminé par évaporation sous pression réduite. Le résidu d'évaporation est repris par de l'acétate d'éthyle. La phase organique est lavée par une solution saturée de chlorure d'ammonium, séchée sur sulfate de magnésium, filtrée puis concentrée sous pression réduite. Le résidu d'évaporation est purifié par chromatographie sur gel de silice.

Elution : cyclohexane/acétate d'éthyle : gradient 95/5 à 8/2. Silice 40-63µm. RMN ¹H (300MHz, CDCl₃, δ en ppm) : 1,45 (s, 9H); 1,57 (s, 6H); 3,19 (m, 4H); 6,79 (d, 1 H, J=8,6Hz); 6,87 (d, 1H, J=3,7Hz); 7,10 (d, 1 H, J=8,6Hz); 7,27 (d, 1 H, J=3,7Hz); 7,69 (d, 2H, J=8,5Hz); 7,88 (d, 2H, J=8,5Hz).

### Composé 38 : Acide 2-(2,3-dichloro-4-(3-oxo-3-(5-(4-(trifluorométhyl)phényl)fur-2-yl)-propyl)phénoxy)-2-méthylpropanoïque

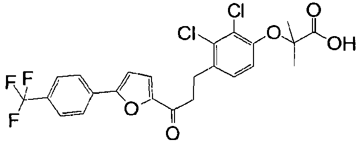

L'acide 2-(2,3-dichloro-4-(3-oxo-3-(5-(4-(trifluorométhyl)phényl)fur-2-yl)propyl)-phénoxy)-2-méthylpropanoïque est préparé à partir du 2-(2,3-dichloro-4-(3-oxo-3-(5-(4-(trifluorométhyl)phényl)fur-2-yl)propyl)phénoxy)-2-méthylpropanoate de tertiobutyle selon la procédure générale E au moyen de 10 équivalents d'acide trifluoroacétique.

Après 2 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice. Elution : dichlorométhane/méthanol: 9/1. Silice 40-63µm.

RMN ¹H (300MHz, CDCl₃, δ en ppm) : 1,63 (s, 6H); 3,19 (m, 4H); 6,86 (d, 1 H, J=3,7Hz); 6,92 (d, 1H, J=8,5Hz); 7,13 (d, 1H, J=8,5Hz); 7,29 (d, 1H, J=3,7Hz); 7,65 (d, 2H, J=8,5Hz); 7,84 (d, 2H, J=8,5Hz); 11,07 (s, 1 H).

Masse (ES⁻) : 513/515 (M-1).

F=85°C.

### Composé 39 : Acide 2-(2,3-dichloro-4-(3-(pyridin-3-ylméthoxy)-3-(5-(4-(trifluoro-méthyl)phényl)thièn-2-yl)propyl)phénoxy)-2-méthylpropanoïque

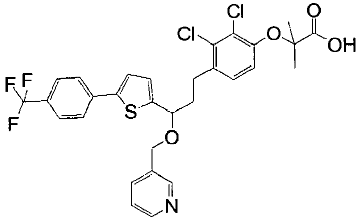

L'acide 2-(2,3-dichloro-4-(3-(pyridin-3-ylméthoxy)-3-(5-(4-(trifluorométhyl)phényl)-thièn-2-yl)propyl)phénoxy)-2-méthylpropanoïque est préparé à partir de l'acide 2-(2,3-dichloro-4-(3-hydroxy-3-(5-(4-(trifluorométhyl)phényl)thièn-2-yl)propyl)phénoxy)-2-méthylpropanoïque au moyen de 8 équivalents d'hydrure de sodium et de 2,1 équivalents de bromohydrate de 3-(bromométhyl)pyriine selon la procédure générale H.

Après 12 heures d'agitation à 70 °C, le milieu réactionnel est dilué par de l'eau, acidifié par une solution d'acide chlorhydrique 1 N et, extrait par de l'acétate d'éthyle. La phase organique est concentrée sous pression réduite puis, traitée par une solution de soude 2N, et extraite par du dichlorométhane. Après acidification par une solution d'acide citrique 1 N, le dichlorométhane est éliminé par évaporation sous pression réduite et le résidu d'évaporation purifié par chromatographie flash sur gel de silice.

Elution : dichlorométhane/méthanol : 100/0 à 95/5. Silice 40-63µm.

RMN ¹H (300MHz, CDCl₃, δ en ppm) : 1,73 (s, 6H); 2,22-2,35 (m, 2H); 2,85-2,97 (m, 2H); 4,35 (d, 1 H, J=12,5Hz); 4,55 (d, 1 H, J=12,5Hz); 4,59-4,67 (m, 1 H); 6,86 (d, 1H, J=7,6Hz); 6,96 (d, 1H, J=3,5Hz); 7,01 (d, 1H, J=7,6Hz); 7,27-7,36 (m, 1 H); 7,61-7,64 (m, 3H); 7,68 (d, 2H, J=8,4Hz); 8,06 (sl, 1 H); 8,52 (d, 2H, J=4,1 Hz).

Masse (ES⁻) : 622 / 624 / 623 (M-1).

F=75-77°C.

### Composé 39a et 39b :

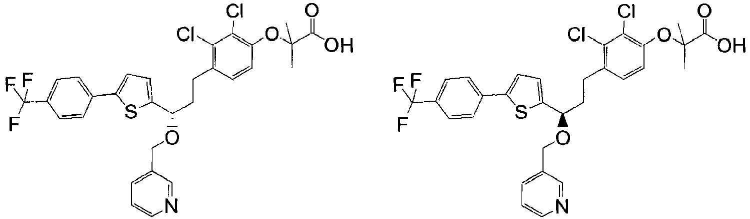

Les deux énantiomères du composé 39 sont séparés par HPLC semi-préparative sur colonne chirale Chiralpak®AD-H (250*20mm, 5µm, Chiral Technologies Europe) à température ambiante. L'élution est réalisée en mode isocratique avec une phase mobile n-heptane-éthanol (87-13) au débit de 12 ml/min.

La pureté énantiomérique de chacun des deux énantiomères ainsi obtenus est contrôlée par HPLC analytique : colonne Chiralpak®AD-H (250*46mm, 5µm, Daicel Chemical Industries, LTD) à 30°C ; élution isocratique avec phase mobile n-heptane-isopropanol (87-13); débit 1 ml/min ; détection UV à 205 nm.

Composé 39a : tR = 16,2 min, ee = 96%

Composé 39b : tR = 19,3 min, ee = 99,1%

### Composé 40: Acide 2-(2,3-dichloro-4-(3-méthoxy-3-(5-(4-(trifluorométhyl)phényl)-thièn-2-yl)propyl)phénoxy)-2-méthylpropanoïque

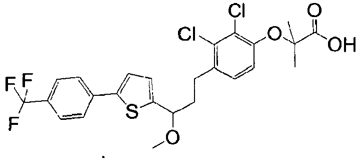

L'acide 2-(2,3-dichloro-4-(3-méthoxy-3-(5-(4-(trifluorométhyl)phényl)thièn-2-yl)-propyl)phénoxy)-2-méthylpropanoïque est préparé à partir de l'acide 2-(2,3-dichloro-4-(3-hydroxy-3-(5-(4-(trifluorométhyl)phényl)thièn-2-yl)propyl)phénoxy)-2-méthylpropanoïque au moyen de 2,2 équivalents d'hydrure de sodium et de 2,2 équivalents de iodométhane selon la procédure générale H.

Le résidu d'évaporation est purifié par chromatographie sur gel de silice (Elution : cyclohexane/acétate d'éthyle : 9/1. Silice 40-63µm.). L'huile isolée est solubilisée dans de l'éthanol en présence de soude 2N (20 éq.). Après 16 heures sous agitation, les solvants sont éliminés par évaporation sous pression réduite. Le résidu d'évaporation est acidifié à l'aide d'une solution diluée d'acide chlorhydrique puis extrait par du dichlorométhane. La phase organique est séchée sur sulfate de magnésium, filtrée puis concentrée sous pression réduite. RMN ¹H (300MHz, CDCl₃, δ en ppm) : 1,65 (s, 6H); 2,03-2,29 (m, 2H); 2,75-2,97 (m, 2H); 3,34 (s, 3H); 4,38 (dd, 1 H, J=5,7Hz J=7,7Hz); 6,95 (d, 1 H, J=8,6Hz); 6,99 (d, 1 H, J=3,6Hz); 7,07 (d, 1 H, J=8,6Hz); 7,27 (d, 1 H, J=3,6Hz); 7,62 (d, 2H, J=8,5Hz); 7,68 (d, 2H, J=8,5Hz).

Masse (ES⁺) : 564/566 (M+NH₄)⁺, 569 / 571 (M+Na)⁺, 585/587 (M+K)⁺. F=46-48°C.

### Composé 40a et 40b :

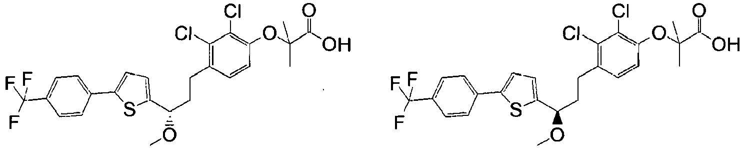

Les deux énantiomères du composé 40 sont séparés par HPLC semi-préparative sur colonne chirale Chiralpak®AD-H (250*20mm, 5µm, Chiral Technologies Europe) à température ambiante. L'élution est réalisée en mode isocratique avec une phase mobile n-heptane-éthanol (93-7) additionnée de 0.1% d'acide trifluoroacétique au débit de 16 à 18 ml/min.

La pureté énantiomérique de chacun des deux énantiomères ainsi obtenus est contrôlée par HPLC analytique : colonne Chiralpak®AD-H (250*46mm, 5µm, Daicel Chemical Industries, LTD) à 30°C ; élution isocratique avec phase mobile n-heptane-éthanol (93-7) additionnée de 0.1% d'acide trifluoroacétique ; débit 1 ml/min ; détection UV à 292 nm.

Composé 40a : tR = 13,4 min, ee = 100 %

Composé 40b : tR = 18,3 min, ee = 99,4%

### Composé 41 : Acide 2-(2,3-dichloro-4-(3-éthoxy-3-(5-(4-(trifluorométhyl)phényl)thièn-2-yl)propyl)phénoxy)-2-méthylpropanoïque

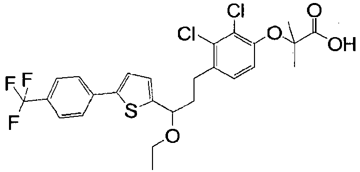

L'acide 2-(2,3-dichloro-4-(3-éthoxy-3-(5-(4-(trifluorométhyl)phényl)thièn-2-yl)propyl)-phénoxy)-2-méthylpropanoïque est préparé à partir de l'acide 2-(2,3-dichloro-4-(3-hydroxy-3-(5-(4-(trifluorométhyl)phényl)thièn-2-yl)propyl)phénoxy)-2-méthylpropanoïque au moyen de 8 équivalents d'hydrure de sodium et de 2,1 équivalents de iodoéthane selon la procédure générale H.

Après 20 minutes d'agitation à 70 °C, le milieu réactionnel est ramené à température ambiante, traité par une solution de soude 2N, puis, acidifié par une solution d'acide chlorhydrique 1N et extrait par de l'acétate d'éthyle. La phase organique est concentrée sous pression réduite et le résidu d'évaporation purifié par chromatographie flash sur gel de silice.

Elution : dichlorométhane/méthanol : 100/0 à 95/5. Silice 40-63µm.

RMN ¹H (300MHz, CDCl₃, δ en ppm) : 1,24 (t, 3H, J=7,0Hz); 1,64 (s, 6H); 2,03-2,28 (m, 2H); 2,78-2,99 (m, 2H); 3,37-3,47 (m, 1 H); 3,53-3,63 (m, 1 H); 4,48 (dd, 1 H, J=5,5Hz, J=7,6Hz); 6,92-6,97 (m, 2H); 7,10 (d, 1 H, J=8,5Hz); 7,25 (d, 1 H, J=3,9Hz); 7,27 (d, 1 H, J=3,8Hz); 7,62 (d, 2H, J=8,3Hz); 7,69 (d, 2H, J=8,3Hz).

Masse (ES⁻) : 559 / 561 (M-1).

Aspect : huile jaune visqueuse.

### Composé 42: Acide 2-(2,3-dichloro-4-(3-(cyclohexylméthoxy)-3-(5-(4-(trifluoro-méthyl)phényl)thièn-2-yl)propyl)phénoxy)-2-méthylpropanoïque

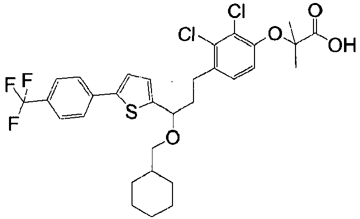

L'acide 2-(2,3-dichloro-4-(3-(cyclohexylméthoxy)-3-(5-(4-(trifluorométhyl)phényl)thièn-2-yl)propyl)phénoxy)-2-méthylpropanoïque est préparé à partir de l'acide 2-(2,3-dichloro-4-(3-hydroxy-3-(5-(4-(trifluorométhyl)phényl)thièn-2-yl)propyl)phénoxy)-2-méthylpropanoïque au moyen de 8 équivalents d'hydrure de sodium et de 2,5 équivalents de bromométhylcyclohexane selon la procédure générale H.

Après 18 heures d'agitation à 70 °C, le milieu réactionnel est acidifié par une solution d'acide chlorhydrique 1N et extrait par de l'acétate d'éthyle. La phase organique est concentrée sous pression réduite et le résidu d'évaporation est purifié par chromatographie flash sur gel de silice.

Elution : dichlorométhane/méthanol : 100/0 à 9/1. Silice 40-63µm.

RMN ¹H (300MHz, CDCl₃, δ en ppm) : 0,89-0,99 (m, 3H); 1,14-1,28 (m, 4H); 1,55-1,84 (m, 10H); 2,03-2,27 (m,2H); 2,78-3,01 (m, 2H); 3,14 (dd, 1H, J=6,4Hz, J=9,0Hz); 3,22 (dd, 1H, J=6,7Hz, J=8,7Hz); 4,43 (dd, 1H, J=4,9Hz, J=7,8Hz); 6,94-6,97 (m, 2H); 7,11 (d, 1 H, J=8,4Hz); 7,25 (d, 1H, J=3,9Hz); 7,27 (d, 1H, J=3,8Hz); 7,62 (d, 2H, J=8,3Hz); 7,69 (d, 2H, J=8,3Hz).

Masse (ES⁻) : 627 / 629 (M-1).

Aspect : huile jaune visqueuse.

### Composé 43: 2-(2,3-dichloro-4-(3-oxo-3-(5-(4-(trifluorométhoxy)phényl)thièn-2-yl)-propyl)phénoxy)-2-méthylpropanoate de tertiobutyle

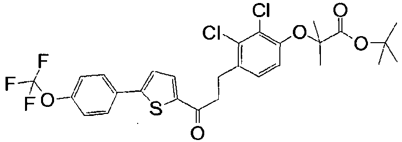

Le 2-(2,3-dichloro-4-(3-oxo-3-(5-(4-(trifluorométhoxy)phényl)thièn-2-yl)propyl)-phénoxy)-2-méthylpropanoate de tertiobutyle est préparé à partir de la 3-(2,3-dichloro-4-hydroxyphényl)-1-(5-(4-(trifluorométhoxy)phényl)thièn-2-yl)propan-1-one et du bromoisobutyrate de tertiobutyle selon la procédure générale D. Après 6 heures d'agitation, le milieu est dilué par une solution de chlorure d'ammonium saturée et extrait par de l'acétate d'éthyle. La phase organique est séchée sur sulfate de magnésium, filtrée puis concentrée sous pression réduite. Le résidu d'évaporation est purifié par chromatographie flash sur gel de silice.

Elution : cyclohexane/acétate d'éthyle : 9/1. Silice 40-63µm.

RMN ¹H (300MHz, CDCl₃, δ en ppm) : 1,44 (s, 9H); 1,58 (s, 6H); 3,14-3,24 (m, 4H); 6,79 (d, 1 H, J=8,6Hz); 7,09 (d, 1 H, J=8,6Hz); 7,25-7,29 (m, 3H); 7,64-7,68 (m, 3H).

### Composé 44 : Acide 2-(2,3-dichloro-4-(3-oxo-3-(5-(4-(trifluorométhoxy)phényl)thièn-2-yl)propyl)phénoxy)-2-méthylpropanoïque

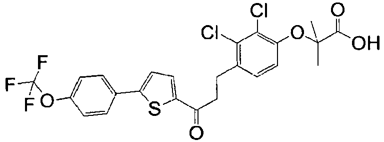

L'acide 2-(2,3-dichloro-4-(3-oxo-3-(5-(4-(trifluorométhoxy)phényl)thièn-2-yl)propyl)-phénoxy)-2-méthylpropanoïque est préparé à partir du 2-(2,3-dichloro-4-(3-oxo-3-(5-(4-(trifluorométhoxy)phényl)thièn-2-yl)propyl)phénoxy)-2-méthylpropanoate de tertiobutyle selon la procédure générale E au moyen de 10 équivalents d'acide trifluoroacétique.

Après 48 heures d'agitation à température ambiante, les solvants sont éliminés par évaporation sous pression réduite. Le résidu d'évaporation est purifié par chromatographie sur gel de silice.

Elution : cyclohexane/acétate d'éthyle : 9/1 à 0/100. Silice 40-63µm.

RMN ¹H (300MHz, CDCl₃, δ en ppm) : 1,63 (s, 6H); 3,17-3,26 (m, 4H); 6,93 (d, 1 H, J=8,4Hz); 7,18 (d, 1H, J=8,4Hz); 7,17-7,29 (m, 3H); 7,64-7,68 (m, 3H). Masse (ES⁻) : 545/547 (M-1).

F=135-136°C.

### Composé 45 : 2-(2,3-difluoro-4-(3-oxo-3-(5-(4-(trifluorométhyl)phényl)thièn-2-yl)-propyl)phénoxy)-2-méthylpropanoate de tertiobutyle

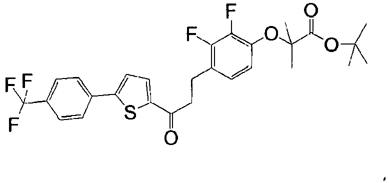

Le 2-(2,3-difluoro-4-(3-oxo-3-(5-(4-(trifluorométhyl)phényl)thièn-2-yl)propyl)phénoxy)-2-méthylpropanoate de tertiobutyle est préparé à partir de la 3-(2,3-difluoro-4-hydroxyphényl)-1-(5-(4-(trifluorométhyl)phényl)thièn-2-yl)propan-1-one et du bromoisobutyrate de tertiobutyle selon la procédure générale D.

Après 4 heures d'agitation, le milieu est acidifié à l'aide d'une solution diluée d'acide citrique 1 N puis extrait par du dichlorométhane. La phase organique est séchée sur sulfate de magnésium, filtrée puis concentrée sous pression réduite. Le résidu d'évaporation est purifié par chromatographie flash sur gel de silice.

Elution : cyclohexane/acétate d'éthyle : 92/8. Silice 40-63µm.

RMN ¹H (300MHz, CDCl₃, δ en ppm) : 1,46 (s, 9H); 1,56 (s, 6H); 3,08 (t, 2H, J=7,0Hz); 3,22 (t, 2H, J=7,0Hz); 6,70 (m, 1 H); 6,86 (m, 1H); 7,38 (d, 1H, J=4,1 Hz); 7,67 (m, 3H); 7,75 (d, 2H, J=8,2Hz).

### Composé 46 : Acide 2-(2,3-difluoro-4-(3-oxo-3-(5-(4-(trifluorométhyl)phényl)thièn-2-yl)propyl)phénoxy)-2-méthylpropanoïque

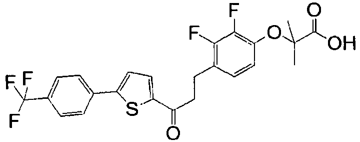

L'acide 2-(2,3-difluoro-4-(3-oxo-3-(5-(4-(trifluorométhyl)phényl)thièn-2-yl)propyl)-phénoxy)-2-méthylpropanoïque est préparé à partir du 2-(2,3-difluoro-4-(3-oxo-3-(5-(4-(trifluorométhyl)phényl)thièn-2-yl)propyl)phénoxy)-2-méthylpropanoate de tertiobutyle selon la procédure générale E au moyen de 15 équivalents d'acide trifluoroacétique.

Après 18 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice.

Elution : dichlorométhane/méthanol: 9/1. Silice 40-63µm.

RMN ¹H (300MHz, CDCl₃, δ en ppm) : 1,59 (s, 6H); 3,11 (t, 2H, J=7,6Hz); 3,25 (t, 2H, J=7,6Hz); 6,80 (t, 1 H, J=8,2Hz); 6,96 (t, 1 H, J=8,2Hz); 7,39 (d, 1 H, J=3,8Hz); 7,68 (m, 3H); 7,76 (d, 2H, J=8,2Hz).

Masse (MALDI-TOF) : 499 (M+1), 516 (M+NH4⁺), 521 (M+Na⁺).

F=165-166°C.

### Composé 47 : 2-(2,6-diméthyl-4-(3-oxo-3-(5-(4-(trifluorométhyl)phényl)thièn-2-yl)-propyl)phénoxy)-2-méthylpropanoate de tertiobutyle

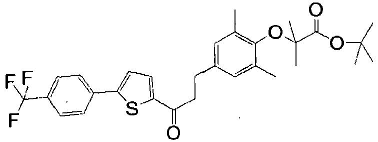

Le 2-(2,6-diméthyl-4-(3-oxo-3-(5-(4-(trifluorométhyl)phényl)thièn-2-yl)propyl)-phénoxy)-2-méthylpropanoate de tertiobutyle est préparé à partir de la 3-(4-hydroxy-3,5-diméthylphényl)-1-(5-(4-(trifluorométhyl)phényl)thièn-2-yl)propan-1-one selon la procédure générale D, au moyen de 15 équivalents de bromoisobutyrate de tertiobutyle et de 15 équivalents de carbonate de potassium ajoutés par portions de 3 équivalents en cours de réaction.

Après 4 jours d'agitation à 100°C, les solvants sont éliminés par évaporation sous pression réduite. Le résidu d'évaporation est repris à l'acétate d'éthyle et lavé par une solution de chlorure d'ammonium saturée. Le résidu d'évaporation est purifié par chromatographie flash sur gel de silice.

Elution : cyclohexane/acétate d'éthyle : 95/5 à 9/1. Silice 40-63µm.

RMN ¹H (300MHz, CDCl₃, δ en ppm) : 1,41 (s, 9H); 1,51 (s, 6H); 2,20 (s, 6H); 2,92-2,97 (m, 2H); 3,15-3,21 (m, 2H); 6,83 (s, 2H); 7,37 (d, 1H, J=3,9Hz); 7,65 (d, 1 H, J=3,9Hz); 7,67 (d, 2H, J=8,4Hz); 7,75 (d, 2H, J=8,4Hz).

### Composé 48 : Acide 2-(2,6-diméthyl-4-(3-oxo-3-(5-(4-(trifluorométhyl)phényl)thièn-2-yl)propyl)phénoxy)-2-méthylpropanoïque

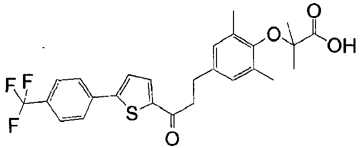

L'acide 2-(2,6-diméthyl-4-(3-oxo-3-(5-(4-(trifluorométhyl)phényl)thièn-2-yl)propyl)-phénoxy)-2-méthylpropanoïque est préparé à partir du -(2,6-diméthyl-4-(3-oxo-3-(5-(4-(trifluorométhyl)phényl)thièn-2-yl)propyl)phénoxy)-2-méthylpropanoate de tertiobutyle selon la procédure générale E au moyen de 10 équivalents d'acide trifluoroacétique.

Après 48 heures d'agitation à température ambiante, les solvants sont éliminés par évaporation sous pression réduite. Le résidu d'évaporation est purifié par chromatographie sur gel de silice.

Elution : dichlorométhane/méthanol : 97/3 à 0/100. Silice 40-63µm.

RMN ¹H (300MHz, CDCl₃, δ en ppm) : 1,51 (s, 6H); 2,22 (s, 6H); 2,96 (t, 2H, J=7,5Hz); 3,19 (t, 2H, J=7,5Hz); 6,88 (s, 2H); 7,37 (d, 1 H, J=3,9Hz); 7,65-7,67 (m, 3H); 7,74 (d, 2H, J=8,4Hz).

Masse (ES⁻) : 489 (M-1).

F=157-158°C.

### Composé 49 : Acide 2-(2,3-dichloro-4-(3-(hydroxyimino)-3-(5-(4-(trifluorométhyl)-phényl)thièn-2-yl)propyl)phénoxy)-2-méthylpropanoïque

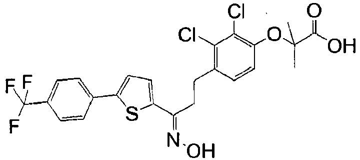

L'acide 2-(2,3-dichloro-4-(3-(hydroxyimino)-3-(5-(4-(trifluorométhyl)phényl)thièn-2-yl)propyl)phénoxy)-2-méthylpropanoïque est préparé à partir de l'acide 2-(2,3-dichloro-4-(3-oxo-3-(5-(4-(trifluorométhyl)phényl)thièn-2-yl)propyl)phénoxy)-2-méthyl propanoïque et de chlorhydrate d'hydroxylamine selon la procédure générale I.

Elution : dichlorométhane/méthanol : 98/2 à 95/5. Silice 40-63µm.

### Conformation majoritaire :

RMN ¹H (300MHz, MeOD, δ en ppm) : 1,39 (s, 6H); 2,92-2,97 (m, 4H); 6,7 (d, 1 H, J=8,4Hz); 6,99 (d, 1H, J=8,4Hz); 7,03 (d, 1H, J=3,9Hz); 7,28 (d, 1H, J=3,9Hz); 7,57 (d, 2H, J=8,4Hz); 7,69 (d, 2H, J=8,4Hz).

### Conformation minoritaire:

RMN ¹H (300MHz, MeOD, δ en ppm) : 1,47 (s, 6H); 2,82-3,01 (m, 4H); 6,81 (d, 1 H, J=8,4Hz); 7,03 (d, 1 H, J=8,4Hz); 7,43 (d, 1H, J=4,2Hz); 7,48 (d, 1H, J=4,2Hz); 7,60 (d, 2H, J=8,2Hz); 7,78 (d, 2H, J=8,2Hz).

Masse (ES⁻) : 544/546 (M-1).

F=135-136°C.

### Composé 50: Acide 2-(2,3-dichloro-4-(3-(méthoxyimino)-3-(5-(4-(trifluorométhyl)-phényl)thièn-2-yl)propyl)phénoxy)-2-méthylpropanoïque

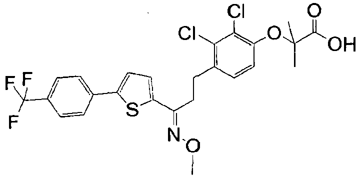

L'acide 2-(2,3-dichloro-4-(3-(méthoxyimino)-3-(5-(4-(trifluorométhyl)phényl)thièn-2-yl)propyl)phénoxy)-2-méthylpropanoïque est préparé à partir de l'acide 2-(2,3-dichloro-4-(3-oxo-3-(5-(4-(trifluorométhyl)phényl)thièn-2-yl)propyl)phénoxy)-2-méthyl propanoïque et de chlorhydrate de O-méthylhydroxylamine selon la procédure générale I.

Elution : dichlorométhane/méthanol : 98/2. Silice 40-63µm.

### Conformation majoritaire :

RMN ¹H (300MHz, MeOD, δ en ppm) : 1,58 (s, 6H); 2,91-3,17 (m, 4H); 3,96 (s, 3H); 6,91 (d, 1 H, J=8,4Hz); 7,11 (d, 1 H, J=8,4Hz); 7,12 (d, 1 H, J=3,9Hz); 7,24 (d, 1 H, J=3,9Hz); 7,61 (d, 2H, J=8,4Hz); 7,67 (d, 2H, J=8,4Hz).

### Conformation minoritaire:

RMN ¹H (300MHz, MeOD, δ en ppm) : 1,64 (s, 6H); 2,95-3,15 (m, 4H); 4,07 (s, 3H); 6,94 (d, 1 H, J=8,4Hz); 7,05 (d, 1 H, J=8,4Hz); 7,36 (d, 1 H, J=4,2Hz); 7,51 (d, 1 H, J=4,2Hz); 7,64 (d, 2H, J=8,2Hz); 7,76 (d, 2H, J=8,2Hz).

Masse (ES⁻) : 558/560 (M-1).

F=68-69°C.

### Composé 51 : 2-(4-(3-(5-(4-bromophényl)thièn-2-yl)-3-oxopropyl)-2,3-dichloro-phénoxy)-2-méthylpropanoate de tertiobutyle

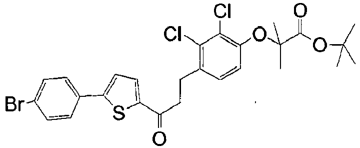

Le 2-(4-(3-(5-(4-bromophényl)thièn-2-yl)-3-oxopropyl)-2,3-dichlorophénoxy)-2-méthyl-propanoate de tertiobutyle est préparé à partir de la 1-(5-(4-bromophényl)thièn-2-yl)-3-(2,3-dichloro-4-hydroxyphényl)propan-1-one selon la procédure générale D, au moyen de 4 équivalents de bromoisobutyrate de tertiobutyle et de 5 équivalents de carbonate de potassium.

Après 16 heures d'agitation à 80°C, le milieu est dilué par une solution de chlorure d'ammonium saturée et extrait par de l'acétate d'éthyle. La phase organique est séchée sur sulfate de magnésium, filtrée puis concentrée sous pression réduite. Le résidu d'évaporation est purifié par chromatographie flash sur gel de silice.

Elution : cyclohexane/acétate d'éthyle : 95/5. Silice 40-63µm.

RMN ¹H (300MHz, CDCl₃, δ en ppm) : 1,44 (s, 9H); 1,58 (s, 6H); 3,12-3,25 (m, 4H); 6,79 (d, 1H, J=8,6Hz); 7,08 (d, 1H, J=8,6Hz); 7,28 (d, 1H, J=4,1Hz); 7,49 (d, 2H, J=9,1 Hz); 7,54 (d, 2H, J=9,1 Hz); 7,64 (d, 1 H, J=4,2Hz).

### Composé 52: Acide 2-(4-(3-(5-(4-bromophényl)thièn-2-yl)-3-oxopropyl)-2,3-dichloro-phénoxy)-2-méthylpropanoïque

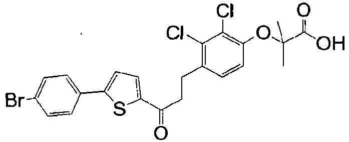

L'acide 2-(4-(3-(5-(4-bromophényl)thièn-2-yl)-3-oxopropyl)-2,3-dichlorophénoxy)-2-méthylpropanoïque est préparé à partir du 2-(4-(3-(5-(4-bromophényl)thièn-2-yl)-3-oxopropyl)-2,3-dichlorophénoxy)-2-méthylpropanoate de tertiobutyle selon la procédure générale E au moyen de 38 équivalents d'acide trifluoroacétique. Après 1 heure d'agitation à température ambiante, les solvants sont éliminés par évaporation sous pression réduite. Le résidu d'évaporation est purifié par chromatographie sur gel de silice.

Elution : dichlorométhane/méthanol : 97/3 à 0/100. Silice 40-63µm.

RMN ¹H (300MHz, MeOD, δ en ppm) : 1,58 (s, 6H); 3,11-3,19 (m, 2H); 3,23-3,30 (m, 2H); 6,93 (d, 1H, J=8,7Hz); 7,21 (d, 1 H, J=8,7Hz); 7,49 (d, 1 H, J=4,1 Hz); 7,61 (d, 2H, J=8,7Hz); 7,66 (d, 2H, J=8,7Hz); 7,81 (d, 1 H, J=4,1 Hz).

Masse (ES⁻) : 539 / 541 / 542 / 543 (M-1).

F=155-157°C.

### Composé 53: 2-(2,3-dichloro-4-(3-(5-(4-(méthylthio)phényl)thièn-2-yl)-3-oxopropyl)-phénoxy)-2-méthylpropanoate de tertiobutyle

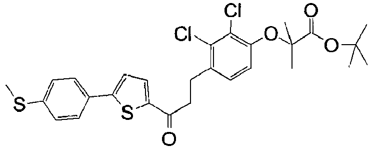

Le 2-(2,3-dichloro-4-(3-(5-(4-(méthylthio)phényl)thièn-2-yl)-3-oxopropyl)phénoxy)-2-méthylpropanoate de tertiobutyle est préparé à partir de la 3-(2,3-dichloro-4-hydroxyphényl)-1-(5-(4-(méthylthio)phényl)thièn-2-yl)propan-1-one solubilisée dans du tétrahydrofurane, selon la procédure générale D.

Après 20 heures d'agitation à 70°C, le milieu est dilué par une solution de chlorure d'ammonium saturée et extrait par de l'acétate d'éthyle. La phase organique est séchée sur sulfate de magnésium, filtrée puis concentrée sous pression réduite. Le résidu d'évaporation est purifié par chromatographie flash sur gel de silice.

Elution : cyclohexane/acétate d'éthyle : 9/1. Silice 40-63µm.

RMN ¹H (300MHz, CDCl₃, δ en ppm) : 1,44 (s, 9H); 1,59 (s, 6H); 2,52 (s, 3H); 3,16-3,23 (m, 4H); 6,95 (d, 1 H, J=8,5Hz); 7,21 (d, 1 H, J=8,5Hz); 7,27-7,30 (m, 3H); 7,56 (d, 2H, J=8,5Hz); 7,66 (d, 1 H, J=4,1 Hz).

### Composé 54: Acide 2-(2,3-dichloro-4-(3-(5-(4-(méthylthio)phényl)thièn-2-yl)-3-oxo-propyl)phénoxy)-2-méthylpropanoïque

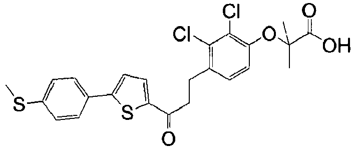

L'acide 2-(2,3-dichloro-4-(3-(5-(4-(méthylthio)phényl)thièn-2-yl)-3-oxopropyl)-phénoxy)-2-méthylpropanoïque est préparé à partir du 2-(2,3-dichloro-4-(3-(5-(4-(méthylthio)phényl)thièn-2-yl)-3-oxopropyl)phénoxy)-2-méthylpropanoate de tertiobutyle selon la procédure générale E au moyen de 10 équivalents d'acide trifluoroacétique.

Après 18 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice.

Elution : dichlorométhane/méthanol: 9/1. Silice 40-63µm.

RMN ¹H (300MHz, CDCl₃, δ en ppm) : 1,63 (s, 6H); 2,53 (s, 3H); 3,18-3,25 (m, 4H); 6,95 (d, 1 H, J=8,5Hz); 7,20 (d, 1 H, J=8,5Hz); 7,26-7,29 (m, 3H); 7,57 (d, 2H, J=8,5Hz); 7,65 (d, 1 H, J=4,1 Hz).

Masse (ES⁻) : 507 / 508 (M-1).

F=171-172°C.

### Composé 55: Acide 2-(2,3-dichloro-4-(3-isopropoxy-3-(5-(4-(trifluorométhyl)-phényl)thièn-2-yl)propyl)phénoxy)-2-méthylpropanoïque

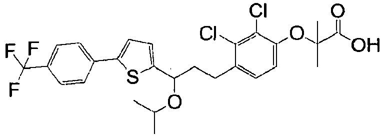

L'acide 2-(2,3-dichloro-4-(3-isopropoxy-3-(5-(4-(trifluorométhyl)phényl)thièn-2-yl)propyl)phénoxy)-2-méthylpropanoïque est préparé à partir de l'acide 2-(2,3-dichloro-4-(3-hydroxy-3-(5-(4-(trifluorométhyl)phényl)thièn-2-yl)propyl)phénoxy)-2-méthylpropanoïque au moyen de 5 équivalents d'hydrure de sodium et de 4 équivalents de 2-bromopropane selon la procédure générale H.

Après 12 heures d'agitation à 70 °C, le milieu réactionnel est dilué par de l'eau, acidifié par une solution d'acide chlorhydrique 0,5N et, extrait par de l'acétate d'éthyle. La phase organique est concentrée sous pression réduite. Le résidu d'évaporation purifié par chromatographie flash sur gel de silice.

Elution : cyclohexane/acétate d'éthyle : 8/2. Silice 40-63µm.

RMN ¹H (300MHz, CDCl₃, δ en ppm) : 1,24 (d, 6H, J=6,3Hz); 1,59 (s, 6H); 2,12-2,23 (m, 2H); 2,76-2,97 (m, 2H); 4,91-4,95 (m, 1H); 5,09 (sep, 1H, J=6,3); 6,78 (d, 1 H, J=8,6Hz); 6,98 (d, 1H, J=3,7Hz); 7,02 (d, 1H, J=8,6Hz); 7,24 (d, 1H, J=3,7Hz); 7,61 (d, 2H, J=8,6Hz); 7,66 (d, 2H, J=8,6Hz).

Masse (ES⁺) : 557 / 559 (M+1), 592 / 594 (NH₄)⁺, 597 / 599 (M+Na)⁺. F=88-90°C.

### Composé 56: 2-(2,3-dichloro-4-(3-oxo-3-(5-phénylthièn-2-yl)propyl)phénoxy)-2-méthylpropanoate de tertiobutyle

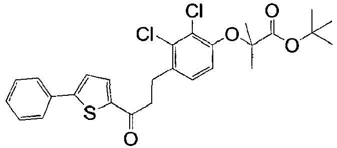

Le 2-(2,3-dichloro-4-(3-oxo-3-(5-phénylthièn-2-yl)propyl)phénoxy)-2-méthyl-propanoate de tertiobutyle est préparé à partir de la 3-(2,3-dichloro-4-hydroxyphényl)-1-(5-phénylthièn-2-yl)propan-1-one solubilisée dans du tétrahydrofurane, selon la procédure générale D.

Après 12 heures d'agitation à 80°C, le milieu est dilué par une solution de chlorure d'ammonium saturée et extrait par de l'acétate d'éthyle. La phase organique est concentrée sous pression réduite. Le résidu d'évaporation est lavé par du cyclohexane.

RMN ¹H (300MHz, CDCl₃, δ en ppm) : 1,44 (s, 9H); 1,57 (s, 6H); 3,11-3,25 (m, 4H); 6,79 (d, 1 H, J=8,4Hz); 7,09 (d, 1 H, J=8,4Hz); 7,31 (d, 1 H, J=3,9Hz); 7,34-7,44 (m, 3H); 7,63-7,66 (m, 3H).

### Composé 57: Acide 2-(2,3-dichloro-4-(3-oxo-3-(5-phénylthièn-2-yl)propyl)phénoxy)-2-méthylpropanoïque

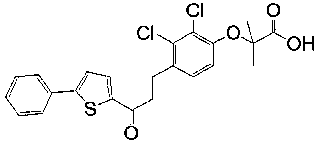

L'acide 2-(2,3-dichloro-4-(3-oxo-3-(5-phénylthièn-2-yl)propyl)phénoxy)-2-méthyl-propanoïque est préparé à partir du 2-(2,3-dichloro-4-(3-oxo-3-(5-phénylthièn-2-yl)propyl)phénoxy)-2-méthylpropanoate de tertiobutyle selon la procédure générale E au moyen de 12 équivalents d'acide trifluoroacétique.

Après 12 heures d'agitation à température ambiante, le milieu réactionnel est lavé avec de l'eau puis le dichlorométhane est éliminé par évaporation sous pression réduite.

RMN ¹H (300MHz, CDCl₃, δ en ppm) : 1,63 (s, 6H); 3,13-3,28 (m, 4H); 6,94 (d, 1 H, J=8,6Hz); 7,18 (d, 1H, J=8,6Hz); 7,31 (d, 1H, J=4,2Hz); 7,35-7,46 (m, 3H); 7,61-7,67 (m, 3H).

Masse (ES⁻) : 461 / 463 (M-1).

F=179-180°C.

### Composé 58 : 2-méthyl-2-(2-méthyl-4-(3-oxo-3-(5-(4-(trifluorométhyl)phényl)thièn-2-yl)propyl)phénoxy) propanoate de tertiobutyle

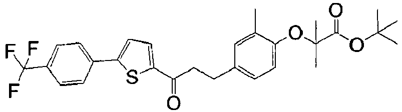

Le 2-méthyl-2-(2-méthyl-4-(3-oxo-3-(5-(4-(trifluorométhyl)phényl)thièn-2-yl)propyl)-phénoxy) propanoate de tertiobutyle est préparé à partir de la 3-(4-hydroxy-3-méthylphényl)-1-(5-(4-(trifluorométhyl)phényl)thièn-2-yl)propan-1-one solubilisée dans du tétrahydrofurane, selon la procédure générale D.

Après 12 heures d'agitation à 70°C, le milieu est dilué par une solution de chlorure d'ammonium saturée et extrait par de l'acétate d'éthyle. La phase organique est concentrée sous pression réduite. Le résidu d'évaporation purifié par chromatographie flash sur gel de silice.

Elution : cyclohexane/acétate d'éthyle : 8/2. Silice 40-63µm.

RMN ¹H (300MHz, CDCl₃, δ en ppm) : 1,45 (s, 9H); 1,55 (s, 6H); 2,23 (s, 3H); 2,93-2,98 (m, 2H); 3,17-3,22 (m, 2H); 6,79 (d, 1 H, J=8,4Hz); 6,98-7,01 (m, 1 H); 7,08 (m, 1 H); 7,39 (d, 1 H, J=3,9Hz); 7,66-7,72 (m, 3H); 7,75 (d, 2H, J=8,4Hz).

### Composé 59: Acide 2-méthyl-2-(2-méthyl-4-(3'oxo-3-(5-(4-(trifluorométhyl)phényl)-thièn-2-yl)propyl)phénoxy)propanoïque

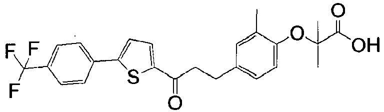

L'acide 2-méthyl-2-(2-méthyl-4-(3-oxo-3-(5-(4-(trifluorométhyl)phényl)thièn-2-yl)-propyl)phénoxy)propanoïque est préparé à partir du 2-méthyl-2-(2-méthyl-4-(3-oxo-3-(5-(4-(trifluorométhyl)phényl)thièn-2-yl)propyl)phénoxy) propanoate de tertiobutyle selon la procédure générale E au moyen de 10 équivalents d'acide trifluoroacétique.

Après 25 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice.

Elution : dichlorométhane/méthanol: 9/1. Silice 40-63µm.

RMN ¹H (300MHz, CDCl₃, δ en ppm) : 1,60 (s, 6H); 2,24 (s, 3H); 3,02 (t, 2H, J=8,2Hz); 3,21 (t, 2H, J=8,2Hz); 6,79 (d, 1H, J=8,4Hz); 6,98-7,01 (m, 1 H); 7,08 (s, 1 H); 7,39 (d, 1 H, J=4,0Hz); 7,67-7,70 (m, 3H); 7,76 (d, 2H, J=8,4Hz).

Masse (ES⁻) : 475 (M-1).

F=130-131°C.

### Composé 60 : Acide 2-(2,3-dichloro-4-(3-hydroxy-3-(4-(4-(trifluorométhyl)phényl)-thièn-2-yl)propyl)phénoxy)-2-méthylpropanoïque

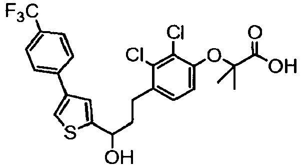

L'acide 2-(2,3-dichloro-4-(3-hydroxy-3-(4-(4-(trifluorométhyl)phényl)thièn-2-yl)propyl)-phénoxy)-2-méthylpropanoïque est préparé à partir de l'acide 2-(2,3-dichloro-4-(3-oxo-3-(4-(4-(trifluorométhyl)phényl)thièn-2-yl)propyl)phénoxy)-2-méthylpropanoïque selon la procédure générale G. Le résidu d'évaporation est utilisé tel quel pour la réalisation de l'étape suivante.

RMN ¹H (300MHz, CDCl₃, δ en ppm) : 1,64 (s, 6H); 2,14-2,22 (m, 2H); 3,01-2,78 (m, 2H); 4,99 (t, 1 H, J=6,5Hz); 6,92 (d, 1 H, J=8,6Hz); 7,07 (d, 1 H, J=8,6Hz); 7,29 (d, 1 H, J=1,2Hz); 7,45 (d, 1H, J=1,2Hz); 7,61-7,67 (m, 4H).

### Composé 61 : Acide 2-(4-(3-(benzyloxy)-3-(4-(4-(trifluorométhyl)phényl)thièn-2-yl)propyl)-2,3-dichlorophénoxy)-2-méthylpropanoïque

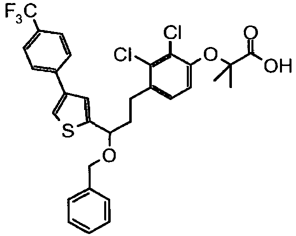

L'acide 2-(4-(3-(benzyloxy)-3-(4-(4-(trifluorométhyl)phényl)thièn-2-yl)propyl)-2,3-dichlorophénoxy)-2-méthylpropanoïque est préparé à partir de l'acide 2-(2,3-dichloro-4-(3-hydroxy-3-(4-(4-(trifluorométhyl)phényl)thièn-2-yl)propyl)phénoxy)-2-méthylpropanoïque au moyen de 2,2 équivalents d'hydrure de sodium et de 2,2 équivalents de bromure de benzyle selon la procédure générale H.

Le résidu d'évaporation est solubilisé dans de l'éthanol en présence de soude 2N (20 éq.). Après 16 heures sous agitation, les solvants sont éliminés par évaporation sous pression réduite. Le résidu d'évaporation est acidifié à l'aide d'une solution diluée d'acide chlorhydrique puis extrait par du dichlorométhane. La phase organique est séchée sur sulfate de magnésium, filtrée puis concentrée sous pression réduite. Le résidu d'évaporation est purifié par chromatographie sur gel de silice.

Elution : cyclohexane/acétate d'éthyle 7/3 puis dichlorométhane/méthanol: 9/1. Silice 40-63µm.

RMN ¹H (300MHz, CDCl₃, δ en ppm) : 1,62 (s, 6H); 2,08-2,18 (m, 1H); 2,21-2,29 (m, 1 H); 2,75-2,85 (m, 1 H); 3,01-2,92 (m, 1 H); 4,40 (t, 1 H, J=11,7Hz); 4,62-4,65 (m, 2H); 6,90 (d, 1 H, J=8,5Hz); 7,01 (d, 1 H, J=8,5Hz); 7,29-7,37 (m, 6H); 7,51 (d, 1 H, J=1,2Hz); 7,67-7,70 (m, 4H).

Masse (ES⁻) : 621 / 623 (M-1).

F=58-59°C.

### Composé 62 : Acide 2-(2,3-difluoro-4-(3-hydroxy-3-(5-(4-(trifluorométhyl)phényl)-thièn-2-yl)propyl)phénoxy)-2-méthylpropanoïque

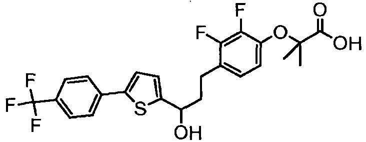

L'acide 2-(2,3-difluoro-4-(3-hydroxy-3-(5-(4-(trifluorométhyl)phényl)thièn-2-yl)propyl)-phénoxy)-2-méthylpropanoïque est préparé à partir de l'acide 2-(2,3-difluoro-4-(3-oxo-3-(5-(4-(trifluorométhyl)phényl)thièn-2-yl)propyl)phénoxy)-2-méthylpropanoïque selon la procédure générale G. Le résidu d'évaporation est utilisé tel quel pour la réalisation de l'étape suivante.

RMN ¹H (300MHz, CDCl₃, δ en ppm) : 1,56 (s, 6H); 2,14-2,23 (m, 2H); 2,74-2,87 (m, 2H); 4,95 (t, 1 H, J=7,4Hz); 6,79 (m, 2H); 6,99 (d, 1 H, J=3,8Hz); 7,25 (d, 1 H, J=3,7Hz); 7,62 (d, 2H, J=8,6Hz); 7,64 (d, 2H, J=8,6Hz).

### Composé 63 : Acide 2-(4-(3-(benzyloxy)-3-(5-(4-(trifluorométhyl)phényl)thièn-2-yl)-propyl)-2,3-difluorophénoxy)-2-méthylpropanoïque

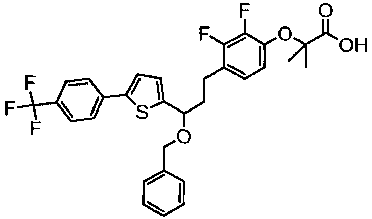

L'acide 2-(4-(3-(benzyloxy)-3-(5-(4-(trifluorométhyl)phényl)thièn-2-yl)propyl)-2,3-difluorophénoxy)-2-méthylpropanoïque est préparé à partir de l'acide 2-(4-(3-(benzyloxy)-3-(5-(4-(trifluorométhyl)phényl)thièn-2-yl)propyl)-2,3-difluorophénoxy)-2-méthylpropanoïque au moyen de 2,2 équivalents d'hydrure de sodium et de 2,2 équivalents de bromure de benzyle selon la procédure générale H.

Le résidu d'évaporation est purifié par par chromatographie sur gel de silice (Elution : cyclohexane/acétate d'éthyle : 9/1. Silice 40-63µm.). L'huile isolée est solubilisée dans de l'éthanol en présence de soude 2N (20 éq.). Après 16 heures sous agitation, les solvants sont éliminés par évaporation sous pression réduite. Le résidu d'évaporation est acidifié à l'aide d'une solution diluée d'acide chlorhydrique puis extrait par du dichlorométhane. La phase organique est concentrée sous pression réduite. Le résidu d'évaporation est purifié par chromatographie sur gel de silice.

Elution : cyclohexane/acétate d'éthyle 7/3 puis dichlorométhane/méthanol: 9/1. Silice 40-63µm.

RMN ¹H (300MHz, CDCl₃, δ en ppm) : 1,59 (s, 6H); 2,03-2,33 (m, 2H); 2,63-2,82 (m, 2H); 4,37 (d, 1H, J=11,7Hz); 4,58 (m, 2H); 6,77 (m, 2H); 6,98 (d, 1H, J=3,5Hz); 7,30 (m, 6H); 7,63 (d, 2H, J=8,5Hz); 7,70 (d, 2H, J=8,5Hz).

Masse (ES⁻) : 589 (M-1).

Aspect : huile visqueuse.

### Composé 64 : 2-(4-(3-oxo-3-(5-(4-(trifluorométhyl)phényl)thièn-2-yl)propyl)phénoxy)-butanoate de tertiobutyle

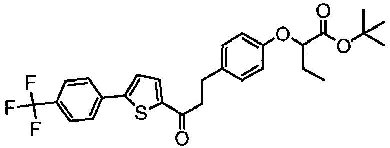

Le 2-(4-(3-oxo-3-(5-(4-(trifluorométhyl)phényl)thièn-2-yl)propyl)phénoxy)butanoate de tertiobutyle est préparé à partir de la 3-(4-hydroxyphényl)-1-(5-(4-(trifluorométhyl)-phényl)thièn-2-yl)propan-1-one et de 2-bromobutanoate de tertiobutyle selon la procédure générale D.

Après 16 heures d'agitation, le solvant est éliminé par évaporation sous pression réduite. Le résidu d'évaporation est repris par de l'acétate d'éthyle. La phase organique est lavée par une solution saturée de chlorure d'ammonium, séchée sur sulfate de magnésium, filtrée puis concentrée sous pression réduite. Le résidu d'évaporation est utilisé tel quel pour la réalisation de l'étape suivante.

### Composé 65 : Acide 2-(4-(3-oxo-3-(5-(4-(trifluorométhyl)phényl)thièn-2-yl)propyl)-phénoxy)butanoïque

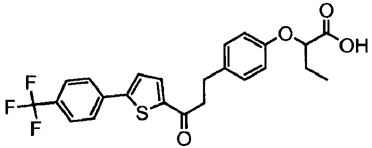

L'acide 2-(4-(3-oxo-3-(5-(4-(trifluorométhyl)phényl)thièn-2-yl)propyl)phénoxy)-butanoïque est préparé à partir du 2-(4-(3-oxo-3-(5-(4-(trifluorométhyl)phényl)thièn-2-yl)propyl)phénoxy)butanoate de tertiobutyle selon la procédure générale E au moyen de 10 équivalents d'acide trifluoroacétique. Après 12 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice (HPLC préparative, lichrospher (Merck) RP18 12µm 100A, colonne : 25*250 mm).

Elution : gradient eau, méthanol + 0,1% acide trifluoroacétique : 30/70 à 0/100. Elution : dichlorométhane/méthanol: 9/1. Silice 40-63µm.

RMN ¹H (300MHz, CDCl₃, δ en ppm) : 1,10 (t, 3H, J=7,3Hz); 1,98-2,08 (m, 2H); 3,03 (t, 2H, J=7,5Hz); 3,18-3,24 (m, 2H); 4,61 (t, 1H, J=6,0Hz); 6,86 (d, 2H, J=8,8Hz); 7,18 (d, 2H, J=8,8Hz); 7,38 (d, 1H, J=3,8Hz); 7,65-7,69 (m, 3H); 7,76 (d, 2H, J=8,5Hz).

Masse (ES⁺) : 461 (M+1).

F=116-117°C.

### Composé 66: 2-méthyl-2-(4-(3-oxo-3-(5-(4-(trifluorométhyl)phényl)thièn-2-yl)propyl)-phénoxy)propanoate de tertiobutyle

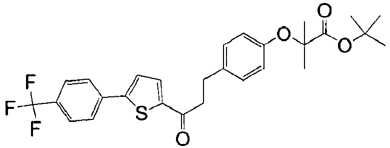

Le 2-méthyl-2-(4-(3-oxo-3-(5-(4-(trifluorométhyl)phényl)thièn-2-yl)propyl)phénoxy)-propanoate de tertiobutyle est préparé à partir de la 3-(4-hydroxyphényl)-1-(5-(4-(trifluorométhyl)phényl)thièn-2-yl)propan-1-one et de 2-bromoisobutyrate de tertiobutyle selon la procédure générale D.

Après 16 heures d'agitation, le solvant est éliminé par évaporation sous pression réduite. Le résidu d'évaporation est repris par de l'acétate d'éthyle. La phase organique est lavée par une solution saturée de chlorure d'ammonium, séchée sur sulfate de magnésium, filtrée puis concentrée sous pression réduite. Le résidu d'évaporation est utilisé tel quel pour la réalisation de l'étape suivante.

### Composé 67 : Acide 2-(4-(3-oxo-3-(5-(4-(trifluorométhyl)phényl)thièn-2-yl)propyl)-phénoxy)-2-méthylpropanoïque

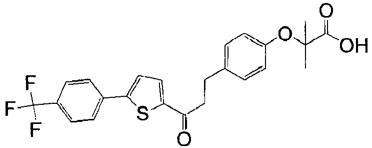

L'acide 2-(4-(3-oxo-3-(5-(4-(trifluorométhyl)phényl)thièn-2-yl)propyl)phénoxy)-2-méthylpropanoïque est préparé à partir du 2-méthyl-2-(4-(3-oxo-3-(5-(4-(trifluorométhyl)phényl)thièn-2-yl)propyl)phénoxy)propanoate de tertiobutyle selon la procédure générale E au moyen de 10 équivalents d'acide trifluoroacétique.

Après 12 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice (HPLC préparative, lichrospher (Merck) RP18 12µm 100A, colonne : 25*250 mm).

Elution : gradient eau, méthanol + 0,1% acide trifluoroacétique : 30/70 à 0/100. RMN ¹H (300MHz, CDCl₃, δ en ppm) : 1,57 (s, 6H); 3,06 (t, 2H, J=7,9Hz); 3,23 (t, 2H, J=6,7Hz); 6,89 (d, 2H, J=8,5Hz); 7,18 (d, 2H, J=8,5Hz); 7,38 (d, 1H, J=4,1 Hz); 7,66-7,69 (m, 3H); 7,75 (d, 2H, J=8,2Hz).

Masse (ES⁺) : 463 (M+1).

F=154-155°C.

### Composé 68 : 2-(4-(3-oxo-3-(5-(4-(trifluorométhyl)phényl)thièn-2-yl)propyl)phénoxy)-acétate de tertiobutyle

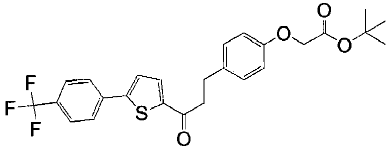

Le 2-méthyl-2-(4-(3-oxo-3-(5-(4-(triffuorométhyl)phényl)thièn-2-yl)propyl)phénoxy)-propanoate de tertiobutyle est préparé à partir de la 3-(4-hydroxyphényl)-1-(5-(4-(trifluorométhyl)phényl)thièn-2-yl)propan-1-one et de bromoacétate de tertiobutyle selon la procédure générale D.

Après 18 heures d'agitation, le milieu est acidifié par une solution d'acide citrique et extrait par de l'acétate d'éthyle. La phase organique est lavée par une solution saturée de chlorure d'ammonium, séchée sur sulfate de magnésium, filtrée puis concentrée sous pression réduite. Le résidu d'évaporation est purifié par chromatographie sur gel de silice.

Elution : cylohexane/acétate d'éthyle: 85/15.

RMN ¹H (300MHz, CDCl₃, δ en ppm) : 1,49 (s, 9H); 3,01-3,06 (m, 2H); 3,18-3,23 (m, 2H); 4,50 (s, 2H); 6,85 (d, 2H, J=8,5Hz); 7,17 (d, 2H, J=8,5Hz); 7,39 (d, 1 H, J=4,1 Hz); 7,65-7,69 (m, 3H); 7,76 (d, 2H, J=8,4Hz).

### Composé 69: Acide 2-(4-(3-oxo-3-(5-(4-(trifluorométhyl)phényl)thièn-2-yl)propyl)-phénoxy)acétique

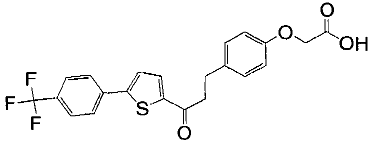

L'acide 2-(4-(3-oxo-3-(5-(4-(trifluorométhyl)phényl)thièn-2-yl)propyl)phénoxy)acétique est préparé à partir du 2-(4-(3-oxo-3-(5-(4-(trifluorométhyl)phényl)thièn-2-yl)propyl)phénoxy)acétate de tertiobutyle selon la procédure générale E au moyen de 10 équivalents d'acide trifluoroacétique. Après 12 heures d'agitation à température ambiante, le milieu réactionnel est lavé avec de l'eau puis le dichlorométhane est éliminé par évaporation sous pression réduite. Le résidu d'évaporation est purifié par chromatographie sur gel de silice.

Elution : dichlorométhane/méthanol: 9/1. Silice 40-63µm.

RMN ¹H (300MHz, CDCl₃, δ en ppm) : 3,03-3,08 (m, 2H); 3,19-3,24 (m, 2H); 4,66 (s, 2H); 6,89 (d, 2H, J=8,8Hz); 7,22 (d, 2H, J=8,8Hz); 7,38 (d, 1 H J=4,1 Hz); 7,66-7,69 (m, 3H); 7,76 (d, 2H, J=8,5Hz).

Masse (ES⁺) : 435 (M+1).

F=182-183°C.

### Composé 70: Acide 2-(4-(3-(benzyloxy)-3-(5-(4-(trifluorométhyl)phényl)thièn-2-yl)propyl)-2-fluorophénoxy)butanoïque

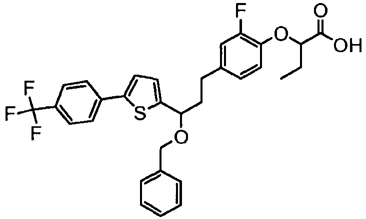

L'acide 2-(4-(3-(benzyloxy)-3-(5-(4-(trifluorométhyl)phényl)thièn-2-yl)propyl)-2-fluoro-phénoxy)butanoïque est préparé à partir de l'acide 2-(2-fluoro-4-(3-hydroxy-3-(5-(4-(trifluorométhyl)phényl)thièn-2-yl)propyl)phénoxy)butanoïque au moyen de 2,1 équivalents d'hydrure de sodium et de 2,1 équivalents de bromure de benzyle selon la procédure générale H.

Le résidu d'évaporation est purifié par chromatographie sur gel de silice (Elution : cyclohexane/acétate d'éthyle : 95/5 à 8/2. Silice 40-63µm.). L'huile isolée est solubilisée dans de l'éthanol en présence de soude 2N (20 éq.). Après 18 heures sous agitation, les solvants sont éliminés par évaporation sous pression réduite. Le résidu d'évaporation est acidifié à l'aide d'une solution diluée d'acide chlorhydrique puis extrait par du dichlorométhane. La phase organique est concentrée sous pression réduite. Le résidu d'évaporation est purifié par chromatographie sur gel de silice (HPLC préparative, lichrospher (Merck) RP18 12µm 100A, colonne : 25*250 mm). Elution : gradient eau, méthanol + 0,1% acide trifluoroacétique : 28/72 à 10/90.

RMN ¹H (300MHz, CDCl₃, δ en ppm) : 1,13 (t, 3H, J=7,3Hz); 2,01-2,11 (m, 3H); 2,23-2,34 (m, 1H); 2,58-2,98 (m, 2H); 4,32 (d, 1H, J=11,7Hz); 4,52 (dd, 1H, J=5,6Hz, J=7,6Hz); 4,59-4,63 (m, 2H); 6,79-6,92 (m, 3H); 6,97 (d, 1H, J=3,5Hz); 7,26-7,27 (m, 1H); 7,29-7,39 (m, 5H); 7,62 (d, 2H, J=8,5Hz); 7,70 (d, 2H, J=8,5Hz).

Masse (ES⁺) : 573 (M+1).

Aspect : huile visqueuse.

### Composé 71 : Acide 2-(2,3-dichloro-4-(3-hydroxy-3-(5-(4-(trifluorométhyl)phényl)fur-2-yl)propyl)phénoxy)-2-méthylpropanoïque

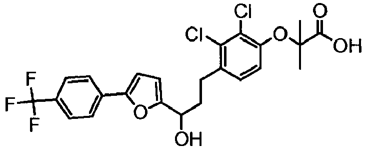

L'acide 2-(2,3-dichloro-4-(3-hydroxy-3-(5-(4-(trifluorométhyl)phényl)fur-2-yl)propyl)-phénoxy)-2-méthylpropanoïque est préparé à partir de l'acide 2-(2,3-dichloro-4-(3-oxo-3-(5-(4-(trifluorométhyl)phényl)fur-2-yl)propyl)phénoxy)-2-méthylpropanoïque selon la procédure générale G. Le résidu d'évaporation est utilisé tel quel pour la réalisation de l'étape suivante.

### Composé 72: Acide 2-(2,3-dichloro-4-(3-méthoxy-3-(5-(4-(trifluorométhyl)phényl)fur-2-yl)propyl)phénoxy)-2-méthylpropanoïque

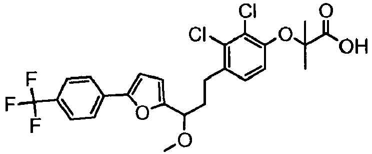

L'acide 2-(2,3-dichloro-4-(3-méthoxy-3-(5-(4-(trifluorométhyl)phényl)fur-2-yl)propyl)-phénoxy)-2-méthylpropanoïque est préparé à partir de l'acide 2-(2,3-dichloro-4-(3-hydroxy-3-(5-(4-(trifluorométhyl)phényl)fur-2-yl)propyl)phénoxy)-2-méthylpropanoïque au moyen de 2,1 équivalents d'hydrure de sodium et de 2,1 équivalents de iodométhane selon la procédure générale H.

Le résidu d'évaporation est purifié par chromatographie sur gel de silice (Elution : cyclohexane/acétate d'éthyle : 9/1. Silice 40-63µm.). L'huile isolée est solubilisée dans de l'éthanol en présence de soude 2N (5 éq.). Après 18 heures sous agitation, les solvants sont éliminés par évaporation sous pression réduite. Le résidu d'évaporation est acidifié à l'aide d'une solution diluée d'acide chlorhydrique puis extrait par de l'acétate d'éthyle. La phase organique est concentrée sous pression réduite.

RMN ¹H (300MHz, CDCl₃, δ en ppm) : 1,63 (s, 6H); 2,11-2,32 (m, 2H); 2,75-2,97 (m, 2H); 3,34 (s, 3H); 4,25 (dd, 1H, J=5,8Hz J=7,6Hz); 6,42 (d, 1 H, J=3,4Hz); 6,73 (d, 1 H, J=3,4Hz); 6,93 (d, 1 H, J=8,5Hz); 7,05 (d, 1 H, J=8,5Hz); 7,62 (d, 2H, J=8,3Hz); 7,75 (d, 2H, J=8,3Hz).

Masse (ES⁻) : 529 / 531 (M-1).

Aspect : huile visqueuse.

### Composé 73: Acide 2-(4-(3-hydroxy-3-(5-(4-(trifluorométhyl)phényl)thièn-2-yl)-propyl)-2,6-diméthylphénoxy)-2-méthylpropanoïque

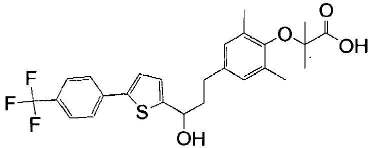

L'acide 2-(4-(3-hydroxy-3-(5-(4-(trifluorométhyl)phényl)thièn-2-yl)propyl)-2,6-diméthyl-phénoxy)-2-méthylpropanoïque est préparé à partir de l'acide 2-(2,6-diméthyl-4-(3-oxo-3-(5-(4-(trifluorométhyl)phényl)thièn-2-yl)propyl)phénoxy)-2-méthylpropanoïque selon la procédure générale G. Le résidu d'évaporation est utilisé tel quel pour la réalisation de l'étape suivante.

RMN ¹H (300MHz, CDCl₃, δ en ppm) : 1,51 (s, 6H); 2,07-2,28 (m, 8H); 2,56-2,76 (m, 2H); 4,90-4,95 (m, 1 H); 6,84 (s, 2H); 6,97 (d, 1 H, J=3,7Hz); 7,25 (d, 1 H, J=3,7Hz); 7,61 (d, 2H, J=8,6Hz); 7,66 (d, 2H, J=8,6Hz).

### Composé 74: Acide 2-(4-(3-méthoxy-3-(5-(4-(trifluorométhyl)phényl)thièn-2-yl)propyl)-2,6-diméthylphénoxy)-2-méthylpropanoïque

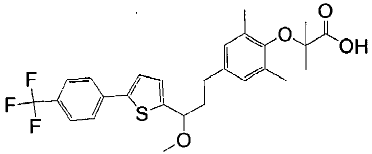

L'acide 2-(4-(3-méthoxy-3-(5-(4-(trifluorométhyl)phényl)thièn-2-yl)propyl)-2,6-diméthylphénoxy)-2-méthylpropanoïque est préparé à partir de l'acide 2-(4-(3-hydroxy-3-(5-(4-(trifluorométhyl)phényl)thièn-2-yl)propyl)-2,6-diméthylphénoxy)-2-méthylpropanoïque au moyen de 2,1 équivalents d'hydrure de sodium et de 2,1 équivalents de iodométhane selon la procédure générale H.

Le résidu d'évaporation est purifié par chromatographie sur gel de silice (Elution : cyclohexane/acétate d'éthyle : 9/1. Silice 40-63µm.). L'huile isolée est solubilisée dans de l'éthanol en présence de soude 2N (5 éq.). Après 18 heures sous agitation, les solvants sont éliminés par évaporation sous pression réduite. Le résidu d'évaporation est acidifié à l'aide d'une solution diluée d'acide chlorhydrique puis extrait par de l'acétate d'éthyle. La phase organique est concentrée sous pression réduite.

RMN ¹H (300MHz, MeOD, δ en ppm) : 1,43 (s, 6H); 1,91-2,06 (m, 1H); 2,11-2,23 (m, 7H); 2,48-2,68 (m, 2H); 3,28 (s, 3H); 4,37 (t, 1 H, J=6,7Hz); 6,81 (s, 2H); 7,01 (d, 1 H, J=3,8Hz); 7,41 (d, 1 H, J=3,8Hz); 7,67 (d, 2H, J=8,2Hz); 7,80 (d, 2H, J=8,2Hz).

Masse (ES⁺) : 524 (M+NH₄⁺), 530 (M+Na⁺), 545 (M+K⁺).

Aspect : huile visqueuse.

### Composé 75 : 2-(2,3-dichloro-4-(3-oxo-3-(5-(3-(trifluorométhyl)phényl)thièn-2-yl)-propyl)phénoxy)-2-méthylpropanoate d'éthyle

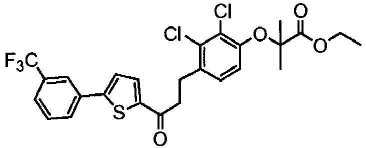

Le 2-(2,3-dichloro-4-(3-oxo-3-(5-(3-(trifluorométhyl)phényl)thièn-2-yl)propyl)phénoxy)-2-méthylpropanoate d'éthyle est préparé à partir de la 3-(2,3-dichloro-4-hydroxy-phényl)-1-(5-(3-(trifluorométhyl)phényl)thièn-2-yl)propan-1-one et de bromo-isobutyrate d'éthyle selon la procédure générale D.

Après 16 heures d'agitation, le milieu est acidifié par une solution d'acide citrique et extrait par de l'acétate d'éthyle. La phase organique est lavée par une solution saturée de chlorure d'ammonium, séchée sur sulfate de magnésium, filtrée puis concentrée sous pression réduite. Le résidu d'évaporation est purifié par chromatographie sur gel de silice.

Elution : cylohexane/acétate d'éthyle: 100/0 à 8/2.

RMN ¹H (300MHz, CDCl₃, δ en ppm) : 1,27 (t, 3H, J=7,0Hz); 1,61 (s, 6H); 3,18-3,24 (m, 4H); 4,25 (q, 2H, J=7,0Hz); 6,77 (d, 1H, J=8,4Hz); 7,11 (d, 1H, J=8,4Hz); 7,38 (d, 1H, J=3,9Hz); 7,56 (m, 1H); 7,63 (d, 1H, J=8,2Hz); 7,68 (d, 1 H, J=3,9Hz); 7,82 (d, 1 H, J=7,6Hz); 7,88 (s, 1 H).

### Composé 76 : Acide 2-(2,3-dichloro-4-(3-oxo-3-(5-(3-(trifluorométhyl)phényl)thièn-2-yl)propyl)phénoxy)-2-méthylpropanoïque

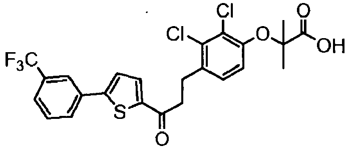

L'acide 2-(2,3-dichloro-4-(3-oxo-3-(5-(3-(trifluorométhyl)phényl)thièn-2-yl)propyl)-phénoxy)-2-méthylpropanoïque est préparé à partir du 2-(2,3-dichloro-4-(3-oxo-3-(5-(3-(trifluorométhyl)phényl)thièn-2-yl)propyl)phénoxy)-2-méthylpropanoate d'éthyle selon la procédure générale F au moyen de 10 équivalents d'une solution de soude 2N.

Après 16 heures à température ambiante, le milieu réactionnel est concentré sous pression réduite, acidifié à l'aide d'une solution diluée d'acide chlorhydrique puis extrait par du dichlorométhane. La phase organique est séchée sur sulfate de magnésium, filtrée puis concentrée sous pression réduite. Le résidu d'évaporation est purifié par chromatographie sur gel de silice.

Elution : dichlorométhane/méthanol: 9/1. Silice 40-63µm.

RMN ¹H (300MHz, CDCl₃, δ en ppm) : 1,64 (s, 6H); 3,18-3,28 (m, 4H); 6,95 (d, 1 H, J=8,4Hz); 7,19 (d, 1 H, J=8,4Hz); 7,37 (d, 1 H, J=3,9Hz); 7,56 (m, 1 H); 7,63 (d, 1 H, J=7,6Hz); 7,69 (d, 1 H, J=3,9Hz); 7,82 (d, 1 H, J=7,6Hz); 7,88 (s, 1H).

### Composé 77 : 2-(2,3-dichloro-4-(3-hydroxy-3-(5-(3-(trifluorométhyl)phényl)thièn-2-yl)-propyl)phénoxy)-2-méthylpropanoate d'éthyle

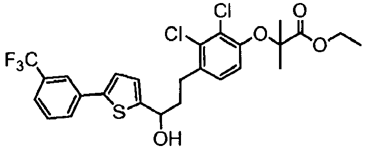

Le 2-(2,3-dichloro-4-(3-hydroxy-3-(5-(3-(trifluorométhyl)phényl)thièn-2-yl)propyl)-phénoxy)-2-méthylpropanoate d'éthyle est préparé à partir du 2-(2,3-dichloro-4-(3-oxo-3-(5-(3-(trifluorométhyl)phényl)thièn-2-yl)propyl)phénoxy)-2-méthylpropanoate d'éthyle selon la procédure générale G. Le résidu d'évaporation est utilisé tel quel pour la réalisation de l'étape suivante.

RMN ¹H (300MHz, CDCl₃, δ en ppm) : 1,28 (t, 3H, J=7,1Hz); 1,62 (s, 6H); 2,14-2,22 (m, 2H); 2,81-2,97 (m, 2H); 4,27 (q, 2H, J=7,1Hz); 4,95 (t, 1H, J=6,4Hz); 6,78 (d, 1 H, J=8,6Hz); 7,00 (d, 1 H, J=3,5Hz); 7,05 (d, 1 H, J=8,6Hz); 7,24 (d, 1H, J=3,5Hz); 7,47-7,55 (m, 2H); 7,75 (d, 1 H, J=7,1 Hz); 7,82 (s, 1 H).

### Composé 78 : 2-(2,3-dichloro-4-(3-méthoxy-3-(5-(3-(trifluorométhyl)phényl) thièn-2-yl)propyl)phénoxy)-2-méthylpropanoate d'éthyle

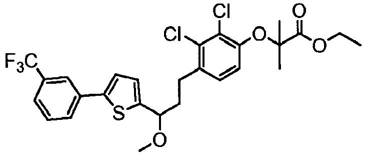

Le 2-(2,3-dichloro-4-(3-méthoxy-3-(5-(3-(trifluorométhyl)phényl)thièn-2-yl)propyl)-phénoxy)-2-méthylpropanoate d'éthyle est préparé à partir du 2-(2,3-dichloro-4-(3-hydroxy-3-(5-(3-(trifluorométhyl)phényl)thièn-2-yl)propyl)phénoxy)-2-méthylpropanoate d'éthyle au moyen de 1,1 équivalents d'hydrure de sodium et de 1,1 équivalents de iodométhane selon la procédure générale H.

Après 1 heure d'agitation à température ambiante, le milieu réactionnel est hydrolysé et extrait par de l'acétate d'éthyle. La phase organique est concentrée sous pression réduite et le résidu d'évaporation est purifié par chromatographie sur gel de silice.

Elution : cyclohexane/acétate d'éthyle: 9/1. Silice 40-63µm.

RMN ¹H (300MHz, CDCl₃, δ en ppm) : 1,28 (t, 3H, J=7,1Hz); 1,61 (s, 6H); 2,02-2,23 (m, 2H); 2,71-2,94 (m, 2H); 4,25 (q, 2H, J=7,1 Hz); 3,32 (s, 3H); 4,32-4,36 (m, 1 H); 6,73 (d, 1 H, J=8,5Hz); 6,96 (d, 1 H, J=3,5Hz); 7,01 (d, 1H, J=8,5Hz); 7,24 (d, 1 H, J=3,5Hz); 7,48-7,50 (m, 2H); 7,74 (d, 1 H, J=7,3Hz); 7,81 (s, 1 H).

### Composé 79: Acide 2-(2,3-dichloro-4-(3-hydroxy-3-(5-(4-(trifluorométhoxy)phényl)-thièn-2-yl)propyl)phénoxy)-2-méthylpropanoïque

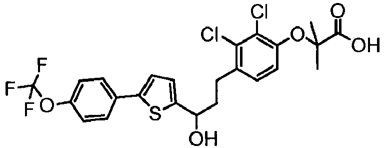

L'acide 2-(2,3-dichloro-4-(3-hydroxy-3-(5-(4-(trifluorométhoxy)phényl)thièn-2-yl)-propyl)phénoxy)-2-méthylpropanoïque est préparé à partir de l'acide 2-(2,3-dichloro-4-(3-oxo-3-(5-(4-(trifluorométhoxy)phényl)thièn-2-yl)propyl)phénoxy)-2-méthylpropanoïque selon la procédure générale G. Le résidu d'évaporation est utilisé tel quel pour la réalisation de l'étape suivante.

### Composé 80 : Acide 2-(2,3-dichloro-4-(3-méthoxy-3-(5-(4-(trifluorométhoxy)phényl)-thièn-2-yl)propyl)phénoxy)-2-méthylpropanoïque

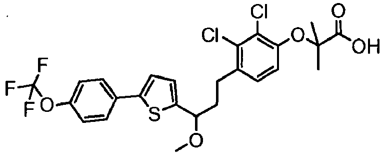

L'acide 2-(2,3-dichloro-4-(3-méthoxy-3-(5-(4-(trifluorométhoxy)phényl)thièn-2-yl)-propyl)phénoxy)-2-méthylpropanoïque est préparé à partir de l'acide 2-(2,3-dichloro-4-(3-hydroxy-3-(5-(4-(trifluorométhoxy)phényl)thièn-2-yl)propyl)phénoxy)-2-méthylpropanoïque au moyen de 2,1 équivalents d'hydrure de sodium et de 2,1 équivalents de iodométhane selon la procédure générale H. Le résidu d'évaporation est purifié par chromatographie sur gel de silice (Elution : cyclohexane/acétate d'éthyle : 9/1. Silice 40-63µm.). L'huile isolée est solubilisée dans de l'éthanol en présence de soude 2N (6 éq.). Après 18 heures sous agitation, les solvants sont éliminés par évaporation sous pression réduite. Le résidu d'évaporation est acidifié à l'aide d'une solution diluée d'acide chlorhydrique puis extrait par de l'acétate d'éthyle. La phase organique est concentrée sous pression réduite.

RMN ¹H (300MHz, CDCl₃, δ en ppm) : 1,60 (s, 6H); 1,98-2,29 (m, 2H); 2,72-2,97 (m, 2H); 3,32 (s, 3H); 4,35 (t, 1 H, J=6,5Hz); 6,89-6,97 (m, 2H); 7,01-7,08 (m, 1 H); 7,16 (d, 1 H, J=3,5Hz); 7,22 (d, 2H, J=8,6Hz); 7,59 (d, 2H, J=8,6Hz).

Masse (ES⁺) : 563/565 (M+1).

Aspect : huile visqueuse.

### Exemple 5 : Evaluation in vitro des propriétés activatrices de PPAR des composés selon l'invention - Méthode 1

### Principe

L'activation des PPAR a été évaluée *in vitro* sur une lignée de fibroblastes de rein de singe (COS-7) par la mesure de l'activité transcriptionnelle de chimères constituées du domaine de liaison à l'ADN du facteur de transcription Gal4 de la levure et du domaine de liaison au ligand des différents PPAR. Les composés ont été testés à des doses comprises entre 10⁻⁵ et 100µM sur les chimères Gal4-PPARα, γ ou δ, et les EC50 correspondants ont été déterminées.

### Protocole

### Culture des cellules

Les cellules COS-7 proviennent de l'ATCC et ont été cultivées dans du milieu DMEM supplémenté de 10% (vol/vol) de sérum de veau foetal, 100 U/ml de pénicilline (Gibco, Paisley, UK) et 2 mM de L-Glutamine (Gibco, Paisley, UK). Les cellules ont été incubées à 37°C dans une atmosphère humide contenant 5% de CO₂.

### Description des plasmides utilisés en transfection

Les plasmides Gal4(RE)_TkpGL3, pGal4-hPPARα, pGal4-hPPARγ, pGal4-PPARδ et pGal4-φ ont été décrits dans la littérature (Raspe E *et al.,* 1999). Les constructions pGal4-hPPARα, pGal4-hPPARγ et pGal4-hPPARδ ont été obtenues par clonage dans le vecteur pGal4-φ de fragments d'ADN amplifiés par PCR correspondants aux domaines DEF des récepteurs nucléaires PPARα, PPARγ et PPARδ humains.

### Transfection

Les cellules COS-7 en suspension ont été transfectées avec 150 ng d'ADN par puits, avec un ratio pGal4-PPAR / Gal4(RE)_TkpGL3 de 1/10, en présence de 10% sérum de veau foetal. Les cellules ont ensuite été ensemencées dans des plaques de 96 puits (4x10⁴ cellules/puits) puis incubées pendant 24 heures à 37°C. L'activation avec les composés à tester s'effectue pendant 24h à 37°C dans du milieu sans sérum. A l'issue de l'expérience, les cellules ont été lysées et l'activité luciférase a été déterminée à l'aide du Steady-Lite™ HTS (Perkin Elmer) selon les recommandations du fournisseur.

### Résultats

De manière inattendue, les inventeurs ont mis en évidence une augmentation significative et dose-dépendante de l'activité luciférase dans les cellules transfectées avec les plasmides pGal4-hPPAR et traitées avec les composés selon l'invention. Les données expérimentales sont résumées dans le tableau 1 ci-après, qui indique les EC50 mesurées pour chaque isoforme de PPAR, ainsi que le pourcentage de réponse maximale atteint par chaque composé au regard de la référence (Acide fénofibrique pour PPARα, Rosiglitazone pour PPARγ et GW501516 pour PPARδ).

Les activités mesurées diffèrent selon le composé testé et on observe aussi parmi les composés de l'invention plus ou moins de sélectivité vis-à-vis des isoformes de PPAR :
- certains composés selon l'invention sont sélectifs par rapport à un sous-type de PPAR,
- d'autres composés selon l'invention sont simultanément activateurs de deux ou trois sous-types.

**Tableau 1**

| | hPPARalpha | | hPPARgamma | | hPPARdelta | |
|---|---|---|---|---|---|---|
| Composé n° | % max | EC₅₀ µM | % max | EC₅₀ µM | % max | EC₅₀ µM |
| 2 | 43 | 0,053 | 11 | 0,06 | 91 | 0,007 |
| 4 | 50 | 0,125 | 38 | ND | 107 | 0,034 |
| 9 | 54 | 0,006 | 41 | 0,28 | 84 | 0,019 |
| 11 | 39 | 0,288 | 55 | 1,17 | 105 | 0,003 |
| 13 | 20 | 0,977 | 58 | 0,49 | 1 | ND |
| 15 | 46 | 0,163 | 32 | 5,66 | 88 | 0,253 |
| 17 | 5 | 0,228 | 27 | 0,19 | 0 | ND |
| 19 | 47 | 0,001 | 27 | 0,18 | 85 | 0,015 |
| 21 | 50 | 0,017 | 63 | 0,08 | 97 | 0,008 |
| 23 | 48 | 0,000 | 49 | 0,40 | 95 | 0,016 |
| 27 | 47 | 1,314 | 30 | 2,82 | 77 | 0,589 |
| 29 | 56 | 0,002 | 65 | 1,27 | 88 | 0,037 |
| 32 | 52 | 0,008 | 67 | 0,21 | 75 | 0,036 |
| 34 | 61 | 0,060 | 37 | ND | 95 | 0,114 |
| 36 | 47 | 0,005 | 63 | ND | 85 | 0,009 |
| 44 | 54 | 0,016 | 46 | 0,10 | 109 | 0,027 |
| 46 | 58 | 0,011 | 30 | ND | 105 | 0,018 |
| 48 | 49 | 0,000 | 85 | 0,56 | 86 | 0,006 |
| 49 | 46 | 0,043 | 24 | 0,66 | 93 | 0,045 |
| 61 | 42 | 0,591 | 78 | 0,03 | 101 | 0,006 |
| 65 | 52 | 0,004 | 73 | ND | 79 | 0,109 |
| 69 | 61 | 0,033 | 15 | 3,53 | 90 | 0,292 |
| 70 | 47 | 0,227 | 56 | 0,97 | 67 | 0,216 |

### Conclusion :

Ces résultats montrent que les composés selon l'invention lient et activent les récepteurs hPPARα, hPPARγ, et/ou hPPARδ de manière significative. L'activité des composés selon l'invention est variable en fonction de la structure chimique du composé testé et selon le sous-type de PPAR étudié.

### Exemple 6 : Evaluation in vitro des propriétés activatrices de PPAR de composés selon l'invention - Méthode 2

Dans cet exemple, le principe, les cellules et les plasmides utilisés pour l'exemple 5 ont été repris. Seule la mise en oeuvre de l'étape de transfection diffère quelque peu. Elle est ci-après détaillée.

Les cellules COS-7 adhérentes sont transfectées avec 40µg d'ADN par boîte de 225 cm², avec un ratio pGal4-PPAR / Gat4(RE)_TkpGL3 de 1/10, en présence de 10% de sérum de veau foetal. Les cellules sont ensuite détachées et ensemencées en absence de sérum dans des plaques de 384 puits (2x10⁴ cellules/puits) et incubées pendant 4 heures à 37°C. Les composés sont ensuite dilués dans une plaque 96 puits et transférés dans la plaque 384 puits. L'activation avec les composés à tester s'effectue pendant 24h supplémentaires à 37°C en présence de 1% de sérum synthétique Ultroser™ (Biosepra) dépourvu de lipides. Ces 2 dernières étapes sont automatisées à l'aide d'une station Genesis Freedom 200™ (Tecan). A l'issue de l'expérience, les cellules sont lysées et l'activité luciférase est déterminée à l'aide du Steady-Lite™ HTS (Perkin Elmer) selon les recommandations du fournisseur.

### Résultats

De manière inattendue, les inventeurs ont mis en évidence une augmentation significative et dose-dépendante de l'activité luciférase dans les cellules transfectées avec les plasmides pGal4-hPPAR et traitées avec les composés selon l'invention. Les données expérimentales sont résumées dans le tableau 2 ci-après qui indique les EC50 mesurées pour chaque isoforme de PPAR, ainsi que le pourcentage de réponse maximale atteint par chaque composé au regard de la référence (Acide fénofibrique pour PPARα, Rosiglitazone pour PPARγ et GW501516 pour PPARδ).

Les activités mesurées diffèrent selon le composé testé et on observe aussi parmi les composés de l'invention plus ou moins de sélectivité vis-à-vis des isoformes PPAR :
- certains composés selon l'invention sont sélectifs par rapport à un sous-type de PPAR,
- d'autres composés selon l'invention sont simultanément activateurs de deux ou trois sous-types.

**Tableau 2**

| | hPPARalpha | | hPPARgamma | | hPPARdelta | |
|---|---|---|---|---|---|---|
| Composé n° | % max | EC₅₀ µM | % max | EC₅₀ µM | % max | EC₅₀ µM |
| 6 | 41 | 7,86 | 55 | 4,11 | 92 | 0,53 |
| 7 | 20 | 5,46 | 40 | 1,70 | 92 | 0,03 |
| 25 | 55 | 1,68 | 27 | 36,47 | 75 | 7,45 |
| 30 | 51 | 0,05 | 45 | 3,93 | 80 | 1,53 |
| 38 | 44 | 0,20 | 37 | 1,84 | 89 | 0,014 |
| 38 | 40 | 0,32 | 39 | 0,81 | 94 | 0,009 |
| 39 | 36 | 1,01 | 52 | 0,55 | 90 | 0,006 |
| 40 | 35 | 0,59 | 32 | 0,99 | 78,29 | 0,005 |
| 41 | 49 | 0,61 | 36 | 1,49 | 76 | 0,01 |
| 42 | 28 | 2,00 | 47 | 0,89 | 82,12 | 0,26 |
| 50 | 38 | 0,26 | 32 | 0,27 | 78 | 0,05 |
| 52 | 53 | 0,21 | 57 | 1,02 | 104 | 0,05 |
| 54 | 43 | 0,15 | 62 | 0,86 | 107 | 0,07 |
| 55 | 25 | 2,80 | 2 | ND | 34 | 4,71 |
| 57 | 44 | 1,00 | 39 | 1,52 | 88 | 0,35 |
| 59 | 47 | 0,00 | 57 | 0,23 | 107 | 0,03 |
| 63 | 46 | 4,21 | 44 | 7,91 | 97 | 0,18 |
| 67 | 45 | 0,01 | 37 | 4,82 | 75 | 0,36 |
| 72 | 59 | 0,27 | 70 | 0,16 | 97 | ND |

### Conclusion :

Ces résultats montrent que les composés selon l'invention lient et activent les récepteurs hPPARα, hPPARγ, et/ou hPPARδ de manière significative. L'activité des composés selon l'invention est variable en fonction de la structure chimique du composé testé et selon le sous-type de PPAR étudié.

### Exemple 7 : Evaluation in vitro des propriétés activatrices de PPARδ des composés selon l'invention par mesure de l'expression de gènes cibles de PPARδ dans des myocytes murins

### Principe

Les effets stimulateurs du métabolisme lipidique, glucidique et de la dépense énergétique des composés selon l'invention ont été évalués par la mesure de l'expression de la Pyruvate Dehydrogenase Kinase 4 (PDK4), de la Carnitine Palmotoyl Transferase 1 b (CPT1 b), de l'Uncoupling Protein 2 (UCP2) et de l'Uncoupling Protein 3 (UCP3) par des myocytes murins traités pendant 24 heures avec les composés selon l'invention. Il est établi que la régulation de l'expression de ces gènes est directement contrôlée par PPARδ dans ce type cellulaire. Plus l'expression des gènes est augmentée, plus le composé selon l'invention est stimulateur du métabolisme dans les cellules musculaires.

### Protocole

### Différenciation des cellules C2C12 en myocytes

Les cellules issues de la lignée cellulaire murine C2C12 (provenance ECACC) sont cultivées dans du milieu DMEM (Gibco ; 41965-039) additionné de 1% de L-Glutamine (Gibco; 25030), de 1% de pénicilline/streptomycine (VWR; BWSTL0022/100) et 10% de sérum de veau foetal décomplémenté (SVF. Gibco ; 10270-106).

Les cellules sont ensemencées sur des plaques de 24 puits à la densité de 50.10³ cellules/puits. A confluence, le milieu est remplacé par un milieu de différenciation (milieu de culture de base additionné de 2% de sérum de cheval (Gibco ; 26050-088)) puis incubées à 37°C et 5% de CO₂ pendant 4 jours afin de les différencier en myocytes.

### Traitement

Après 5 jours de différenciation, les cellules sont placées dans un milieu de déprivation (milieu de culture de base sans sérum) pendant 6 heures. Les cellules sont ensuite traitées avec les composés selon l'invention dans le milieu de culture de déprivation. Les composés 2, 4, 7, 11, 39, 40, 46, 49 et 72 ont été testés en effet dose, correspondant à 1x, 10x et 100x leur EC50 PPARδ. Les composés selon l'invention ont été dissous dans du diméthyl sulfoxide (DMSO, Sigma ; D5879). Les cellules ont été traitées pendant 24h à 37°C, 5% CO2. L'effet des composés selon l'invention ont été comparés à l'effet du DMSO seul.

### Extraction des ARN, transcription inverse et PCR quantitative.

Après traitement, l'ARN total est extrait des cellules en utilisant le kit NucleoSpin^{®} 96 RNA (Macherey Nagel, Hoerdt, France) selon les instructions du fabricant.

1µg d'ARN total (quantifié par lecture au spectrophotomètre UV) a ensuite été retro-transcrit en ADN complémentaire par une réaction d'1 heure à 37°C dans un volume total de 30µl contenant du tampon 1X (Invitrogen), 1,5mM de DTT, 0,18mM de dNTPs (Promega), 200ng de pdN6 (Amersham), 30U d'inhibiteur de RNase (Promega) et 1µl de MMLV-RT (Invitrogen).

Les expériences de PCR quantitative ont été effectuées en utilisant le MyiQ Single-Color Real-Time PCR Détection System (Biorad, Marnes-la-Coquette, France) et ont été réalisées en utilisant le kit iQ SYBR Green Supermix selon les recommandations du fournisseur, en plaques 96 puits, sur 5µl de réactions de retro-transcription diluées avec une température d'hybridation de 60°C. Des paires d'amorces spécifiques des gènes PDK4, CPT1 b, UCP2 et UCP3 étudiés ont été utilisées :
- mPDK4 : amorce sens : 5'-TACTCCACTGCTCCAACACCTG-3' (SEQ ID n°11) et amorce antisens 5'-GTTCTTCGGTTCCCTGCTTG-3' (SEQ ID n°12)
- mCPT1 b : amorce sens : 5'-GGACTGAGACTGTGCGTTCCTG-3' (SEQ ID n°7) et amorce antisens : 5'-AGTGCTTGGCGGATGTGGTT-3' (SEQ ID n°8)
- mUCP2 : amorce sens : 5'- GTCGGAGATACCAGAGCACTGTCG-3' (SEQ ID n°13) et amorce antisens : 5'- CACATCAACAGGGGAGGCGA-3' (SEQ ID n°14)
- mUCP3 : amorce sens : 5'- GCACCGCCAGATGAGTTTTG-3' (SEQ ID n°15) et amorce antisens : 5'- GACGCTGGAGTGGTCCGCTC-3' (SEQ ID n°16)

La quantité de fluorescence émise est directement proportionnelle à la quantité d'ADN complémentaire présent au début de la réaction et amplifié au cours de la PCR. Pour chaque cible étudiée, une gamme est réalisée par des dilutions successives d'un pool constitué de quelques microlitres de différentes réactions de retro-transcription. Les niveaux d'expression relatifs de chaque cible sont ainsi déterminés en utilisant les courbes d'efficacité obtenues avec les points de gamme.

Les niveaux d'expression des gènes d'intérêt ont ensuite été normalisés par rapport au niveau d'expression du gène de référence 36B4 (dont les amorces spécifiques sont : amorce sens : 5'-CATGCTCAACATCTCCCCCTTCTCC-3' (SEQ ID n°19) et amorce antisens : 5'-GGGAAGGTGTAATCCGTCTCCACAG-3' (SEQ ID n°20)). Le facteur d'induction, c'est-à-dire le rapport entre le signal relatif (induit par le composé selon l'invention) et la moyenne des valeurs relatives du groupe contrôle, a ensuite été calculé. Plus ce facteur est élevé, plus les composés selon l'invention ont un caractère activateur de l'expression génique. Le résultat final est représenté comme moyenne des valeurs d'induction dans chaque groupe expérimental.

### Résultats

Les inventeurs ont aussi mis en évidence, sur des myocytes murins *in vitro,* que les composés 2, 4, 7, 11, 39, 40, 46, 48, 63 et 72 possèdent des effets stimulateurs de l'expression de gènes impliqués dans le métabolisme glucidique, lipidique et dans la thermorégulation. Les résultats obtenus sont présentés dans le tableau 3 ci-après. Ces résultats montrent que les composés selon l'invention, dès 1x l'EC50 PPARδ, induisent une augmentation significative et dose dépendante de l'expression de PDK4, CPT1 b, UCP2 et UCP3 dans les myocytes.

**Tableau 3**

| | | | **PDK4** | | | **CPT1b** | | | **UCP2** | | | **UCP3** | | |
|---|---|---|---|---|---|---|---|---|---|---|---|---|---|---|
| Cpd | Dose (µM) | | Induction | SD | Test-t | Induction | SD | Test-t | Induction | SD | Test-t | Induction | SD | Test-t |
| 2 | 1x EC50 | 0,00 6 | 1,62 | 0,24 | *** | 1,81 | 0,40 | * | 3,46 | 0,34 | *** | 4,29 | 0,43 | *** |
| | 10x EC50 | 0,06 | 2,11 | 0,02 | *** | 1,66 | 0,91 | | 3,98 | 0,47 | *** | 4,62 | 1,31 | *** |
| | 100x EC50 | 0,6 | 2,39 | 0,41 | *** | 2,97 | 0,62 | *** | 4,67 | 1,34 | *** | 6,95 | 1,82 | *** |
| 4 | 1x EC50 | 0,02 | 2,19 | 0,08 | *** | 1,78 | 0,34 | *** | 2,79 | 0,27 | *** | 2,45 | 0,25 | ** |
| | 10x EC50 | 0,2 | 2,35 | 0,13 | *** | 1,93 | 0,27 | *** | 2,41 | 0,13 | *** | 2,34 | 0,36 | ** |
| | 100x EC50 | 2 | 2,13 | 0,11 | *** | 1,98 | 0,38 | *** | 2,92 | 0,22 | *** | 2,37 | 0,36 | ** |
| 7 | 1xEC50 | 0,01 | 3,45 | 0,41 | *** | 1,71 | 0,34 | ** | 3,41 | 1,16 | *** | 4,30 | 0,32 | *** |
| | 10x EC50 | 0,1 | 5,30 | 0,56 | *** | 2,14 | 0,41 | *** | 3,95 | 0,88 | *** | 5,09 | 1,32 | *** |
| | 100x EC50 | 1 | 5,14 | 0,57 | *** | 2,50 | 0,62 | *** | 2,14 | 0,27 | *** | 5,24 | 1,45 | *** |
| 11 | 1x EC50 | 0,00 3 | 1,32 | 0,04 | | 0,96 | 0,25 | | 2,15 | 0,49 | *** | 1,45 | 0,53 | |
| | 10x EC50 | 0,03 | 2,85 | 0,42 | *** | 1,42 | 0,29 | | 1,68 | 1,16 | | 2,56 | 0,81 | *** |
| | 100x EC50 | 0,3 | 4,58 | 0,47 | *** | 1,51 | 0,08 | * | 3,85 | 1,06 | *** | 4,96 | 1,02 | *** |
| 39 | 1x EC50 | 0,00 7 | 3,66 | 0,27 | *** | 2,46 | 0,85 | *** | 4,38 | 0,75 | *** | 3,39 | 0,82 | *** |
| | 10x EC50 | 0,07 | 6,22 | 0,63 | *** | 2,16 | 0,39 | ** | 4,56 | 2,07 | *** | 3,85 | 0,87 | *** |
| | 100x EC50 | 0,7 | 5,17 | 0,50 | *** | 3,81 | 0,21 | *** | 5,63 | 1,28 | *** | 5,24 | 0,63 | *** |
| 40 | 1x EC50 | 0,00 6 | 3,79 | 0,14 | *** | 1,94 | 0,55 | ** | 4,86 | 0,36 | *** | 4,12 | 0,41 | *** |
| | 10x EC50 | 0,06 | 4,05 | 0,12 | *** | 2,13 | 0,31 | *** | 3,47 | 0,73 | *** | 2,97 | 0,74 | *** |
| | 100x EC50 | 0,6 | 3,88 | 0,81 | *** | 1,91 | 0,77 | ** | 2,99 | 1,60 | ** | 2,72 | 0,39 | *** |
| 46 | 1x EC50 | 0,01 | 1,81 | 0,04 | *** | 2,03 | 0,36 | *** | 3,27 | 0,64 | *** | 3,38 | 0,92 | *** |
| | 10x EC50 | 0,1 | 2,68 | 0,21 | *** | 2,63 | 0,59 | *** | 5,37 | 0,82 | *** | 5,60 | 0,36 | *** |
| | 100x EC50 | 1 | 2,67 | 0,21 | *** | 2,91 | 0,40 | *** | 4,58 | 0,34 | *** | 5,61 | 1,63 | *** |
| 48 | 1x EC50 | 0,00 6 | 0,84 | 0,08 | | 1,32 | 0,42 | | 1,13 | 0,18 | | 1,42 | 0,08 | * |
| | 10x EC50 | 0,06 | 1,01 | 0,04 | | 1,95 | 0,39 | *** | 2,04 | 0,33 | *** | 1,82 | 0,53 | ** |
| | 100x EC50 | 0,6 | 1,78 | 0,16 | *** | 2,17 | 0,50 | *** | 4,64 | 0,18. | *** | 3,83 | 1,19 | *** |
| 63 | 1x EC50 | 0,06 | 1,86 | 0,14 | *** | 1,84 | 0,65 | ** | 3,36 | 0,41 | *** | 4,60 | 0,29 | *** |
| | 10x EC50 | 0,60 | 2,04 | 0,21 | *** | 2,04 | 0,32 | *** | 4,44 | 0,47 | *** | 5,44 | 0,43 | *** |
| | 100x EC50 | 1,6 | 2,63 | 0,86 | *** | 2,61 | 0,63 | *** | 4,23 | 0,44 | *** | 5,52 | 1,45 | *** |
| 72 | 1x EC50 | 0,01 | 4,01 | 0,30 | *** | 2,24 | 0,25 | *** | 3,09 | 0,87 | *** | 3,17 | 0,68 | *** |
| | 10x EC50 | 0,1 | 5,05 | 0,20 | *** | 2,96 | 0,43 | *** | 4,25 | 0,97 | *** | 3,79 | 0,73 | *** |
| | 100x EC50 | 1 | 5,64 | 0,52 | *** | 3,67 | 0,64 | *** | 4,31 | 1,10 | *** | 4,48 | 0,25 | *** |

### Conclusion

De manière inattendue, les données expérimentales présentées montrent que les composés selon l'invention ont une action métabolique dans les myocytes murins par activation de PPARδ.

### Exemple 8 Evaluation in vivo chez la souris E2/E2, des propriétés hypolipémiantes et stimulatrices de la synthèse de HDL-cholestérol des composés selon l'invention par dosages lipidiques et mesure de l'expression de gènes impliqués dans le métabolisme lipidique et glucidique et la dissipation d'énergie

### Principe

Les propriétés hypolipémiantes et stimulatrices de la synthèse du HDL-cholestérol des composés selon l'invention ont été évaluées *in vivo* par le dosage des lipides plasmatiques, l'analyse de la répartition du cholestérol et des triglycérides dans les différentes fractions lipoprotéiques plasmatiques et par la mesure de l'expression des gènes cibles des PPAR dans le foie et le muscle squelettique après traitement, avec le composé 2 de souris E2/E2 dyslipidémiques.

Le modèle murin utilisé est la souris de type ApoE2/E2, souris transgénique pour l'isoforme E2 de l'apolipoprotéine E humaine (Sullivan PM *et al.,* 1998). Chez l'homme, cette apolipoprotéine, constituant des lipoprotéines de faible et très faible densité (LDL-VLDL), est présente sous trois isoformes E2, E3 et E4. La forme E2 présente une mutation sur un acide aminé en position 158, ce qui affaiblit considérablement l'affinité de cette protéine pour le récepteur aux LDL. La clairance des VLDL est de ce fait quasi nulle. Il se produit alors une accumulation des lipoprotéines de faible densité et une hyperlipidémie mixte dite de type III (cholestérol et triglycérides élevés).

PPARα régule l'expression de gènes impliqués dans le transport des lipides (apolipoprotéines telles que Apo Al, Apo All et Apo CIII, transporteurs membranaires tels que FAT) ou le catabolisme des lipides (ACOX1, CPT-I ou CPT-II, enzymes de la β-oxydation des acides gras). Un traitement par les activateurs de PPARα se traduit donc, chez l'homme comme chez le rongeur, par une diminution des taux circulants de triglycérides et des acides gras libres. La mesure des lipides et acides gras libres plasmatiques après traitement par les composés selon l'invention est donc un indicateur du caractère agoniste des PPAR et donc du caractère hypolipémiant des composés selon l'invention.

Les propriétés agonistes de PPARα des molécules selon l'invention préalablement mesurées *in vitro* doivent se traduire au niveau hépatique par une modulation de l'expression des gènes cibles directement sous le contrôle du récepteur PPARα. Les gènes qui ont été étudiés dans cette expérience sont les gènes codant pour PDK4 (Pyruvate Deshydrogénase Kinase isoforme 4, enzyme du métabolisme glucidique), l'Acox1 (L'Acox1 présent chez la souris correspond au gène de l'ACO chez l'homme (Acyl Co-enzymeA Oxydase, une enzyme clé dans le mécanisme de la β-oxydation des acides gras)) et pour Apo CIII (une apolipoprotéine impliquée dans le métabolisme lipidique).

Un traitement par les activateurs de PPARδ se traduit chez l'homme comme chez le rongeur, par une augmentation du taux de HDL-cholestérol plasmatique. L'analyse de la répartition du cholestérol après traitement par les composés selon l'invention permet donc de mettre en évidence le caractère stimulateur de la synthèse du HDL-cholestérol des composés selon l'invention.

Les propriétés agonistes de PPARδ des molécules selon l'invention préalablement mesurées *in vitro* doivent aussi se traduire au niveau du muscle squelettique par une sur-expression des gènes cibles directement sous le contrôle du récepteur PPARδ. Les gènes qui ont été étudiés dans cette expérience sont les gènes codant pour PDK4 (Pyruvate Deshydrogénase Kinase isoforme 4, enzyme du métabolisme glucidique) et UCP2 (Uncoupling Protein 2, transporteur mitochondrial impliqué dans la thermorégulation).

La mesure de l'activité transcriptionnelle des gènes cibles des PPAR après traitement par les composés selon l'invention est donc aussi un indicateur du caractère hypolipémiant des composés selon l'invention.

### Protocole

### Traitement des animaux

Des souris transgéniques Apo E2/E2 ont été maintenues sous un cycle lumière/obscurité de 12/12 heures à une température constante de 20 ± 3°C. Après une acclimatation d'une semaine, les souris ont été pesées et rassemblées par groupes de 6 animaux sélectionnés de telle sorte que la distribution de leurs poids corporels et de leurs taux de lipides plasmatiques déterminés une première fois avant l'expérience soient uniformes. Les composés testés ont été suspendus dans la carboxyméthylcellulose (Sigma C4888) et administrés par gavage intra-gastrique, à raison d'une fois par jour pendant 13 jours à la dose choisie. Les animaux ont eu un accès libre à l'eau et à la nourriture (régime standard). A l'issue de l'expérience, les animaux ont été anesthésiés après un jeûne de 4 heures, un prélèvement sanguin a été effectué sur anticoagulant (EDTA) puis les souris ont été pesées et euthanasiées. Le plasma a été séparé par centrifugation à 3000 tours/minute pendant 20 minutes, les échantillons ont été conservés à + 4°C.

Des échantillons de foie et de tissu musculaire squelettique ont été prélevés et congelés immédiatement dans de l'azote liquide puis conservés à -80°C pour les analyses ultérieures.

### Analyse de la répartition du cholestérol dans les fractions lipoprotéiques plasmatiques.

Les différentes fractions lipidiques (VLDL, LDL, HDL) du plasma ont été séparées par une chromatographie Gel - Filtration. Les concentrations de cholestérol et de triglycérides ont ensuite été mesurées dans chaque fraction par dosages enzymatiques (bioMérieux-Lyon-France) selon les recommandations du fournisseur.

### Mesure du cholestérol total plasmatique

Les concentrations plasmatiques de cholestérol total ont été mesurées par dosages enzymatiques (bioMérieux-Lyon-France) selon les recommandations du fournisseur.

### Mesure du HDL-cholestérol

Les lipoprotéines de basse densité (VLDL et LDL) ont été précipitées par Phosphotungstate. Le précipité a été éliminé par centrifugation. Le HDL-cholestérol présent dans le surnageant a été quantifié par dosages enzymatiques (bioMérieux-Lyon-France) selon les recommandations du fournisseur.

### Analyse d'expression génique par RT-PCR quantitative

### Tissu hépatique

L'ARN total a été extrait à partir de fragments de foie en utilisant le kit NucleoSpin® 96 RNA (Macherey Nagel, Hoerdt, France) selon les instructions du fabricant.

### Tissu squelettique

L'ARN total a été extrait à partir de fragments de muscle squelettique gastrocnémien en utilisant le kit RNeasy^{®} Fibrous Tissue kit (Qiagen) selon les instructions du fabricant.

1µg d'ARN total (quantifié par spectrophotométrie) a ensuite été reverse transcrit en ADN complémentaire par une réaction d'1 heure à 37°C dans un volume total de 30µl contenant du tampon 1X (Invitrogen), 1,5mM de DTT, 0,18mM de dNTPs (Promega), 200ng de pdN6 (Amersham), 30U d'inhibiteur de RNase (Promega) et 1µl de MMLV-RT (Invitrogen).

Les expériences de PCR quantitative ont été effectuées en utilisant le MyiQ Single-Color Real-Time PCR Detection System (Biorad, Marnes-la-Coquette, France) et ont été réalisées en utilisant le kit iQ SYBR Green Supermix selon les recommandations du fournisseur, en plaques 96 puits, sur 5µl de réaction de reverse transcription diluée avec une température d'hybridation de 60°C. Des paires d'amorces spécifiques des gènes PDK4, Acox1, ApoCIII et UCP2 étudiés ont été utilisées :
- mPDK4 : amorce sens : 5'-TACTCCACTGCTCCAACACCTG-3' (SEQ ID n°11) et amorce antisens 5'-GTTCTTCGGTTCCCTGCTTG-3' (SEQ ID n°12)
- mACOX1 : amorce sens : 5'- GAAGCCAGCGTTACGAGGTG-3' (SEQ ID n°3) et amorce antisens : 5'- TGGAGTTCTTGGGACGGGTG-3' (SEQ ID n°4)
- mApoCIII: amorce sens: 5'-CTCTTGGCTCTCCTGGCATC-3' (SEQ ID n°5) et amorce antisens 5'-GCATCCTGGACCGTCTTGGA-3' (SEQ ID n°6)
- mUCP2 : amorce sens : 5'-GTCGGAGATACCAGAGCACTGTCG-3' (SEQ ID n°13) et amorce antisens : 5'-CACATCAACAGGGGAGGCGA-3' (SEQ ID n°14)

Dans les deux cas (tissu hépatique et tissu musculaire squelettique), la quantité de fluorescence émise est directement proportionnelle à la quantité d'ADN complémentaire présent au début de la réaction et amplifié au cours de la PCR. Pour chaque cible étudiée, une gamme est réalisée par des dilutions successives d'un pool constitué de quelques microlitres de différentes réactions de retro-transcription. Les niveaux d'expression relatifs de chaque cible sont ainsi déterminés en utilisant les courbes d'efficacité obtenues avec les points de gamme.

Les niveaux d'expression des gènes d'intérêt ont ensuite été normalisés, dans le tissu hépatique par rapport au niveau d'expression du gène de référence 36B4 (dont les amorces spécifiques sont : amorce sens : 5'-CATGCTCAACATCTCCCCCTTCTCC-3' (SEQ ID n°19) et amorce antisens : 5'-GGGAAGGTGTAATCCGTCTCCACAG-3' (SEQ ID n°20)) et, dans le tissu musculaire squelettique par rapport au niveau d'expression du gène de référence 18S (dont les amorces spécifiques sont: amorce sens: 5'-CGGACACGGACAGGATTGACAG-3' (SEQ ID n°21) et l'amorce antisens: 5'-AATCTCGGGTGGCTGAACGC-3' (SEQ ID n°22).

Le facteur d'induction, c'est-à-dire le rapport entre le signal relatif (induit par le composé selon l'invention) et la moyenne des valeurs relatives du groupe contrôle, a ensuite été calculé pour chaque échantillon. Plus ce facteur est élevé, plus le composé a un caractère activateur d'expression génique. Le résultat final est représenté comme moyenne des valeurs d'induction dans chaque groupe expérimental.

### Résultats

La figure 1-1 compare les taux de cholestérol total plasmatique après 7 et 13 jours de traitement avec le composé 2 à 50 mpk aux taux obtenus avec les animaux contrôles. De manière inattendue, les taux de cholestérol total plasmatique ont été significativement diminués par le traitement dès 7 jours.
La figure 1-2 compare les taux de HDL-cholestérol plasmatique après 7 et 13 jours de traitement avec le composé 2 à 50 mpk aux taux obtenus avec les animaux contrôles. De manière inattendue, les taux de HDL-cholestérol plasmatique ont été significativement augmentés par le traitement dès 7 jours.
La figure 1-3 présente la répartition du cholestérol dans les différentes fractions lipoprotéiques plasmatiques des souris E2/E2 contrôles ou traitées pendant 13 jours avec le composé 2 à 50 mpk. De manière inattendue, le taux de HDL-cholestérol plasmatique a été augmenté par le traitement avec le composé 2, administré à la dose de 50 mpk. On observe également une diminution significative des taux de LDL et VLDL plasmatiques par le traitement avec le composé 2, administré à la dose de 50 mpk.
La figure 1-4 présente la répartition des triglycérides dans les différentes fractions lipoprotéiques plasmatiques des souris E2/E2 contrôles ou traitées pendant 13 jours avec le composé 2 à 50 mpk. De manière inattendue, le taux de triglycérides présent dans les VLDL a été diminué par le traitement avec le composé 2, administré à la dose de 50 mpk.

### Analyse de l'expression génique par RT-PCR quantitative

Les inventeurs ont aussi mis en évidence *in vivo* que les composés selon l'invention sont des régulateurs de l'expression de gènes cibles des PPAR. Les résultats présentés sur les figures 1-5 à 1-9 montrent que le composé 2, administré à 50 mpk pendant 13 jours à des souris E2/E2, induit une augmentation significative de l'expression hépatique des gènes codant pour PDK4 (figure 1-5), l'Acox1 (figure 1-6), une diminution de l'expression hépatique du gène codant pour ApoCIII (figure 1-7) ainsi qu'une augmentation significative dans le muscle squelettique des gènes codant pour PDK4 (figure 1-8) et UCP2 (figure 1-9). Ces gènes codent pour des enzymes impliquées dans le métabolisme des lipides et des glucides ainsi que la dissipation d'énergie et le fait que leur expression soit modulée par les composés selon l'invention renforce l'idée que ces composés présentent un intérêt majeur dans le cadre des pathologies métaboliques.

### Conclusion

Les données expérimentales présentées montrent que les composés selon l'invention stimulent *in vivo* la synthèse de HDL-cholestérol tout en ayant un effet hypolipémiant (diminution des taux plasmatiques de cholestérol et de triglycérides). De plus, les données expérimentales présentées montrent que les composés selon l'invention modulent l'expression de gènes régulés par l'activation des PPAR qui codent pour des enzymes impliquées dans le métabolisme des lipides et des glucides et dans la dissipation d'énergie.

### Exemple 9 : Evaluation in vivo, chez la souris C57BI6, des propriétés hypolipémiantes et stimulatrices de la synthèse de HDL cholestérol des composés selon l'invention

### Principe

L'effet du composé 2, administré en effet dose, est évalué *in vivo* chez la souris C57BI6 après 14 jours de traitement par voie orale. A l'issue du traitement, l'effet hypolipémiant du composé 2 est évalué par la mesure des taux de cholestérol total, de HDL-cholestérol, de triglycérides et d'acides gras libres plasmatiques.

Il a été montré qu'un traitement par les activateurs de PPARδ se traduit chez l'homme comme chez le rongeur, par une augmentation du taux de HDL-cholestérol plasmatique.

Les propriétés agonistes de PPARδ des molécules selon l'invention préalablement mesurées *in vitro* doivent se traduire au niveau du muscle squelettique par une sur-expression des gènes cibles directement sous le contrôle du récepteur PPARδ : les gènes qui ont été étudiés dans cette expérience sont les gènes codant pour UCP2 (Uncoupling Protein 2, transporteur mitochondrial impliqué dans la thermorégulation) et PDK4 (Pyruvate Deshydrogénase Kinase isoforme 4, enzyme du métabolisme glucidique).

La mesure de l'activité transcriptionnelle des gènes cibles des PPAR après traitement par les composés selon l'invention est donc aussi un indicateur du caractère hypolipémiant des composés selon l'invention.

### Protocole

### Traitement des animaux

Des souris C57BI6 femelles ont été maintenues sous un cycle lumière/obscurité de 12/12 heures à une température constante de 20 ± 3°C. Après une acclimatation d'une semaine, les souris ont été pesées et rassemblées par groupes de 6 animaux sélectionnés de telle sorte que la distribution de leur poids corporel, de leur taux de lipides plasmatiques et de cholestérol total déterminés une première fois avant l'expérience soit uniforme. Les composés testés ont été suspendus dans la carboxyméthylcellulose (Sigma C4888) et administrés par gavage intra-gastrique, à raison d'une fois par jour pendant 14 jours à la dose choisie. Les animaux ont eu un accès libre à l'eau et à la nourriture (régime standard). A l'issue de l'expérience, les animaux ont été anesthésiés après un jeûne de 4 heures. Un prélèvement sanguin a été effectué sur anticoagulant (EDTA) puis les souris ont été pesées et euthanasiées. Le plasma a été séparé par centrifugation à 3000 tours/minutes pendant 20 minutes, les échantillons ont été conservés à +4°C.

Des échantillons de tissu musculaire squelettique ont été prélevés et congelés immédiatement dans de l'azote liquide puis conservés à -80°C pour les analyses ultérieures.

### Mesure du cholestérol total et des triglycérides plasmatiques

Les concentrations plasmatiques de cholestérol total et des triglycérides ont été mesurées par dosages enzymatiques (bioMérieux-Lyon-France) selon les recommandations du fournisseur.

### Mesure du HDL-cholestérol

Les lipoprotéines de basse densité (VLDL et LDL) ont été précipitées par Phosphotungstate. Le précipité a été éliminé par centrifugation. Le HDL-cholestérol présent dans le surnageant a été quantifié par dosages enzymatiques (bioMérieux-Lyon-France) selon les recommandations du fournisseur.

### Mesure des acides gras libres plasmatiques

Les concentrations plasmatiques d'acides gras libres ont été mesurées par dosages enzymatiques (WACO chemicals) selon les recommandations du fournisseur.

### Analyse d'expression génique par RT-PCR quantitative

### Tissu squelettique

L'ARN total a été extrait à partir de fragments de muscle squelettique gastrocnémien en utilisant le kit RNeasy^{®} Fibrous Tissue kit (Qiagen) selon les instructions du fabricant.

1µg d'ARN total (quantifié par lecture au spectrophotomètre) a ensuite été reverse -transcrit en ADN complémentaire par une réaction d'1 heure à 37°C dans un volume total de 30µl contenant du tampon 1X (Invitrogen), 1,5mM de DTT, 0,18mM de dNTPs (Promega), 200ng de pdN6 (Amersham), 30U d'inhibiteur de RNase (Promega) et 1µl de MMLV-RT (Invitrogen).

Les expériences de PCR quantitative ont été effectuées en utilisant le MyiQ Single-Color Real-Time PCR Détection System (Biorad, Marnes-la-Coquette, France) et ont été réalisées en utilisant le kit iQ SYBR Green Supermix selon les recommandations du fournisseur, en plaques 96 puits, sur 5µl de réaction de reverse transcription diluée avec une température d'hybridation de 60°C. Des paires d'amorces spécifiques des gènes étudiés ont été utilisées :
- mPDK4 : amorce sens : 5'-TACTCCACTGCTCCAACACCTG-3' (SEQ ID n°11) et amorce antisens 5'-GTTCTTCGGTTCCCTGCTTG-3' (SEQ ID n°12)
- mUCP2 : amorce sens : 5'-GTCGGAGATACCAGAGCACTGTCG-3' (SEQ ID n°13) et amorce antisens : 5'-CACATCAACAGGGGAGGCGA-3' (SEQ ID n°14)

La quantité de fluorescence émise est directement proportionnelle à la quantité d'ADN complémentaire présent au début de la réaction et amplifié au cours de la PCR. Pour chaque cible étudiée, une gamme est réalisée par des dilutions successives d'un pool constitué de quelques microlitres de différentes réactions de reverse transcription. Les niveaux d'expression relatifs de chaque cible sont ainsi déterminés en utilisant les courbes d'efficacité obtenues avec les points de gamme.

Les niveaux d'expression des gènes d'intérêt ont ensuite été normalisés par rapport au niveau d'expression du gène de référence 18S (dont les amorces spécifiques sont : amorce sens : 5'-CGGACACGGACAGGATTGACAG-3' (SEQ ID n°21) et amorce antisens: 5'-AATCTCGGGTGGCTGAACGC-3' (SEQ ID n°22)).

Le facteur d'induction, c'est-à-dire le rapport entre le signal relatif (induit par le composé selon l'invention) et la moyenne des valeurs relatives du groupe contrôle, a ensuite été calculé pour chaque échantillon. Plus ce facteur est élevé, plus le composé a un caractère activateur d'expression génique. Le résultat final est représenté comme moyenne des valeurs d'induction dans chaque groupe expérimental.

### Résultats

De manière générale, les résultats obtenus montrent que le composé 2 améliore le profil lipidique des souris C57BI6 traitées pendant 14 jours.

La figure 2-1 compare les taux de cholestérol total plasmatique après 14 jours de traitement avec le composé 2, administré à 1, 5, 10 et 50 mpk aux taux obtenus avec les animaux contrôles. De manière inattendue, les taux de cholestérol total plasmatique ont été significativement augmentés à 5 et 50mpk.

La figure 2-2 compare les taux de HDL-cholestérol plasmatique après 14 jours de traitement avec le composé 2, adminisré à 1, 5, 10 et 50 mpk aux taux obtenus avec les animaux contrôles. De manière inattendue, les taux de HDL-cholestérol plasmatique ont été significativement augmentés par le traitement de manière dose dépendante dès 1 mpk.

La figure 2-3 compare les taux de triglycérides plasmatiques après 14 jours de traitement avec le composé 2, administré à 1, 5, 10 et 50 mpk aux taux obtenus avec les animaux contrôles. De manière inattendue, les taux de triglycérides plasmatiques ont été diminués de manière dose dépendante, avec un effet significatif à 50 mpk.

La figure 2-4 compare les taux d'acides gras libres plasmatiques après 14 jours de traitement avec le composé 2, administré à 1, 5, 10 et 50 mpk aux taux obtenus avec les animaux contrôles. De manière inattendue, les taux d'acides gras libres plasmatiques ont été diminués significativement dès 10 mpk.

### Analyse de l'expression génique par RT-PCR quantitative

Les inventeurs ont aussi mis en évidence que les composés selon l'invention sont, *in vivo,* des régulateurs de l'expression de gènes cibles des PPAR. Les résultats présentés sur les figures 2-5 et 2-6 montrent que le composé 2, administré à 50 mpk pendant 14 jours à des souris C57BI6, induit dans le muscle squelettique une augmentation significatique de l'expression des gènes codant pour PDK4 (figure 2-5) et UCP2 (figure 2-6). Ces gènes codent pour des protéines impliquées dans le métabolisme des glucides et la dissipation d'énergie et le fait que leur expression soit modulée par les composés selon l'invention renforce l'idée que ces composés présentent un intérêt majeur dans le cadre des pathologies métaboliques.

### Conclusion

De manière inattendue, les données expérimentales montrent que les composés selon l'invention stimulent *in vivo* la synthèse de HDL-cholestérol tout en ayant un effet hypolipémiant (diminution des taux plasmatiques de triglycérides et d'acides gras libres). De plus, les données expérimentales montrent que les composés selon l'invention modulent dans le muscle squelettique l'expression de gènes régulés par l'activation des PPAR. Gènes qui codent pour des enzymes impliquées dans le métabolisme des glucides et dans la dissipation d'énergie.

### Exemple 10 : Evaluation in vivo, chez la souris C57BI6, des propriétés stimulatrices de la synthèse de HDL cholestérol des composés selon l'invention par dosages lipidigues et mesure de l'expression de gènes impliqués dans le métabolisme lipidique, glucidique et la dissipation d'énergie

### Principe

L'effet des composés selon l'invention a été évalué *in vivo* chez la souris C57BI6 après 14 jours de traitement par voie orale. A l'issu du traitement, la répartition du cholestérol dans les différentes fractions lipoprotéiques plasmatiques a été déterminée. Celle ci a été comparée au profil obtenu chez les animaux contrôles (non traités par les composés selon l'invention). L'effet des composés selon l'invention a été évalué chez la souris C57BI6 par la mesure des taux plasmatiques de cholestérol total et de HDL-cholestérol après 14 jours de traitement par voie orale. Ces taux ont été comparés à ceux obtenus avec des animaux contrôles (non traités par les composés selon l'invention). La différence mesurée témoigne de l'effet hypolipémiant des composés selon l'invention.

Il a été montré qu'un traitement par les activateurs de PPARδ se traduit chez l'homme comme chez le rongeur, par une augmentation du taux de HDL-cholestérol plasmatique.

Les propriétés agonistes de PPARδ, des molécules selon l'invention préalablement mesurées *in vitro* doivent se traduire au niveau du muscle squelettique par une sur-expression des gènes cibles directement sous le contrôle du récepteur PPARδ: les gènes qui ont été étudiés dans cette expérience sont les gènes codant pour PDK4 (enzyme du métabolisme glucidique), CPT1 b (enzyme du métabolisme lipidique), UCP2 et UCP3 (transporteurs mitochondriaux impliqués dans la thermorégulation).

La mesure de l'activité transcriptionnelle des gènes cibles des PPAR après traitement par les composés selon l'invention est donc aussi un indicateur du caractère hypolipémiant des composés selon l'invention.

### Protocole

### Traitement des animaux

Des souris C57BI6 femelles ont été maintenues sous un cycle lumière/obscurité de 12/12 heures à une température constante de 20 ± 3°C. Après une acclimatation d'une semaine, les souris ont été pesées et rassemblées par groupes de 6 animaux sélectionnés de telle sorte que la distribution de leur poids corporel, de leur taux de lipides plasmatiques et de cholestérol total déterminés une première fois avant l'expérience soit uniforme. Les composés testés ont été suspendus dans la carboxyméthylcellulose (Sigma C4888) et administrés par gavage intra-gastrique, à raison d'une fois par jour pendant 14 jours à la dose choisie. Les animaux ont eu un accès libre à l'eau et à la nourriture (régime standard). A l'issue de l'expérience, les animaux ont été anesthésiés après un jeûne de 4 heures, un prélèvement sanguin a été effectué sur anticoagulant (EDTA) puis les souris ont été pesées et euthanasiées. Le plasma a été séparé par centrifugation à 3000 tours/minute pendant 20 minutes, les échantillons ont été conservés à + 4°C.

Des échantillons de tissu musculaire squelettique ont été prélevés et congelés immédiatement dans de l'azote liquide puis conservés à -80°C pour les analyses ultérieures.

### Mesure du cholestérol total plasmatique

Les concentrations plasmatiques de cholestérol total ont été mesurées par dosages enzymatiques (bioMérieux-Lyon-France) selon les recommandations du fournisseur.

### Mesure du HDL-cholestérol

Les lipoprotéines de basse densité (VLDL et LDL) ont été précipitées par Phosphotungstate. Le précipité a été éliminé par centrifugation. Le HDL-cholestérol présent dans le surnageant a été quantifié par dosages enzymatiques (bioMérieux-Lyon-France) selon les recommandations du fournisseur.

### Analyse de la répartition du cholestérol dans les factions lipoprotéiques plasmatiques

Les différentes fractions lipidiques (VLDL, LDL, HDL) du plasma ont été séparées par une chromatographie Gel - Filtration. Les concentrations de cholestérol ont ensuite été mesurées dans chaque fraction par dosages enzymatiques (bioMérieux-Lyon-France) selon les recommandations du fournisseur.

### Analyse d'expression génique par RT-PCR quantitative

L'ARN total a été extrait à partir de fragments de muscle squelettique gastrocnémien en utilisant le kit RNeasy^{®} Fibrous Tissue kit (Qiagen) selon les instructions du fabricant.

1µg d'ARN total (quantifié par lecture au spectrophotomètre) a ensuite été reverse -transcrit en ADN complémentaire par une réaction d'1 heure à 37°C dans un volume total de 30µl contenant du tampon 1X (Invitrogen), 1,5mM de DTT, 0,18mM de dNTPs (Promega), 200ng de pdN6 (Amersham), 30U d'inhibiteur de RNase (Promega) et 1µl de MMLV-RT (Invitrogen).

Les expériences de PCR quantitative ont été effectuées en utilisant le MyiQ Single-Color Real-Time PCR Detection System (Biorad, Marnes-la-Coquette, France) et ont été réalisées en utilisant le kit iQ SYBR Green Supermix selon les recommandations du fournisseur, en plaques 96 puits, sur 5µl de réaction de reverse transcription diluée avec une température d'hybridation de 60°C. Des paires d'amorces spécifiques des gènes étudiés ont été utilisées :
- mPDK4 : amorce sens : 5'-TACTCCACTGCTCCAACACCTG-3' (SEQ ID n°11) et amorce antisens 5'-GTTCTTCGGTTCCCTGCTTG-3' (SEQ ID n°12)
- mCPT1b : amorce sens : 5'-GGACTGAGACTGTGCGTTCCTG-3' (SEQ ID n°7) et amorce antisens : 5'-AGTGCTTGGCGGATGTGGTT-3' (SEQ ID n°8)
- mUCP2 : amorce sens : 5'- GTCGGAGATACCAGAGCACTGTCG-3' (SEQ ID n°13) et amorce antisens : 5'- CACATCAACAGGGGAGGCGA-3' (SEQ ID n°14)
- mUCP3 : amorce sens : 5'- GCACCGCCAGATGAGTTTTG-3' (SEQ ID n°15) et amorce antisens : 5'- GACGCTGGAGTGGTCCGCTC-3' (SEQ ID n°16)

La quantité de fluorescence émise est directement proportionnelle à la quantité d'ADN complémentaire présent au début de la réaction et amplifié au cours de la PCR. Pour chaque cible étudiée, une gamme est réalisée par des dilutions successives d'un pool constitué de quelques microlitres de différentes réactions de reverse transcription. Les niveaux d'expression relatifs de chaque cible sont ainsi déterminés en utilisant les courbes d'efficacité obtenues avec les points de gamme.

Les niveaux d'expression des gènes d'intérêt ont ensuite été normalisés par rapport au niveau d'expression du gène de référence 18S (dont les amorces spécifiques sont : amorce sens : 5'-CGGACACGGACAGGATTGACAG-3' (SEQ ID n°21) et amorce antisens: 5'-AATCTCGGGTGGCTGAACGC-3' (SEQ ID n°22)).

Le facteur d'induction, c'est-à-dire le rapport entre le signal relatif (induit par le composé selon l'invention) et la moyenne des valeurs relatives du groupe contrôle, a ensuite été calculé pour chaque échantillon. Plus ce facteur est élevé, plus le composé a un caractère activateur de l'expression génique. Le résultat final est représenté comme moyenne des valeurs d'induction dans chaque groupe expérimental.

### Résultats

De manière générale, les résultats obtenus montrent que les composés 4 et 7 selon l'invention améliorent le profil lipidique des souris C57BI6 traitées pendant 14 jours.

La figure 3-1 compare les taux de cholestérol total plasmatique après 14 jours de traitement avec les composés 4 et 7 selon l'invention, administrés à 50 mpk aux taux obtenus avec les animaux contrôles. De manière inattendue, les taux de cholestérol total plasmatique ont été significativement augmentés par le traitement avec les composés 4 et 7 à 50mpk.

La figure 3-2 compare les taux de HDL-cholestérol plasmatique après 14 jours de traitement avec les composés 4 et 7 selon l'invention, adminisrés à 50 mpk aux taux obtenus avec les animaux contrôles. De manière inattendue, les taux de HDL-cholestérol plasmatique ont été significativement augmentés par le traitement avec le composé 4 à 50mpk. Le traitement par le composé 7 selon l'invention à 50 mpk a également induit une augmentation, non significative, du taux de HDL-cholestérol.

La figure 3-3 présente la répartition du cholestérol dans les fractions lipoprotéiques plasmatiques des souris C57BI6 contrôles ou traitées pendant 14 jours avec les composés 4 et 7 à 50 mpk. De manière inattendue, le taux de HDL-cholestérol plasmatique a été augmenté par les traitements avec les composés 4 et 7.

### Analyse de l'expression génique par RT-PCR quantitative

Les inventeurs ont aussi mis en évidence que les composés selon l'invention sont, *in vivo,* des régulateurs de l'expression de gènes cibles des PPAR. Les résultats présentés sur les figures 3-4 à 3-7 montrent que les composés 4 et 7 selon l'invention, administrés à 50 mpk pendant 14 jours à des souris C57BI6, induisent dans le muscle squelettique une augmentation significative des gènes codant pour PDK4 (figure 3-4), CPT1 b (figure 3-5), UCP2 (figure 3-6) et UCP3 (figure 3-7). Ces gènes codent pour des enzymes fortement impliquées dans le métabolisme lipidiques, glucidiques et la dissipation d'énergie, et le fait que leur expression soit modulée par les composés selon l'invention renforce l'idée que ces composés présentent un intérêt potentiel majeur dans le cadre des pathologies métaboliques.

### Conclusion

De manière inattendue, les données expérimentales présentées montrent que les composés selon l'invention stimulent *in vivo* la synthèse de HDL-cholestérol. De plus, les données expérimentales présentées montrent que les composés selon l'invention modulent dans le muscle squelettique l'expression de gènes régulés par l'activation des PPAR qui codent pour des enzymes fortement impliquées dans le métabolisme des glucides et dans la dissipation d'énergie.

### Exemple 11 : Evaluation in vivo, chez la souris db/db, des propriétés hypolipémiantes, antidiabétigue et activatrices des PPAR des composés selon l'invention

### Principe

L'effet des composés selon l'invention est évalué *in vivo* chez la souris db/db par la mesure des triglycérides plasmatiques et de l'insulinémie après 28 jours de traitement par voie orale avec le composé 2. Ces taux sont comparés à ceux obtenus avec des animaux contrôles (non traités par le composé selon l'invention). La différence mesurée témoigne de l'effet hypolipémiant et sur l'insulino-résistance du composé selon l'invention.

L'efficacité du composé selon l'invention est aussi évaluée par la mesure, dans les tissus hépatiques et musculaires de l'expression de gènes impliqués dans le métabolisme glucidique, lipidique, et la dissipation d'énergie. Les niveaux d'expression de chaque gène sont normalisés par rapport au niveau d'expression des gènes de référence 36B4 dans le foie et 18S dans le muscle squelettique. Le facteur d'induction, c'est-à-dire le rapport entre le signal relatif (induit par le composé selon l'invention) et la moyenne des valeurs relatives du groupe contrôle, est ensuite calculé. Plus ce facteur est élevé, plus le composé a un caractère activateur d'expression génique. Le résultat final est représenté comme moyenne des valeurs d'induction dans chaque groupe expérimental.

### Protocole

### Traitement des animaux

Des souris db/db femelles ont été maintenues sous un cycle lumière/obscurité de 12/12 heures à une température constante de 20 ± 3°C. Après une acclimatation d'une semaine, les souris ont été pesées et rassemblées par groupe de 8 animaux sélectionnés de telle sorte que la distribution de leurs poids corporel et de leurs taux de lipides plasmatiques déterminés une première fois avant l'expérience soient uniformes. Les composés testés ont été suspendus dans la carboxyméthylcellulose (Sigma C4888) et administrés par gavage intra-gastrique, à raison d'une fois par jour pendant 28 jours à la dose choisie. Les animaux ont eu un accès libre à l'eau et à la nourriture (régime standard). La prise de nourriture et la prise de poids sont enregistrées tout au long de l'expérience. A l'issue de l'expérience, les animaux ont été anesthésiés après un jeûne de 4 heures, un prélèvement sanguin a été effectué sur anticoagulant (EDTA) puis les souris ont été pesées et euthanasiées. Le plasma a été séparé par centrifugation à 3000 tours/minutes pendant 20 minutes, les échantillons ont été conservés à + 4°C.

Des échantillons de tissus hépatique et musculaire ont été prélevés et congelés immédiatement dans de l'azote liquide puis conservés à -80°C pour les analyses ultérieures.

### Mesure du taux de triglycérides plasmatiques

Les concentrations plasmatiques des triglycérides ont été mesurées par dosages enzymatiques (bioMérieux-Lyon-France) selon les recommandations du fournisseur.

### Mesure de l'insulinémie plasmatique

Le dosage de l'insuline murine est effectué par la méthode Elisa (en utilisant le kit INSKR020 du fournisseur Crystal chem). Un anticorps anti-insuline de souris est fixé sur une microplaque. Le sérum à doser pour l'insuline est ensuite déposé sur cette plaque. Un anticorps anti-insuline de cobaye va reconnaître le complexe insuline/anticorps monoclonal anti-insuline de souris et s'y fixer. Enfin un anticorps anti-cobaye marqué à la péroxydase est ajouté se fixant à l'anticorps anti-insuline de cobaye. La réaction colorimétrique est réalisée par addition du substrat de l'enzyme OPD (Ortho Phényl Diamine). L'intensité de la coloration est proportionnelle à la quantité d'insuline présente dans l'échantillon.

### Analyse d'expression génique par RT-PCR quantitative

### Tissu hépatique

L'ARN total a été extrait à partir de fragments de foie en utilisant le kit NucleoSpin® 96 RNA (Macherey Nagel, Hoerdt, France) selon les instructions du fabricant.

### Tissu musculaire

L'ARN total a été extrait à partir de fragments de muscle en utilisant le kit RNeasy^{®} Fibrous Tissue kit (Qiagen) selon les instructions du fabricant.

1µg d'ARN total (quantifié par spectrophotométrie UV) a ensuite été retro-transcrit en ADN complémentaire par une réaction d'1 heure à 37°C dans un volume total de 30µl contenant du tampon 1X (Invitrogen), 1,5mM de DTT, 0,18mM de dNTPs (Promega), 200ng de pdN6 (Amersham), 30U d'inhibiteur de RNase (Promega) et 1µl de MMLV-RT (Invitrogen).

Les expériences de PCR quantitative ont été effectuées en utilisant le MyiQ Single-Color Real-Time PCR Detection System (Biorad, Marnes-la-Coquette, France) et ont été réalisées en utilisant le kit iQ SYBR Green Supermix selon les recommandations du fournisseur, en plaques 96 puits, sur 5µL de réaction de retro-transcription diluée avec une température d'hybridation de 60°C. Les paires d'amorces spécifiques des gènes étudiés sont les suivantes.
- mPDK4 : amorce sens : 5'-TACTCCACTGCTCCAACACCTG-3' (SEQ ID n°11) et amorce antisens 5'-GTTCTTCGGTTCCCTGCTTG-3' (SEQ ID n°12)
- mACOX1 : amorce sens : 5'- GAAGCCAGCGTTACGAGGTG-3' (SEQ ID n°3) et amorce antisens : 5'- TGGAGTTCTTGGGACGGGTG-3' (SEQ ID n°4)
- mCPT1b : amorce sens : 5'-GGACTGAGACTGTGCGTTCCTG-3' (SEQ ID n°7) et amorce antisens : 5'-AGTGCTTGGCGGATGTGGTT-3' (SEQ ID n°8)
- mUCP3 : amorce sens : 5'- GCACCGCCAGATGAGTTTTG-3' (SEQ ID n°15) et amorce antisens : 5'- GACGCTGGAGTGGTCCGCTC-3' (SEQ ID n°16)

La quantité de fluorescence émise est directement proportionnelle à la quantité d'ADN complémentaire présent au début de la réaction et amplifié au cours de la PCR. Pour chaque cible étudiée, une gamme est réalisée par des dilutions successives d'un pool constitué de quelques microlitres de différentes réactions de retro-transcription. Les niveaux d'expression relatifs de chaque cible sont ainsi déterminés en utilisant les courbes d'efficacité obtenues avec les points de gamme.

Les niveaux d'expression des gènes d'intérêt ont ensuite été normalisés, dans le tissu hépatique par rapport au niveau d'expression du gène de référence 36B4 (dont les amorces spécifiques sont : amorce sens : 5'-CATGCTCAACATCTCCCCCTTCTCC-3' (SEQ ID n°19) et amorce antisens : 5'-GGGAAGGTGTAATCCGTCTCCACAG-3' (SEQ ID n°20)) et, dans le tissu musculaire par rapport au niveau d'expression du gène de référence 18S (dont les amorces spécifiques sont : amorce sens : 5'-CGGACACGGACAGGATTGACAG-3' (SEQ ID n°21) et l'amorce antisens: 5'-AATCTCGGGTGGCTGAACGC-3' (SEQ ID n°22)). Le facteur d'induction a été calculé pour chaque échantillon. Plus ce facteur est élevé, plus le composé a un caractère activateur d'expression génique. Le résultat final est représenté comme moyenne des valeurs d'induction dans chaque groupe expérimental.

### Résultats

### Mesure des taux triglycérides plasmatiques

La figure 4-1 compare les taux plasmatiques de triglycérides après 28 jours de traitement avec le composé 2, administré à 50mpk aux taux obtenus pour les animaux contrôles. De manière inattendue, le taux de triglycérides est significativement diminué par le traitement avec le composé selon l'invention.

### Mesure de l'insulinémie

La figure 4-2 compare les taux plasmatiques d'insuline après 28 jours de traitement avec le composé 2, administré à 50mpk aux taux obtenus avec les animaux contrôles. De manière inattendue, l'insulinémie est significativement diminuée après 28 jours de traitement des animaux avec le composé 2.

### Analyse de l'expression génique par RT-PCR quantitative

Les inventeurs ont aussi mis en évidence *in vivo* que les composés selon l'invention sont des régulateurs de l'expression de gènes cibles des PPAR. Les résultats présentés sur les figures 4-3 et 4-4 montrent que le composé 2, administré à 50 mpk pendant 28 jours chez des souris db/db, induit dans le foie une augmentation significative de l'expression des gènes codant pour PDK4 (figure 4-3) et pour l'ACOX1 (figure 4-4).

Ces gènes codent pour des enzymes impliquées dans le métabolisme des lipides et des glucides, et sont reconnus comme étant des gènes cibles de PPARα dans le foie. Le fait que leur expression soit modulée par le composé 2 renforce l'idée que ce composé présente un intérêt majeur dans le cadre des pathologies métaboliques.

D'autre part, les inventeurs ont aussi mis en évidence *in vivo* que le composé 2 est un régulateur de l'expression de gènes cibles de PPARδ dans le muscle squelettique. Les résultats présentés sur les figures 4-5 à 4-7 montrent que le composé 2 administré à 50 mpk pendant 28 jours chez des souris db/db, induit dans le muscle squelettique une augmentation significative de l'expression des gènes codant pour CPT1 b (figure 4-5), PDK4 (figure 4-6) et UCP2 (figure 4-7). Ces gènes codent pour des protéines impliquées dans le métabolisme des lipides, des glucides et de la thermorégulation, et sont connus comme étant des gènes cibles de PPARδ dans le muscle. Le fait que leur expression soit modulée par le composé 2 renforce l'idée que ce composé présente un intérêt majeur dans le cadre des pathologies métaboliques.

### Conclusion

De manière inattendue, les données expérimentales présentées montrent que le composé 2 possède des propriétés hypolipémiantes et antidiabétiques chez la souris db/db. De plus, les données expérimentales montrent que les composés selon l'invention modulent l'expression de gènes régulés par l'activation des PPAR qui codent pour des enzymes impliquées dans le métabolisme des lipides, des glucides et la thermorégulation.

### Exemple 12: Evaluation in vitro des propriétés métaboliques des composés selon l'invention dans un modèle de myocytes murins

### Principe

Les effets stimulateurs des composés selon l'invention ont été évalués par la mesure de la β-oxydation des acides gras par des myocytes murins pré-traités pendant 24 heures avec les composés selon l'invention. Plus l'induction de la β-oxydation des acides gras est augmentée, plus le composé selon l'invention est stimulateur du métabolisme dans les cellules musculaires.

L'objectif est de mesurer la quantité d'eau tritiée formée durant l'oxydation mitochondriale des acides gras marqués au ³H.

### Protocole

### Différenciation des cellules C2C12 en myocytes.

La lignée cellulaire murine C2C12 (provenance ECACC) sont cultivées dans du milieu DMEM (Gibco; 41965-039) additionné de 1% L-Glutamine (Gibco; 25030), de 1% pénicilline/streptomycine (VWR ; BWSTL0022/100) et 10% sérum de veau foetal décomplémenté (SVF. Gibco ; 10270-106).

Les cellules sont ensemencées sur des plaques de 48 puits à la densité de 25.10³ cellules/puits. A confluence, le milieu est remplacé par un milieu de différenciation (milieu de culture de base additionné de 2% de sérum de cheval (Gibco ; 26050-088)) puis incubées à 37°C et 5% de CO₂ pendant 4 jours afin de les différencier en myocytes.

### Traitement

Après 4 jours de différenciation, les cellules sont traitées avec les composés selon l'invention ajouté au milieu de culture de différenciation. Le composé 2 a été testé en effet dose de 0,01 à 1µM, et les autres ont été testés à la dose de 1µM. Les composés selon l'invention sont dissous dans du diméthyl sulfoxide (DMSO, Sigma ; D5879). Les cellules sont traitées pendant 24h à 37°C, 5% CO₂. L'effet des composés selon l'invention est comparé à l'effet du DMSO seul.

### Mesure de la β-oxydation

Après 24 heures de traitement, le milieu de culture est remplacé par un milieu DMEM 1g/L glucose (Gibco ; 21885-025) additionné de 1% L-Glutamine (Gibco ; 25030), 1% pénicilline/streptomycine (VWR; BWSTL0022/100) et 1 mM L-carnitine (Alfa Aefar; A 17618). Les cellules sont ensuite incubées avec le complexe de palmitate tritié / BSA (0,1 mM) à 37°C en présence de 5% CO₂. La réaction est stoppée après 1, 2 et 3 heures et les protéines sont précipitées avec distribution de TCA 10%.

Dans un premier temps, le comptage du tritium est réalisé sur 100µL de surnageant de culture précipité.

Dans un second temps, 50µL de surnageant précipité est mis en évaporation pendant 2 jours puis le tritium résiduel mesuré afin d'évaluer la quantité de palmitate tritié non converti en eau.

Une gamme étalon est réalisée par dilutions successives du complexe palmitate tritié / BSA afin de déterminer la quantité de palmitate transformée en eau.

Pour chaque temps de la cinétique, 1 h, 2h et 3h, la quantité en nanomoles de palmitate oxidé est calculée par régression linéaire. Les aires sous la courbe sont déterminées pour chaque condition et les résultats sont normalisés au DMSO.

Le résultat final est représenté comme moyenne des valeurs d'induction dans chaque groupe expérimental.

### Résultats

Les inventeurs ont aussi mis en évidence *in vitro,* sur des myocytes, que les composés selon l'invention ont des effets stimulateurs de la β-oxydation des acides gras. Les résultats présentés sur la figure 5 montrent que le composé 2, en effet dose dès 0,01 µM et les composés 4 et 11 selon l'invention, à 1µM, induisent une augmentation significative de la β-oxydation des acides gras dans les myocytes murins.

### Conclusion

De manière inattendue, les données expérimentales présentées montrent que les composés selon l'invention ont une action métabolique dans les myocytes murins en induisant le catabolisme des acides gras.

### Exemple 13 : Evaluation in vitro des propriétés activatrices du transport inverse du cholestérol des composés selon l'invention.

### Principe

L'effet des composés selon l'invention sur le transport inverse de cholestérol a été évalué par la mesure de l'expression du gène ABCA1 (ATP-binding cassette, sub-family A, member 1 ; transporteur membranaire impliqué dans l'efflux de cholestérol) dans des macrophages humains. Plus l'expression d'ABCA1 est augmentée, plus le composé selon l'invention stimule le transport inverse du cholestérol.

### Protocole

### Différenciation des cellules THP-1 en macrophages.

La lignée de monocytes humains THP1 (provenance ATCC) est cultivée dans du milieu RPM11640 additionné de 25mM Hepes (Gibco; 42401-018), 1% glutamine (Gibco; 25030-24), 1% pénicilline/streptomycine (Biochrom AG; A 2213) et 10% de sérum de veau foetal décomplémenté (SVF. Gibco ; 26050-088).

Les cellules sont ensemencées sur des plaques de 24 puits (Primaria BD Falcon) à la densité de 3.10⁵ cellules/puits et incubées à 37°C et 5% de CO₂ pendant 72h en présence de 30 ng/ml de phorbol 12-myristate 13-acétate (PMA) afin de les différencier en macrophages.

### Traitement

Le milieu de différenciation est aspiré et remplacé par le milieu de traitement (même composition que le milieu de culture mais sans sérum de veau foetal et avec 1% d'ultroser (Pall Life Science ; P/N267051)).

Les composés selon l'invention sont dissous dans du diméthyl sulfoxide (DMSO, Fluka ; 41640). Le composé 2 a été testé à la dose de 1µM. Les cellules sont traitées pendant 24h à 37°C, 5% CO2. L'effet des composés selon l'invention est comparé à l'effet du DMSO seul.

### Extraction des ARN, transcription inverse et PCR quantitative.

Après traitement, l'ARN total est extrait des cellules en utilisant le kit NucleoSpin^{®} 96 RNA (Macherey Nagel, Hoerdt, France) selon les instructions du fabricant.

1µg d'ARN total (quantifié par lecture au spectrophotomètre) a ensuite été reverse transcrit en ADN complémentaire par une réaction d'1 heure à 37°C dans un volume total de 30µl contenant du tampon 1X (Invitrogen), 1,5mM de DTT, 0,18mM de dNTPs (Promega), 200ng de pdN6 (Amersham), 30U d'inhibiteur de RNase (Promega) et 1µl de MMLV-RT (Invitrogen).

Les expériences de PCR quantitative ont été effectuées en utilisant le MyiQ Single-Color Real-Time PCR Detection System (Biorad, Marnes-la-Coquette, France) et ont été réalisées en utilisant le kit iQ SYBR Green Supermix selon les recommandations du fournisseur, en plaques 96 puits, sur 5µl de réactions de reverse transcription diluées avec une température d'hybridation de 60°C. Des paires d'amorces spécifiques du gène étudié ont été utilisées :
- hABCA1 : amorce sens : 5'-CTGAGGTTGCTGCTGTGGAAG-3' (SEQ ID n°1) amorce antisens: 5'-CATCTGAGAACAGGCGAGCC-3' (SEQ ID n°2)

La quantité de fluorescence émise est directement proportionnelle à la quantité d'ADN complémentaire présent au début de la réaction et amplifié au cours de la PCR. Pour chaque cible étudiée, une gamme est réalisée par des dilutions successives d'un pool constitué de quelques µl de différentes réactions de reverse transcription. Les niveaux d'expression relatifs de chaque cible sont ainsi déterminés en utilisant les courbes d'efficacité obtenues avec les points de gamme.

Les niveaux d'expression des gènes d'intérêt ont ensuite été normalisés, par rapport au niveau d'expression du gène de référence 36B4 (dont les amorces spécifiques sont : amorce sens : 5'-CATGCTCAACATCTCCCCCTTCTCC-3' (SEQ ID n°19) et amorce antisens : 5'-GGGAAGGTGTAATCCGTCTCCACAG - 3' (SEQ ID n°20)).

Le facteur d'induction, c'est-à-dire le rapport entre le signal relatif (induit par le composé selon l'invention) et la moyenne des valeurs relatives du groupe contrôle, a ensuite été calculé. Plus ce facteur est élevé, plus le composé a un caractère activateur d'expression génique. Le résultat final est représenté comme moyenne des valeurs d'induction dans chaque groupe expérimental.

### Résultats

Les inventeurs ont mis en évidence que les composés selon l'invention sont, *in vitro* dans les macrophages humains, des stimulateurs du transport inverse du cholestérol. Les résultats présentés sur la figure 6-1 montrent que le composé 2, à 1µM, induit une augmentation significative de l'expression du gène codant pour ABCA1 dans les macrophages humains. Les résultats présentés sur la figure 6-2 montrent que les composés 4 et 7 selon l'invention, à 1µM et 300nM respectivement, induisent une augmentation significative de l'expression du gène codant pour ABCA1 dans les macrophages humains.

### Conclusion

De manière inattendue, les données expérimentales présentées montrent que les composés selon l'invention stimulent le transport inverse du cholesterol.

### Exemple 14: Evaluation in vifro des propriétés anti-inflammatoires des composés selon l'invention dans un modèle de monocytes humains

### Principe

Les effets anti-inflammatoires des composés selon l'invention ont été évalués par la mesure de la sécrétion et de l'expression de la Macrophages Chemoattractant Protein (MCP1) et de la Matrix metalloproteinase 9 (MMP9) par des monocytes traités pendant 24 heures avec les composés selon l'invention et stimulés avec du PMA (phorbol 12-myristate 13-acétate, provoque une réponse inflammatoire des cellules). Plus la quantité de MCP1 sécrétée est diminuée, plus les composés selon l'invention inhibent la réaction inflammatoire. De la même manière, plus l'expression des gènes codant pour MCP1 et MMP9 est inhibée, plus les composés selon l'invention sont anti-inflammatoires.

### Protocole

### Culture des cellules THP-1

La lignée de monocytes humains THP1 (provenance ATCC) est cultivée dans du milieu RPM11640 additionné de 25mM Hepes (Gibco; 42401-018), 1% glutamine (Gibco; 25030-24) 1% pénicilline/streptomycine (Biochrom AG; A 2213) et 10% sérum de veau foetal décomplémenté (SVF. Gibco ; 10270-106).

### Traitement

Les cellules sont ensemencées sur des plaques de 24 puits (Primaria BD Falcon) à la densité de 8,70.10⁵ cellules/puits dans du milieu de traitement (même composition que le milieu de culture mais avec 0,2% de sérum de veau foetal décomplémenté) et en présence de 5 ng/mL de phorbol 12-myristate 13-acetate (PMA) pour induire la réponse inflammatoire.

Les composés selon l'invention ont été testés à 0,1, 0,3 et/ou à 1µM, et dissous dans du diméthyl sulfoxide (DMSO, Fluka ; 41640). Les cellules sont traitées pendant 24h à 37°C, 5% CO₂. L'effet du composé selon l'invention est comparé à l'effet du DMSO seul.

### Mesure de la sécrétion de MCP1

Le milieu de traitement est récupéré et la concentration de MCP1 est mesurée en utilisant la trousse Elisa « Human MCP1 Elisa set » (BD OptEIA ; 555179) selon les recommandations du fabricant.

Un anticorps monoclonal anti-MCP1 humain est fixé sur une plaque, puis les surnageants, contenant les MCP1 secrétés par les cellules sont distrubés. Un anticorps anti-MCP1 biotinylé va ensuite se fixer au complexe. Un troisième anticorps conjugué et couplé à une enzyme peroxydase permet en présence de substrat d'initier une réaction enzymatique dont le résultat est une coloration proportionnelle à la quantité de MCP1 fixée et peut être mesurée par spectrophotométrie. Une gamme est réalisée à partir d'un point de concentration connue et permet de calculer la concentration en MCP1 de chaque échantillon. Le facteur d'induction, c'est-à-dire le rapport entre le signal induit par le composé selon l'invention et le signal du groupe contrôle, a ensuite été calculé. Plus ce facteur est faible, plus le composé a un caractère inhibiteur de la sécrétion de MCP1. Le résultat final est représenté comme moyenne des valeurs d'induction de chaque groupe expérimental.

### Extraction des ARN, transcription inverse et PCR quantitative.

Après traitement, l'ARN total est extrait des cellules en utilisant le kit NucIeoSpin^{®} 96 RNA (Macherey Nagel, Hoerdt, France) selon les instructions du fabricant.

1µg d'ARN total (quantifié par lecture au spectrophotomètre UV) a ensuite été retro-transcrit en ADN complémentaire par une réaction d'1 heure à 37°C dans un volume total de 30µl contenant du tampon 1X (Invitrogen), 1,5mM de DTT, 0,18mM de dNTPs (Promega), 200ng de pdN6 (Amersham), 30U d'inhibiteur de RNase (Promega) et 1µl de MMLV-RT (Invitrogen).

Les expériences de PCR quantitative ont été effectuées en utilisant le MyiQ Single-Color Real-Time PCR Detection System (Biorad, Marnes-la-Coquette, France) et ont été réalisées en utilisant le kit iQ SYBR Green Supermix selon les recommandations du fournisseur, en plaques 96 puits, sur 5µl de réaction de retro-transcription diluées avec une température d'hybridation de 60°C. Des paires d'amorces spécifiques des gènes MCP1 et MMP9 étudiés ont été utilisées :
- hMCP1 : amorce sens : 5'-AGGAAGATCTCAGTGCAGAGG-3' (SEQ ID n°9) et amorce antisens : 5'-AGTCTTCGGAGTTTGGGTTTG-3' (SEQ ID n°10)
- hMMP9 : amorce sens : 5'-TGGCACCACCACAACATCAC-3' (SEQ ID n°17) et amorce antisens : 5'-ACCACAACTCGTCATCGTCG-3' (SEQ ID n°18)

La quantité de fluorescence émise est directement proportionnelle à la quantité d'ADN complémentaire présent au début de la réaction et amplifié au cours de la PCR. Pour chaque cible étudiée, une gamme est réalisée par des dilutions successives d'un pool constitué de quelques microlitres de différentes réactions de retro-transcription. Les niveaux d'expression relatifs de chaque cible sont ainsi déterminés en utilisant les courbes d'efficacité obtenues avec les points de gamme.

Les niveaux d'expression des gènes d'intérêt ont ensuite été normalisés, dans le tissu hépatique par rapport au niveau d'expression du gène de référence 36B4 (dont les amorces spécifiques sont : amorce sens : 5'-CATGCTCAACATCTCCCCCTTCTCC-3' (SEQ ID n°19) et amorce antisens : 5'-GGGAAGGTGTAATCCGTCTCCACAG-3' (SEQ ID n°20)). Le facteur d'induction, c'est-à-dire le rapport entre le signal relatif (induit par le composé selon l'invention) et la moyenne des valeurs relatives du groupe contrôle, a ensuite été calculé. Plus ce facteur est faible, plus le composé a un caractère inhibiteur de l'expression génique. Le résultat final est représenté comme moyenne des valeurs d'induction dans chaque groupe expérimental.

### Résultats

Les inventeurs ont aussi mis en évidence, sur des monocytes *in vitro,* que les composés selon l'invention ont des effets anti-inflammatoires. Les résultats présentés sur la figure 7-1 montrent que les composés 7 et 11 selon l'invention, à 1µM, induisent une diminution significative de la secrétion de MCP1 dans les monocytes humains. Les résultats présentés sur les figures 7-2 et 7-3 montrent que les composés 7 et 11 selon l'invention, à 1µM, induisent une diminution significative de l'expression de MCP1 et MMP9 dans les monocytes humains. Les résultats présentés sur la figure 7-4 montrent que le composé 2, à 0,1 et 0,3µM, induit une diminution significative de l'expression de MCP1 dans les monocytes humains.

### Conclusion

De manière inattendue, les données expérimentales présentées montrent que les composés selon l'invention ont une action anti-inflammatoire dans les monocytes stimulés avec du PMA.

### Conclusion générale

Les inventeurs ont mis en évidence que les composés selon l'invention ont des propriétés stimulatrices de la synthèse de HDL-cholestérol, des propriétés hypolipémiantes (en baissant les taux plasmatiques de triglycérides et d'acides gras libres) ainsi que des propriétés anti-diabétiques. De plus, les inventeurs ont mis en évidence que les composés selon l'invention sont des régulateurs de l'expression de gènes codant pour des enzymes impliquées dans le métabolisme des lipides, des glucides et dans la dissipation d'énergie. Ces résultats, obtenus *in vivo,* témoignent du potentiel thérapeutique des composés selon l'invention vis-à-vis de pathologies associées au syndrome métabolique telles que les dyslipidémies, l'obésité, l'athérosclérose etc.

D'autre part, les inventeurs ont mis en évidence le caractère activateur des PPAR dans différents modèles cellulaires se traduisant notamment par la régulation de l'expression de gènes codant pour des enzymes impliquées dans le métabolisme des lipides, des glucides, de la thermorégulation, et par une augmentation du catabolisme des acides gras, ainsi que par une action anti-inflammatoire.

### BIBLIOGRAPHIE

de la Monte SM, et al., Therapeutic rescue of neurodegeneration in experimental type 3 diabetes: Relevance to Alzheimer's disease, J Alzheimers Dis, 2006, 10 (1), 89-109
Fox-Tucker J, The Cardiovasular Market Outlook to 2010, BUSINESS INSIGHTS REPORTS, 2005, 1-174
Gross B, et al., Peroxisome Proliferator-Activated Receptor b/d: A novel target for the reduction of atherosclerosis, DRUG DISCOVERY TODAY: THERAPEUTIC STRATEGIES, 2005, 2 (3), 237-243
International Atherosclerosis Society, Harmonized Clinical. Guidelines on Prevention of Atherosclerotic Vascular Disease, 2003,
Kota BP, et al., An overview on biological mechanisms of PPARs, Pharmacol Res, 2005, 51 (2), 85-94
Lefebvre P, et al., Sorting out the roles of PPARalpha in energy metabolism and vascular homeostasis, J Clin Invest, 2006, 116 (3), 571-580
Lehrke M and Lazar MA, The many faces of PPARgamma, Cell, 2005, 123 (6), 993-9
Liu Y and Miller A, Ligands to peroxisome proliferator-activated receptors as therapeutic options for metabolic syndrome, DRUG DISCOVERY TODAY: THERAPEUTIC STRATEGIES, 2005, 2 (3), 165-169
Mensah M, The Atlas of Heart Disease and Stroke, 2004,
Polak, Oral administration of the selective PPARd agonist GW0742 reduced clinical symptoms and reduces astroglial and microglial inflammatory activation in a model of EAE, J Neuroimmunol, 2005,
Raspe E, et al., Modulation of rat liver apolipoprotein gene expression and serum lipid levels by tetradecylthioacetic acid (TTA) via PPARalpha activation, J Lipid Res, 1999, 40 (11), 2099-110

## Revendications

1. Composés dérivés de 3-phényl-1-(phénylthiènyl)propan-1-one et de 3-phényl-1-(phénylfuranyl)propan-1-one substitués de formule générale (I) : 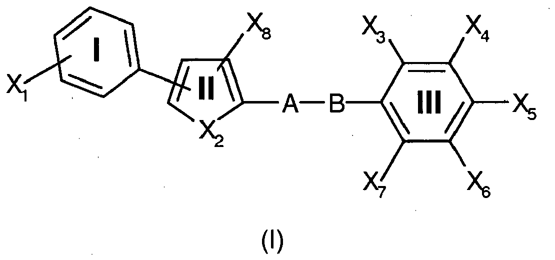 dans laquelle :
X1 représente un halogène, un groupement R1, -SR1 ou -OR1 ;
X2 représente un atome de soufre ou d'oxygène ;
X3 représente un halogène ou un groupement R3 ;
X4 représente un halogène ou un groupement R4 ;
X5 représente un groupement -OR5 ;
X6 représente un halogène ou un groupement R6 ;
X7 représente un halogène ou un groupement R7 ;
X8 représente un atome d'hydrogène ;
R1 représentant un hydrogène ou un groupement alkyle ayant 1 à 4 atomes de carbone, ledit groupement alkyle étant éventuellement halogéné ;
R3, R4, R6 et R7 identiques ou différents, choisis parmi un hydrogène ou un groupement alkyle ayant 1 à 4 atomes de carbone ;
R5 représentant un radical alkyle formé d'une chaîne carbonée linéaire et saturée, ayant 1 à 4 atomes de carbone, ladite chaîne carbonée étant :
- liée, par son extrémité opposée au groupement phényle (III), à un substituant -COOR12, R12 représentant un hydrogène ou un groupement alkyle ayant 1 à 4 atomes de carbone ;
- non ramifiée ou ramifiée avec au moins un groupement alkyle ayant 1 à 4 atomes de carbone ;
A représente :
(i) un groupement carbonyle (CO), ou
(ii) un groupement -CR9R10, R9 représentant un atome d'hydrogène et R10 représentant un groupement -OR11, R11 étant choisi parmi un atome d'hydrogène ou un groupement alkyle (linéaire ou ramifié) ayant 1 à 7 atomes de carbone, ledit groupement alkyle étant non substitué ou substitué par un groupement cycloalkyle, notamment cyclohexyl, un groupement aryle, notamment phényle ou un groupement hétéroaryle, notamment pyridinyl;
B représente un groupement alkyle non substitué, saturé présentant deux atomes de carbone (CH₂-CH₂);
leurs stéréoisomères (diastéréoisomères, énantiomères), purs ou en mélange, mélanges racémiques, isomères géométriques, tautomères, sels, hydrates, solvates, formes solides ainsi que leurs mélanges.

2. Composés selon la revendication 1, **caractérisés en ce que** X5 est choisi parmi les groupements : -OC(CH₃)₂COOR12, -OCH(CH₂CH₃)COOR12 et -OCH₂COOR12.

3. Composés selon la revendication 2, **caractérisés en ce que** R12 représente un hydrogène ou un groupement tertiobutyle.

4. Composé selon l'une des revendications 1 à 3, **caractérisés en ce que** A représente un groupement carbonyle C=O.

5. Composés selon l'une des revendications 1 à 4, **caractérisés en ce que** le cycle II est substitué par le cycle I en position C₄ ou en position C₅.

6. Composés selon l'une des revendications 1 à 5, **caractérisés en ce que** X1 se trouve en *para* par rapport à la position du cycle II.

7. Composés selon l'une des revendications 1 à 6, **caractérisé en ce que** X1 est choisi parmi un groupement trifluorométhyle, un atome de brome, un groupement méthyloxy, un groupement méthylthio et un groupement trifluorométhoxy et un atome d'hydrogène.

8. Composés selon l'une des revendications 1 à 7, **caractérisés en ce qu'**au moins un des groupements X3, X4, X6 et X7 désigne un atome d'halogène.

9. Composés selon l'une des revendications 1 à 8, **caractérisés en ce que** X3 et X4 sont identiques et correspondent à des atomes de chlore ou de fluor.

10. Composés selon l'une des revendications 1 à 7, **caractérisés en ce que** X4 et/ou X6 désigne(nt) un groupement alkyle.

11. Composés selon l'une des revendications 1 à 10, **caractérisés en ce qu'**ils sont choisis parmi :
- le 2-(2,3-dichloro-4-(3-oxo-3-(5-(4-(trifluorométhyl)phényl)thièn-2-yl)propyl)-phénoxy)-2-méthylpropanoate de tertiobutyle ;
- l'acide 2-(2,3-dichloro-4-(3-oxo-3-(5-(4-(trifluorométhyl)phényl)thièn-2-yl)-propyl)phénoxy)-2-méthylpropanoïque ;
- le 2-(2,3-dichloro-4-(3-hydroxy-3-(5-(4-(trifluorométhyl)phényl)thièn-2-yl)-propyl)phénoxy)-2-méthylpropanoate de tertiobutyle ;
- l'acide 2-(2,3-dichloro-4-(3-hydroxy-3-(5-(4-(trifluorométhyl)phényl)thièn-2-yl)propyl)phénoxy)-2-méthylpropanoïque ;
- l'acide 2-(4-(3-(benzyloxy)-3-(5-(4-(trifluorométhyl)phényl)thièn-2-yl)propyl)-2,3-dichlorophénoxy)-2-méthylpropanoïque ;
- le 2-(2,3-dichloro-4-(3-oxo-3-(5-(4-(trifluorométhyl)phényl)thièn-2-yl)propyl)-phénoxy)butanoate de tertiobutyle ;
- l'acide 2-(2,3-dichloro-4-(3-oxo-3-(5-(4-(trifluorométhyl)phényl)thièn-2-yl)-propyl)phénoxy)butanoïque ;
- le 2-(2,3-dichloro-4-(3-oxo-3-(4-(4-(trifluorométhyl)phényl)thièn-2-yl)propyl)-phénoxy)-2-méthylpropanoate de tertiobutyle ;
- l'acide 2-(2,3-dichloro-4-(3-oxo-3-(4-(4-(trifluorométhyl)phényl)thièn-2-yl)-propyl)phénoxy)-2-méthylpropanoïque ;
- le 5-(2,3-dichloro-4-(3-oxo-3-(5-(4-(trifluorométhyl)phényl)thièn-2-yl)propyl)-phénoxy)-2,2-diméthylpentanoate de méthyle ;
- l'acide 5-(2,3-dichloro-4-(3-oxo-3-(5-(4-(trifluorométhyl)phényl)thièn-2-yl)propyl)phénoxy)-2,2-diméthylpentanoïque ;
- le 2-(2,3-dichloro-4-(3-oxo-3-(5-(4-(trifluorométhyl)phényl)thièn-2-yl)propyl)-phénoxy)acétate de tertiobutyle ;
- l'acide 2-(2,3-dichloro-4-(3-oxo-3-(5-(4-(trifluorométhyl)phényl)thièn-2-yl)-propyl)phénoxy)acétique ;
- le 2-(2-chloro-4-(3-oxo-3-(5-(4-(trifluorométhyl)phényl)thièn-2-yl)propyl)-phénoxy)-2-méthylpropanoate de tertiobutyle ;
- l'acide 2-(2-chloro-4-(3-oxo-3-(5-(4-(trifluorométhyl)phényl)thièn-2-yl)-propyl)phénoxy)-2-méthylpropanoïque ;
- le 2-(3-chloro-4-(3-oxo-3-(5-(4-(trifluorométhyl)phényl)thièn-2-yl)propyl)-phénoxy)-2-méthylpropanoate de tertiobutyle ;
- l'acide 2-(3-chloro-4-(3-oxo-3-(5-(4-(trifluorométhyl)phényl)thièn-2-yl)-propyl)phénoxy)-2-méthylpropanoïque ;
- le 2-(2-fluoro-4-(3-oxo-3-(5-(4-(trifluorométhyl)phényl)thièn-2-yl)propyl)-phénoxy)-2-méthylpropanoate de tertiobutyle ;
- l'acide 2-(2-fluoro-4-(3-oxo-3-(5-(4-(trifluorométhyl)phényl)thièn-2-yl)propyl)-phénoxy)-2-méthylpropanoïque ;
- le 2-(2-fluoro-4-(3-oxo-3-(5-(4-(trifluorométhyl)phényl)thièn-2-yl)propyl)-phénoxy)acétate de tertiobutyle ;
- l'acide 2-(2-fluoro-4-(3-oxo-3-(5-(4-(trifluorométhyl)phényl)thièn-2-yl)propyl)-phénoxy)acétique ;
- l'acide 2-(2-fluoro-4-(3-hydroxy-3-(5-(4-(trifluorométhyl)phényl)thièn-2-yl)-propyl)phénoxy)acétique ;
- l'acide 2-(4-(3-(benzyloxy)-3-(5-(4-(trifluorométhyl)phényl)thièn-2-yl)propyl)-2-fluorophénoxy)acétique ;
- le 2-(2-fluoro-4-(3-oxo-3-(5-(4-(trifluorométhyl)phényl)thièn-2-yl)propyl)-phénoxy)butanoate de tertiobutyle ;
- l'acide 2-(2-fluoro-4-(3-oxo-3-(5-(4-(trifluorométhyl)phényl)thièn-2-yl)propyl)-phénoxy)butanoïque ;
- l'acide 2-(2-fluoro-4-(3-hydroxy-3-(5-(4-(trifluorométhyl)phényl)thièn-2-yl)-propyl)phénoxy)butanoïque ;
- le 2-(2-bromo-4-(3-oxo-3-(5-(4-(trifluorométhyl)phényl)thièn-2-yl)propyl)-phénoxy)butanoate de tertiobutyle ;
- l'acide 2-(2-bromo-4-(3-oxo-3-(5-(4-(trifluorométhyl)phényl)thièn-2-yl)-propyl)phénoxy)butanoïque ;
- le 2-(2-bromo-4-(3-oxo-3-(5-(4-(trifluorométhyl)phényl)thièn-2-yl)propyl)-phénoxy)acétate de tertiobutyle ;
- l'acide 2-(2-bromo-4-(3-oxo-3-(5-(4-(trifluorométhyl)phényl)thièn-2-yl)-propyl)phénoxy)acétique ;
- le 2-(2-bromo-4-(3-oxo-3-(5-(4-(trifluorométhyl)phényl)thièn-2-yl)propyl)-phénoxy)-2-méthylpropanoate de tertiobutyle ;
- l'acide 2-(2-bromo-4-(3-oxo-3-(5-(4-(trifluorométhyl)phényl)thièn-2-yl)-propyl)phénoxy)-2-méthylpropanoïque ;
- le 2-(2,3-dichloro-4-(3-oxo-3-(5-(4-(trifluorométhyl)phényl)fur-2-yl)propyl)-phénoxy)-2-méthylpropanoate de tertiobutyle ;
- l'acide 2-(2,3-dichloro-4-(3-oxo-3-(5-(4-(trifluorométhyl)phényl)fur-2-yl)-propyl)phénoxy)-2-méthylpropanoïque ;
- l'acide 2-(2,3-dichloro-4-(3-(pyridin-3-ylméthoxy)-3-(5-(4-(trifluorométhyl)-phényl)thièn-2-yl)propyl)phénoxy)-2-méthylpropano'ique ;
- l'acide 2-(2,3-dichloro-4-(3-méthoxy-3-(5-(4-(trifluorométhyl)phényl)thièn-2-yl)propyl)phénoxy)-2-méthylpropanoïque ;
- l'acide 2-(2,3-dichloro-4-(3-éthoxy-3-(5-(4-(trifluorométhyl)phényl)thièn-2-yl)propyl)phénoxy)-2-méthylpropanoïque ;
- l'acide 2-(2,3-dichloro-4-(3-(cyclohexylméthoxy)-3-(5-(4-(trifluorométhyl)-phényl)thièn-2-yl)propyl)phénoxy)-2-méthylpropanoïque ;
- le 2-(2,3-dichloro-4-(3-oxo-3-(5-(4-(trifluorométhoxy)phényl)thièn-2-yl)-propyl)phénoxy)-2-méthylpropanoate de tertiobutyle ;
- l'acide 2-(2,3-dichloro-4-(3-oxo-3-(5-(4-(trifluorométhoxy)phényl)thièn-2-yl)-propyl)phénoxy)-2-méthylpropanoïque ;
- le 2-(2,3-difluoro-4-(3-oxo-3-(5-(4-(trifluorométhyl)phényl)thièn-2-yl)propyl)-phénoxy)-2-méthylpropanoate de tertiobutyle ;
- l'acide 2-(2,3-difluoro-4-(3-oxo-3-(5-(4-(trifluorométhyl)phényl)thièn-2-yl)-propyl)phénoxy)-2-méthylpropanoïque ;
- le 2-(2,6-diméthyl-4-(3-oxo-3-(5-(4-(trifluorométhyl)phényl)thièn-2-yl)propyl)-phénoxy)-2-méthylpropanoate de tertiobutyle ;
- l'acide 2-(2,6-diméthyl-4-(3-oxo-3-(5-(4-(trifluorométhyl)phényl)thièn-2-yl)-propyl)phénoxy)-2-méthylpropanoïque ;
- le 2-(4-(3-(5-(4-bromophényl)thièn-2-yl)-3-oxopropyl)-2,3-dichlorophénoxy)-2-méthylpropanoate de tertiobutyle ;
- l'acide 2-(4-(3-(5-(4-bromophényl)thièn-2-yl)-3-oxopropyl)-2,3-dichloro-phénoxy)-2-méthylpropanoïque ;
- le 2-(2,3-dichloro-4-(3-(5-(4-(méthylthio)phényl)thièn-2-yl)-3-oxopropyl)-phénoxy)-2-méthylpropanoate de tertio-butyle ;
- l'acide 2-(2,3-dichloro-4-(3-(5-(4-(méthylthio)phényl)thièn-2-yl)-3-oxo-propyl)phénoxy)-2-méthylpropanoïque ;
- l'acide 2-(2,3-dichloro-4-(3-isopropoxy-3-(5-(4-(trifluorométhyl)phényl)thièn-2-yl)propyl)phénoxy)-2-méthylpropanoïque ;
- le 2-(2,3-dichloro-4-(3-oxo-3-(5-phénylthiophèn-2-yl)propyl)phénoxy)-2-méthylpropanoate de tertiobutyle ;
- l'acide 2-(2,3-dichloro-4-(3-oxo-3-(5-phénylthièn-2-yl)propyl)phénoxy)-2-méthylpropanoïque ;
- le 2-méthyl-2-(2-méthyl-4-(3-oxo-3-(5-(4-(trifluorométhyl)phényl)thiophèn-2-yl)propyl)phénoxy) propanoate de tertiobutyle ;
- l'acide 2-méthyl-2-(2-méthyl-4-(3-oxo-3-(5-(4-(trifluorométhyl)phényl)-thiophèn-2-yl)propyl)phénoxy)propanoïque ;
- l'acide 2-(2,3-dichloro-4-(3-hydroxy-3-(4-(4-(trifluorométhyl)phényl)thièn-2-yl)propyl)phénoxy)-2-méthylpropanoïque ;
- l'acide 2-(4-(3-(benzyloxy)-3-(4-(4-(trifluorométhyl)phényl)thièn-2-yl)propyl)-2,3-dichlorophénoxy)-2-méthylpropanoïque ;
- l'acide 2-(2,3-difluoro-4-(3-hydroxy-3-(5-(4-(trifluorométhyl)phényl)thièn-2-yl)propyl)phénoxy)-2-méthylpropanoïque ;
- l'acide 2-(4-(3-(benzyloxy)-3-(5-(4-(trifluorométhyl)phényl)thièn-2-yl)propyl)-2,3-difluorophénoxy)-2-méthylpropano'ique ;
- le 2-(4-(3-oxo-3-(5-(4-(trifluorométhyl)phényl)thièn-2-yl)propyl)phénoxy)-butanoate de tertiobutyle ;
- l'acide 2-(4-(3-oxo-3-(5-(4-(trifluorométhyl)phényl)thièn-2-yl)propyl)-phénoxy)-butanoïque ;
- le 2-méthyl-2-(4-(3-oxo-3-(5-(4-(trifluorométhyl)phényl)thièn-2-yl)propyl)-phénoxy)propanoate de tertiobutyle ;
- l'acide 2-(4-(3-oxo-3-(5-(4-(trifluorométhyl)phényl)thièn-2-yl)propyl)-phénoxy)-2-méthylpropanoïque ;
- le 2-(4-(3-oxo-3-(5-(4-(trifluorométhyl)phényl)thièn-2-yl)propyl)phénoxy)-acétate de tertiobutyle ;
- l'acide 2-(4-(3-oxo-3-(5-(4-(trifluorométhyl)phényl)thièn-2-yl)propyl)-phénoxy)acétique ;
- l'acide 2-(4-(3-(benzyloxy)-3-(5-(4-(trifluorométhyl)phényl)thièn-2-yl)propyl)-2-fluorophénoxy)butanoïque ;
- l'acide 2-(2,3-dichloro-4-(3-hydroxy-3-(5-(4-(trifluorométhyl)phényl)furan-2-yl)propyl)phénoxy)-2-méthylpropanoïque ;
- l'acide 2-(2,3-dichloro-4-(3-méthoxy-3-(5-(4-(trifluorométhyl)phényl)fur-2-yl)propyl)phénoxy)-2-méthylpropanoïque ;
- l'acide 2-(4-(3-hydroxy-3-(5-(4-(trifluorométhyl)phényl)thièn-2-yl)propyl)-2,6-diméthylphénoxy)-2-méthylpropanoïque ;
- l'acide 2-(4-(3-méthoxy-3-(5-(4-(trifluorométhyl)phényl)thièn-2-yl)propyl)-2,6-diméthylphénoxy)-2-méthylpropanoïque ;
- le 2-(2,3-dichloro-4-(3-oxo-3-(5-(3-(trifluorométhyl)phényl)thièn-2-yl)propyl)-phénoxy)-2-méthylpropanoate d'éthyle ;
- l'acide 2-(2,3-dichloro-4-(3-oxo-3-(5-(3-(trifluorométhyl)phényl)thièn-2-yl)-propyl)phénoxy)-2-méthylpropanoïque ;
- le 2-(2,3-dichloro-4-(3-hydroxy-3-(5-(3-(trifluorométhyl)phényl)thièn-2-yl)-propyl)phénoxy)-2-méthylpropanoate d'éthyle ;
- le 2-(2,3-dichloro-4-(3-méthoxy-3-(5-(3-(trifluorométhyl)phényl)thièn-2-yl)-propyl)phenoxy)-2-méthylpropanoate d'éthyle ;
- l'acide 2-(2,3-dichloro-4-(3-hydroxy-3-(5-(4-(trifluorométhoxy)phényl)thièn-2-yl)propyl)phénoxy)-2-méthylpropanoïque ;
- l'acide 2-(2,3-dichloro-4-(3-méthoxy-3-(5-(4-(trifluorométhoxy)phényl)thièn-2-yl)propyl)phénoxy)-2-méthylpropanoïque.

12. Composition pharmaceutique comprenant, dans un support acceptable sur le plan pharmaceutique, au moins un composé tel que défini dans les revendications 1 à 11, éventuellement en association avec un ou plusieurs autres principes actifs thérapeutiques et/ou cosmétiques.

13. Composition pharmaceutique selon la revendication 12, pour traiter les facteurs de risque cardiovasculaire liés aux dérèglements du métabolisme lipidique et/ou glucidique.

14. Composition pharmaceutique selon la revendication 12, pour le traitement du diabète ou des dyslipidémies.

## Patentansprüche

1. Verbindungen substituierter 3-Phenyl-1-(phenylthienyl)propan-1-on- und 3-Phenyl-1-(phenylfuranyl)propan-1-on-Derivate mit der allgemeinen Formel (I): 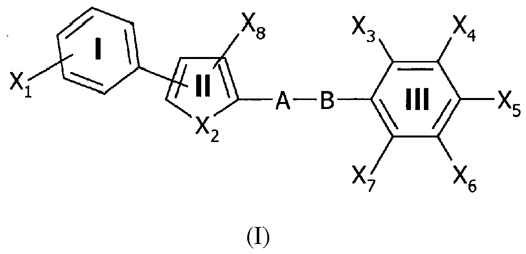 in der:
X für ein Halogen, eine Gruppe R1, -SR1 oder -OR1 steht;
X2 für ein Schwefel- oder Sauerstoffatom steht;
X3 für ein Halogen oder eine Gruppe R3 steht;
X4 für ein Halogen oder eine Gruppe R4 steht;
X5 für eine Gruppe -OR5 steht;
X6 für ein Halogen oder eine Gruppe R6 steht;
X7 für ein Halogen oder eine Gruppe R7 steht;
X8 für ein Wasserstoffatom steht;
wobei R1 für einen Wasserstoff oder eine Alkylgruppe mit 1 bis 4 Kohlenstoffatomen steht, wobei besagte Alkylgruppe eventuell halogeniert ist;
wobei R3, R4, R6 und R7 identisch oder verschieden sind und aus einem Wasserstoff oder einer Alkylgruppe mit 1 bis 4 Kohlenstoffatomen ausgewählt sind;
wobei R5 für einen Alkylrest steht, der aus einer linearen und gesättigten Kohlenstoffkette mit 1 bis 4 Kohlenstoffatomen gebildet ist, wobei besagte Kohlenstoffkette:
- mit ihrem der Phenylgruppe (III) entgegengesetzten Ende an einen -COOR12-Substituenten gebunden ist, wobei R12 für einen Wasserstoff oder eine Alkylgruppe mit 1 bis 4 Kohlenstoffatomen steht;
- mit wenigstens einer Alkylgruppe mit 1 bis 4 Kohlenstoffatomen verzweigt sein kann;
A:
(i) für eine Carbonylgruppe (CO) steht, oder
(ii) für eine Gruppe -CR9R10 steht, wobei R9 für ein Wasserstoffatom steht und R10 für eine Gruppe -OR11 steht, wobei R11 aus einem Wasserstoffatom oder einer (linearen oder verzweigten) Alkylgruppe mit 1 bis 7 Kohlenstoffatomen ausgewählt ist, wobei besagte Alkylgruppe mit einer Cycloalkylgruppe, insbesondere Cyclohexyl, einer Arylgruppe, insbesondere Phenyl, oder einer Heteroarylgruppe, insbesondere Pyridinyl, substituiert sein kann;
B: für eine nichtsubstituierte, gesättigte Alkylgruppe steht, die zwei Kohlenstoffatome (CH₂-CH₂) aufweist;
ihre Stereoisomere (Diastereoisomere, Enantiomere), rein oder im Gemisch, racemischen Gemische, geometrischen Isomere, Tautomere, Salze, Hydrate, Solvate, festen Formen sowie ihre Gemische.

2. Verbindungen gemäß Anspruch 1, **dadurch gekennzeichnet, dass** X5 aus den Gruppen ausgewählt ist: -OC(CH₃)₂COOR12, -OCH(CH₂CH₃)COOR12 und -OCH₂COOR12.

3. Verbindungen gemäß Anspruch 2, **dadurch gekennzeichnet, dass** R12 für einen Wasserstoff oder eine tertiäre Butylgruppe steht.

4. Verbindungen gemäß einem der Ansprüche 1 bis 3, **dadurch gekennzeichnet, dass** A für eine Carbonylgruppe C=O steht.

5. Verbindungen gemäß einem der Ansprüche 1 bis 4, **dadurch gekennzeichnet, dass** der Ring II mit dem Ring I in Position C₄ oder in Position C₅ substituiert ist.

6. Verbindungen gemäß einem der Ansprüche 1 bis 5, **dadurch gekennzeichnet, dass** X1 in para bezüglich der Position des Rings II steht.

7. Verbindungen gemäß einem der Ansprüche 1 bis 6, **dadurch gekennzeichnet, dass** X1 aus einer Trifluormethylgruppe, einem Bromatom, einer Methyloxygruppe, einer Methylthiogruppe und einer Trifluormethoxygruppe und einem Wasserstoffatom ausgewählt ist.

8. Verbindungen gemäß einem der Ansprüche 1 bis 7, **dadurch gekennzeichnet, dass** wenigstens eine der Gruppen X3, X4, X6 und X7 ein Halogenatom bezeichnet.

9. Verbindungen gemäß einem der Ansprüche 1 bis 8, **dadurch gekennzeichnet, dass** X3 und X4 identisch sind und Chlor- oder Fluoratome darstellen.

10. Verbindungen gemäß einem der Ansprüche 1 bis 7, **dadurch gekennzeichnet, dass** X4 und/oder X6 eine Alkylgruppe bezeichnet/bezeichnen.

11. Verbindungen gemäß einem der Ansprüche 1 bis 10, **dadurch gekennzeichnet, dass** sie ausgewählt sind aus:
- 2-(2,3-Dichlor-4-(3-oxo-3-(5-(4-(trifluoimethyl)phenyl)thien-2-yl)propyl)phenoxy)-2-methylpropionsäure-tert-butylester;
- 2-(2,3-Dichlor-4-(3-oxo-3-(5-(4-(trifluoimethyl)phenyl)thien-2-yl)propyl)phenoxy)-2-methylpropionsäure;
- 2-(2,3-Dichlor-4-(3-hydroxy-3-(5-(4-(trifluormethyl)phenyl)thien-2-yl)propyl)phenoxy)-2-methylpropionsäure-tert-butylester;
- 2-(2,3-Dichlor-4-(3-hydroxy-3-(5-(4-(trifluormethyl)phenyl)thien-2-yl)propyl)phenoxy)-2-methylpropionsäure;
- 2-(4-(3-Benzyloxy-3-(5-(4-(trifluormethyl)phenyl)thien-2-yl)propyl)-2,3-dichlorphenoxy)-2-methylpropionsäure;
- 2-(2,3-Dichlor-4-(3-oxo-3-(5-(4-(trifluoimethyl)phenyl)thien-2-yl)propyl)phenoxy)-2-buttersäure-tert-butylester;
- 2-(2,3-Dichlor-4-(3-oxo-3-(5-(4-(trifluoimethyl)phenyl)thien-2-yl)propyl)phenoxy)-2-buttersäure;
- 2-(2,3-Dichlor-4-(3-oxo-3-(4-(4-(trifluoimethyl)phenyl)thien-2-yl)propyl)phenoxy)-2-methylpropionsäure-tert-butylester;
- 2-(2,3-Dichlor-4-(3-oxo-3-(4-(4-(trifluoimethyl)phenyl)thien-2-yl)propyl)phenoxy)-2-methylpropionsäure;
- 5-(2,3-Dichlor-4-(3-oxo-3-(5-(4-(trifluormethyl)phenyl)thien-2-yl)propyl)phenoxy)-2,2-dimethylvaleriansäuremethylester;
- 5-(2,3-Dichlor-4-(3-oxo-3-(5-(4-(trifluormethyl)phenyl)thien-2-yl)propyl)phenoxy)-2,2-dimethylvaleriansäure;
- 2-(2,3-Dichlor-4-(3-oxo-3-(5-(4-(trifluoimethyl)phenyl)thien-2-yl)propyl)phenoxy)essigsäure-tert-butylester;
- 2-(2,3-Dichlor-4-(3-oxo-3-(5-(4-(trifluormethyl)phenyl)thien-2-yl)propyl)phenoxy)essigsäure;
- 2-(2-Chlor-4-(3-oxo-3-(5-(4-(trifluoimethyl)phenyl)thien-2-yl)propyl)phenoxy)-2-methylpropionsäure-tert-butylester;
- 2-(2-Chlor-4-(3-oxo-3-(5-(4-(trifluoimethyl)phenyl)thien-2-yl)propyl)phenoxy)-2-methylpropionsäure;
- 2-(3-Chlor-4-(3-oxo-3-(5-(4-(trifluoimethyl)phenyl)thien-2-yl)propyl)phenoxy)-2-methylpropionsäure-tert-butylester;
- 2-(3-Chlor-4-(3-oxo-3-(5-(4-(trifluoimethyl)phenyl)thien-2-yl)propyl)phenoxy)-2-methylpropionsäure;
- 2-(2-Fluor-4-(3-oxo-3-(5-(4-(trifluoimethyl)phenyl)thien-2-yl)propyl)phenoxy)-2-methylpropionsäure-tert-butylester;
- 2-(2-Fluor-4-(3-oxo-3-(5-(4-(trifluoimethyl)phenyl)thien-2-yl)propyl)phenoxy)-2-methylpropionsäure;
- 2-(2-Fluor-4-(3-oxo-3-(5-(4-(trifluormethyl)phenyl)thien-2-yl)propyl)phenoxy)essigsäure-tert-butylester;
- 2-(2-Fluor-4-(3-oxo-3-(5-(4-(trifluormethyl)phenyl)thien-2-yl)propyl)phenoxy)essigsäure;
- 2-(2-Fluor-4-(3-hydroxy-3-(5-(4-(trifluormethyl)phenyl)thien-2-yl)propyl)phenoxy)essigsäure;
- 2-(4-(3-(Benzyloxy)-3-(5-(4-(trifluormethyl)phenyl)thien-2-yl)propyl)-2-fluorphenoxy)essigsäure;
- 2-(2-Fluor-4-(3-oxo-3-(5-(4-(trifluoimethyl)phenyl)thien-2-yl)propyl)phenoxy)buttersäure-tert-butylester;
- 2-(2-Fluor-4-(3-oxo-3-(5-(4-(trifluormethyl)phenyl)thien-2-yl)propyl)phenoxy)buttersäure;
- 2-(2-Fluor-4-(3-hydroxy-3-(5-(4-(trifluoimethyl)phenyl)thien-2-yl)propyl)phenoxy)-buttersäure;
- 2-(2-Brom-4-(3-oxo-3-(5-(4-(trifluoimethyl)phenyl)thien-2-yl)propyl)phenoxy)buttersäure-tert-butylester;
- 2-(2-Brom-4-(3-oxo-3-(5-(4-(trifluormethyl)phenyl)thien-2-yl)propyl)phenoxy)buttersäure;
- 2-(2-Brom-4-(3-oxo-3-(5-(4-(trifluormethyl)phenyl)thien-2-yl)propyl)phenoxy)essigsäure-tert-butylester;
- 2-(2-Brom-4-(3-oxo-3-(5-(4-(tufluoimethyl)phenyl)thien-2-yl)propyl)phenoxy)essigsäure;
- 2-(2-Brom-4-(3-oxo-3-(5-(4-(trifluoimethyl)phenyl)thien-2-yl)propyl)phenoxy)-2-methylpropionsäure-tert-butylester;
- 2-(2-Brom-4-(3-oxo-3-(5-(4-(trifluoimethyl)phenyl)thien-2-yl)propyl)phenoxy)-2-methylpropionsäure;
- 2-(2,3-Dichlor-4-(3-oxo-3-(5-(4-(trifluoimethyl)phenyl)fur-2-yl)propyl)phenoxy)-2-methylpropionsäure-tert-butylester;
- 2-(2,3-Dichlor-4-(3-oxo-3-(5-(4-(trifluoimethyl)phenyl)fur-2-yl)propyl)phenoxy)-2-methylpropionsäure;
- 2-(2,3-Dichlor-4-(3-pyudin-3-ylmethoxy)-3-(5-(4-(tufluormethyl)phenyl)thien-2-yl)propyl)phenoxy)-2-methylpropionsäure;
- 2-(2,3-Dichlor-4-(3-methoxy-3-(5-(4-(trifluoimethyl)phenyl)thien-2-yl)propyl)phenoxy)-2-methylpropionsäure;
- 2-(2,3-Dichlor-4-(3-ethoxy-3-(5-(4-(trifluormethyl)phenyl)thien-2-yl)propyl)phenoxy)-2-methylpropionsäure;
- 2-(2,3-Dichlor-4-(3-cyclohexylmethoxy-3-(5-(4-(trifluormethyl)phenyl)thien-2-yl)propyl)phenoxy)-2-methylpropionsäure;
- 2-(2,3-Dichlor-4-(3-oxo-3-(5-(4-(trifluormethoxy)phenyl)thien-2-yl)propyl)phenoxy)-2-methylpropionsäure-tert-butylester;
- 2-(2,3-Dichlor-4-(3-oxo-3-(5-(4-(trifluormethoxy)phenyl)thien-2-yl)propyl)phenoxy)-2-methylpropionsäure;
- 2-(2,3-Difluor-4-(3-oxo-3-(5-(4-(trifluoimethyl)phenyl)thien-2-yl)propyl)phenoxy)-2-methylpropionsäure-tert-butylester;
- 2-(2,3-Difluor-4-(3-oxo-3-(5-(4-(trifluoimethyl)phenyl)thien-2-yl)propyl)phenoxy)-2-methylpropionsäure;
- 2-(2,6-Dimethyl-4-(3-oxo-3-(5-(4-(trifluoimethyl)phenyl)thien-2-yl)propyl)phenoxy)-2-methylpropionsäure-tert-butylester;
- 2-(2,6-Dimethyl-4-(3-oxo-3-(5-(4-(trifluoimethyl)phenyl)thien-2-yl)propyl)phenoxy)-2-methylpropionsäure;
- 2-(4-(3-(5-(4-Bromphenyl)thien-2-yl)-3-oxopropyl)-2,3-dichloiphenoxy)-2-methylpropionsäure-tert-butylester;
- 2-(4-(3-(5-(4-Bromphenyl)thien-2-yl)-3-oxopropyl)-2,3-dichloiphenoxy)-2-methylpropionsäure;
- 2-(2,3-Dichlor-4-(3-(5-(4-methylthio)phenyl)thien-2-yl)-3-oxopropyl)phenoxy)-2-methylpropionsäure-tert-butylester;
- 2-(2,3-Dichlor-4-(3-(5-(4-methylthio)phenyl)thien-2-yl)-3-oxopropyl)phenoxy)-2-methylpropionsäure;
- 2-(2,3-Dichlor-4-(3-isopropoxy-3-(5-(4-trifluoimethyl)phenyl)thien-2-yl)propyl)phenoxy)-2-methylpropionsäure;
- 2-(2,3-Dichlor-4-(3-oxo-3-(5-phenylthiophen-2-yl)propyl)phenoxy)-2-methylpropionsäure-tert-butylester;
- 2-(2,3-Dichlor-4-(3-oxo-3-(5-phenylthien-2-yl)propyl)phenoxy)-2-methylpropionsäure;
- 2-Methyl-2-(2-methyl-4-(3-oxo-3-(5-(4-trifluormethyl)phenyl)thiophen-2-yl)propyl)phenoxy)-propionsäure-tert-butylester;
- 2-Methyl-2-(2-methyl-4-(3-oxo-3-(5-(4-trifluormethyl)phenyl)thiophen-2-yl)propyl)phenoxy)-propionsäure;
- 2-(2,3-Dichlor-4-(3-hydroxy-3-(4-(4-(trifluormethyl)phenyl)thien-2-yl)propyl)phenoxy)-2-methylpropionsäure;
- 2-(4-(3-(Benzyloxy)-3-(4-(4-(trifluormethyl)phenyl)thien-2-yl)propyl)-2,3-dichlorphenoxy)-2-methylpropionsäure;
- 2-(2,3-Difluor-4-(3-hydroxy)-3-(5-(4-(trifluormethyl)phenyl)thien-2-yl)propyl)phenoxy)-2-methylpropionsäure;
- 2-(4-(3-(Benzyloxy)-3-(5-(4-(trifluoimethyl)phenyl)thien-2-yl)propyl)-2,3-difluorphenoxy)-2-methylpropionsäure;
- 2-(4-(3-Oxo-3-(5-(4-(trifluoimethyl)phenyl)thien-2-yl)propyl)phenoxy)buttersäure-tert-butylester;
- 2-(4-(3-Oxo-3-(5-(4-(trifluormethyl)phenyl)thien-2-yl)propyl)phenoxy)buttersäure;
- 2-Methyl-2-(4-(3-oxo-3-(5-(4-(trifluormethyl)phenyl)thien-2-yl)propyl)phenoxy)propionsäure-tert-butylester;
- 2-(4-(3-Oxo-3-(5-(4-(tufluormethyl)phenyl)thien-2-yl)propyl)phenoxy)-2-methylpropionsäure;
- 2-(4-(3-Oxo-3-(5-(4-(trifluormethyl)phenyl)thien-2-yl)propyl)phenoxy)essigsäure-tert-butylester;
- 2-(4-(3-Oxo-3-(5-(4-(tufluormethyl)phenyl)thien-2-yl)propyl)phenoxy)essigsäure;
- 2-(4-(3-(Benzyloxy)-3-(5-(4-(trifluoimethyl)phenyl)thien-2-yl)propyl)-2-fluorphenoxy)buttersäure;
- 2-(2,3-Dichlor-4-(3-hydroxy-3-(5-(4-(trifluoimethyl)phenyl)furan-2-yl)propyl)phenoxy)-2-methylpropionsäure;
- 2-(2,3-Dichlor-4-(3-methoxy-3-(5-(4-(trifluoimethyl)phenyl)fur-2-yl)propyl)phenoxy)-2-methylpropionsäure;
- 2-(4-(3-Hydroxy-3-(5-(4-(trifluoiTnethyl)phenyl)thien-2-yl)propyl)-2,6-dimethylphenoxy)-2-methylpropionsäure;
- 2-(4-(3-Methoxy-3-(5-(4-(trifluoimethyl)phenyl)thien-2-yl)propyl)-2,6-dimethylphenoxy)-2-methylpropionsäure;
- 2-(2,3-Dichlor-4-(3-oxo-3-(5-(3-(trifluoimethyl)phenyl)thien-2-yl)propyl)phenoxy)-2-methylpropionsäureethylester;
- 2-(2,3-Dichlor-4-(3-oxo-3-(5-(3-(trifluoimethyl)phenyl)thien-2-yl)propyl)phenoxy)-2-methylpropionsäure;
- 2-(2,3-Dichlor-4-(3-hydroxy-3-(5-(3-(trifluormethyl)phenyl)thien-2-yl)propyl)phenoxy)-2-methylpropionsäureethylester;
- 2-(2,3-Dichlor-4-(3-methoxy-3-(5-(3-(trifluoimethyl)phenyl)thien-2-yl)propyl)phenoxy)-2-methylpropionsäureethylester;
- 2-(2,3-Dichlor-4-(3-hydroxy-3-(5-(4-(tufluormethoxy)phenyl)thien-2-yl)propyl)phenoxy)-2-methylpropionsäure;
- 2-(2,3-Dichlor-4-(3-methoxy-3-(5-(4-(trifluormethoxy)phenyl)thien-2-yl)propyl)phenoxy)-2-methylpropionsäure;

12. Pharmazeutische Zusammensetzung, umfassend in einem pharmazeutisch verträglichen Träger wenigstens eine wie in den Ansprüchen 1 bis 11 definierte Verbindung, eventuell in Kombination mit einem oder mehreren weiteren therapeutischen und/oder kosmetischen Wirkstoffen.

13. Pharmazeutische Zusammensetzung gemäß Anspruch 12 zur Behandlung von kardiovaskulären Risikofaktoren, die mit Störungen des Lipid- und/oder Kohlenhydratstoffwechsels verbunden sind.

14. Pharmazeutische Zusammensetzung gemäß Anspruch 12 zur Behandlung von Diabetes und Dyslipidämien.

## Claims

1. Compounds derived from substituted 3-phenyl-1-(phenylthienyl)propan-1-ones and 3-phenyl-1-(phenylfuranyl)propan-1-ones of general formula (I): 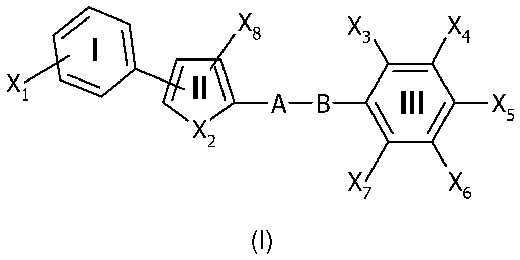 in which:
X1 represents a halogen, a R1, -SR1 or -OR1 group;
X2 represents a sulfur or oxygen atom;
X3 represents a halogen or a R3 group;
X4 represents a halogen or a R4 group;
X5 represents a -OR5 group;
X6 represents a halogen or a R6 group;
X7 represents a halogen or a R7 group;
X8 represents a hydrogen atom;
R1 representing a hydrogen or an alkyl group having 1 to 4 carbon atoms, said alkyl group being optionally halogenated;
R3, R4, R6 and R7, which may be identical or different, selected from hydrogen or an alkyl group having 1 to 4 carbon atoms;
R5 representing an alkyl radical formed from a saturated linear carbon chain, having 1 to 4 carbon atoms, said carbon chain being:
- bound, by its end opposite to the phenyl group (III), to a -COOR12 substituent, R12 representing a hydrogen or an alkyl group having 1 to 4 carbon atoms;
- unbranched or branched with at least one alkyl group having 1 to 4 carbon atoms;
A represents:
(i) a carbonyl group (CO), or
(ii) a-CR9R10 group, R9 representing a hydrogen atom and R10 representing an -OR11 group, R11 being selected from a hydrogen atom or an alkyl group (linear or branched) having 1 to 7 carbon atoms, said alkyl group being unsubstituted or substituted with a cycloalkyl group, notably cyclohexyl, an aryl group, notably phenyl or a heteroaryl group, notably pyridinyl;
B represents an unsubstituted, saturated alkyl group having two carbon atoms (CH₂-CH₂);
their stereoisomers (diastereoisomers, enantiomers), pure or mixed, racemic mixtures, geometric isomers, tautomers, salts, hydrates, solvates, solid forms and mixtures thereof.

2. The compounds according to claim 1, **characterized in that** X5 is selected from the groups: -OC(CH₃)₂COOR12, -OCH(CH₂CH₃)COOR12 and -OCH₂COOR12.

3. The compounds according to claim 2, **characterized in that** R12 represents a hydrogen or a tert-butyl group.

4. The compound according to any one of claims 1 to 3, **characterized in that** A represents a carbonyl group C=O.

5. The compounds according to any one of claims 1 to 4, **characterized in that** ring II is substituted with ring I in position C₄ or in position C₅.

6. The compounds according to any one of claims 1 to 5, **characterized in that** X1 is in the *para* position relative to the position of ring II.

7. The compounds according to any one of claims 1 to 6, **characterized in that** X1 is selected from a trifluoromethyl group, a bromine atom, a methyloxy group, a methylthio group and a trifluoromethoxy group and a hydrogen atom.

8. The compounds according to any one of claims 1 to 7, **characterized in that** at least one of the groups X3, X4, X6 and X7 represents a halogen atom.

9. The compounds according to any one of claims 1 to 8, **characterized in that** X3 and X4 are identical and correspond to chlorine or fluorine atoms.

10. The compounds according to any one of claims 1 to 7, **characterized in that** X4 and/or X6 represents an alkyl group.

11. The compounds according to any one of claims 1 to 10, **characterized in that** they are selected from:
- tert-butyl 2-(2,3-dichloro-4-(3-oxo-3-(5-(4-(trifluoromethyl)phenyl)thien-2-yl)propyl)-phenoxy)-2-methylpropanoate;
- 2-(2,3-dichloro-4-(3-oxo-3-(5-(4-(trifluoromethyl)phenyl)thien-2-yl)-propyl)phenoxy)-2-methylpropanoic acid;
- tert-butyl 2-(2,3-dichloro-4-(3-hydroxy-3-(5-(4-(trifluoromethyl)phenyl)thien-2-yl)-propyl)phenoxy)-2-methylpropanoate;
- 2-(2,3-dichloro-4-(3-hydroxy-3-(5-(4-(trifluoromethyl)phenyl)thien-2-yl)propyl)phenoxy)-2-methylpropanoic acid;
- 2-(4-(3-(benzyloxy)-3-(5-(4-(trifluoromethyl)phenyl)thien-2-yl)propyl)-2,3-dichlorophenoxy)-2-methylpropanoic acid;
- tert-butyl 2-(2,3-dichloro-4-(3-oxo-3-(5-(4-(trifluoromethyl)phenyl)thien-2-yl)propyl)-phenoxy)butanoate;
- 2-(2,3-dichloro-4-(3-oxo-3-(5-(4-(trifluoromethyl)phenyl)thien-2-yl)-propyl)phenoxy)butanoic acid;
- tert-butyl 2-(2,3-dichloro-4-(3-oxo-3-(4-(4-(trifluoromethyl)phenyl)thien-2-yl)propyl)-phenoxy)-2-methylpropanoate;
- 2-(2,3-dichloro-4-(3-oxo-3-(4-(4-(trifluoromethyl)phenyl)thien-2-yl)-propyl)phenoxy)-2-methylpropanoic acid;
- methyl 5-(2,3-dichloro-4-(3-oxo-3-(5-(4-(trifluoromethyl)phenyl)thien-2-yl)propyl)-phenoxy)-2,2-dimethylpentanoate;
- 5-(2,3-dichloro-4-(3-oxo-3-(5-(4-(trifluoromethyl)phenyl)thien-2-yl)propyl)phenoxy)-2,2-dimethylpentanoic acid;
- tert-butyl 2-(2,3-dichloro-4-(3-oxo-3-(5-(4-(trifluoromethyl)phenyl)thien-2-yl)propyl)-phenoxy)acetate;
- 2-(2,3-dichloro-4-(3-oxo-3-(5-(4-(trifluoromethyl)phenyl)thien-2-yl)-propyl)phenoxy)acetic acid;
- tert-butyl 2-(2-chloro-4-(3-oxo-3-(5-(4-(trifluoromethyl)phenyl)thien-2-yl)propyl)-phenoxy)-2-methylpropanoate;
- 2-(2-chloro-4-(3-oxo-3-(5-(4-(trifluoromethyl)phenyl)thien-2-yl)-propyl)phenoxy)-2-methylpropanoic acid;
- tert-butyl 2-(3-chloro-4-(3-oxo-3-(5-(4-(trifluoromethyl)phenyl)thien-2-yl)propyl)-phenoxy)-2-methylpropanoate;
- 2-(3-chloro-4-(3-oxo-3-(5-(4-(trifluoromethyl)phenyl)thien-2-yl)-propyl)phenoxy)-2-methylpropanoic acid;
- tert-butyl 2-(2-fluoro-4-(3-oxo-3-(5-(4-(trifluoromethyl)phenyl)thien-2-yl)propyl)-phenoxy)-2-methylpropanoate;
- 2-(2-fluoro-4-(3-oxo-3-(5-(4-(trifluoromethyl)phenyl)thien-2-yl)propyl)-phenoxy)-2-methylpropanoic acid;
- tert-butyl 2-(2-fluoro-4-(3-oxo-3-(5-(4-(trifluoromethyl)phenyl)thien-2-yl)propyl)-phenoxy)acetate;
- 2-(2-fluoro-4-(3-oxo-3-(5-(4-(trifluoromethyl)phenyl)thien-2-yl)propyl)-phenoxy)acetic acid;
- 2-(2-fluoro-4-(3-hydroxy-3-(5-(4-(trifluoromethyl)phenyl)thien-2-yl)-propyl)phenoxy)acetic acid;
- 2-(4-(3-(benzyloxy)-3-(5-(4-(trifluoromethyl)phenyl)thien-2-yl)propyl)-2-fluorophenoxy)acetic acid;
- tert-butyl 2-(2-fluoro-4-(3-oxo-3-(5-(4-(trifluoromethyl)phenyl)thien-2-yl)propyl)-phenoxy)butanoate;
- 2-(2-fluoro-4-(3-oxo-3-(5-(4-(trifluoromethyl)phenyl)thien-2-yl)propyl)-phenoxy)butanoic acid;
- 2-(2-fluoro-4-(3-hydroxy-3-(5-(4-(trifluoromethyl)phenyl)thien-2-yl)-propyl)phenoxy)butanoic acid;
- tert-butyl 2-(2-bromo-4-(3-oxo-3-(5-(4-(trifluoromethyl)phenyl)thien-2-yl)propyl)-phenoxy)butanoate;
- 2-(2-bromo-4-(3-oxo-3-(5-(4-(trifluoromethyl)phenyl)thien-2-yl)-propyl)phenoxy)butanoic acid;
- tert-butyl 2-(2-bromo-4-(3-oxo-3-(5-(4-(trifluoromethyl)phenyl)thien-2-yl)propyl)-phenoxy)acetate;
- 2-(2-bromo-4-(3-oxo-3-(5-(4-(trifluoromethyl)phenyl)thien-2-yl)-propyl)phenoxy)acetic acid;
- tert-butyl 2-(2-bromo-4-(3-oxo-3-(5-(4-(trifluoromethyl)phenyl)thien-2-yl)propyl)-phenoxy)-2-methylpropanoate;
- 2-(2-bromo-4-(3-oxo-3-(5-(4-(trifluoromethyl)phenyl)thien-2-yl)-propyl)phenoxy)-2-methylpropanoic acid;
- tert-butyl 2-(2,3-dichloro-4-(3-oxo-3-(5-(4-(trifluoromethyl)phenyl)fur-2-yl)propyl)-phenoxy)-2-methylpropanoate;
- 2-(2,3-dichloro-4-(3-oxo-3-(5-(4-(trifluoromethyl)phenyl)fur-2-yl)-propyl)phenoxy)-2-methylpropanoic acid;
- 2-(2,3-dichloro-4-(3-(pyridin-3-ylmethoxy)-3-(5-(4-(trifluoromethyl)-phenyl)thien-2-yl)propyl)phenoxy)-2-methylpropanoic acid;
- 2-(2,3-dichloro-4-(3-methoxy-3-(5-(4-(trifluoromethyl)phenyl)thien-2-yl)propyl)phenoxy)-2-methylpropanoic acid;
- 2-(2,3-dichloro-4-(3-ethoxy-3-(5-(4-(trifluoromethyl)phenyl)thien-2-yl)propyl)phenoxy)-2-methylpropanoic acid;
- 2-(2,3-dichloro-4-(3-(cyclohexylmethoxy)-3-(5-(4-(trifluoromethyl)-phenyl)thien-2-yl)propyl)phenoxy)-2-methylpropanoic acid;
- tert-butyl 2-(2,3-dichloro-4-(3-oxo-3-(5-(4-(trifluoromethoxy)phenyl)thien-2-yl)-propyl)phenoxy)-2-methylpropanoate;
- 2-(2,3-dichloro-4-(3-oxo-3-(5-(4-(trifluoromethoxy)phenyl)thien-2-yl)-propyl)phenoxy)-2-methylpropanoic acid;
- tert-butyl 2-(2,3-difluoro-4-(3-oxo-3-(5-(4-(trifluoromethyl)phenyl)thien-2-yl)propyl)-phenoxy)-2-methylpropanoate;
- 2-(2,3-difluoro-4-(3-oxo-3-(5-(4-(trifluoromethyl)phenyl)thien-2-yl)-propyl)phenoxy)-2-methylpropanoic acid;
- tert-butyl 2-(2,6-dimethyl-4-(3-oxo-3-(5-(4-(trifluoromethyl)phenyl)thien-2-yl)propyl)-phenoxy)-2-methylpropanoate;
- 2-(2,6-dimethyl-4-(3-oxo-3-(5-(4-(trifluoromethyl)phenyl)thien-2-yl)-propyl)phenoxy)-2-methylpropanoic acid;
- tert-butyl 2-(4-(3-(5-(4-bromophenyl)thien-2-yl)-3-oxopropyl)-2,3-dichlorophenoxy)-2-methylpropanoate;
- 2-(4-(3-(5-(4-bromophenyl)thien-2-yl)-3-oxopropyl)-2,3-dichloro-phenoxy)-2-methylpropanoic acid;
- tert-butyl 2-(2,3-dichloro-4-(3-(5-(4-(methylthio)phenyl)thien-2-yl)-3-oxopropyl)-phenoxy)-2-methylpropanoate;
- 2-(2,3-dichloro-4-(3-(5-(4-(methylthio)phenyl)thien-2-yl)-3-oxopropyl)phenoxy)-2-methylpropanoic acid;
- 2-(2,3-dichloro-4-(3-isopropoxy-3-(5-(4-(trifluoromethyl)phenyl)thien-2-yl)propyl)phenoxy)-2-methylpropanoic acid;
- tert-butyl 2-(2,3-dichloro-4-(3-oxo-3-(5-phenylthiophen-2-yl)propyl)phenoxy)-2-methylpropanoate;
- 2-(2,3-dichloro-4-(3-oxo-3-(5-phenylthien-2-yl)propyl)phenoxy)-2-methylpropanoic acid;
- tert-butyl 2-methyl-2-(2-methyl-4-(3-oxo-3-(5-(4-(trifluoromethyl)phenyl)thiophen-2-yl)propyl)phenoxy) propanoate;
- 2-methyl-2-(2-methyl-4-(3-oxo-3-(5-(4-(trifluoromethyl)phenyl)-thiophen-2-yl)propyl)phenoxy)propanoic acid;
- 2-(2,3-dichloro-4-(3-hydroxy-3-(4-(4-(trifluoromethyl)phenyl)thien-2-yl)propyl)phenoxy)-2-methylpropanoic acid;
- 2-(4-(3-(benzyloxy)-3-(4-(4-(trifluoromethyl)phenyl)thien-2-yl)propyl)-2,3-dichlorophenoxy)-2-methylpropanoic acid;
- 2-(2,3-difluoro-4-(3-hydroxy-3-(5-(4-(trifluoromethyl)phenyl)thien-2-yl)propyl)phenoxy)-2-methylpropanoic acid;
- 2-(4-(3-(benzyloxy)-3-(5-(4-(trifluoromethyl)phenyl)thien-2-yl)propyl)-2,3-difluorophenoxy)-2-methylpropanoic acid;
- tert-butyl 2-(4-(3-oxo-3-(5-(4-(trifluoromethyl)phenyl)thien-2-yl)propyl)phenoxy)-butanoate;
- 2-(4-(3-oxo-3-(5-(4-(trifluoromethyl)phenyl)thien-2-yl)propyl)-phenoxy)-butanoic acid;
- tert-butyl 2-methyl-2-(4-(3-oxo-3-(5-(4-(trifluoromethyl)phenyl)thien-2-yl)propyl)-phenoxy)propanoate;
- 2-(4-(3-oxo-3-(5-(4-(trifluoromethyl)phenyl)thien-2-yl)propyl)-phenoxy)-2-methylpropanoic acid;
- tert-butyl 2-(4-(3-oxo-3-(5-(4-(trifluoromethyl)phenyl)thien-2-yl)propyl)phenoxy)-acetate;
- 2-(4-(3-oxo-3-(5-(4-(trifluoromethyl)phenyl)thien-2-yl)propyl)-phenoxy)acetic acid;
- 2-(4-(3-(benzyloxy)-3-(5-(4-(trifluoromethyl)phenyl)thien-2-yl)propyl)-2-fluorophenoxy)butanoic acid;
- 2-(2,3-dichloro-4-(3-hydroxy-3-(5-(4-(trifluoromethyl)phenyl)furan-2-yl)propyl)phenoxy)-2-methylpropanoic acid;
- 2-(2,3-dichloro-4-(3-methoxy-3-(5-(4-(trifluoromethyl)phenyl)fur-2-yl)propyl)phenoxy)-2-methylpropanoic acid;
- 2-(4-(3-hydroxy-3-(5-(4-(trifluoromethyl)phenyl)thien-2-yl)propyl)-2,6-dimethylphenoxy)-2-methylpropanoic acid;
- 2-(4-(3-methoxy-3-(5-(4-(trifluoromethyl)phenyl)thien-2-yl)propyl)-2,6-dimethylphenoxy)-2-methylpropanoic acid;
- ethyl 2-(2,3-dichloro-4-(3-oxo-3-(5-(3-(trifluoromethyl)phenyl)thien-2-yl)propyl)-phenoxy)-2-methylpropanoate;
- 2-(2,3-dichloro-4-(3-oxo-3-(5-(3-(trifluoromethyl)phenyl)thien-2-yl)-propyl)phenoxy)-2-methylpropanoic acid;
- ethyl 2-(2,3-dichloro-4-(3-hydroxy-3-(5-(3-(trifluoromethyl)phenyl)thien-2-yl)-propyl)phenoxy)-2-methylpropanoate;
- ethyl 2-(2,3-dichloro-4-(3-methoxy-3-(5-(3-(trifluoromethyl)phenyl)thien-2-yl)-propyl)phenoxy)-2-methylpropanoate;
- 2-(2,3-dichloro-4-(3-hydroxy-3-(5-(4-(trifluoromethoxy)phenyl)thien-2-yl)propyl)phenoxy)-2-methylpropanoic acid;
- 2-(2,3-dichloro-4-(3-methoxy-3-(5-(4-(trifluoromethoxy)phenyl)thien-2-yl)propyl)phenoxy)-2-methylpropanoic.

12. A pharmaceutical composition comprising, in a pharmaceutically acceptable carrier, at least one compound as defined in claims 1 to 11, optionally in combination with one or more other therapeutic and/or cosmetic active principles.

13. The pharmaceutical composition according to claim 12, for treating the cardiovascular risk factors connected with disturbances of lipid and/or carbohydrate metabolism.

14. The pharmaceutical composition according to claim 12, for the treatment of diabetes or dyslipidemias.
